# EUROPEAN PATENT SPECIFICATION

(11) **EP 4 388 100 B1**
(45) Date of publication and mention of the grant of the patent: **05.03.2025**
(21) Application number: 23749018.0
(22) Date of filing: 27.07.2023
(51) Int. Cl.: C12N 15/113, A61K 31/713

(54) **DOUBLE STRANDED NUCLEIC ACID COMPOUNDS INHIBITING ZPI**
ZPI INHIBIERENDE DOPPELSTRÄNGIGE NUKLEINSÄUREVERBINDUNGEN
COMPOSÉS ACIDES NUCLÉIQUES DOUBLE BRIN INHIBANT ZPI

(30) Priority: 27.07.2022 US 202263369631 P; 06.02.2023 EP 23155105
(43) Date of publication of application: 26.06.2024
(73) Proprietor: E-Therapeutics plc, London W2 6BD (GB)
(72) Inventor: MCCARTHY, Amy, London W2 6BD (GB); CRAGGS, Graham, London W2 6BD (GB); LONGDEN, James, London W2 6BD (GB); DE SANTIAGO, Ines, London W2 6BD (GB); BROWN, Duncan, London W2 6BD (GB); MORTAZAVI, Ahmad Ali, London W2 6BD (GB); MANNELLA, Viviana, London W2 6BD (GB); JAYARAMAN, Muthusamy, London W2 6BD (GB); DEBACKER, Alexandre, London W2 6BD (GB); MOGG, Adrian James, London W2 6BD (GB)
(74) Representative: CMS Cameron McKenna Nabarro Olswang LLP
(86) International application number: PCT/EP2023/070899
(87) International publication number: WO 2024/023251

(56) References cited:
- WO-A1-2023/059948
- WO-A2-2007/110623
- KITCHENS CAROLYN ANTONIA: "Exploitation of small interfering RNA methodology to identify novel anticancer treatments", 1 January 2011 (2011-01-01), pages 1 - 130, XP093092445, ISBN: 978-1-124-83360-6, Retrieved from the Internet <URL:https://d-scholarship.pitt.edu/6311/1/CAKitchens_ETD2011.pdf> [retrieved on 20231016]

## Description

### FIELD

The present invention provides novel nucleic acid compounds, suitable for therapeutic use. Additionally, the present disclosure provides methods of making these compounds, as well as methods of using such compounds for the treatment of various diseases and conditions. Said methods are not part of the claimed invention.

### BACKGROUND OF THE INVENTION

Nucleic acid compounds have important therapeutic applications in medicine. Nucleic acids can be used to silence genes that are responsible for a particular disease. Gene-silencing prevents formation of a protein by inhibiting translation. Importantly, gene-silencing agents are a promising alternative to traditional small, organic compounds that inhibit the function of the protein linked to the disease. siRNA, antisense RNA, and micro-RNA are oligonucleotides / oligonucleosides that prevent the formation of proteins by gene-silencing.

A number of modified siRNA compounds in particular have been developed in the last two decades for diagnostic and therapeutic purposes, including siRNA / RNAi therapeutic agents for the treatment of various diseases including central-nervous-system diseases, inflammatory diseases, metabolic disorders, oncology, infectious diseases, and ocular diseases.

The present invention relates to nucleic acid compounds, for use in the treatment and / or prevention of disease.

### STATEMENTS OF INVENTION

The invention is as defined in the claims. The following aspects are useful for understanding the invention but are not part of the claimed invention in their entirety.

According to a first aspect, there is provided a nucleic acid for inhibiting expression of ZPI, comprising a duplex region that comprises a first strand and a second strand that is at least partially complementary to the first strand, wherein said first strand is: (i) at least partially complementary to a portion of RNA transcribed from the ZPI gene, and (ii) comprises at least 17 contiguous nucleosides differing by 0 or 1 nucleosides from any one of the first strand sequences as listed in Table 2.

According to a second aspect, there is provided a nucleic acid for inhibiting expression of ZPI, comprising a duplex region that comprises a first strand and a second strand that is at least partially complementary to the first strand, wherein said first strand is: (i) at least partially complementary to a portion of RNA transcribed from the ZPI gene, and (ii) comprises at least 17 contiguous nucleosides differing by 0 or 1 nucleosides from any one of the first strand modified sequences as listed in Table 3.

A nucleic acid as described herein, wherein the first strand comprises nucleosides 2-18 of any one of the sequences according to the above first and second aspects

A nucleic acid according to the above first aspect, wherein the second strand comprises a nucleoside sequence of at least 17 contiguous nucleosides differing by 0 or 1 nucleosides from any one of the second strand sequences as listed in Table 2, and wherein the second strand has a region of at least 85% complementarity over the 17 contiguous nucleosides to the first strand.

A nucleic acid according to the above first aspect, wherein the second strand comprises a nucleoside sequence of at least 17 contiguous nucleosides differing by 0 or 1 nucleosides from any one of the second strand sequences as listed in Table 2, and wherein the duplex region comprises at least 14, 15, 16 or 17 complementary base pairs.

A nucleic acid according to the above second aspect, wherein the second strand comprises a nucleoside sequence of at least 17 contiguous nucleosides differing by 0 or 1 nucleosides from any one of the second strand modified sequences as listed in Table 4, and wherein the second strand has a region of at least 85% complementarity over the 17 contiguous nucleosides to the first strand.

A nucleic acid according to the above second aspect, wherein the second strand comprises a nucleoside sequence of at least 17 contiguous nucleosides differing by 0 or 1 nucleosides from any one of the second strand modified sequences as listed in Table 4, and wherein the duplex region comprises at least 14, 15, 16 or 17 complementary base pairs.

A nucleic acid according to the above first aspect, wherein the first strand comprises any one of the first strand sequences as listed in Table 2.

A nucleic acid according to the above second aspect, wherein the first strand comprises any one of the first strand modified sequences as listed in Table 3.

A nucleic acid according to the above first aspect, wherein the second strand comprises any one of the second strand sequences as listed in Table 2.

A nucleic acid according to the above second aspect, wherein the second strand comprises any one of the second strand modified sequences as listed in Table 4.

A nucleic acid according to the above first aspect, wherein the first strand comprises any one of the following sequences: SEQ ID NO: 126, SEQ ID NO: 127, SEQ ID NO: 128, SEQ ID NO: 129, SEQ ID NO: 131, SEQ ID NO: 132, SEQ ID NO: 138, SEQ ID NO: 139, SEQ ID NO: 144, SEQ ID NO: 145, SEQ ID NO: 147, SEQ ID NO: 148, SEQ ID NO: 149, SEQ ID NO: 226, SEQ ID NO: 227, SEQ ID NO: 228, SEQ ID NO: 229, SEQ ID NO: 231, SEQ ID NO: 232, SEQ ID NO: 238, SEQ ID NO: 239.

A nucleic acid according to the above second aspect, wherein the first strand comprises any one of the following sequences: SEQ ID NO: 366, SEQ ID NO: 367, SEQ ID NO: 368, SEQ ID NO: 369, SEQ ID NO: 371, SEQ ID NO: 372, SEQ ID NO: 378, SEQ ID NO: 379, SEQ ID NO: 384, SEQ ID NO: 385, SEQ ID NO: 387, SEQ ID NO: 388, SEQ ID NO: 389, SEQ ID NO: 466, SEQ ID NO: 467, SEQ ID NO: 468, SEQ ID NO: 469, SEQ ID NO: 471, SEQ ID NO: 472, SEQ ID NO: 478, SEQ ID NO: 479, SEQ ID NO: 498, SEQ ID NO: 518, SEQ ID NO: 538, SEQ ID NO: 546, SEQ ID NO: 547, SEQ ID NO: 548, SEQ ID NO: 549, SEQ ID NO: 551, SEQ ID NO: 552, SEQ ID NO: 558, SEQ ID NO: 559.

A nucleic acid according to the above first aspect, wherein the second strand comprises any one of the following sequences: SEQ ID NO: 246, SEQ ID NO: 247, SEQ ID NO: 248, SEQ ID NO: 249, SEQ ID NO: 251, SEQ ID NO: 252, SEQ ID NO: 258, SEQ ID NO: 259, SEQ ID NO: 264, SEQ ID NO: 265, SEQ ID NO: 267, SEQ ID NO: 268, SEQ ID NO: 269, SEQ ID NO: 346, SEQ ID NO: 347, SEQ ID NO: 348, SEQ ID NO: 349, SEQ ID NO: 351, SEQ ID NO: 352,SEQ ID NO: 358, SEQ ID NO: 359.

A nucleic acid according to the above second aspect, wherein the second strand comprises any one of the following sequences: SEQ ID NO: 566, SEQ ID NO: 567, SEQ ID NO: 568, SEQ ID NO: 569, SEQ ID NO: 571, SEQ ID NO: 572, SEQ ID NO: 578, SEQ ID NO: 579, SEQ ID NO: 584, SEQ ID NO: 585, SEQ ID NO: 587, SEQ ID NO: 588, SEQ ID NO: 589, SEQ ID NO: 666, SEQ ID NO: 667, SEQ ID NO: 668, SEQ ID NO: 669, SEQ ID NO: 671, SEQ ID NO: 672, SEQ ID NO: 678, SEQ ID NO: 679, SEQ ID NO: 698, SEQ ID NO: 718, SEQ ID NO: 738, SEQ ID NO: 746, SEQ ID NO: 747, SEQ ID NO: 748, SEQ ID NO: 749, SEQ ID NO: 751, SEQ ID NO: 752, SEQ ID NO: 758, SEQ ID NO: 759.

A nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO: 126 | SEQ ID NO: 246 |
| SEQ ID NO: 127 | SEQ ID NO: 247 |
| SEQ ID NO: 128 | SEQ ID NO: 248 |
| SEQ ID NO: 129 | SEQ ID NO: 249 |
| SEQ ID NO: 131 | SEQ ID NO: 251 |
| SEQ ID NO: 132 | SEQ ID NO: 252 |
| SEQ ID NO: 138 | SEQ ID NO: 258 |
| SEQ ID NO: 139 | SEQ ID NO: 259 |
| SEQ ID NO: 144 | SEQ ID NO: 264 |
| SEQ ID NO: 145 | SEQ ID NO: 265 |
| SEQ ID NO: 147 | SEQ ID NO: 267 |
| SEQ ID NO: 148 | SEQ ID NO: 268 |
| SEQ ID NO: 149 | SEQ ID NO: 269 |
| SEQ ID NO: 226 | SEQ ID NO: 346 |
| SEQ ID NO: 227 | SEQ ID NO: 347 |
| SEQ ID NO: 228 | SEQ ID NO: 348 |
| SEQ ID NO: 229 | SEQ ID NO: 349 |
| SEQ ID NO: 231 | SEQ ID NO: 351 |
| SEQ ID NO: 232 | SEQ ID NO: 352 |
| SEQ ID NO: 238 | SEQ ID NO: 358 |
| SEQ ID NO: 239 | SEQ ID NO: 359 |
| SEQ ID NO: 138 | SEQ ID NO: 258 |
| SEQ ID NO: 138 | SEQ ID NO: 258 |
| SEQ ID NO: 138 | SEQ ID NO: 258 |
| SEQ ID NO: 126 | SEQ ID NO: 246 |
| SEQ ID NO: 127 | SEQ ID NO: 247 |
| SEQ ID NO: 128 | SEQ ID NO: 248 |
| SEQ ID NO: 129 | SEQ ID NO: 249 |
| SEQ ID NO: 131 | SEQ ID NO: 251 |
| SEQ ID NO: 132 | SEQ ID NO: 252 |
| SEQ ID NO: 138 | SEQ ID NO: 258 |
| SEQ ID NO: 139 | SEQ ID NO: 259 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 366 | SEQ ID NO: 566 |
| SEQ ID NO: 367 | SEQ ID NO: 567 |
| SEQ ID NO: 368 | SEQ ID NO: 568 |
| SEQ ID NO: 369 | SEQ ID NO: 569 |
| SEQ ID NO: 371 | SEQ ID NO: 571 |
| SEQ ID NO: 372 | SEQ ID NO: 572 |
| SEQ ID NO: 378 | SEQ ID NO: 578 |
| SEQ ID NO: 379 | SEQ ID NO: 579 |
| SEQ ID NO: 384 | SEQ ID NO: 584 |
| SEQ ID NO: 385 | SEQ ID NO: 585 |
| SEQ ID NO: 387 | SEQ ID NO: 587 |
| SEQ ID NO: 388 | SEQ ID NO: 588 |
| SEQ ID NO: 389 | SEQ ID NO: 589 |
| SEQ ID NO: 466 | SEQ ID NO: 666 |
| SEQ ID NO: 467 | SEQ ID NO: 667 |
| SEQ ID NO: 468 | SEQ ID NO: 668 |
| SEQ ID NO: 469 | SEQ ID NO: 669 |
| SEQ ID NO: 471 | SEQ ID NO: 671 |
| SEQ ID NO: 472 | SEQ ID NO: 672 |
| SEQ ID NO: 478 | SEQ ID NO: 678 |
| SEQ ID NO: 479 | SEQ ID NO: 679 |
| SEQ ID NO: 498 | SEQ ID NO: 698 |
| SEQ ID NO: 518 | SEQ ID NO: 718 |
| SEQ ID NO: 538 | SEQ ID NO: 738 |
| SEQ ID NO: 546 | SEQ ID NO: 746 |
| SEQ ID NO: 547 | SEQ ID NO: 747 |
| SEQ ID NO: 548 | SEQ ID NO: 748 |
| SEQ ID NO: 549 | SEQ ID NO: 749 |
| SEQ ID NO: 551 | SEQ ID NO: 751 |
| SEQ ID NO: 552 | SEQ ID NO: 752 |
| SEQ ID NO: 558 | SEQ ID NO: 758 |
| SEQ ID NO: 559 | SEQ ID NO: 759 |

A nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO: 128 | SEQ ID NO: 248 |
| SEQ ID NO: 144 | SEQ ID NO: 264 |
| SEQ ID NO: 148 | SEQ ID NO: 268 |
| SEQ ID NO: 149 | SEQ ID NO: 269 |
| SEQ ID NO: 138 | SEQ ID NO: 258 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 368 | SEQ ID NO: 568 |
| SEQ ID NO: 384 | SEQ ID NO: 584 |
| SEQ ID NO: 388 | SEQ ID NO: 588 |
| SEQ ID NO: 389 | SEQ ID NO: 589 |
| SEQ ID NO: 538 | SEQ ID NO: 738 |

A nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO: 148 | SEQ ID NO: 268 |
| SEQ ID NO: 145 | SEQ ID NO: 265 |
| SEQ ID NO: 144 | SEQ ID NO: 264 |
| SEQ ID NO: 165 | SEQ ID NO: 285 |
| SEQ ID NO: 202 | SEQ ID NO: 322 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 388 | SEQ ID NO: 588 |
| SEQ ID NO: 385 | SEQ ID NO: 585 |
| SEQ ID NO: 384 | SEQ ID NO: 584 |
| SEQ ID NO: 405 | SEQ ID NO: 605 |
| SEQ ID NO: 442 | SEQ ID NO: 642 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 762 | SEQ ID NO: 772 |
| SEQ ID NO: 763 | SEQ ID NO: 773 |
| SEQ ID NO: 764 | SEQ ID NO: 774 |
| SEQ ID NO: 765 | SEQ ID NO: 775 |
| SEQ ID NO: 766 | SEQ ID NO: 776 |
| SEQ ID NO: 767 | SEQ ID NO: 777 |
| SEQ ID NO: 768 | SEQ ID NO: 778 |
| SEQ ID NO: 769 | SEQ ID NO: 779 |
| SEQ ID NO: 770 | SEQ ID NO: 780 |
| SEQ ID NO: 771 | SEQ ID NO: 781 |
| SEQ ID NO: 782 | SEQ ID NO: 773 |
| SEQ ID NO: 783 | SEQ ID NO: 775 |
| SEQ ID NO: 784 | SEQ ID NO: 777 |
| SEQ ID NO: 785 | SEQ ID NO: 779 |
| SEQ ID NO: 786 | SEQ ID NO: 781 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 385 | SEQ ID NO: 585 |
| SEQ ID NO: 388 | SEQ ID NO: 588 |
| SEQ ID NO: 764 | SEQ ID NO: 774 |
| SEQ ID NO: 765 | SEQ ID NO: 775 |
| SEQ ID NO: 766 | SEQ ID NO: 776 |
| SEQ ID NO: 767 | SEQ ID NO: 777 |

A nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO: 145 | SEQ ID NO: 265 |
| SEQ ID NO: 148 | SEQ ID NO: 268 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 385 | SEQ ID NO: 585 |
| SEQ ID NO: 764 | SEQ ID NO: 774 |
| SEQ ID NO: 765 | SEQ ID NO: 775 |
| SEQ ID NO:783 | SEQ ID NO:775 |
| SEQ ID NO: 801 | SEQ ID NO: 819 |
| SEQ ID NO: 802 | SEQ ID NO: 819 |
| SEQ ID NO: 803 | SEQ ID NO: 819 |
| SEQ ID NO: 804 | SEQ ID NO: 819 |
| SEQ ID NO: 805 | SEQ ID NO: 819 |
| SEQ ID NO: 806 | SEQ ID NO: 819 |
| SEQ ID NO: 807 | SEQ ID NO: 819 |
| SEQ ID NO: 808 | SEQ ID NO: 819 |
| SEQ ID NO: 809 | SEQ ID NO: 819 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 388 | SEQ ID NO: 588 |
| SEQ ID NO: 766 | SEQ ID NO: 776 |
| SEQ ID NO: 767 | SEQ ID NO: 777 |
| SEQ ID NO: 810 | SEQ ID NO: 820 |
| SEQ ID NO: 811 | SEQ ID NO: 820 |
| SEQ ID NO: 812 | SEQ ID NO: 820 |
| SEQ ID NO: 813 | SEQ ID NO: 820 |
| SEQ ID NO: 814 | SEQ ID NO: 820 |
| SEQ ID NO: 815 | SEQ ID NO: 820 |
| SEQ ID NO: 816 | SEQ ID NO: 820 |
| SEQ ID NO: 817 | SEQ ID NO: 820 |
| SEQ ID NO: 818 | SEQ ID NO: 820 |

A nucleic acid comprising first and second strands that comprise, consist of, or consist essentially of a nucleoside sequence differing by 0 or 1 nucleosides from any one of the following first and second sequences:

| Modified first strand | Modified second strand |
|---|---|
| SEQ ID NO: 764 | SEQ ID NO: 774 |
| SEQ ID NO: 766 | SEQ ID NO: 776 |

A conjugate for inhibiting expression of ZPI target gene in a cell, said conjugate comprising a nucleic acid as disclosed herein and one or more ligand moieties.

A pharmaceutical composition comprising a nucleic acid as disclosed herein, in combination with a pharmaceutically acceptable excipient or carrier.

A nucleic acid or pharmaceutical composition, for use in therapy.

A nucleic acid or pharmaceutical composition, for use in prevention or treatment of a disease related to a disorder of haemostasis, such as a disease related to a disorder of haemostasis, such as haemophilia.

### FIGURES

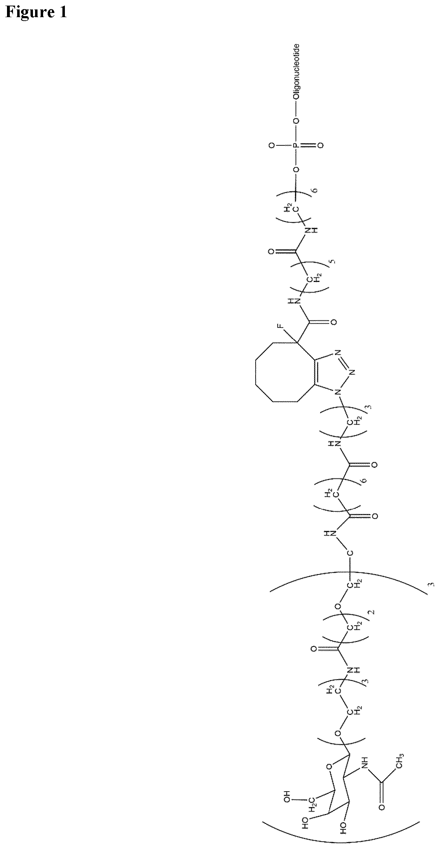

**Figure 1****:** Linker and ligand portions of constructs suitable for use according to the present invention including tether 1a. While Figure 1 depicts the linker to be conjugated to an oligonucleotide, it is to be understood that the present invention also encompasses conjugates of the same linker with an oligonucleoside as disclosed herein.
   It should also be understood that while Figure 1 depicts as a product molecules based on the linker and ligand portions as specifically depicted in Figure 1 attached to an oligonucleoside moiety as also depicted herein, this product may alternatively further comprise, or consist essentially of, molecules wherein the linker and ligand portions are essentially as depicted in Figure 1 attached to an oligonucleoside moiety but having the F substituent as shown in Figure 1 on the cyclo-octyl ring replaced by a substituent occurring as a result of hydrolytic displacement, such as an OH substituent. In this way, (a) tether 1a constructs can consist essentially of molecules having linker and ligand portions specifically as depicted in Figure 1, with a F substituent on the cyclo-octyl ring; or (b) tether 1a constructs can consist essentially of molecules having linker and ligand portions essentially as depicted in Figure 1 but having the F substituent as shown in Figure 1 on the cyclo-octyl ring replaced by a substituent occurring as a result of hydrolytic displacement, such as an OH substituent, or (c) tether 1a constructs can comprise a mixture of molecules as defined in (a) and/or (b).

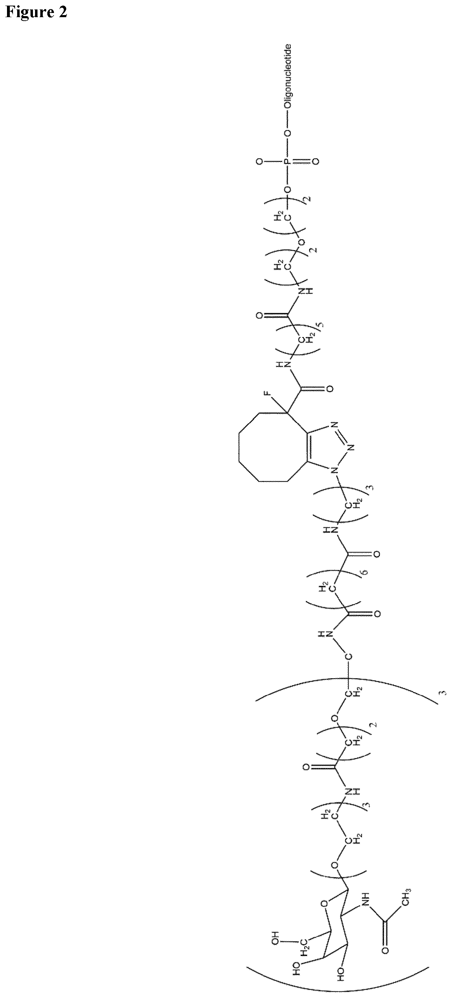

**Figure 2****:** Linker and ligand portions of constructs suitable for use according to the present invention including tether 1b. While Figure 2 depicts the linker to be conjugated to an oligonucleotide, it is to be understood that the present invention also encompasses conjugates of the same linker with an oligonucleoside as disclosed herein.
   The comments made in relation to Figure 1 and the possible replacement of the F substituent as shown in Figure 1 on the cyclo-octyl ring replaced by a substituent occurring as a result of hydrolytic displacement, such as an OH substituent, apply equally to tether 1b constructs. In this way, (a) tether 1b constructs can consist essentially of molecules having linker and ligand portions specifically as depicted in Figure 2, with a F substituent on the cyclo-octyl ring; or (b) tether 1b constructs can consist essentially of molecules having linker and ligand portions essentially as depicted in Figure 2 but having the F substituent as shown in Figure 2 on the cyclo-octyl ring replaced by a substituent occurring as a result of hydrolytic displacement, such as an OH substituent, or (c) tether 1b constructs can comprise a mixture of molecules as defined in (a) and/or (b).

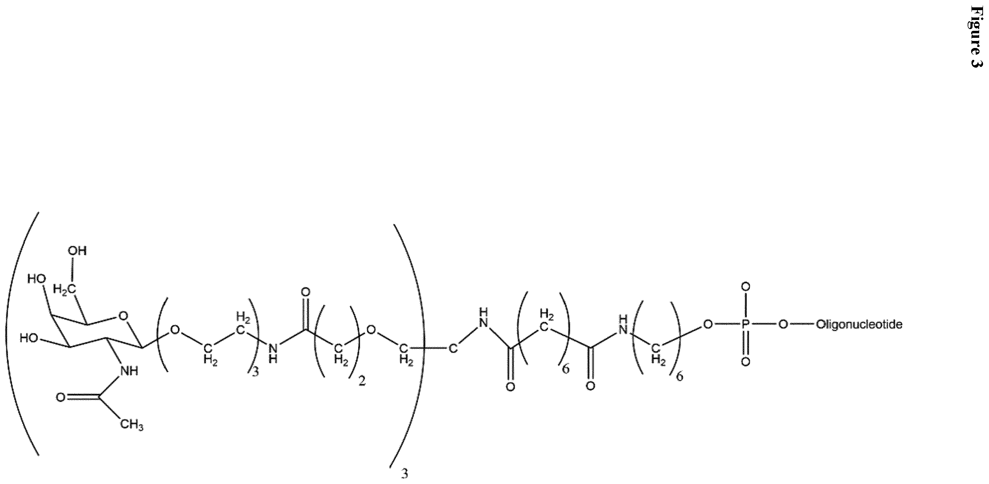

**Figure 3****:** Linker and ligand portions of constructs suitable for use according to the present invention including tether 2a. While Figure 3 depicts the linker to be conjugated to an oligonucleotide, it is to be understood that the present invention also encompasses conjugates of the same linker with an oligonucleoside as disclosed herein.

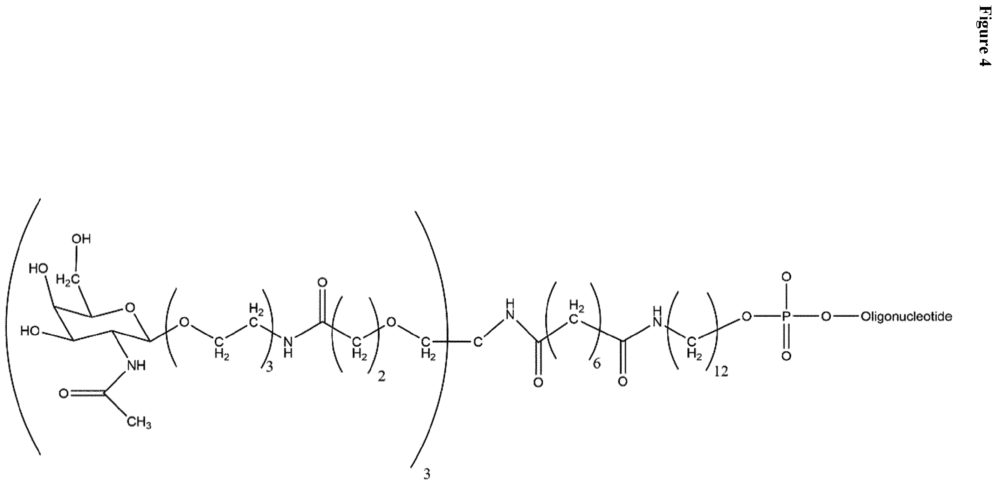

**Figure 4****:** Linker and ligand portions of constructs suitable for use according to the present invention including tether 2b. While Figure 4 depicts the linker to be conjugated to an oligonucleotide, it is to be understood that the present invention also encompasses conjugates of the same linker with an oligonucleoside as disclosed herein.

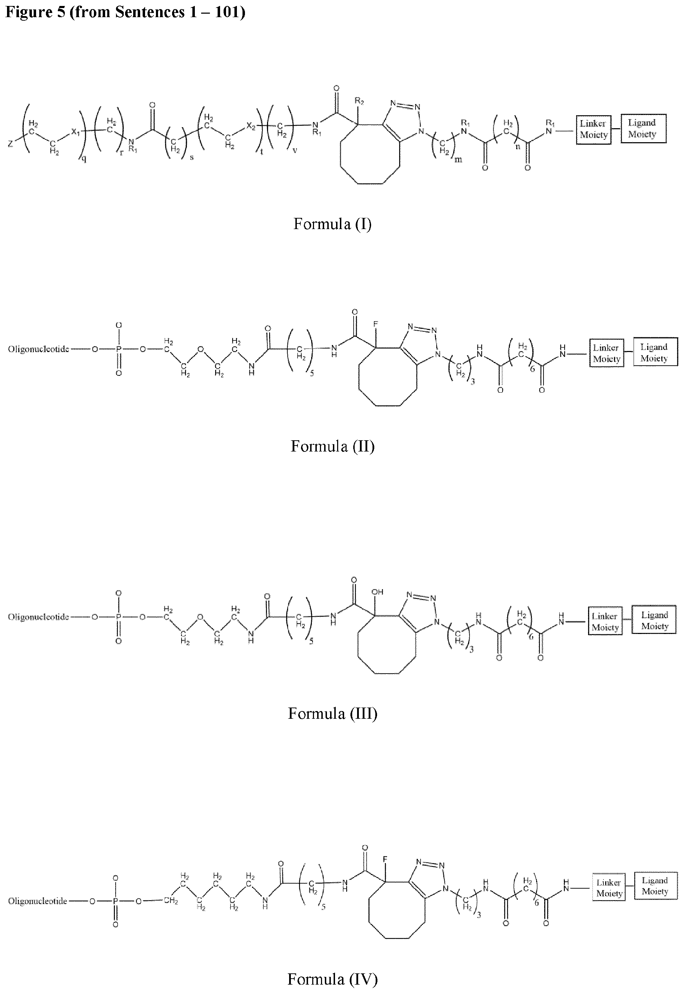

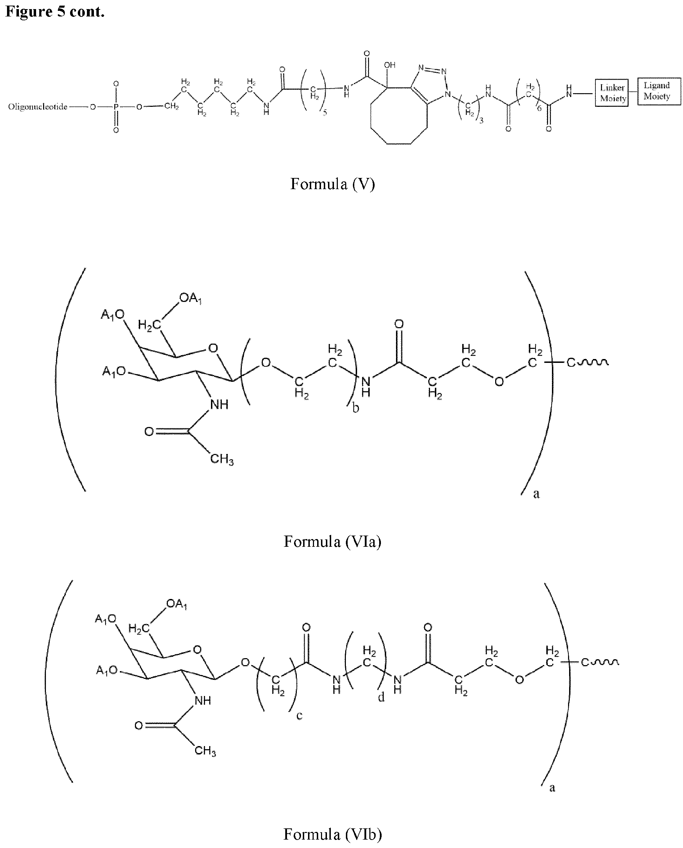

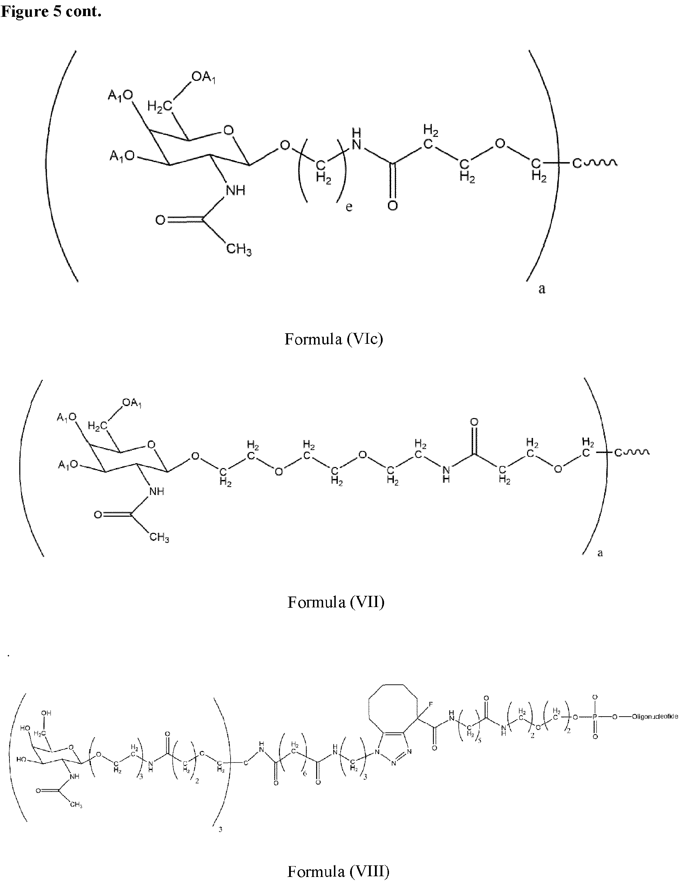

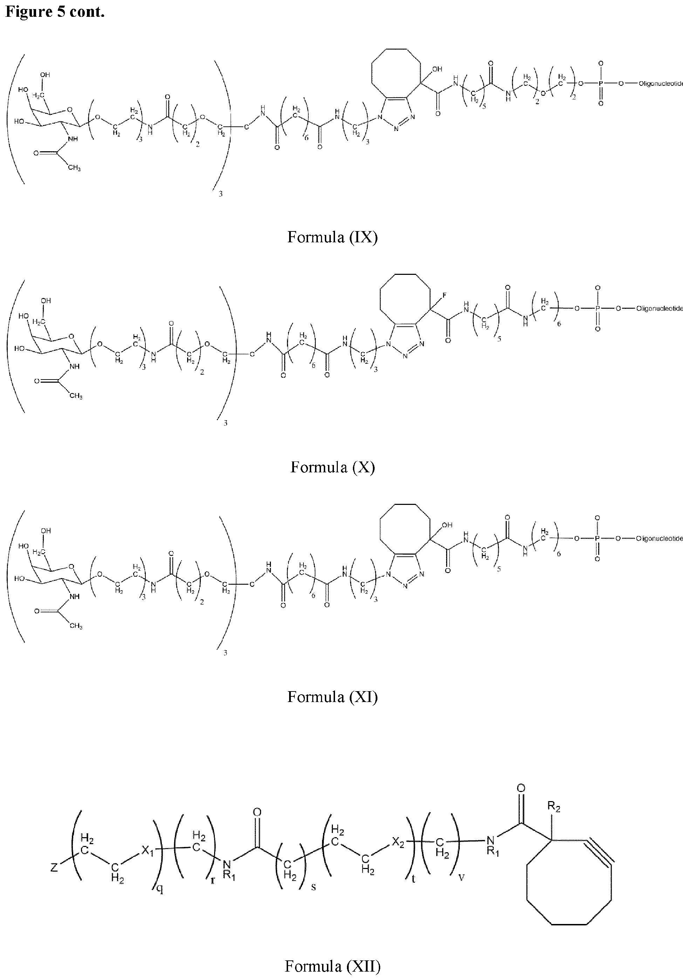

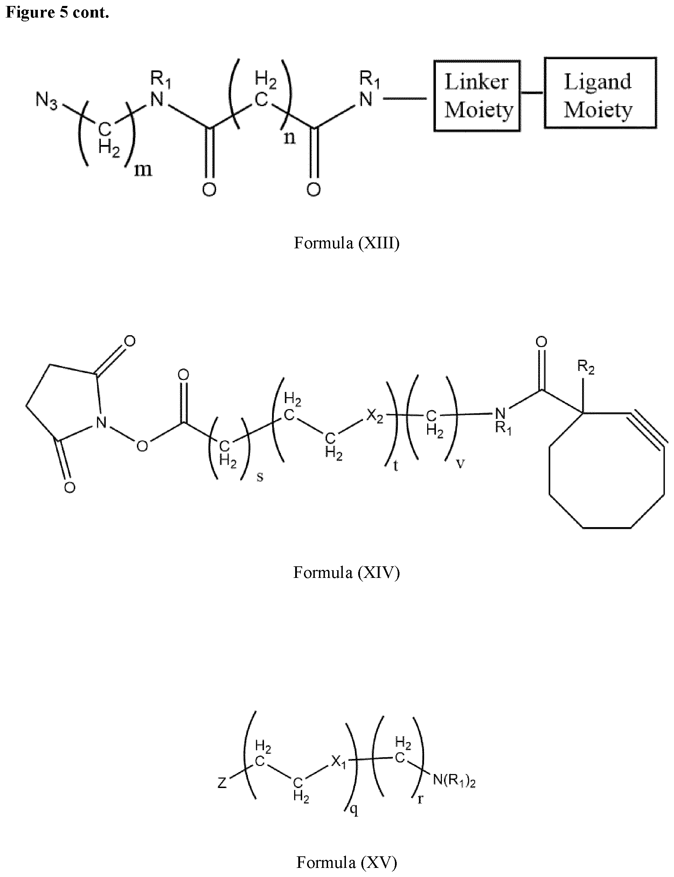

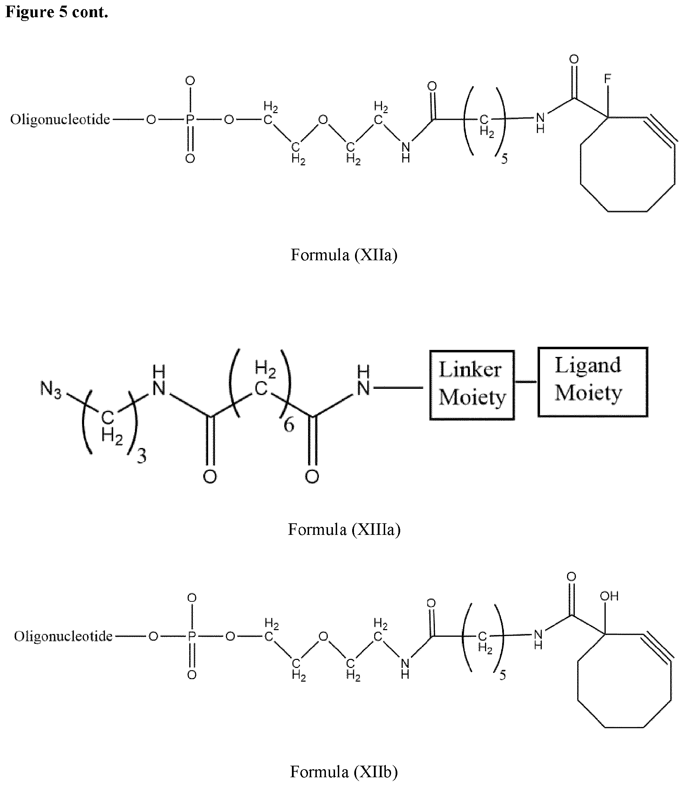

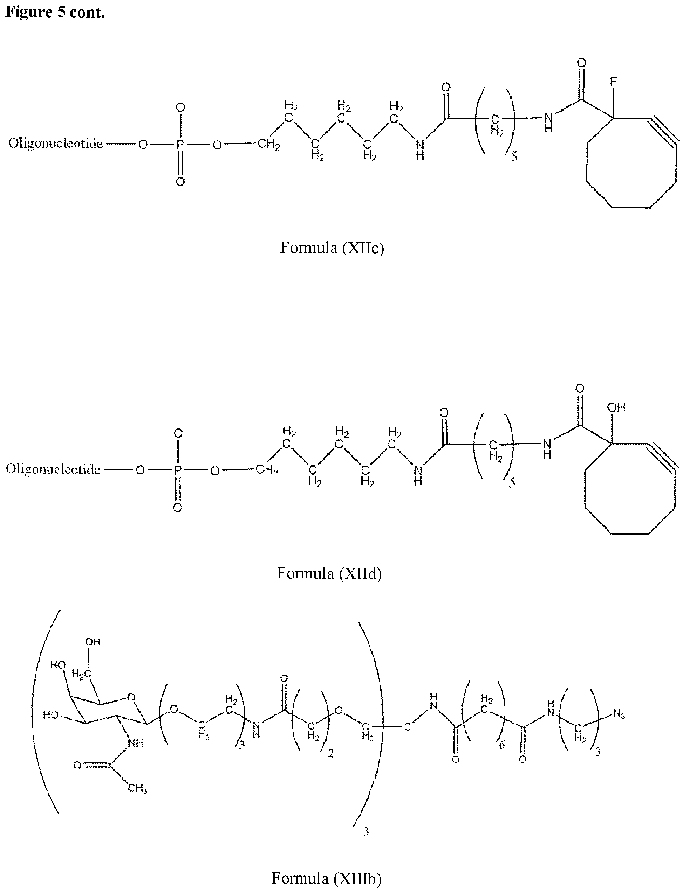

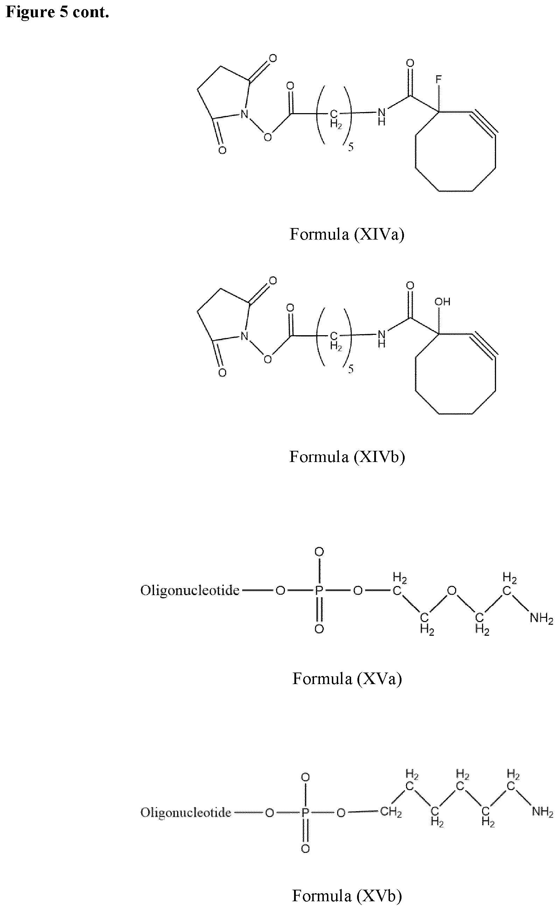

**Figure 5****:** Formulae described in Sentences 1-101 disclosed herein.

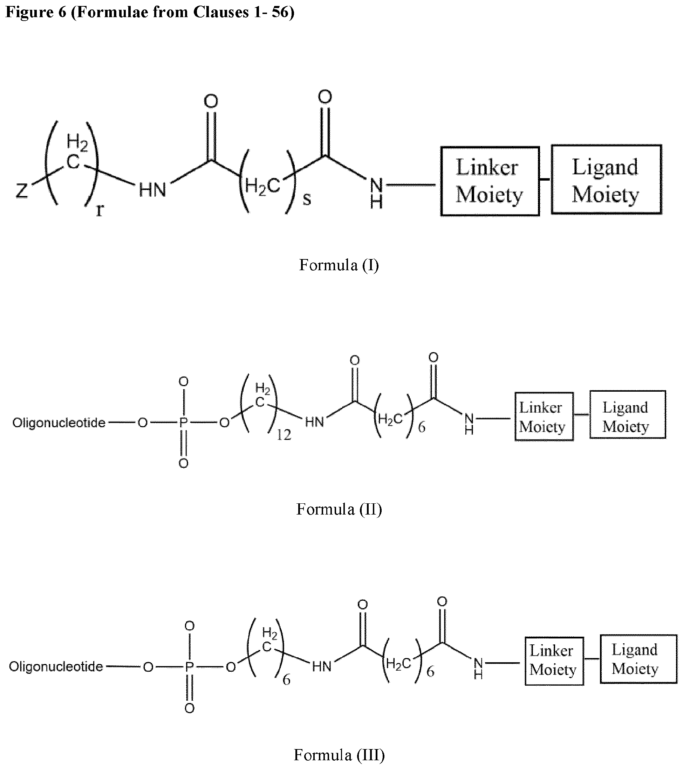

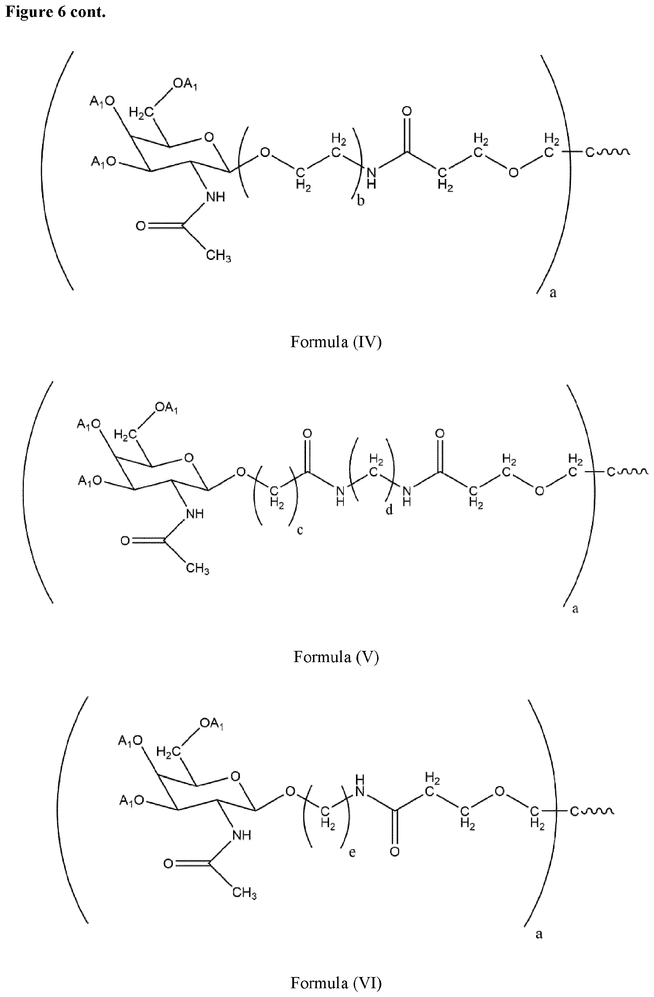

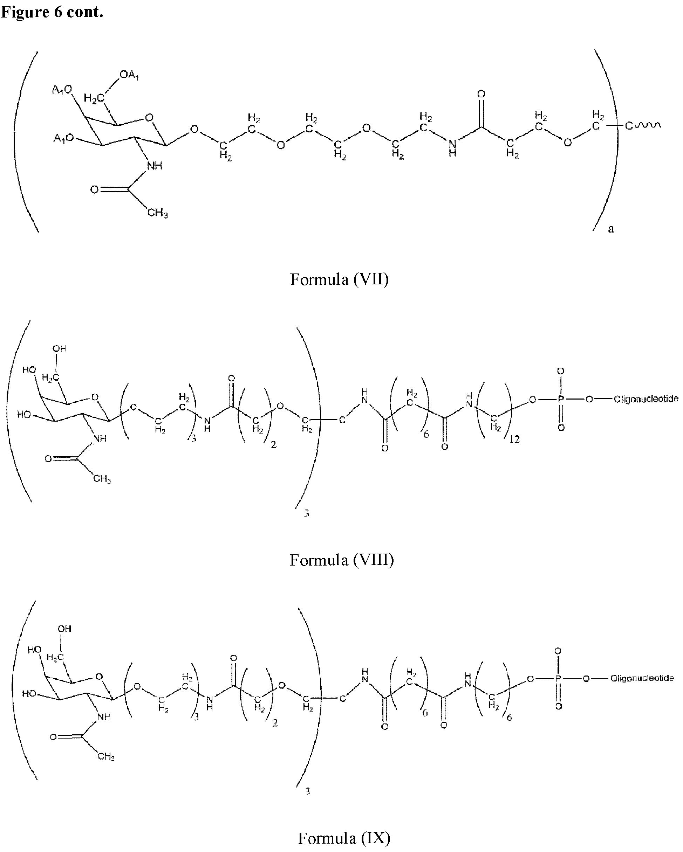

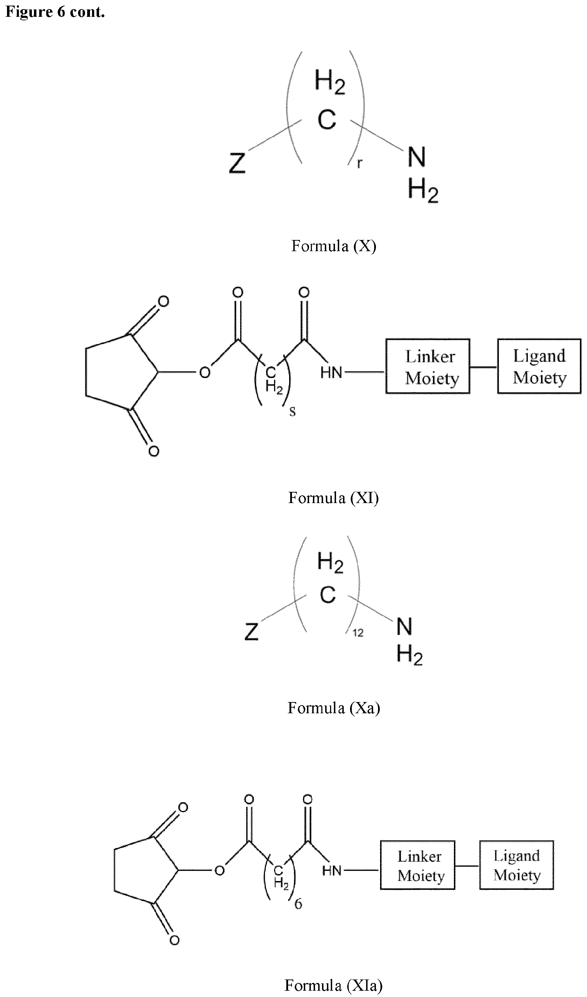

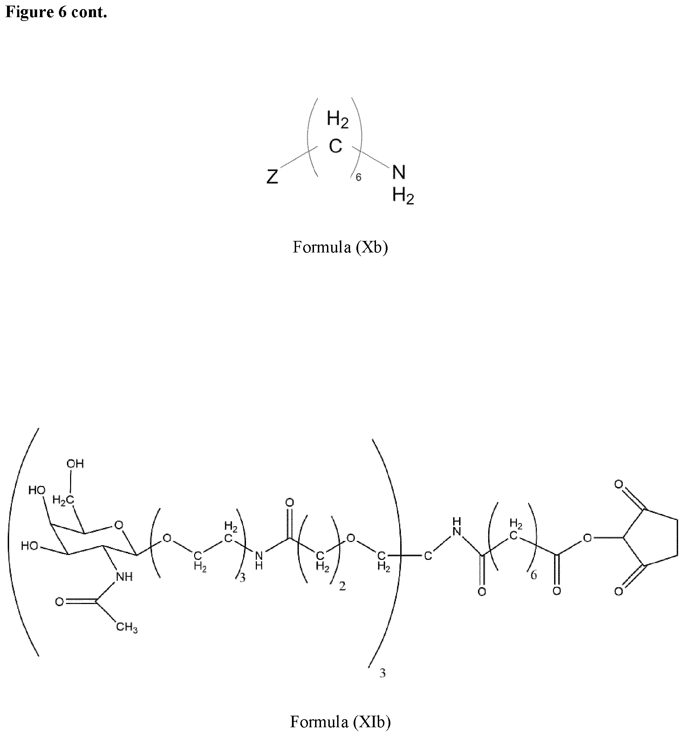

**Figure 6****:** Formulae described in Clauses 1-56 disclosed herein

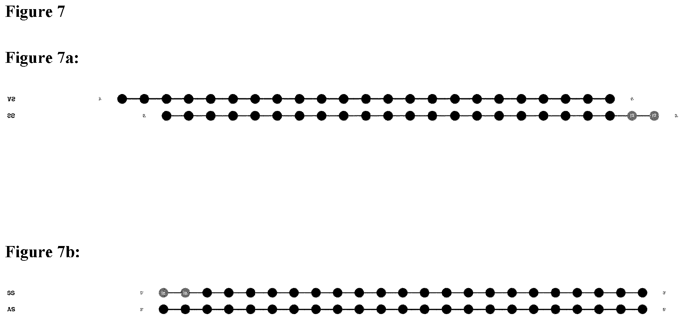

**Figures 7a and 7b****:** Inverted abasic constructs that can be used with nucleic acid sequences according to the present invention as described herein. For Figure 7a, a galnac linker is attached to the 5' end region of the sense strand in use (not depicted in Figure 7a). For Figure 7b, a galnac linker is attached to the 3' end region of the sense strand in use (not depicted in Figure 7b).
   iaia as shown at the 3' end region of the sense strand in Figure 7a represents (i) two abasic nucleosides provided as the penultimate and terminal nucleosides at the 3' end region of the sense strand, (ii) wherein a 3'-3' reversed linkage is provided between the antepenultimate nucleoside (namely at position 21 of the sense strand, wherein position 1 is the terminal 5' nucleoside of the sense strand) and the adjacent penultimate abasic residue of the sense strand, and (iii) the linkage between the terminal and penultimate abasic nucleosides is 5'-3' when reading towards the 3' end region comprising the terminal and penultimate abasic nucleosides.
   iaia as shown at the 5' end region of the sense strand in Figure 7b represents (i) two abasic nucleosides provided as the penultimate and terminal nucleosides at the 5' end region of the sense strand, (ii) wherein a 5'-5' reversed linkage is provided between the antepenultimate nucleoside (namely at position 1 of the sense strand, not including the iaia motif at the 5' end region of the sense strand in the nucleoside position numbering on the sense strand) and the adjacent penultimate abasic residue of the sense strand, and (iii) the linkage between the terminal and penultimate abasic nucleosides is 3'-5' when reading towards the 5' end region comprising the terminal and penultimate abasic nucleosides.

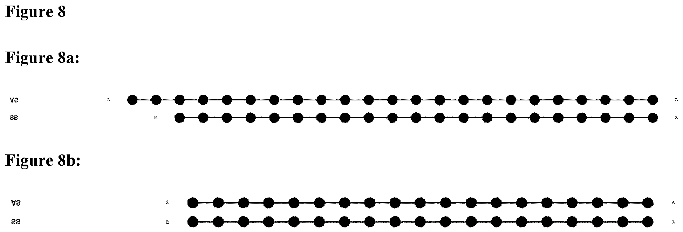

**Figures 8a and 8b****:** Duplex constructs according to Table 5.

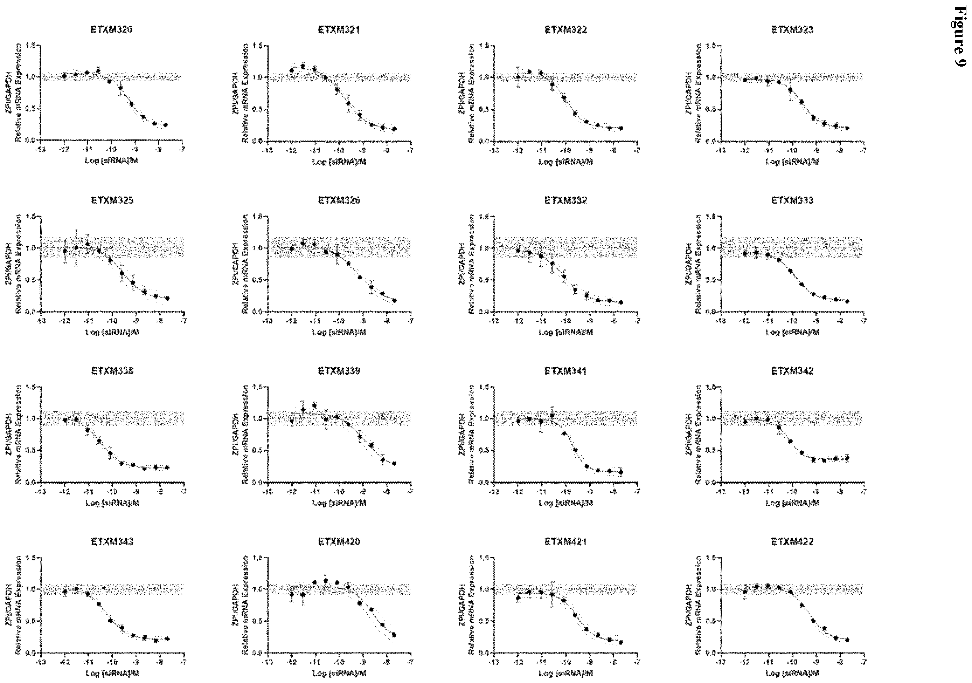

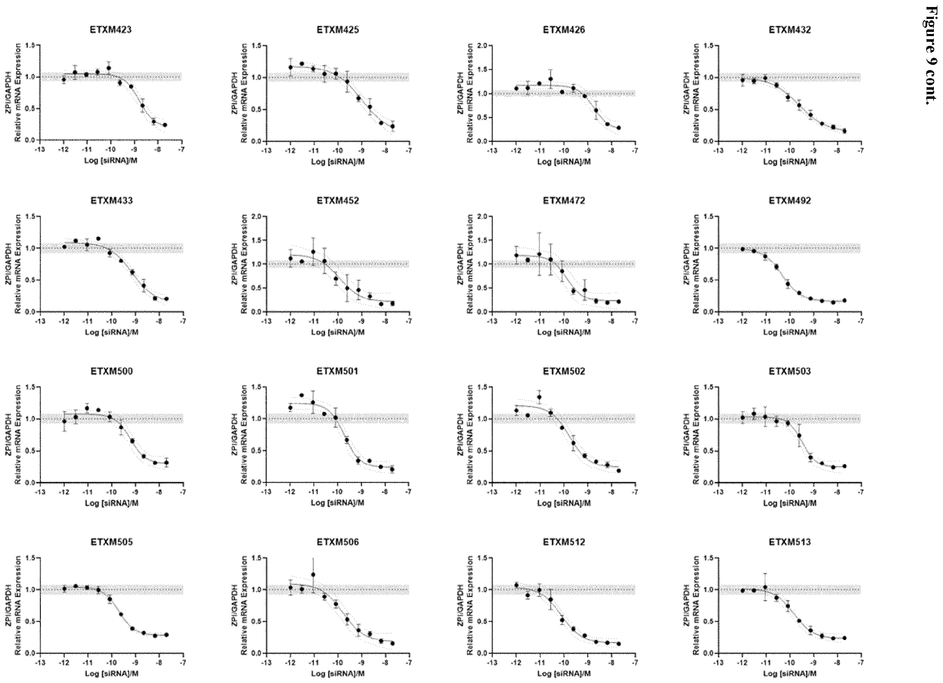

**Figure 9****:** Results of dose-response experiments for inhibition of ZPI mRNA expression in human Huh7 cells. Points represent mean relative expression of ZPI mRNA compared to untreated wells after treatment with siRNA construct at the indicated concentrations on the x-axis. Error bars represent standard deviation of the mean. Dotted curves represent 95% confidence intervals. Dotted lines and shaded areas represent the mean relative expression +/standard deviation from untreated wells on the same plate.

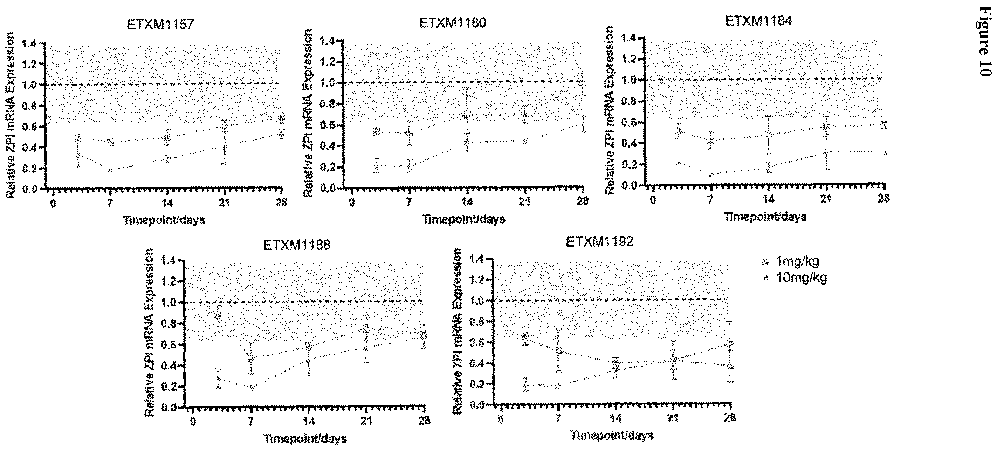

**Figure 10****:** Change in liver ZPI mRNA expression over time following subcutaneous delivery of GalNAc conjugated siRNAs in C57BL/6 mice. ETXM1180, 1188, and 1192 were all murinised for this assay as described in Example 11. Data are mean +/- standard deviation, n=3 mice per timepoint.

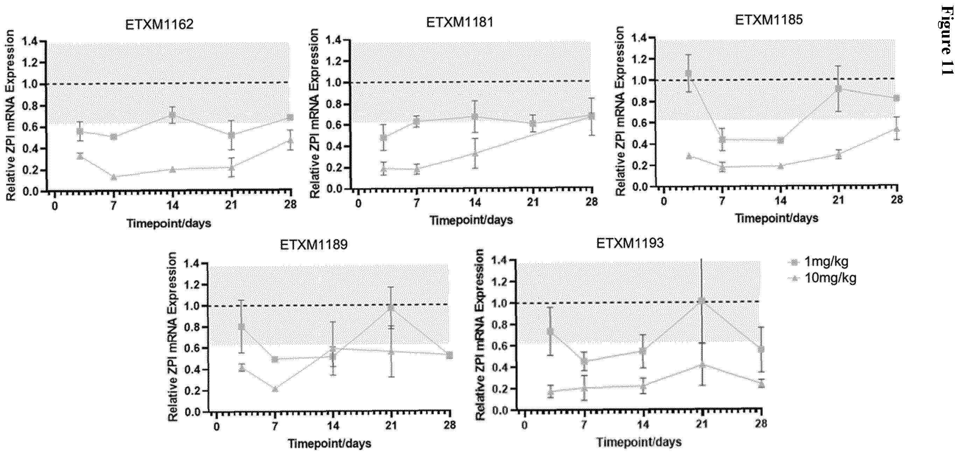

**Figure 11****:** Change in liver ZPI mRNA expression over time following subcutaneous delivery of GalNAc conjugated siRNAs in C57BL/6 mice. ETXM1181, 1189 and 1193 were all murinised for this assay as described in Example 11. Data are mean +/- standard deviation, n=3 mice per timepoint.

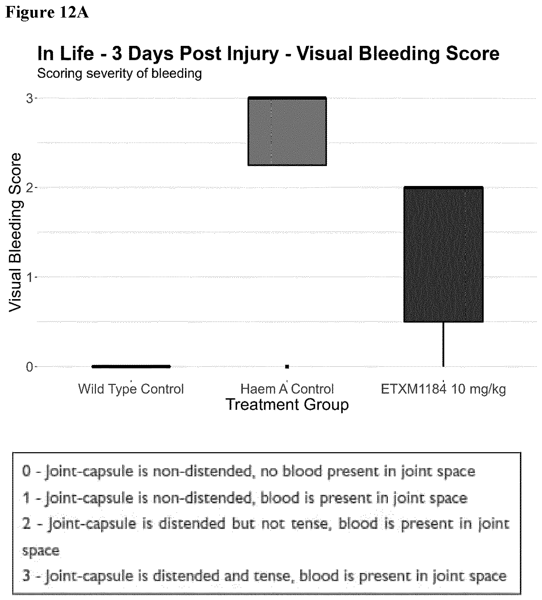

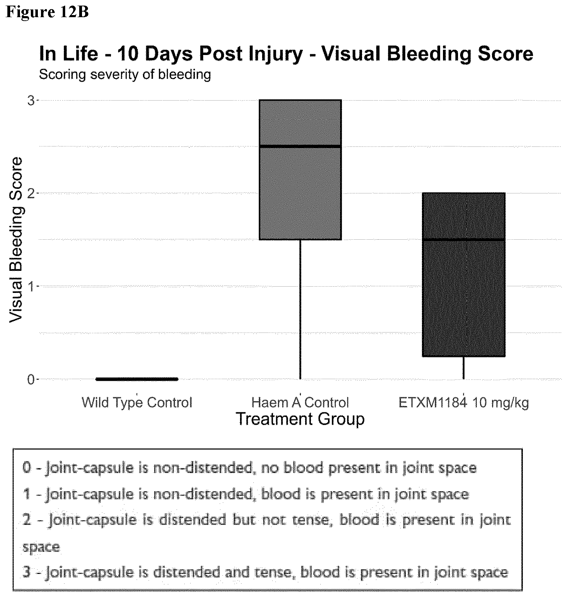

**Figure 12****:** Visual bleeding score of mice in three different treatment groups (wild type control group, Haem A mice that received a vehicle (0.9% saline), and Haem A mice that received the GalNAc-siRNA construct ETXM1184) 3 days (Fig.10A) and 10 days (Fig.10B) post injury. Definition of the bleeding scores is provided below.

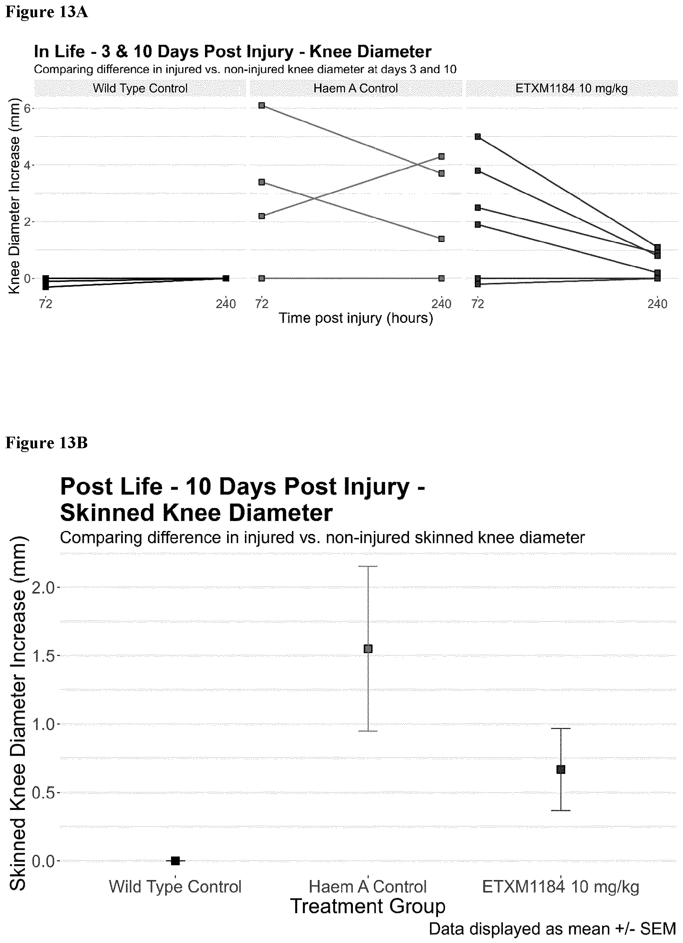

**Figure 13****:** (A) Comparison of knee diameters at day 3 and 10 post injury of mice in three different treatment groups (wild-type control group, Haem A mice receiving vehicle (0.9% saline), and Haem A mice receiving the GalNAc-siRNA construct ETXM1184). (B) Comparison of skinned knee diameter at day 10 post injury of mice in the same three treatment groups.

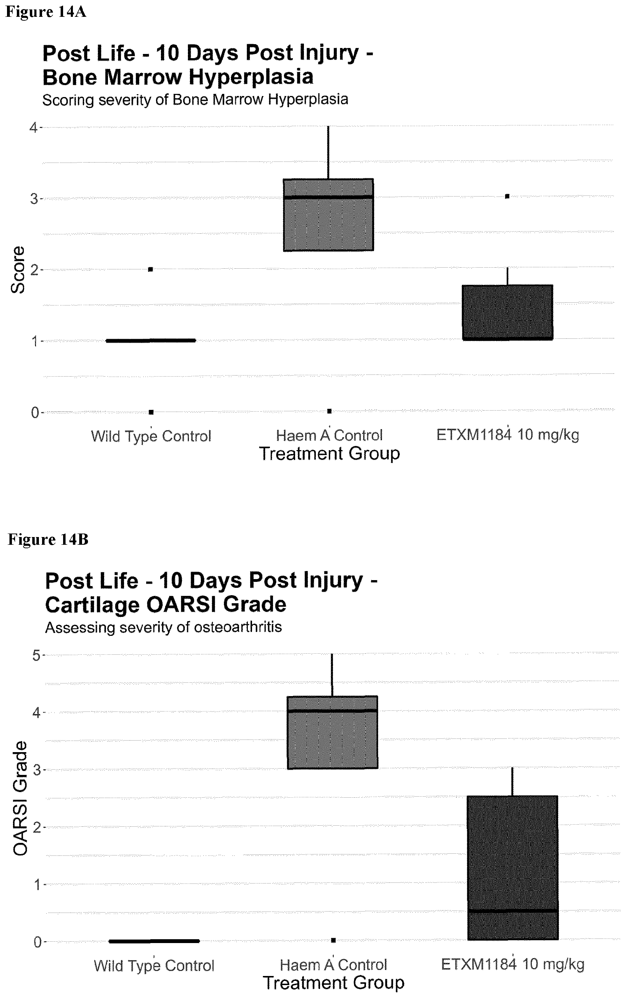

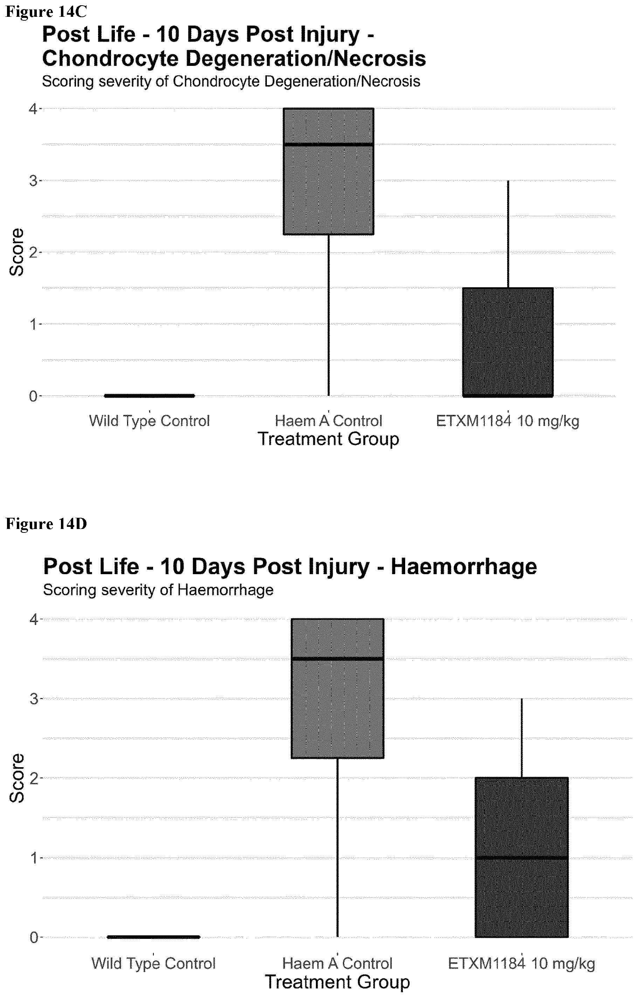

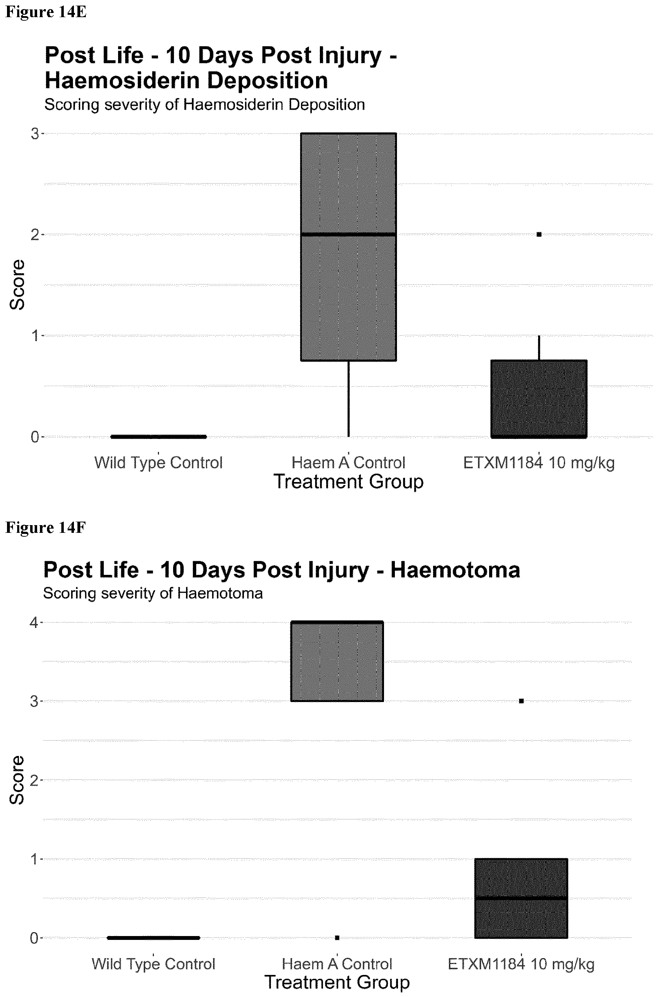

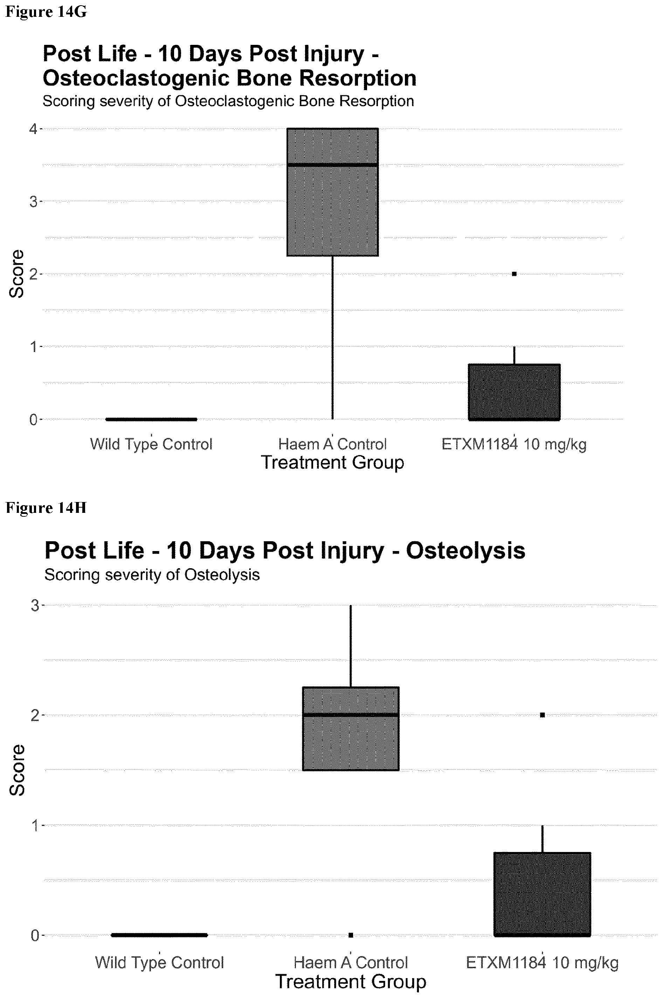

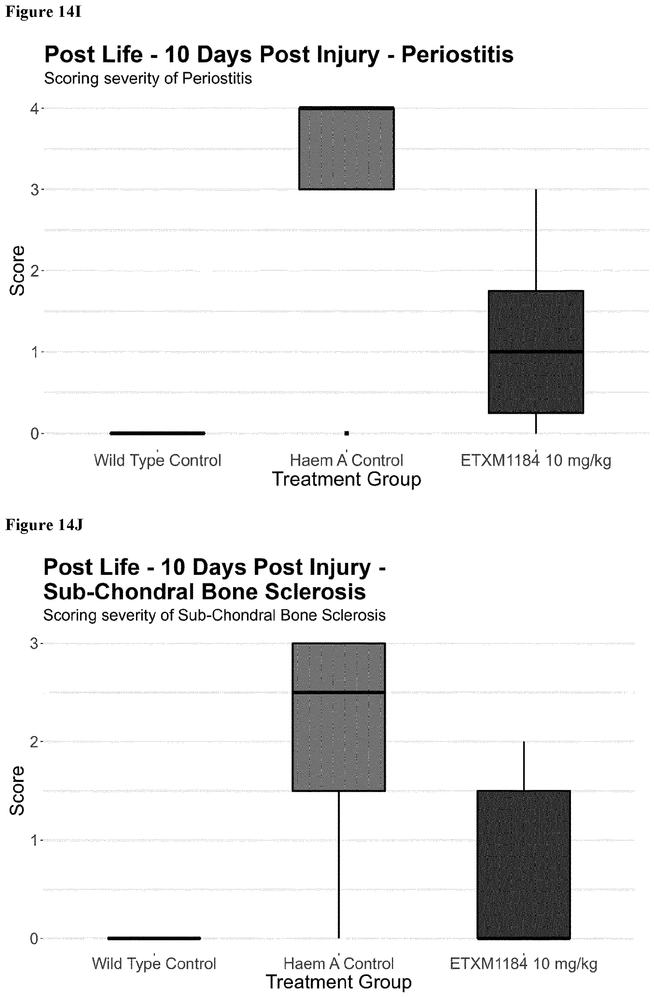

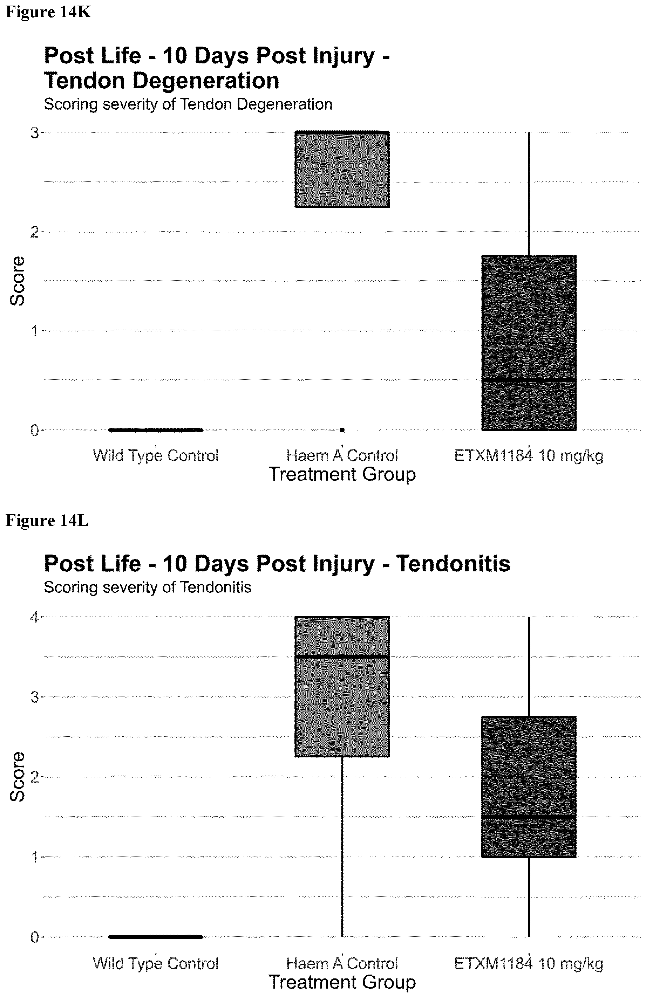

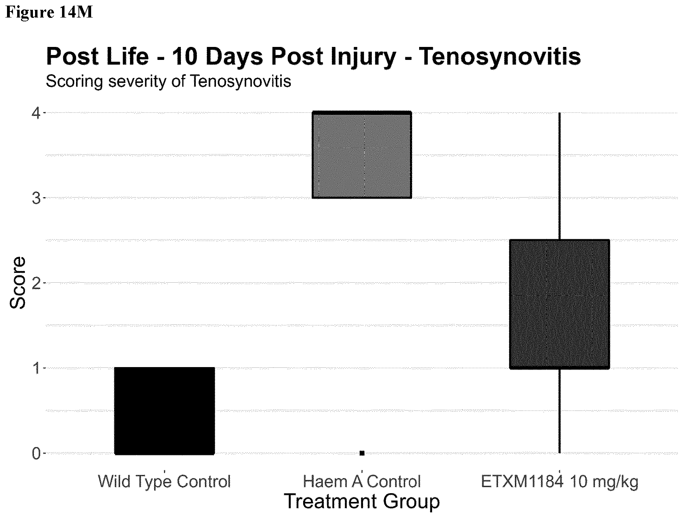

**Figure 14****:** Comparison of the severity of (A) bone marrow hyperplasia, (B) osteoarthritis, (C) chondrocyte degeneration/necrosis, (D) haemorrhage, (E) haemosiderin deposition, (F) haematoma, (G) osteoclastogenic bone resorption, (H) osteolysis, (I) periostitis, (J) sub-chondral bone sclerosis, (K) tendon degeneration, (L) tendonitis and (M) tenosynovitis (Fig.12M) in mice in three different treatment groups (wild-type control group, Haem A mice receiving vehicle (0.9% saline), and Haem A mice receiving the GalNAc-siRNA construct ETXM1184).

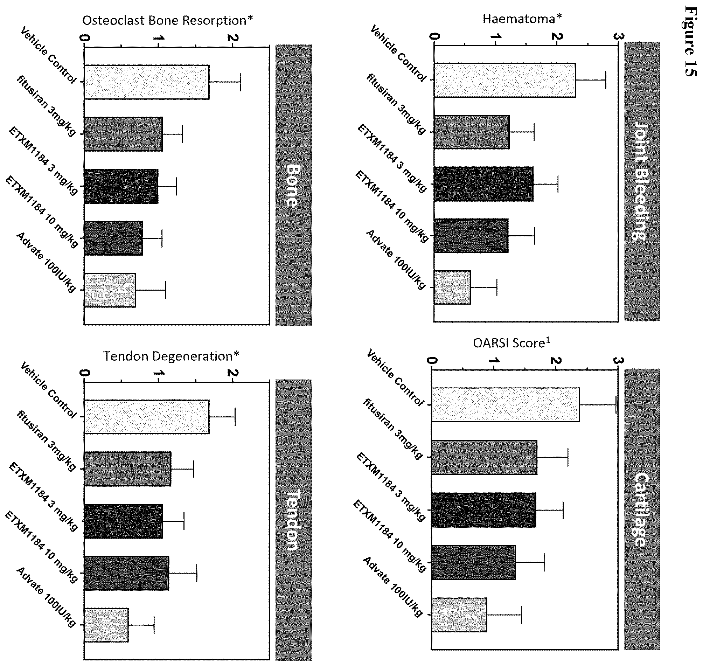

**Figure 15****:** Joint Protection: Several Endpoints Document Dose-Responsive Effect. Prophylactic administration of ETXM1184 shows dose-dependent protection in key tissue readouts at 10 days post-injury. ETXM1184 shows efficacy in the same range as clinical comparators: FVIII replacement therapy as gold-standard for emergency treatments (Advate) and siRNA-based rebalancing agent for prophylaxis that demonstrated good bleed protection in late-stage clinical development (fitusiran). * Scale: 0 = Normal; 1 = Minimal; 2 = Moderate; 3 = Marked; 4 = Severe. [1] Glasson et al., Osteoarthritis Cartilage. 2010 Oct; 18 Suppl 3:S17-23. doi: 10.1016/j.joca.2010.05.025. PMID: 20864019.

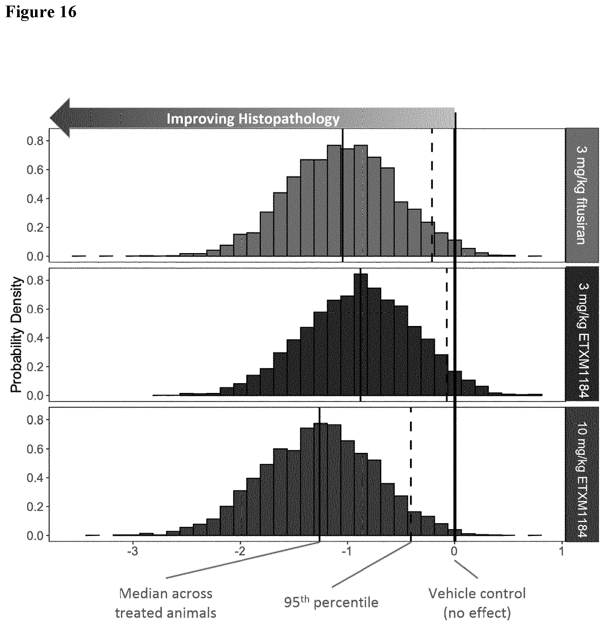

**Figure 16****:** Composite haemarthrosis histopathology score quantifies: Tendonitis, Tendon degeneration, Tenosynovitis, Periostitis, Osteolysis, Osteoclastic bone resorption, Haemorrhage, Haematoma, Haemosiderin deposition, Chondrocyte necrosis, Cartilage OARSI Grade, Subchondral bone sclerosis and Bone marrow hyperplasia. ETX-148 shows significant dose-responsive effect (Bayesian linear model fitted to composite score). Median reduction of composite score compared to control: -1.25 for the ETXM1184 10 mg/kg group (significance level equivalent to p<0.01); -0.91 for the ETXM1184 3 mg/kg group (significance level equivalent to p<0.05). Comparator fitusiran shows median reduction of - 1.04 for the 3 mg/kg group (significance level equivalent to p<0.05).

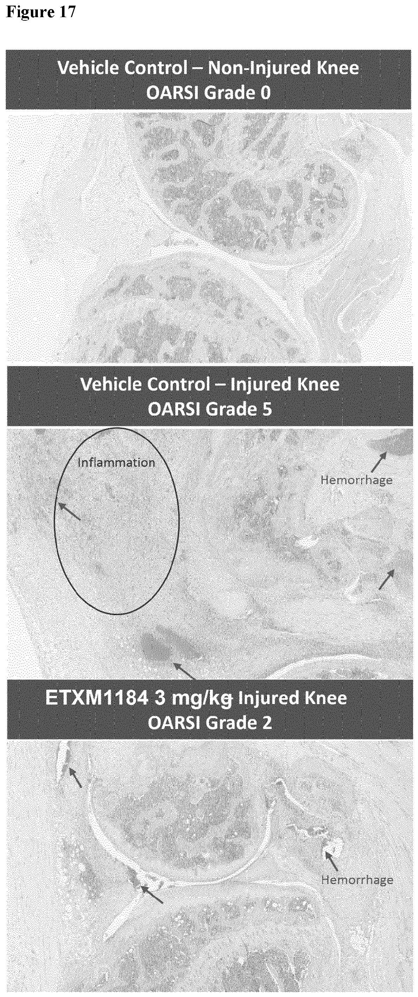

**Figure 17****:** Prophylactic administration of ETXM1184 improves haemarthrosis joint pathology in haemophilia A mice. Administration of 3 mg/kg ETXM1184 resulted in improved hemarthrosis knee joint pathology, reduced inflammation, and resulted in smaller areas of haemorrhage.

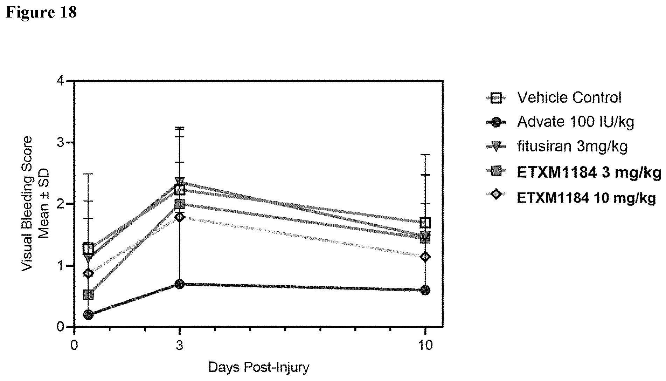

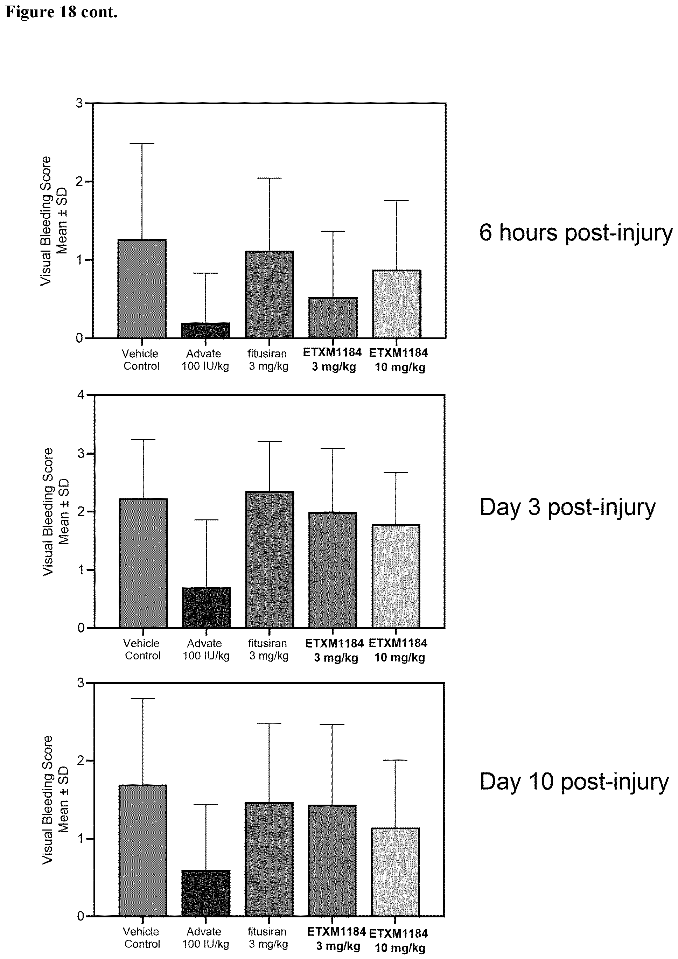

**Figure 18****:** Prophylactic administration of ETXM1184 reduces post-injury bleeding in hemophilia A mice (in-life visual bleeding score (VBS)). A bleeding event was introduced into the knee joint of Hemophilia A mice 8 days after siRNA administration. Bleeding was monitored for 10 days post-injury and terminal histological analysis was conducted. Prophylactic administration of a single 10 mg/kg dose of ETXM1184 effectively reduced visual bleeding score (VBS) comparably to Factor VIII replacement (Advate) by 10 days post-injury.

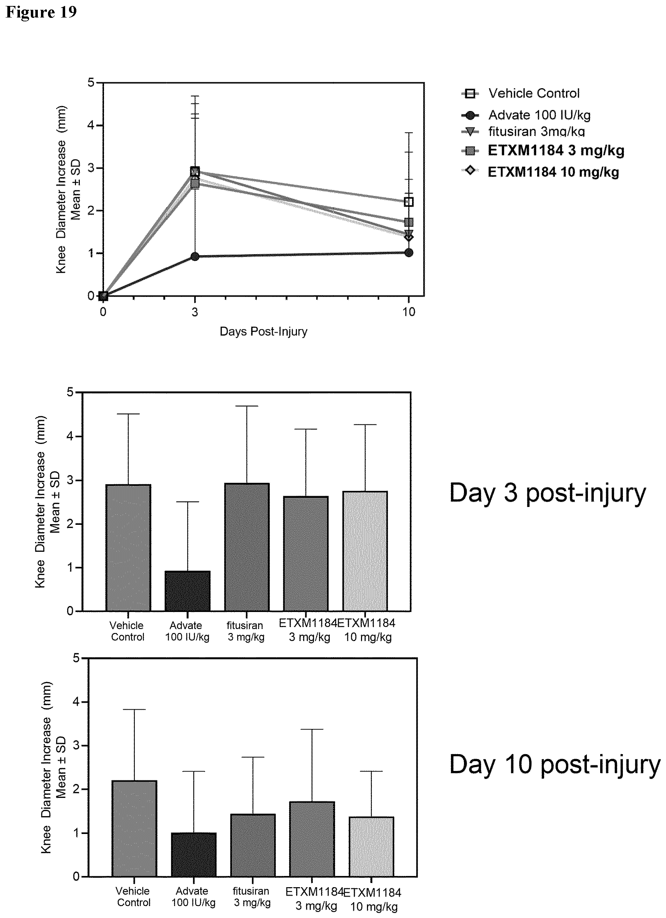

**Figure 19****:** Prophylactic administration of ETXM1184 reduces post-injury bleeding into the knee joint of hemophilia A mice (in-life measurement of injured knee diameter compared to non-injured knee diameter). A bleeding event was introduced into the knee joint of Hemophilia A mice 8 days after siRNA administration. Bleeding was monitored for 10 days post-injury and terminal histological analysis conducted. Prophylactic ETXM1184 administered as a single 10 mg/kg dose effectively reduced blood accumulation in knee joint comparably to Factor VIII replacement (Advate) by 10 days post-injury.

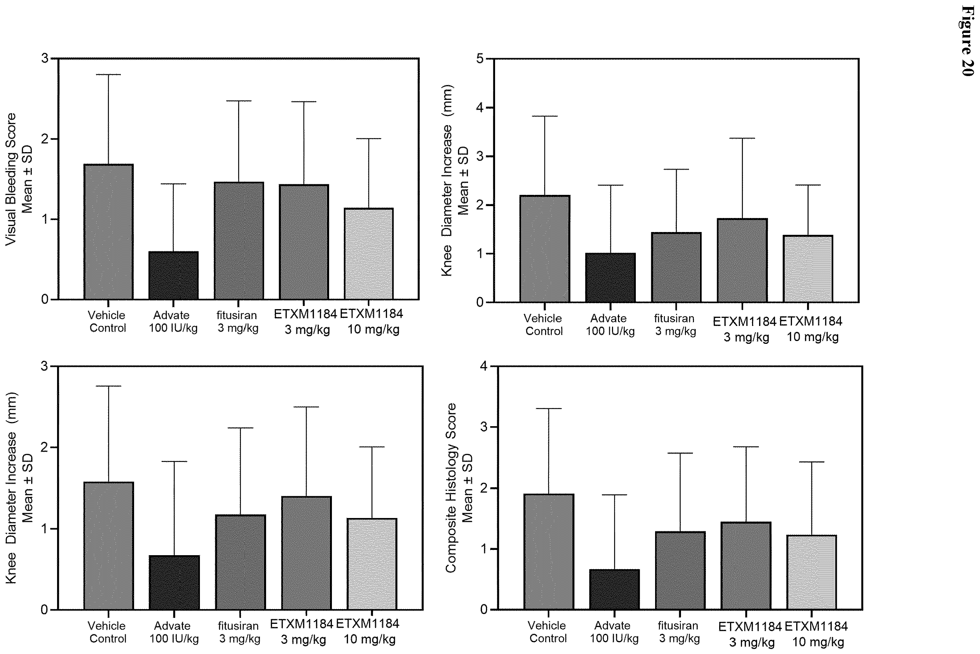

**Figure 20****:** Prophylactic administration of ETXM1184 reduces hemarthrosis in a Hemophilia A mouse model (terminal measurements taken 18 days post-siRNA dosing and 10 days post-injury). Prophylactic ETXM1184 administered as a single 10 mg/kg dose effectively reduced joint bleeding and characteristics of hemophilic arthropathy comparably to Factor VIII replacement (Advate) by 10 days post-injury.

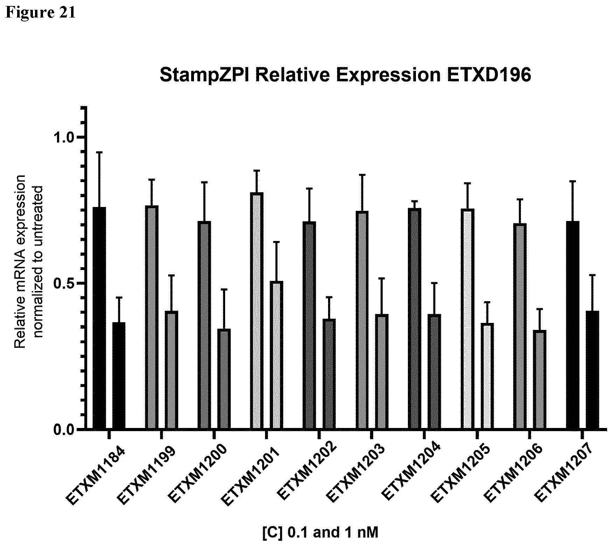

**Figure 21****:** Inhibition of ZPI expression by ETXM1184 (ETXS1036 & ETXS1035), ETXM1199 (ETXS2398 & ETXS2397), ETXM1200 (ETXS2400 & ETXS2397), ETXM1201 (ETXS2402 & ETXS2397), ETXM1202 (ETXS2404 & ETCS2397), ETXM1203 (ETXS2406 & ETXS2397), ETXM 1204 (ETXS2408 & ETXS2397), ETXM 1205 (ETXS2410 & ETXS2397), ETXM1206 (ETXS2412 & ETXS2397) and ETXM1207 (ETXS2414 & ETXS2397).

### DEFINITIONS

The "first strand", also called the antisense strand or guide strand herein and which can be used interchangeably herein, refers to the nucleic acid strand, e.g. the strand of an siRNA, e.g. a dsiRNA, which includes a region that is substantially complementary to a target sequence, e.g. to an mRNA. As used herein, the term "region of complementarity" refers to the region on the antisense strand that is substantially complementary to a sequence, for example a target sequence. Where the region of complementarity is not fully complementary to the target sequence, the mismatches can typically be in the internal or terminal regions of the molecule. In some embodiments, a double stranded nucleic acid e.g. an siRNA agent of the invention includes a nucleoside mismatch in the antisense strand.

The "second strand" (also called the sense strand or passenger strand herein, and which can be used interchangeably herein), refers to the strand of a nucleic acid e.g. siRNA that includes a region that is substantially complementary to a region of the antisense strand as that term is defined herein.

In the context of molecule comprising a nucleic acid provided with a ligand moiety, optionally also with a linker moiety, the nucleic acid of the invention may be referred to as an oligonucleoside or an oligonucleoside moiety.

Oligonucleotides are short nucleic acid polymers. Whilst oligonucleotides contain phosphodiester bonds between the nucleoside component thereof (base plus sugar), the present invention is not limited to oligonucleotides always joined by such a phosphodiester bond between adjacent nucleosides, and other oligomers of nucleosides joined by bonds which are bonds other than a phosphodiester bond are contemplated. For example, a bond between nucleosides may be a phosphorothioate bond. Therefore, the term "oligonucleoside" as used herein covers both oligonucleotides and other oligomers of nucleosides. An oligonucleoside which is a nucleic acid having at least a portion which is an oligonucleotide is preferred according to the present invention. An oligonucleoside having one or more, or a majority of, phosphodiester backbone bonds between nucleosides is also preferred according to the present invention. An oligonucleoside having one or more, or a majority of, phosphodiester backbone bonds between nucleosides, and also having one or more phosphorothioate backbone bonds between nucleosides (typically in a terminal region of the first and / or second strands) is also preferred according to the present invention.

It is preferred herein that the nucleic acid according to the invention is a double stranded oligonucleoside comprising one or more phosphorothioate backbone bonds between nucleosides. Accordingly, in all instances in which the present application refers to an oligonucleotide, particularly in the chemical structures disclosed herein, the oligonucleotide may equally be an oligonucleoside as defined herein.

In some embodiments, a double stranded nucleic acid e.g. siRNA agent of the invention includes a nucleoside mismatch in the sense strand. In some embodiments, the nucleoside mismatch is, for example, within 5, 4, 3, 2, or 1 nucleosides from the 3 '-end of the nucleic acid e.g. siRNA.

In another embodiment, the nucleoside mismatch is, for example, in the 3'- terminal nucleoside of the nucleic acid e.g. siRNA.

A "target sequence" (which may also be called a target RNA or a target mRNA) refers to a contiguous portion of the nucleoside sequence of an mRNA molecule formed during the transcription of a gene, including mRNA that is a product of RNA processing of a primary transcription product.

The target sequence may be from about 10-35 nucleosides in length, e.g., about 15-30 nucleosides in length. For example, the target sequence can be from about 15-30 nucleosides, 15-29, 15-28, 15-27, 15-26, 15-25, 15-24, 15-23, 15-22, 15-21, 15-20, 15-19, 15-18, 15-17, 18-30, 18-29, 18- 28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24, 20-23, 20-22, 20- 21, 21-30, 21-29, 21-28, 21-27, 21-26, 21-25, 21-24, 21-23, or 21-22 nucleosides in length. Ranges and lengths intermediate to the above recited ranges and lengths are also contemplated to be part of the disclosure.

The term "ribonucleoside" or "nucleoside" can also refer to a modified nucleoside, as further detailed below.

A nucleic acid can be a DNA or an RNA, and can comprise modified nucleosides. RNA is a preferred nucleic acid.

The terms "iRNA", "siRNA", "RNAi agent," and "iRNA agent," "RNA interference agent" as used interchangeably herein, refer to an agent that contains RNA, and which mediates the targeted cleavage of an RNA transcript via an RNA-induced silencing complex (RISC) pathway. siRNA directs the sequence-specific degradation of mRNA through RNA interference (RNAi).

A double stranded RNA is referred to herein as a "double stranded siRNA (dsiRNA) agent", "double stranded siRNA (dsiRNA) molecule", "double stranded RNA (dsRNA) agent", "double stranded RNA (dsRNA) molecule", "dsiRNA agent", "dsiRNA molecule", or "dsiRNA", which refers to a complex of ribonucleic acid molecules, having a duplex structure comprising two anti-parallel and substantially complementary nucleic acid strands, referred to as having "sense" and "antisense" orientations with respect to a target RNA.

The majority of nucleosides of each strand of the nucleic acid, e.g. a dsiRNA molecule, are preferably ribonucleosides, but in that case each or both strands can also include one or more non-ribonucleosides, e.g., a deoxyribonucleoside or a modified nucleoside. In addition, as used in this specification, an "siRNA" may include ribonucleosides with chemical modifications.

The term "modified nucleoside" refers to a nucleoside having, independently, a modified sugar moiety, a modified internucleoside linkage, or modified nucleobase, or any combination thereof. Thus, the term modified nucleoside encompasses substitutions, additions or removal of, e.g., a functional group or atom, to internucleoside linkages, sugar moieties, or nucleobases. Any such modifications, as used in an siRNA type molecule, are encompassed by "iRNA" or "RNAi agent" or "siRNA" or "siRNA agent" for the purposes of this specification and claims.

The two strands forming the duplex structure may be different portions of one larger molecule, or they may be separate molecules e.g. RNA molecules.

The term "nucleoside overhang" refers to at least one unpaired nucleoside that extends from the duplex structure of a nucleic acid according to the present invention. A nucleic acid according to the present invention can comprise an overhang of at least one nucleoside; alternatively the overhang can comprise at least two nucleosides, at least three nucleosides, at least four nucleosides, at least five nucleosides or more. A nucleoside overhang can comprise or consist of a nucleoside/nucleoside analog, including a deoxynucleoside. The overhang(s) can be on the sense strand, the antisense strand, or any combination thereof. Furthermore, the nucleoside(s) of an overhang can be present on the 5'-end, 3'-end, or both ends of either an antisense or sense strand.

In certain embodiments, the antisense strand has a 1-10 nucleoside, e.g., 0-3, 1-3, 2-4, 2-5, 4-10, 5-10, 1, 2, 3, 4, 5, 6, 7, 8, 9, or 10 nucleoside, overhang at the 3'-end or the 5'-end.

"Blunt" or "blunt end" means that there are no unpaired nucleosides at that end of the double stranded nucleic acid, i.e., no nucleoside overhang. The nucleic acids of the invention include those with no nucleoside overhang at one end or with no nucleoside overhangs at either end.

Unless otherwise indicated, the term "complementary," when used to describe a first nucleoside sequence in relation to a second nucleoside sequence, refers to the ability of an oligonucleoside comprising the first nucleoside sequence to hybridize and form a duplex structure under certain conditions with an oligonucleoside comprising the second nucleoside sequence, as will be understood by the skilled person. Such conditions can, for example, be stringent conditions, where stringent conditions can include: 400 mM NaCl, 40 mM PIPES pH 6.4, 1 mM EDTA, 50°C or 70°C for 12-16 hours followed by washing (see, e.g., "Molecular Cloning: A Laboratory Manual, Sambrook, et al. (1989) Cold Spring Harbor Laboratory Press).

Complementary sequences within nucleic acid e.g. a dsiRNA, as described herein, include base-pairing of the oligonucleoside comprising a first nucleoside sequence to an oligonucleoside comprising a second nucleoside sequence over the entire length of one or both nucleoside sequences. Such sequences can be referred to as "fully complementary" with respect to each other herein. However, where a first sequence is referred to as "substantially complementary" or "partially complementary" with respect to a second sequence herein, the two sequences can be fully complementary, or they can form one or more mismatched base pairs, such as 2, 4, or 5 mismatched base pairs, but preferably not more than 5 , while retaining the ability to hybridize under the conditions most relevant to their ultimate application, e.g. , inhibition of gene expression via a RISC pathway. Overhangs shall not be regarded as mismatches with regard to the determination of complementarity. For example, a nucleic acid e.g. dsiRNA comprising one oligonucleoside 17 nucleosides in length and another oligonucleoside 19 nucleosides in length, wherein the longer oligonucleoside comprises a sequence of 17 nucleosides that is fully complementary to the shorter oligonucleoside, can yet be referred to as "fully complementary" .

"Complementary" sequences, as used herein, can also include, or be formed entirely from, non- Watson-Crick base pairs or base pairs formed from non-natural and modified nucleosides, in so far as the above requirements with respect to their ability to hybridize are fulfilled. Such non- Watson- Crick base pairs include, but are not limited to, G:U Wobble or Hoogstein base pairing.

The terms "complementary," "fully complementary" and "substantially/partially complementary" herein can be used with respect to the base matching between the sense strand and the antisense strand of a nucleic acid eg dsiRNA, or between the antisense strand of a double stranded nucleic acid e.g. siRNA agent and a target sequence.

Within the present invention, the second strand of the nucleic acid according to the invention, in particular a dsiRNA for inhibiting ZPI, is at least partially complementary to the first strand of said nucleic acid. In certain embodiments, a first and second strand of a nucleic acid according to the invention are partially complementary if they form a duplex region having a length of at least 17 base pairs and comprising not more than 1, 2, 3, 4, or 5 mismatched base pairs.

In certain embodiments, a first and second strand of the nucleic acid according to the invention are partially complementary if they form a duplex region having a length of 19 base pairs and comprising not more than 1, 2, 3, 4, or 5 mismatched base pairs. In certain embodiments, a first and second strand of the nucleic acid according to the invention are partially complementary if they form a duplex region having a length of 21 base pairs comprising not more than 1, 2, 3, 4, or 5 mismatched base pairs.

Alternatively, a first and second strand of the nucleic acid according to the invention are partially complementary if they form a duplex region having a length of at least 17 base pairs, wherein at least 14, 15, 16 or 17 of said base pairs are complementary base pairs, in particular Watson-Crick base pairs.

In certain embodiments, a first and second strand of the nucleic acid according to the invention are partially complementary if they form a duplex region having a length of 19 base pairs, wherein at least 14, 15, 16, 17, 18 or all 19 base pairs are complementary base pairs, in particular Watson-Crick base pairs. In certain embodiments, a first and second strand of the nucleic acid according to the invention are partially complementary if they form a duplex region having a length of 21 base pairs, wherein at least 16, 17, 18, 19, 20 or all 21 base pairs are complementary base pairs, in particular Watson-Crick base pairs.

As used herein, a nucleic acid that is "substantially complementary" or "partially complementary" to at least part of a messenger RNA (mRNA) refers to a nucleic acid that is substantially or partially complementary to a contiguous portion of the mRNA of interest (e.g., an mRNA encoding a gene). In certain embodiments, the contiguous portion of the mRNA is a sequence as listed in Table 1, i.e., any one of SEQ ID NOs:2-121. For example, a nucleic acid is complementary to at least a part of an mRNA of a gene of interest if the sequence is substantially or partially complementary to a non-interrupted portion of an mRNA encoding that gene.

Accordingly, in some preferred embodiments, the antisense oligonucleosides as disclosed herein are fully complementary to the target gene sequence.

In other embodiments, the antisense oligonucleosides disclosed herein are substantially or partially complementary to a target RNA sequence and comprise a contiguous nucleoside sequence which is at least about 80% complementary over its entire length to the equivalent region of the target RNA sequence, such as at least about 85%, 86%, 87%, 88%, 89%, about 90%, 91 %, 92%, 93%, 94%, 95%, 96%, 97%, 98%, or 99% complementary or 100% complementary.

In certain embodiments, the first (antisense) strand of a nucleic acid according to the invention is partially or fully complementary to a contiguous portion of RNA transcribed from the ZPI gene. In certain embodiments, the first strand of the nucleic acid according to the invention is partially or fully complementary to a contiguous portion of at least 17 nucleosides of the ZPI mRNA. In certain embodiments, the first strand of the nucleic acid according to the invention is partially or fully complementary to a contiguous portion of 17, 18, 19, 20, 21, 22 or 23 nucleosides of the ZPI mRNA. In certain embodiments, the first strand of the nucleic acid according to the invention is partially or fully complementary to a contiguous portion of 17, 18, 19, 20, 21, 22 or 23 nucleosides of any one of the sequences as listed in Table 1, i.e., any one of SEQ ID NOs: 2-121.

In certain embodiments, the first (antisense) strand of the nucleic acid according to the invention is partially complementary to a contiguous portion of the ZPI mRNA if it comprises a contiguous nucleoside sequence of at least 17 nucleosides, wherein at least 14, 15, 16 or 17 nucleosides of said contiguous nucleoside sequence are complementary to a contiguous portion of the ZPI mRNA. In certain embodiments, the first strand of the nucleic acid according to the invention comprises a contiguous nucleoside sequence of at least 17 nucleosides, wherein at least 14, 15, 16 or 17 nucleosides of said contiguous nucleoside sequence are complementary to a contiguous portion of any one of the sequences listed in Table 1, i.e., any one of SEQ ID NOs: 2-121. In certain embodiments, the first strand of the nucleic acid according to the invention comprises a contiguous nucleoside sequence of 19 nucleosides, wherein at least 14, 15, 16, 17, 18 or all 19 nucleosides of said contiguous nucleoside sequence are complementary to a contiguous portion of any one of the sequences listed in Table 1, i.e., any one of SEQ ID NOs: 2-121. In certain embodiments, the first strand of the nucleic acid according to the invention comprises a contiguous nucleoside sequence of 23 nucleosides, wherein at least 18, 19, 20, 21, 22 or all 23 nucleosides of said contiguous nucleoside sequence are complementary to a contiguous portion of any one of the sequences listed in Table 1, i.e., any one of SEQ ID NOs: 2-101.

In some embodiments, a nucleic acid e.g. an siRNA of the invention includes a sense strand that is substantially or partially complementary to an antisense oligonucleoside which, in turn, is complementary to a target gene sequence and comprises a contiguous nucleoside sequence. The nucleoside sequence of the sense strand is typically at least about 80% complementary over its entire length to the equivalent region of the nucleoside sequence of the antisense strand, such as about 85%, 86%, 87%, 88%, 89%, 90%, 91 %, 92%, 93%, 94%, 95%, 96%, 97%, 98%, or 99% complementary, or 100% complementary.

In some embodiments, a nucleic acid e.g. an siRNA of the invention includes an antisense strand that is substantially or partially complementary to the target sequence and comprises a contiguous nucleoside sequence which is at least 80% complementary over its entire length to the target sequence such as about 85%, 86%, 87%, 88%, 89%, 90%, 91 %, 92%, 93%, 94%, 95%, 96%, 97%, 98%, or 99% complementary, or 100% complementary.

As used herein, a "subject" is an animal, such as a mammal, including a primate (such as a human, a non-human primate, e.g., a monkey, and a chimpanzee), or a non-primate or a bird that expresses the target gene, either endogenously or heterologously, when the target gene sequence has sufficient complementarity to the nucleic acid e.g. siRNA agent to promote target knockdown. In certain preferred embodiments, the subject is a human.

The terms "treating" or "treatment" refer to a beneficial or desired result including, but not limited to, alleviation or amelioration of one or more symptoms associated with gene expression. "Treatment" can also mean prolonging survival as compared to expected survival in the absence of treatment. Treatment can include prevention of development of comorbidities, e.g. , reduced liver damage in a subject with a hepatic infection.

"Therapeutically effective amount," as used herein, is intended to include the amount of a nucleic acid e.g. an siRNA that, when administered to a patient for treating a subject having disease, is sufficient to effect treatment of the disease (e.g. , by diminishing, ameliorating or maintaining the existing disease or one or more symptoms of disease or its related comorbidities).

The phrase "pharmaceutically acceptable" is employed herein to refer to compounds, materials, compositions, or dosage forms which are suitable for use in contact with the tissues of human subjects and animal subjects without excessive toxicity, irritation, allergic response, or other problem or complication, commensurate with a reasonable benefit/risk ratio.

The phrase "pharmaceutically-acceptable carrier" as used herein means a pharmaceutically- acceptable material, composition, or vehicle, such as a liquid or solid filler, diluent, excipient, manufacturing aid or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject being treated.

Where a value or range of values of a parameter are recited, it is intended that values and ranges intermediate to the recited values are also intended to be part of this invention.

The articles "a" and "an" are used herein to refer to one or to more than one (i.e. to at least one) of the grammatical object of the article.

The term "including" is used herein to mean, and is used interchangeably with, the phrase "including but not limited to".

The term "or" is used herein to mean, and is used interchangeably with, the term "and/or," unless context clearly indicates otherwise. For example, "sense strand or antisense strand" is understood as "sense strand or antisense strand or sense strand and antisense strand. "

The term "about" is used herein to mean within the typical ranges of tolerances in the art. For example, "about" can be understood as about 2 standard deviations from the mean. In certain embodiments, about means +10%. In certain embodiments, about means +5%. When about is present before a series of numbers or a range, it is understood that "about" can modify each of the numbers in the series or range.

The term "at least" prior to a number or series of numbers is understood to include the number adjacent to the term "at least", and all subsequent numbers or integers that could logically be included, as clear from context. For example, the number of nucleosides in a nucleic acid molecule must be an integer. For example, "at least 18 nucleosides of a 21 nucleoside nucleic acid molecule" means that 18, 19, 20, or 21 nucleosides have the indicated property. When at least is present before a series of numbers or a range, it is understood that "at least" can modify each of the numbers in the series or range.

As used herein, "no more than" or "less than" is understood as the value adjacent to the phrase and logical lower values or integers, as logical from context, to zero. For example, a duplex with an overhang of "no more than 2 nucleosides" has a 2, 1, or 0 nucleoside overhang. When "no more than" is present before a series of numbers or a range, it is understood that "no more than" can modify each of the numbers in the series or range.

The terminal region of a strand is the last 5 nucleosides from the 5' or the 3' end.

Various embodiments of the invention can be combined as determined appropriate by one of skill in the art.

### Abasic Nucleosides

In certain embodiments, there are 1, e.g. 2, e.g. 3, e.g. 4 or more abasic nucleosides present in nucleic acids according to the present invention. Abasic nucleosides are modified nucleosides because they lack the base normally seen at position 1 of the sugar moiety. Typically, there will be a hydrogen at position 1 of the sugar moiety of the abasic nucleosides present in a nucleic acid according to the present invention.

The abasic nucleosides are in the terminal region of the second strand, preferably located within the terminal 5 nucleosides of the end of the strand. The terminal region may be the terminal 5 nucleosides, which includes abasic nucleosides.

The second strand may comprise, as preferred features (which are all specifically contemplated in combination unless mutually exclusive):
2, or more than 2, abasic nucleosides in a terminal region of the second strand; and / or
2, or more than 2, abasic nucleosides in either the 5' or 3' terminal region of the second strand; and / or
2, or more than 2, abasic nucleosides in either the 5' or 3' terminal region of the second strand, wherein the abasic nucleosides are present in an overhang as herein described; and/or
2, or more than 2, consecutive abasic nucleosides in a terminal region of the second strand, wherein preferably one such abasic nucleoside is a terminal nucleoside; and / or
2, or more than 2, consecutive abasic nucleosides in either the 5' or 3' terminal region of the second strand, wherein preferably one such abasic nucleoside is a terminal nucleoside in either the 5' or 3' terminal region of the second strand; and / or
a reversed internucleoside linkage connects at least one abasic nucleoside to an adjacent basic nucleoside in a terminal region of the second strand; and / or
a reversed internucleoside linkage connects at least one abasic nucleoside to an adjacent basic nucleoside in either the 5' or 3' terminal region of the second strand; and /or
an abasic nucleoside as the penultimate nucleoside which is connected via the reversed linkage to the nucleoside which is not the terminal nucleoside (called the antepenultimate nucleoside herein); and/or
abasic nucleosides as the 2 terminal nucleosides connected via a 5'-3' linkage when reading the strand in the direction towards the terminus comprising the terminal nucleosides;
abasic nucleosides as the 2 terminal nucleosides connected via a 3'-5' linkage when reading the strand in the direction towards the terminus comprising the terminal nucleosides;
abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
abasic nucleosides as the terminal 2 positions, wherein the penultimate nucleoside is connected via the reversed linkage to the antepenultimate nucleoside, and wherein either
   (1) the reversed linkage is a 5-5' reversed linkage and the linkage between the terminal and penultimate abasic nucleosides is 3'5' when reading towards the terminus comprising the terminal and penultimate abasic nucleosides; or
   (2) the reversed linkage is a 3-3' reversed linkage and the linkage between the terminal and penultimate abasic nucleosides is 5'3' when reading towards the terminus comprising the terminal and penultimate abasic nucleosides.

Preferably there is an abasic nucleoside at the terminus of the second strand.

Preferably there are 2 or at least 2 abasic nucleosides in the terminal region of the second strand, preferably at the terminal and penultimate positions.

Preferably 2 or more abasic nucleosides are consecutive, for example all abasic nucleosides may be consecutive. For example, the terminal 1 or terminal 2 or terminal 3 or terminal 4 nucleosides may be abasic nucleosides.

An abasic nucleoside may also be linked to an adjacent nucleoside through a 5'-3' phosphodiester linkage or reversed linkage unless there is only 1 abasic nucleoside at the terminus, in which case it will have a reversed linkage to the adjacent nucleoside.

A reversed linkage (which may also be referred to as an inverted linkage, which is also seen in the art), comprises either a 5'-5', a 3'3', a 3'-2' or a 2'-3' phosphodiester linkage between the adjacent sugar moieties of the nucleosides.

Abasic nucleosides which are not terminal will have 2 phosphodiester bonds, one with each adjacent nucleoside, and these may be a reversed linkage or may be a 5'-3 phosphodiester bond or may be one of each.

A preferred embodiment comprises 2 abasic nucleosides at the terminal and penultimate positions of the second strand, and wherein the reversed internucleoside linkage is located between the penultimate (abasic) nucleoside and the antepenultimate nucleoside.

Preferably there are 2 abasic nucleosides at the terminal and penultimate positions of the second strand and the penultimate nucleoside is linked to the antepenultimate nucleoside through a reversed internucleoside linkage and is linked to the terminal nucleoside through a 5'-3' or 3'-5' phosphodiester linkage (reading in the direction of the terminus of the molecule).

Preferably a nucleic acid according to the present invention comprises one or more abasic nucleosides, optionally wherein the one or more abasic nucleosides are in a terminal region of the second strand, and/or wherein at least one abasic nucleoside is linked to an adjacent basic nucleoside through a reversed internucleoside linkage.

Typically the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein: (a) said penultimate abasic nucleoside is connected to an adjacent first basic nucleoside in an adjacent 5' near terminal region through a reversed internucleoside linkage; and (b) the reversed linkage is a 5-5' reversed linkage; and (c) the linkage between the terminal and penultimate abasic nucleosides is 3'5' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 3' terminal region of the second strand.

Alternatively the second strand comprises 2 consecutive abasic nucleosides preferably in an overhang in the 3' terminal region of the second strand, wherein one such abasic nucleoside is a terminal nucleoside at the 3' terminal region of the second strand and the other abasic nucleoside is a penultimate nucleoside at the 3' terminal region of the second strand, wherein: (a) said penultimate abasic nucleoside is connected to an adjacent first basic nucleoside in an adjacent 3' near terminal region through a reversed internucleoside linkage; and (b) the reversed linkage is a 3-3' reversed linkage; and (c) the linkage between the terminal and penultimate abasic nucleosides is 5'-3' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 3' near terminal region of the second strand, wherein a first phosphorothioate internucleoside linkage is present between said adjacent first basic nucleoside of (a) and an adjacent second basic nucleoside in said 3' near terminal region of the second strand, and a second phosphorothioate internucleoside linkage is present between said adjacent second basic nucleoside and an adjacent third basic nucleoside in said 3' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 5' terminal region of the second strand.

Examples of the structures are as follows (where the specific RNA nucleosides shown are not limiting and could be any RNA nucleoside):
A A 3'-3' reversed bond (and also showing the 5'-3 direction of the last phosphodiester bond between the two abasic molecules reading towards the terminus of the molecule) 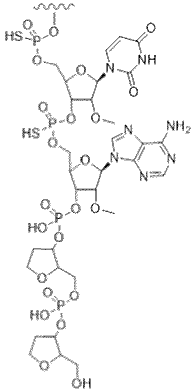
B Illustrating a 5'-5' reversed bond (and also showing the 3'-5' direction of the last phosphodiester bond between the two abasic molecules reading towards the terminus of the molecule)

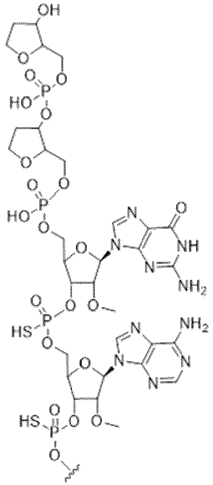

The abasic nucleoside or abasic nucleosides present in the nucleic acid are provided in the presence of a reversed internucleoside linkage or linkages, namely a 5'-5' or a 3'-3' reversed internucleoside linkage. A reversed linkage occurs as a result of a change of orientation of an adjacent nucleoside sugar, such that the sugar will have a 3' - 5' orientation as opposed to the conventional 5' - 3' orientation (with reference to the numbering of ring atoms on the nucleoside sugars). The abasic nucleoside or nucleosides as present in the nucleic acids of the invention preferably include such inverted nucleoside sugars.

In the case of a terminal nucleoside having an inverted orientation, then this will result in an "inverted" end configuration for the overall nucleic acid. Whilst certain structures drawn and referenced herein are represented using conventional 5' - 3' direction (with reference to the numbering of ring atoms on the nucleoside sugars), it will be appreciated that the presence of a terminal nucleoside having a change of orientation and a proximal 3'-3' reversed linkage, will result in a nucleic acid having an overall 5'- 5' end structure (i.e. the conventional 3' end nucleoside becomes a 5' end nucleoside). Alternatively, it will be appreciated that the presence of a terminal nucleoside having a change of orientation and a proximal 5'-5' reversed linkage will result in a nucleic acid with an overall 3'- 3' end structure.

The proximal 3'-3' or 5'-5' reversed linkage as herein described, may comprise the reversed linkage being directly adjacent / attached to a terminal nucleoside having an inverted orientation, such as a single terminal nucleoside having an inverted orientation. Alternatively, the proximal 3'-3' or 5'-5' reversed linkage as herein described, may comprise the reversed linkage being adjacent 2, or more than 2, nucleosides having an inverted orientation, such as 2, or more than 2, terminal region nucleosides having an inverted orientation, such as the terminal and penultimate nucleosides. In this way, the reversed linkage may be attached to a penultimate nucleoside having an inverted orientation. While a skilled addressee will appreciate that inverted orientations as described above can result in nucleic acid molecules having overall 3' - 3' or 5'- 5' end structures as described herein, it will also be appreciated that with the presence of one or more additional reversed linkages and / or nucleosides having an inverted orientation, then the overall nucleic acid may have 3' - 5' end structures corresponding to the conventionally positioned 5' / 3' ends.

In one aspect the nucleic acid may have a 3'-3' reversed linkage, and the terminal sugar moiety may comprise a 5' OH rather than a 5' phosphate group at the 5' position of that terminal sugar.

A skilled person would therefore clearly understand that 5'- 5', 3'-3' and 3' - 5' (reading in the direction of that terminus) end variants of the more conventional 5'- 3' structures (with reference to the numbering of ring atoms on the end nucleoside sugars) drawn herein are included in the scope of the disclosure, where a reversed linkage or linkages is / are present.

In the situation of eg a reversed internucleoside linkage and / or one or more nucleosides having an inverted orientation creating an inverted end, and where the relative position of a linkage (eg to a linker) or the location of an internal feature (such as a modified nucleoside) is defined relative to the 5' or 3' end of the nucleic acid, then the 5' or 3' end is the conventional 5' or 3' end which would have existed had a reversed linkage not been in place, and wherein the conventional 5' or 3' end is determined by consideration of the directionality of the majority of the internal nucleoside linkages and / or nucleoside orientation within the nucleic acid. It is possible to tell from these internal bonds and / or nucleoside orientation which ends of the nucleic acid would constitute the conventional 5' and 3' ends (with reference to the numbering of ring atoms on the end nucleoside sugars) of the molecule absent the reversed linkage.

For example, in the structure shown below there are abasic residues in the first 2 positions located at the 5' end. Where the terminal nucleoside has an inverted orientation then the 5' end indicated in the diagram below, which is the conventional 5' end, can in fact comprise a 3' OH in view of the inverted nucleoside at the terminal position. Nevertheless the majority of the molecule will comprise conventional internucleoside linkages that run from the 3' OH of the sugar to the 5' phosphate of the next sugar, when reading in the standard 5' [PO4] to 3' [OH] direction of a nucleic acid molecule (with reference to the numbering of ring atoms on the nucleoside sugars), which can be used to determine the conventional 5' and 3' ends that would be found absent the inverted end configuration.
5' A-A-Me-Me-Me-Me-Me-Me-F-Me-F-F-F-Me-Me-Me-Me-Me-Me-Me-Me-Me-Me 3'

In some embodiments, the second (sense) strand of the nucleic acid according to the invention comprises 2 consecutive abasic nucleosides in the 5' terminal region as shown in the following 5' terminal motif 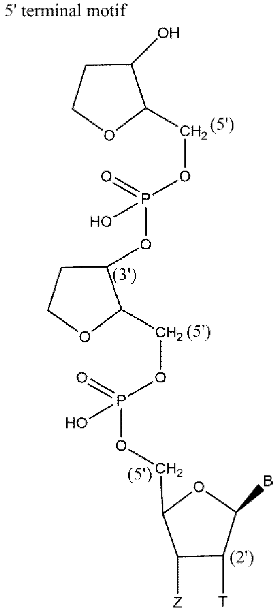 wherein:
B represents a nucleoside base,
T represent H, OH or a 2' ribose modification,
Z represents the remaining nucleosides of said second strand.

In some embodiments, the second (sense) strand of the nucleic acid according to the invention comprises 2 consecutive abasic nucleosides in the 5' terminal region as shown in the following 5' terminal motif 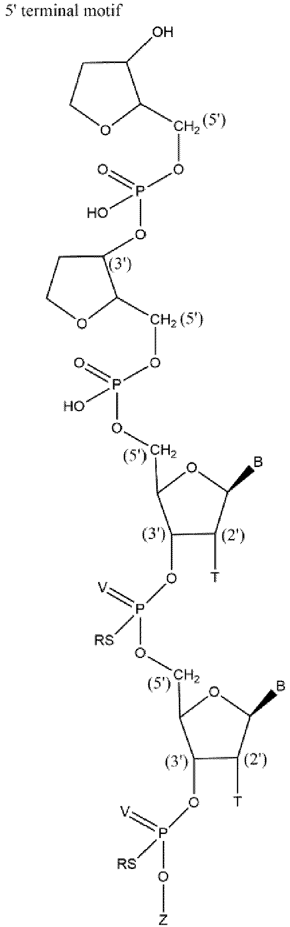 wherein:
B represents a nucleoside base,
T represents H, OH or a 2' ribose modification (preferably a 2' ribose modification, more preferably a 2'Me or 2'F ribose modification),
V represents O or S (preferably O),
R represents H or C₁₋₄ alkyl (preferably H),
Z represents the remaining nucleosides of said second strand,
more preferably the following 5' terminal motif 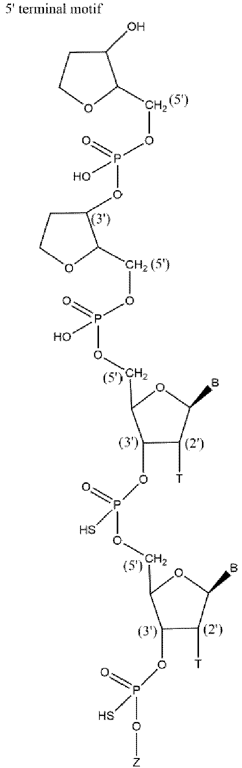 wherein:
   B represents a nucleoside base,
   T represents a 2' ribose modification (preferably a 2'Me or 2'F ribose modification),
   Z represents the remaining nucleosides of said second strand.

The reversed bond is preferably located at the end of the nucleic acid eg RNA which is distal to a ligand moiety, such as a GalNAc containing portion, of the molecule.

GalNAc-siRNA constructs with a 5'-GalNAc on the sense strand can have a reversed linkage on the opposite end of the sense strand.

GalNAc-siRNA constructs with a 3'-GalNAc on the sense strand can have a reversed linkage on the opposite end of the sense strand.

In a preferred embodiment, the second (sense) strand of the nucleic acid according to the invention comprises 2 consecutive abasic nucleosides in the 5' terminal region as shown in the following 5' terminal motif 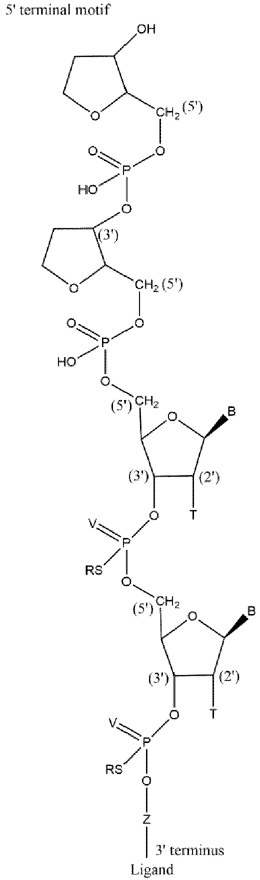 wherein:
B represents a nucleoside base,
T represent H, OH or a 2' ribose modification (preferably a 2' ribose modification, more preferably a 2'Me or 2'F ribose modification),
V represent O or S (preferably O),
R represent H or C₁₋₄ alkyl (preferably H),
Z comprises 11 to 26 contiguous nucleosides, preferably 15 to 21 contiguous nucleosides, and more preferably 19 contiguous nucleosides,
more preferably the following 5' terminal motif 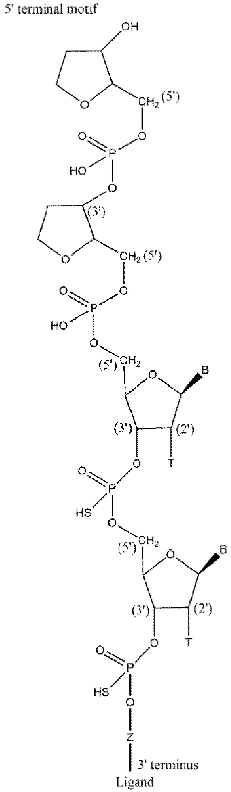 wherein:
   B represents a nucleoside base,
   T represents a 2' ribose modification (preferably a 2'Me or 2'F ribose modification),
   Z comprises 19 contiguous nucleosides.

### Nucleic Acid Lengths

In one aspect the i) the first strand of the nucleic acid has a length in the range of 17 to 30 nucleosides, preferably 19 to 25 nucleosides, more preferably 19 or 23 nucleosides; and / or ii) the second strand of the nucleic acid has a length in the range of 17 to 30 nucleosides, preferably 19 to 25 nucleosides, more preferably 19 or 21 nucleosides.

Typically the duplex region of the nucleic acid is between 17 and 30 nucleosides in length, more preferably is 19 or 21 nucleosides in length. Similarly, the region of complementarity between the first strand and the portion of RNA transcribed from the ZPI gene is between 17 and 30 nucleosides in length.

### Nucleic Acid Modifications

In certain embodiments, the nucleic acid e.g. an RNA of the invention e.g., a dsiRNA, does not comprise further modifications, e.g., chemical modifications or conjugations known in the art and described herein.

In other preferred embodiments, the nucleic acid e.g. RNA of the invention, e.g., a dsiRNA, is further chemically modified to enhance stability or other beneficial characteristics.

In certain embodiments of the invention, substantially all of the nucleosides are modified.

The nucleic acids featured in the invention can be synthesized or modified by methods well established in the art, such as those described in "Current protocols in nucleic acid chemistry," Beaucage, S.L. et al. (Edrs.), John Wiley & Sons, Inc., New York, NY, USA.

Modifications include, for example, end modifications, e.g., 5'-end modifications (phosphorylation, conjugation, inverted linkages) or 3 '-end modifications (conjugation, DNA nucleosides within an RNA, or RNA nucleosides within a DNA, inverted linkages, etc.); base modifications, e.g., replacement with stabilizing bases, destabilizing bases, or bases that base pair with an expanded repertoire of partners, conjugated bases; sugar modifications (e.g. , at the 2'-position or 4'- position) or replacement of the sugar; or backbone modifications, including modification or replacement of the phosphodiester linkages.

Specific examples of nucleic acids such as siRNA compounds useful in the embodiments described herein include, but are not limited to RNAs containing modified backbones or no natural internucleoside linkages. Nucleic acids such as RNAs having modified backbones include, among others, those that do not have a phosphorus atom in the backbone. For the purposes of this specification, and as sometimes referenced in the art, modified nucleic acids e.g. RNAs that do not have a phosphorus atom in their internucleoside backbone can also be considered to be oligonucleosides. In some embodiments, a modified nucleic acid e.g. an siRNA will have a phosphorus atom in its internucleoside backbone.

Modified nucleic acid e.g. RNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5'-linked analogs of these, and those having inverted polarity wherein the adjacent pairs of nucleoside units are linked 5'-3' or 5'-2'. Various salts, mixed salts and free acid forms are also included.

Modified nucleic acids e.g. RNAs can also contain one or more substituted sugar moieties. The nucleic acids e.g. siRNAs, e.g., dsiRNAs, featured herein can include one of the following at the 2'-position: OH; F; 0-, S-, or N-alkyl; 0-, S-, or N-alkenyl; 0-, S- or N-alkynyl; or O-alkyl-O-alkyl, wherein the alkyl, alkenyl and alkynyl can be substituted or unsubstituted. 2' O- methyl and 2' -F are preferred modifications.

In certain preferred embodiments, the nucleic acid comprises at least one modified nucleoside.

The nucleic acid of the disclosure may comprise one or more modified nucleosides on the first strand and/or the second strand.

In some embodiments, substantially all of the nucleosides of the sense strand and all of the nucleosides of the antisense strand comprise a modification.

In some embodiments, all of the nucleosides of the sense strand and substantially all of the nucleosides of the antisense strand comprise a modification.

In some embodiments, all of the nucleosides of the sense strand and all of the nucleosides of the antisense strand comprise a modification.

In one embodiment, at least one of the modified nucleosides is selected from the group consisting of a deoxy- nucleoside, a 3 '-terminal deoxy-thymine (dT) nucleoside, a 2'-O-methyl modified nucleoside (also called herein 2'-Me, where Me is a methoxy) , a 2'-fluoro modified nucleoside, a 2'-deoxy- modified nucleoside, a locked nucleoside, an unlocked nucleoside, a conformationally restricted nucleoside, a constrained ethyl nucleoside, an abasic nucleoside, a 2' -amino- modified nucleoside, a 2'-O-allyl- modified nucleoside, 2' -O-alkyl- modified nucleoside, 2'-hydroxly-modified nucleoside, a 2'- methoxyethyl modified nucleoside, a 2'-O-alkyl-modified nucleoside, a morpholino nucleoside, a phosphoramidate, a non-natural base comprising nucleoside, a tetrahydropyran modified nucleoside, a 1 ,5-anhydrohexitol modified nucleoside, a cyclohexenyl modified nucleoside, a nucleoside comprising a phosphorothioate group, a nucleoside comprising a methylphosphonate group, a nucleoside comprising a 5 '-phosphate, and a nucleoside comprising a 5 '-phosphate mimic. In another embodiment, the modified nucleosides comprise a short sequence of 3 '-terminal deoxy-thymine nucleosides (dT).

Modifications on the nucleosides may preferably be selected from the group including, but not limited to, LNA, HNA, CeNA, 2'-methoxyethyl, 2'-O-alkyl, 2'-O-allyl, 2'-C-allyl, 2'-fluoro, 2'-deoxy, 2'- hydroxyl, and combinations thereof. In another embodiment, the modifications on the nucleosides are 2-O-methyl ("2'-Me") or 2'-fluoro modifications.

One preferred modification is a modification at the 2'-OH group of the ribose sugar, optionally selected from 2'-Me or 2'-F modifications.

Preferred nucleic acid comprise one or more nucleosides on the first strand and / or the second strand which are modified, to form modified nucleosides, as follows:

A nucleic acid wherein the modification is a modification at the 2'-OH group of the ribose sugar, optionally selected from 2'-Me or 2'-F modifications.

A nucleic acid wherein the first strand comprises a 2'-F modification at any of position 2, position 6, position 14, or any combination thereof, counting from position 1 of said first strand.

A nucleic acid wherein the second strand comprises a 2'-F modification at any of position 7, position 9, position 11, or any combination thereof, counting from position 1 of said second strand.

A nucleic acid wherein the first and second strand each comprise 2'-Me and 2'-F modifications.

A nucleic which comprises at least one thermally destabilizing modification, suitably at one or more of positions 1 to 9 of the first strand counting from position 1 of the first strand, and / or at one or more of positions on the second strand aligned with positions 1 to 9 of the first strand, wherein the destabilizing modification is selected from a modified unlocked nucleic acid (UNA) and a glycol nucleic acid (GNA), preferably a glycol nucleic acid, more preferably an (S)-glycol nucleic acid.

A nucleic acid which comprises at least one thermally destabilizing modification at position 7 of the first strand, counting from position 1 of the first strand.

A nucleic acid which is an siRNA oligonucleoside, wherein the siRNA oligonucleoside comprises 3 or more 2'-F modifications at positions 6 to 12 of the second strand, such as 4, 5, 6 or 7 2'-F modifications at positions 6 to 12 of the second strand, counting from position 1 of said second strand.

A nucleic acid which is an siRNA oligonucleoside, wherein said second strand comprises at least 3, such as 4, 5 or 6, 2'-Me modifications at positions 1 to 6 of the second strand, counting from position 1 of said second strand.

A nucleic acid which is an siRNA oligonucleoside, wherein said first strand comprises at least 5 2'-Me consecutive modifications at the 3' terminal region, preferably including the terminal nucleoside at the 3' terminal region, or at least within 1 or 2 nucleosides from the terminal nucleoside at the 3' terminal region.

A nucleic acid which is an siRNA oligonucleoside, wherein said first strand comprises 7 2'-Me consecutive modifications at the 3' terminal region, preferably including the terminal nucleoside at the 3' terminal region.

A nucleic acid which is an siRNA oligonucleoside, wherein each of the first and second strands comprises an alternating modification pattern, preferably a fully alternating modification pattern along the entire length of each of the first and second strands, wherein the nucleosides of the first strand are modified by (i) 2'Me modifications on the odd numbered nucleosides counting from position 1 of the first strand, and (ii) 2'F modifications on the even numbered nucleosides counting from position 1 of the first strand, and nucleosides of the second strand are modified by (i) 2'F modifications on the odd numbered nucleosides counting from position 1 of the second strand, and (ii) 2'Me modifications on the even numbered nucleosides counting from position 1 of the second strand. Typically such fully alternating modification patterns are present in a blunt ended oligonucleoside, wherein each of the first and second strands are 19 nucleosides in length.

Position 1 of the first or the second strand is the nucleoside which is the closest to the end of the nucleic acid (ignoring any abasic nucleosides) and that is joined to an adjacent nucleoside (at Position 2) via a 3' to 5' internal bond, with reference to the bonds between the sugar moieties of the backbone, and reading in a direction away from that end of the molecule.

It can therefore be seen that "position 1 of the sense strand" is the 5' most nucleoside (not including abasic nucleosides) at the conventional 5' end of the sense strand. Typically, the nucleoside at this position 1 of the sense strand will be equivalent to the 5' nucleoside of the selected target nucleic acid sequence, and more generally the sense strand will have equivalent nucleosides to those of the target nucleic acid sequence starting from this position 1 of the sense strand, whilst also allowing for acceptable mismatches between the sequences.

As used herein, "position 1 of the antisense strand" is the 5' most nucleoside (not including abasic nucleosides) at the conventional 5' end of the antisense strand. As hereinbefore described, there will be a region of complementarity between the sense and antisense strands, and in this way the antisense strand will also have a region of complementarity to the target nucleic acid sequence as referred to above.

In certain embodiments, the nucleic acid e.g. siRNA agent further comprises at least one phosphorothioate or methylphosphonate internucleoside linkage. For example the phosphorothioate or methylphosphonate internucleoside linkage can be at the 3 'terminus or in the terminal region of one strand, i.e. , the sense strand or the antisense strand; or at the ends of both strands, the sense strand and the antisense strand.

In certain embodiments, the phosphorothioate or methylphosphonate internucleoside linkage is at the 5 'terminus or in the terminal region of one strand, i.e. , the sense strand or the antisense strand; or at the ends of both strands, the sense strand and the antisense strand.

In certain embodiments, a phosphorothioate or a methylphosphonate internucleoside linkage is at both the 5'- and 3 '-terminus or in the terminal region of one strand, i.e. , the sense strand or the antisense strand; or at the ends of both strands, the sense strand and the antisense strand.

Any nucleic acid may comprise one or more phosphorothioate (PS) modifications within the nucleic acid, such as at least two PS internucleoside bonds at the ends of a strand.

At least one of the oligoribonucleoside strands preferably comprises at least two consecutive phosphorothioate modifications in the last 3 nucleosides of the oligonucleoside.

The invention therefore also relates to: A nucleic acid disclosed herein which comprises phosphorothioate internucleoside linkages respectively between at least two or three consecutive positions, such as in a 5' and/or 3' terminal region and/or near terminal region of the second strand, whereby said near terminal region is preferably adjacent said terminal region wherein said one or more abasic nucleosides of said second strand is / are located.

A nucleic acid disclosed herein which comprises phosphorothioate internucleoside linkages respectively between at least two or three consecutive positions in a 5' and / or 3' terminal region of the first strand, whereby preferably the terminal position at the 5' and / or 3' terminal region of said first strand is attached to its adjacent position by a phosphorothioate internucleoside linkage.

The nucleic acid strand may be an RNA comprising a phosphorothioate internucleoside linkage between the three nucleosides contiguous with 2 terminally located abasic nucleosides.

A preferred nucleic acid is a double stranded RNA comprising 2 adjacent abasic nucleosides at the 5' terminus of the second strand and a ligand moiety comprising one or more GalNAc ligand moieties at the opposite 3' end of the second strand. Further preferred, the same nucleic acid may also comprise a phosphorothioate bond between nucelotides at positions 3-4 and 4-5 of the second strand, reading from the position 1 of the second strand. Further preferred, the same nucleic acid may also comprise a 2' F modification at positions 7, 9 and 11 of the second strand.

Preferred modifications are as follows.

A nucleic acid wherein modified nucleosides of said second strand comprise a modification pattern according to any one of the following (5'-3'):
Me - Me - Me - Me - Me - Me - F - F - F - F - F - Me - Me - Me - Me - Me - Me - Me - F - Me - Me, or
Me - Me - Me - Me - Me - F - F - Me - F - F - F - F - Me - Me - Me - Me - Me - Me - Me - Me - Me, or
Me - Me - Me - Me - Me - Me - F - Me - F - F - F - F - Me - Me - Me - Me - Me - Me - Me - Me - Me, or
Me - Me - Me - Me - Me - Me - Me - Me - F - F - F - Me - Me - Me - Me - Me - Me - Me - Me - Me - Me, or
Me - Me - Me - Me - Me - Me - F - Me - F - F - F - Me - Me - Me - Me - Me - Me - Me - Me - Me - Me.

A nucleic acid wherein modified nucleosides of said second strand comprise a modification pattern according to any one of the following (5'-3'):
Me(s)Me(s)Me - Me - Me - Me - F - F - F - F - F - Me - Me - Me - Me - Me - Me - Me - F - Me - Me, or
Me(s)Me(s)Me - Me - Me - F - F - Me - F - F - F - F - Me - Me - Me - Me - Me - Me - Me - Me - Me, or
Me(s)Me(s)Me - Me - Me - Me - F - Me - F - F - F - F - Me - Me - Me - Me - Me - Me - Me - Me - Me, or
Me(s)Me(s)Me - Me - Me - Me - Me - Me - F - F - F - Me - Me - Me - Me - Me - Me - Me - Me - Me - Me, or
Me(s)Me(s)Me - Me - Me - Me - F - Me - F - F - F - Me - Me - Me - Me - Me - Me - Me - Me - Me - Me, or
Me - Me - Me - Me - Me - Me - F - F - F - F - F - Me - Me - Me - Me - Me - Me - Me - F(s)Me(s)Me, or
Me - Me - Me - Me - Me - F - F - Me - F - F - F - F - Me - Me - Me - Me - Me - Me - Me(s)Me(s)Me, or
Me - Me - Me - Me - Me - Me - F - Me - F - F - F - F - Me - Me - Me - Me - Me - Me - Me(s)Me(s)Me, or
Me - Me - Me - Me - Me - Me - Me - Me - F - F - F - Me - Me - Me - Me - Me - Me - Me - Me(s)Me(s)Me, or
Me - Me - Me - Me - Me - Me - F - Me - F - F - F - Me - Me - Me - Me - Me - Me - Me - Me(s)Me(s)Me,
wherein (s) is a phosphorothioate internucleoside linkage.

A nucleic acid wherein modified nucleosides of said second strand comprise a modification pattern according to any one of the following (5'-3'):
ia - ia - Me - Me - Me - Me - Me - Me - F - F - F - F - F - Me - Me - Me - Me - Me - Me - Me - F - Me - Me, or
ia - ia - Me - Me - Me - Me - Me - F - F - Me - F - F - F - F - Me - Me - Me - Me - Me - Me - Me - Me - Me, or
ia - ia - Me - Me - Me - Me - Me - Me -F - Me - F - F - F - F - Me - Me - Me - Me - Me - Me - Me - Me - Me, or
ia - ia - Me - Me - Me - Me - Me - Me - Me - Me - F - F - F - Me - Me - Me - Me - Me - Me - Me - Me - Me - Me, or
ia - ia - Me - Me - Me - Me - Me - Me - F - Me - F - F - F - Me - Me - Me - Me - Me - Me - Me - Me - Me - Me, or
Me - Me - Me - Me - Me - Me - F - F - F - F - F - Me - Me - Me - Me - Me - Me - Me - F - Me - Me - ia - ia, or
Me - Me - Me - Me - Me - F - F - Me - F - F - F - F - Me - Me - Me - Me - Me - Me - Me - Me - Me - ia - ia, or
Me - Me - Me - Me - Me - Me - F - Me - F - F - F - F - Me - Me - Me - Me - Me - Me - Me - Me - Me - ia - ia, or
Me - Me - Me - Me - Me - Me - Me - Me - F - F - F - Me - Me - Me - Me - Me - Me - Me - Me - Me - Me - ia - ia, or
Me - Me - Me - Me - Me - Me - F - Me - F - F - F - Me - Me - Me - Me - Me - Me - Me - Me - Me - Me- ia - ia,
wherein ia represents an inverted abasic nucleoside, and when the inverted abasic nucleosides as represented by ia - ia are present at the 3' terminus of the second strand, said inverted abasic nucleosides are present in a 2 nucleoside overhang.

A nucleic acid wherein modified nucleosides of said second strand comprise a modification pattern according to any one of the following (5'-3'):
ia - ia - Me(s)Me(s)Me - Me - Me - Me - F - F - F - F - F - Me - Me - Me - Me - Me - Me - Me - F - Me - Me, or
ia - ia - Me(s)Me(s)Me - Me - Me - F - F - Me - F - F - F - F - Me - Me - Me - Me - Me - Me - Me - Me - Me, or
ia - ia - Me(s)Me(s)Me - Me - Me - Me -F- Me - F - F - F - F - Me - Me - Me - Me - Me - Me - Me - Me - Me, or
ia - ia - Me(s)Me(s)Me - Me - Me - Me - Me - Me - F - F - F - Me - Me - Me - Me - Me - Me - Me - Me - Me - Me, or
ia - ia - Me(s)Me(s)Me - Me - Me - Me - F - Me - F - F - F - Me - Me - Me - Me - Me - Me - Me - Me - Me - Me, or
Me - Me - Me - Me - Me - Me - F - F - F - F - F - Me - Me - Me - Me - Me - Me - Me - F(s)Me(s)Me - ia - ia, or
Me - Me - Me - Me - Me - F - F - Me - F - F - F - F - Me - Me - Me - Me - Me - Me - Me(s)Me(s)Me - ia - ia, or
Me - Me - Me - Me - Me - Me -F- Me - F - F - F - F - Me - Me - Me - Me - Me - Me - Me(s)Me(s)Me - ia - ia, or
Me - Me - Me - Me - Me - Me - Me - Me - F - F - F - Me - Me - Me - Me - Me - Me - Me - Me(s)Me(s)Me - ia - ia , or
Me - Me - Me - Me - Me - Me - F - Me - F - F - F - Me - Me - Me - Me - Me - Me - Me - Me(s)Me(s)Me - ia - ia,
wherein:
   (s) is a phosphorothioate internucleoside linkage, ia represents an inverted abasic nucleoside, and when the inverted abasic nucleosides as represented by ia - ia are present at the 3' terminus of the second strand, said inverted abasic nucleosides are present in a 2 nucleoside overhang.

A nucleic acid wherein modified nucleosides comprise any one of the following modification patterns:
Modification pattern 1: Second strand (5'-3'): Me - Me - Me - Me - Me - Me - F - F - F - F - F - Me - Me - Me - Me - Me - Me - Me - F - Me - Me, First strand (5'-3'): Me - F - Me - F - Me - F - Me - F - F - Me - Me - Me - Me - F - Me - F - Me - Me - Me - Me - Me - Me - Me
Or Modification pattern 2: Second strand (5'-3'): Me - Me - Me - Me - Me - F - F - Me - F - F - F - F - Me - Me - Me - Me - Me - Me - Me - Me - Me, First strand (5'-3'): Me - F - Me - F - Me - F - Me - F - F - Me - Me - Me - Me - F - Me - F - Me - Me - Me - Me - Me - Me - Me
Or Modification pattern 3: Second strand (5'-3'): Me - Me - Me - Me - Me - Me -F- Me - F - F - F - F - Me - Me - Me - Me - Me - Me - Me - Me - Me, First strand (5'-3'): Me - F - Me - F - Me - F - Me - F - F - Me - Me - Me - Me - F - Me - F - Me - Me - Me - Me - Me - Me - Me
Or Modification pattern 4: Second strand (5'-3'): Me - Me - Me - Me - Me - Me - F - Me - F - F - F - F - Me - Me - Me - Me - Me - Me - Me - Me - Me, First strand (5'-3'): Me - F - Me - F - Me - F - Me - Me - Me - Me - Me - Me - Me - F - Me - F - Me - Me - Me - Me - Me - Me - Me
Or Modification pattern 5: Second strand (5'-3'): Me - Me - Me - Me - Me - Me - Me - Me - F - F - F - Me - Me - Me - Me - Me - Me - Me - Me - Me - Me, First strand (5'-3'): Me - F - Me - Me - Me - F - Me - Me - F - Me - Me - Me - Me - F - Me - F - Me - Me - Me - Me - Me - Me - Me
Or Modification pattern 6: Second strand (5'-3'): Me - Me - Me - Me - Me - Me - F - Me - F - F - F - Me - Me - Me - Me - Me - Me - Me - Me - Me - Me, First strand (5'-3'): Me - F - Me - Me - Me - F - Me - Me - F - Me - Me - Me - Me - F - Me - F - Me - Me - Me - Me - Me - Me - Me.

A nucleic acid wherein modified nucleosides comprise any one of the following modification patterns:
Modification pattern 1: Second strand (5'-3'): Me(s)Me(s)Me - Me - Me - Me - F - F - F - F - F - Me - Me - Me - Me - Me - Me - Me - F - Me - Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 2: Second strand (5'-3'): Me(s)Me(s)Me - Me - Me - F - F - Me - F - F - F - F - Me - Me - Me - Me - Me - Me - Me - Me - Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 3: Second strand (5'-3'): Me(s)Me(s)Me - Me - Me - Me -F- Me - F - F - F - F - Me - Me - Me - Me - Me - Me - Me - Me - Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 4: Second strand (5'-3'): Me(s)Me(s)Me - Me - Me - Me - F - Me - F - F - F - F - Me - Me - Me - Me - Me - Me - Me - Me - Me, First strand (5'-3'): Me(s)F(s)Me - F - Me - F - Me - Me - Me - Me - Me - Me - Me - F - Me - F - Me - Me - Me - Me - Me(s)Me(s)Me
Or Modification pattern 5: Second strand (5'-3'): Me(s)Me(s)Me - Me - Me - Me - Me - Me - F - F - F - Me - Me - Me - Me - Me - Me - Me - Me - Me - Me, First strand (5'-3'): Me(s)F(s)Me - Me - Me - F - Me - Me - F - Me - Me - Me - Me - F - Me - F - Me - Me - Me - Me - Me(s)Me(s)Me
Or Modification pattern 6: Second strand (5'-3'): Me(s)Me(s)Me - Me - Me - Me - F - Me - F - F - F - Me - Me - Me - Me - Me - Me - Me - Me - Me - Me, First strand (5'-3'): Me(s)F(s)Me - Me - Me - F - Me - Me - F - Me - Me - Me - Me - F - Me - F - Me - Me - Me - Me - Me(s)Me(s)Me
wherein (s) is a phosphorothioate internucleoside linkage.

A nucleic acid wherein modified nucleosides comprise any one of the following modification patterns:
Modification pattern 1: Second strand (5'-3'): Me - Me - Me - Me - Me - Me - F - F - F - F - F - Me - Me - Me - Me - Me - Me - Me - F(s)Me(s)Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 2: Second strand (5'-3'): Me - Me - Me - Me - Me - F - F - Me - F - F - F - F - Me - Me - Me - Me - Me - Me - Me(s)Me(s)Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 3: Second strand (5'-3'): Me - Me - Me - Me - Me - Me -F- Me - F - F - F - F - Me - Me - Me - Me - Me - Me - Me(s)Me(s)Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 4: Second strand (5'-3'): Me - Me - Me - Me - Me - Me - F - Me - F - F - F - F - Me - Me - Me - Me - Me - Me - Me(s)Me(s)Me, First strand (5'-3'): Me(s)F(s)Me - F - Me - F - Me - Me - Me - Me - Me - Me - Me - F - Me - F - Me - Me - Me - Me - Me(s)Me(s)Me
Or Modification pattern 5: Second strand (5'-3'): Me - Me - Me - Me - Me - Me - Me - Me - F - F - F - Me - Me - Me - Me - Me - Me - Me - Me(s)Me(s)Me, First strand (5'-3'): Me(s)F(s)Me - Me - Me - F - Me - Me - F - Me - Me - Me - Me - F - Me - F - Me - Me - Me - Me - Me(s)Me(s)Me
Or Modification pattern 6: Second strand (5'-3'): Me - Me - Me - Me - Me - Me - F - Me - F - F - F - Me - Me - Me - Me - Me - Me - Me - Me(s)Me(s)Me, First strand (5'-3'): Me(s)F(s)Me - Me - Me - F - Me - Me - F - Me - Me - Me - Me - F - Me - F - Me - Me - Me - Me - Me(s)Me(s)Me
wherein (s) is a phosphorothioate internucleoside linkage.

A nucleic acid wherein modified nucleosides comprise any one of the following modification patterns:
Modification pattern 1: Second strand (5'-3'): ia - ia - Me - Me - Me - Me - Me - Me - F - F - F - F - F - Me - Me - Me - Me - Me - Me - Me - F - Me - Me, First strand (5' - 3'): Me - F - Me - F - Me - F - Me - F - F - Me - Me - Me - Me - F - Me - F - Me - Me - Me - Me - Me - Me - Me
Or Modification pattern 2: Second strand (5'-3'): ia - ia - Me - Me - Me - Me - Me - F - F - Me - F - F - F - F - Me - Me - Me - Me - Me - Me - Me - Me - Me, First strand (5'-3'): Me - F - Me - F - Me - F - Me - F - F - Me - Me - Me - Me - F - Me - F - Me - Me - Me - Me - Me - Me - Me
Or Modification pattern 3: Second strand (5'-3'): ia - ia - Me - Me - Me - Me - Me - Me -F-Me - F - F - F - F - Me - Me - Me - Me - Me - Me - Me - Me - Me, First strand (5'-3'): Me-F - Me - F - Me - F - Me - F - F - Me - Me - Me - Me - F - Me - F - Me - Me - Me - Me - Me - Me - Me
Or Modification pattern 4: Second strand (5'-3'): ia - ia - Me - Me - Me - Me - Me - Me - F - Me - F - F - F - F - Me - Me - Me - Me - Me - Me - Me - Me - Me, First strand (5'-3'): Me - F - Me - F - Me - F - Me - Me - Me - Me - Me - Me - Me - F - Me - F - Me - Me - Me - Me - Me - Me - Me
Or Modification pattern 5: Second strand (5'-3'): ia - ia - Me - Me - Me - Me - Me - Me - Me - Me - F - F - F - Me - Me - Me - Me - Me - Me - Me - Me - Me - Me, First strand (5'-3'): Me - F - Me - Me - Me - F - Me - Me - F - Me - Me - Me - Me - F - Me - F - Me - Me-Me - Me - Me - Me - Me
Or Modification pattern 6: Second strand (5'-3'): ia - ia - Me - Me - Me - Me - Me - Me - F - Me - F - F - F - Me - Me - Me - Me - Me - Me - Me - Me - Me - Me, First strand (5'-3'): Me - F - Me - Me - Me - F - Me - Me - F - Me - Me - Me - Me - F - Me - F - Me - Me - Me - Me - Me - Me - Me,
wherein ia represents an inverted abasic nucleoside.

A nucleic acid wherein modified nucleosides comprise any one of the following modification patterns:
Modification pattern 1: Second strand (5'-3'): Me - Me - Me - Me - Me - Me - F - F - F - F - F - Me - Me - Me - Me - Me - Me - Me - F - Me - Me - ia - ia, First strand (5'-3'): Me - F - Me - F - Me - F - Me - F - F - Me - Me - Me - Me - F - Me - F - Me - Me - Me - Me - Me - Me - Me
Or Modification pattern 2: Second strand (5'-3'): Me - Me - Me - Me - Me - F - F - Me - F - F - F - F - Me - Me - Me - Me - Me - Me - Me - Me - Me - ia - ia, First strand (5'-3'): Me - F - Me - F - Me - F - Me - F - F - Me - Me - Me - Me - F - Me - F - Me - Me - Me - Me - Me - Me - Me
Or Modification pattern 3: Second strand (5'-3'): Me - Me - Me - Me - Me - Me -F- Me - F - F - F - F - Me - Me - Me - Me - Me - Me - Me - Me - Me - ia - ia, First strand (5'-3'): Me - F - Me - F - Me - F - Me - F - F - Me - Me - Me - Me - F - Me - F - Me - Me - Me - Me - Me - Me - Me
Or Modification pattern 4: Second strand (5'-3'): Me - Me - Me - Me - Me - Me - F - Me - F - F - F - F - Me - Me - Me - Me - Me - Me - Me - Me - Me - ia - ia, First strand (5'-3'): Me - F - Me - F - Me - F - Me - Me - Me - Me - Me - Me - Me - F - Me - F - Me - Me - Me - Me - Me - Me - Me
Or Modification pattern 5: Second strand (5'-3'): Me - Me - Me - Me - Me - Me - Me - Me - F - F - F - Me - Me - Me - Me - Me - Me - Me - Me - Me - Me - ia - ia, First strand (5'-3'): Me - F - Me - Me - Me - F - Me - Me - F - Me - Me - Me - Me - F - Me - F - Me - Me - Me - Me - Me - Me - Me
Or Modification pattern 6: Second strand (5'-3'): Me - Me - Me - Me - Me - Me - F - Me - F - F - F - Me - Me - Me - Me - Me - Me - Me - Me - Me - Me - ia - ia,- First strand (5'-3'): Me - F - Me - Me - Me - F - Me - Me - F - Me - Me - Me - Me - F - Me - F - Me - Me - Me - Me - Me - Me - Me,
wherein ia represents an inverted abasic nucleoside, and when the inverted abasic nucleosides as represented by ia - ia are present at the 3' terminus of the second strand, said inverted abasic nucleosides are present in a 2 nucleoside overhang.

A nucleic acid wherein modified nucleosides comprise any one of the following modification patterns:
Modification pattern 1: Second strand (5'-3'): ia - ia - Me(s)Me(s)Me - Me - Me - Me - F - F - F - F - F - Me - Me - Me - Me - Me - Me - Me - F - Me - Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 2: Second strand (5'-3'): ia - ia - Me(s)Me(s)Me - Me - Me - F - F - Me - F - F - F - F - Me - Me - Me - Me - Me - Me - Me - Me - Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 3: Second strand (5'-3'): ia - ia - Me(s)Me(s)Me - Me - Me - Me - F- Me - F - F - F - F - Me - Me - Me - Me - Me - Me - Me - Me - Me, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 4: Second strand (5'-3'): ia - ia - Me(s)Me(s)Me - Me - Me - Me - F - Me - F - F - F - F - Me - Me - Me - Me - Me - Me - Me - Me - Me, First strand (5'-3'): Me(s)F(s)Me - F - Me - F - Me - Me - Me - Me - Me - Me - Me - F - Me - F - Me - Me - Me - Me - Me(s)Me(s)Me
Or Modification pattern 5: Second strand (5'-3'): ia - ia - Me(s)Me(s)Me - Me - Me - Me - Me - Me - F - F - F - Me - Me - Me - Me - Me - Me - Me - Me - Me - Me, First strand (5'-3'): Me(s)F(s)Me - Me - Me - F - Me - Me - F - Me - Me - Me - Me - F - Me - F - Me - Me-Me - Me - Me(s)Me(s)Me
Or Modification pattern 6: Second strand (5'-3'): ia - ia - Me(s)Me(s)Me - Me - Me - Me - F - Me - F - F - F - Me - Me - Me - Me - Me - Me - Me - Me - Me - Me, First strand (5'-3'): Me(s)F(s)Me - Me - Me - F - Me - Me - F - Me - Me - Me - Me - F - Me - F - Me - Me - Me - Me - Me(s)Me(s)Me
wherein:
   (s) is a phosphorothioate internucleoside linkage, ia represents an inverted abasic nucleoside.

A nucleic acid wherein modified nucleosides comprise any one of the following modification patterns:
Modification pattern 1: Second strand (5'-3'): Me - Me - Me - Me - Me - Me - F - F - F - F - F - Me - Me - Me - Me - Me - Me - Me - F(s)Me(s)Me - ia - ia, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 2: Second strand (5'-3'): Me - Me - Me - Me - Me - F - F - Me - F - F - F - F - Me - Me - Me - Me - Me - Me - Me(s)Me(s)Me - ia - ia, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 3: Second strand (5'-3'): Me - Me - Me - Me - Me - Me -F- Me - F - F - F - F - Me - Me - Me - Me - Me - Me - Me(s)Me(s)Me - ia - ia, First strand (5'-3'): Me(s)F(s)Me - F - Me - F - Me - F - F - Me - Me - Me - Me - F - Me - F - Me - Me - Me - Me - Me(s)Me(s)Me
Or Modification pattern 4: Second strand (5'-3'): Me - Me - Me - Me - Me - Me - F - Me - F - F - F - F - Me - Me - Me - Me - Me - Me - Me(s)Me(s)Me - ia - ia, First strand (5'-3'): Me(s)F(s)Me - F - Me - F - Me - Me - Me - Me - Me - Me - Me - F - Me - F - Me - Me - Me - Me - Me(s)Me(s)Me
Or Modification pattern 5: Second strand (5'-3'): Me - Me - Me - Me - Me - Me - Me - Me - F - F - F - Me - Me - Me - Me - Me - Me - Me - Me(s)Me(s)Me - ia - ia, First strand (5'-3'): Me(s)F(s)Me - Me - Me - F - Me - Me - F - Me - Me - Me - Me - F - Me - F - Me - Me-Me - Me - Me(s)Me(s)Me
Or Modification pattern 6: Second strand (5'-3'): Me - Me - Me - Me - Me - Me - F - Me - F - F - F - Me - Me - Me - Me - Me - Me - Me - Me(s)Me(s)Me - ia - ia, First strand (5'-3'): Me(s)F(s)Me - Me - Me - F - Me - Me - F - Me - Me - Me - Me - F - Me - F - Me - Me - Me - Me - Me(s)Me(s)Me
wherein: (s) is a phosphorothioate internucleoside linkage, ia represents an inverted abasic nucleoside, and when the inverted abasic nucleosides as represented by ia - ia are present at the 3' terminus of the second strand, said inverted abasic nucleosides are present in a 2 nucleoside overhang.

Particularly preferred is a nucleic acid wherein the modified nucleosides comprise the following modification pattern:
Modification pattern 4: Second strand (5'-3'): ia - ia - Me(s)Me(s)Me - Me - Me - Me - F - Me - F - F - F - F - Me - Me - Me - Me - Me - Me - Me - Me - Me, First strand (5'-3'): Me(s)F(s)Me - F - Me - F - Me - Me - Me - Me - Me - Me - Me - F - Me - F - Me - Me - Me - Me - Me(s)Me(s)Me
wherein: (s) is a phosphorothioate internucleoside linkage, ia represents an inverted abasic nucleoside.

A nucleic acid wherein the first strand comprises a 2' sugar modification pattern wherein said modifications are selected at least from 2'Me and 2'F sugar modifications, provided that the overall number of 2'F sugar modifications in the first strand does not consist of four, or six, 2'F modifications.

A nucleic acid wherein the first strand comprises a 2' sugar modification pattern wherein said modifications are selected at least from 2'Me and 2'F sugar modifications, wherein the overall number of 2'F sugar modifications in the first strand consists of three, five or seven 2'F modifications.

A nucleic acid wherein the first strand comprises a 2' sugar modification pattern wherein said modifications are selected at least from 2'Me and 2'F sugar modifications, wherein the overall number of 2'F sugar modifications in the first strand consists of three 2'F modifications.

A nucleic acid wherein the first strand comprises a 2' sugar modification pattern wherein said modifications are selected at least from 2'Me and 2'F sugar modifications, wherein the overall number of 2'F sugar modifications in the first strand consists of five 2'F modifications.

A nucleic acid wherein the first strand comprises a 2' sugar modification pattern as follows (5'-3'):
Me - F - Me - X₂ - Me - F - (Me)₇ - (F - Me)₂ - X₃ - Me - X₄ - (Me)₃
wherein X₂, X₃ and X₄ are selected from 2'Me and 2'F sugar modifications, provided that for X₂, X₃ and X₄ at least one is a 2'F sugar modification, and the other two sugar modifications are 2'Me sugar modifications.

A nucleic acid wherein the first strand comprises a 2' sugar modification pattern as follows (5'-3'):
Me - F - Me - X₂ - Me - F - (Me)₇ - (F - Me)₂ - X₃ - Me - X₄ - (Me)₃
wherein X₂ is a 2'F sugar modification, and X₃ and X₄ are 2'Me sugar modifications.

A nucleic acid wherein the first strand comprises a 2' sugar modification pattern as follows (5'-3'):
Me - F - Me - X₂ - Me - F - (Me)₇ - (F - Me)₂ - X₃ - Me - X₄ - (Me)₃
wherein X₃ is a 2'F sugar modification, and X₂ and X₄ are 2'Me sugar modifications.

A nucleic acid wherein the first strand comprises a 2' sugar modification pattern as follows (5'-3'):
Me - F - Me - X₂ - Me - F - (Me)₇ - (F - Me)₂ - X₃ - Me - X₄ - (Me)₃
wherein X₄ is a 2'F sugar modification, and X₂ and X₃ are 2'Me sugar modifications.

A nucleic acid wherein the first strand comprises a 2' sugar modification pattern wherein said modifications are selected at least from 2'Me and 2'F sugar modifications, wherein the overall number of 2'F sugar modifications in the first strand consists of seven 2'F modifications.

A nucleic acid wherein the first strand comprises a 2' sugar modification pattern as follows (5'-3'):
Me - F - Me - X₂ - Me - F - Me - (F)₂ - (Me)₄ - (F - Me)₂ - X₃ - Me - X₄ - (Me)₃
wherein X₂, X₃ and X₄ are selected from 2'Me and 2'F sugar modifications, provided that for X₂, X₃ and X₄ at least one is a 2'F sugar modification, and the other two sugar modifications are 2'Me sugar modifications.

A nucleic acid wherein the first strand comprises a 2' sugar modification pattern as follows (5'-3'):
Me - F - Me - X₂ - Me - F - Me - (F)₂ - (Me)₄ - (F - Me)₂ - X₃ - Me - X₄ - (Me)₃
wherein X₂ is a 2'F sugar modification, and X₃ and X₄ are 2'Me sugar modifications.

A nucleic acid wherein the first strand comprises a 2' sugar modification pattern as follows (5'-3'):
Me - F - Me - X₂ - Me - F - Me - (F)₂ - (Me)₄ - (F - Me)₂ - X₃ - Me - X₄ - (Me)₃
wherein X₃ is a 2'F sugar modification, and X₂ and X₄ are 2'Me sugar modifications.

A nucleic acid wherein the first strand comprises a 2' sugar modification pattern as follows (5'-3'):
Me - F - Me - X₂ - Me - F - Me - (F)₂ - (Me)₄ - (F - Me)₂ - X₃ - Me - X₄ - (Me)₃
wherein X₄ is a 2'F sugar modification, and X₂ and X₃ are 2'Me sugar modifications.

A nucleic acid wherein the first strand comprises a 2' sugar modification pattern as follows (5'-3'):
Me - F - (Me)₃ - X₁ - (Me)₇ - F - Me - F - (Me)₇
wherein X₁ is a thermally destabilising modification.

A nucleic acid wherein the first strand comprises a 2' sugar modification pattern as follows (5'-3'):
Me - F - (Me)₃ - X₁ - Me - (F)₂ - (Me)₄ - F - Me - F - (Me)₇
wherein X₁ is a thermally destabilising modification.

A nucleic acid wherein the second strand comprises a 2' sugar modification pattern as follows (5'-3'):
(Me)₈ - (F)₃ - (Me)₁₀

A nucleic acid wherein the second strand comprises a 2' sugar modification pattern as follows (5'-3'):
(Me)₈ - (F)₃ - (Me)₁₀, and
wherein the first strand comprises a 2' sugar modification pattern wherein said modifications are selected at least from 2'Me and 2'F sugar modifications, provided that the overall number of 2'F sugar modifications in the first strand does not consist of four, or six, 2'F modifications.

A nucleic acid wherein the second strand comprises a 2' sugar modification pattern as follows (5'-3'):
(Me)₈ - (F)₃ - (Me)₁₀, and
wherein the first strand comprises a 2' sugar modification pattern wherein said modifications are selected at least from 2'Me and 2'F sugar modifications, wherein the overall number of 2'F sugar modifications in the first strand consists of three, five or seven 2'F modifications.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   Me - F - (Me)₃ - X₁ - (Me)₇ - F - Me - F - (Me)₇, wherein X₁ is a thermally destabilising modification.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   (Me - F)₃ - (Me)₇ - F - Me - F - (Me)₇.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   Me - F - (Me)₃ - F - (Me)₇ - (F - Me)₂ - F - (Me)₅.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   Me - F - (Me)₃ - F - (Me)₇ - F - Me - F - (Me)₃ - F - (Me)₃.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   Me - F - (Me)₃ - X₁ - Me - (F)₂ - (Me)₄ - F - Me - F - (Me)₇, wherein X₁ is a thermally destabilising modification.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   (Me - F)₃ - Me - (F)₂ - (Me)₄ - (F - Me)₂ - (Me)₆.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   Me - F - (Me)₃ - F - Me - (F)₂ - (Me)₄ - (F - Me)₂ - F - (Me)₅.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar modification pattern as follows (5'-3'):
(Me)₈ - (F)₃ - (Me)₁₀, and
wherein nucleosides of said first strand comprise a 2' sugar modification pattern as follows (5'-3'):
   Me-F-(Me)₃-F-Me-(F)₂-(Me)₄-(F-Me)₂-(Me)₂-F-(Me)₃.

A nucleic acid wherein the second strand comprises a 2' sugar, and abasic modification pattern as follows (5'-3'):
ia-ia-(Me)₈ - (F)₃ - (Me)₁₀
wherein ia represents an inverted abasic nucleoside.

A nucleic acid wherein the second strand comprises a 2' sugar, and abasic modification pattern as follows (5'-3'):
ia-ia-(Me)₈ - (F)₃ - (Me)₁₀, wherein ia represents an inverted abasic nucleoside; and
wherein the first strand comprises a 2' sugar modification pattern wherein said modifications are selected at least from 2'Me and 2'F sugar modifications, provided that the overall number of 2'F sugar modifications in the first strand does not consist of four, or six, 2'F modifications.

A nucleic acid wherein the second strand comprises a 2' sugar, and abasic modification pattern as follows (5'-3'):
ia-ia-(Me)₈ - (F)₃ - (Me)₁₀, wherein ia represents an inverted abasic nucleoside; and
wherein the first strand comprises a 2' sugar modification pattern wherein said modifications are selected at least from 2'Me and 2'F sugar modifications, wherein the overall number of 2'F sugar modifications in the first strand consists of three, five or seven 2'F modifications.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   Me - F - (Me)₃ - X₁ - (Me)₇ - F - Me - F - (Me)₇, wherein X₁ is a thermally destabilising modification.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   (Me - F)₃ - (Me)₇ - F - Me - F - (Me)₇.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   Me - F - (Me)₃ - F - (Me)₇ - (F - Me)₂ - F - (Me)₅.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   Me - F - (Me)₃ - F - (Me)₇ - F - Me - F - (Me)₃ - F - (Me)₃.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   Me - F - (Me)₃ - X₁ - Me - (F)₂ - (Me)₄ - F - Me - F - (Me)₇,
wherein X₁ is a thermally destabilising modification.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   (Me - F)₃ - Me - (F)₂ - (Me)₄ - (F - Me)₂ - (Me)₆.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   Me - F - (Me)₃ - F - Me - (F)₂ - (Me)₄ - (F - Me)₂ - F - (Me)₅.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-(Me)₈ - (F)₃ - (Me)₁₀, wherein ia represents an inverted abasic nucleoside; and
wherein nucleosides of said first strand comprise a 2' sugar modification pattern as follows (5'-3'):
   Me - F - (Me)₃ - F - Me - (F)₂ - (Me)₄ - (F - Me)₂ - (Me)₂ - F - (Me)₃.

A nucleic acid wherein the second strand comprises a 2' sugar modification pattern as follows (5'-3'):
ia-ia-Me(s)Me(s) (Me)₆ - (F)₃ - (Me)₁₀,
wherein ia represents an inverted abasic nucleoside, and (s) represents a phosphorothioate linkage.

A nucleic acid wherein the second strand comprises a 2' sugar modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage; and
wherein the first strand comprises a 2' sugar modification pattern wherein said modifications are selected at least from 2'Me and 2'F sugar modifications, provided that the overall number of 2'F sugar modifications in the first strand does not consist of four, or six, 2'F modifications.

A nucleic acid wherein the second strand comprises a 2' sugar modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage; and
wherein the first strand comprises a 2' sugar modification pattern wherein said modifications are selected at least from 2'Me and 2'F sugar modifications, wherein the overall number of 2'F sugar modifications in the first strand consists of three, five or seven 2'F modifications.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)(Me)₃ - X₁ - (Me)₇ - F - Me - F - (Me)₅(s)Me(s)Me, wherein X₁ is a thermally destabilising modification.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)Me - F - Me - F - (Me)₇ - F - Me - F - (Me)₅(s)Me(s)Me.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)(Me)₃ - F - (Me)₇ - (F - Me)₂ - F - (Me)₃(s)Me(s)Me.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)(Me)₃ - F - (Me)₇ - F - Me - F - (Me)₃ - F - Me(s)Me(s)Me.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)(Me)₃ - X₁ - Me - (F)₂ - (Me)₄ - F - Me - F - (Me)₅(s)Me(s)Me, wherein X₁ is a thermally destabilising modification

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)Me - F - Me - F - Me - (F)₂ - (Me)₄ - (F - Me)₂ - (Me)₄(s)Me(s)Me.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)(Me)₃ - F - Me - (F)₂ - (Me)₄ - (F - Me)₂ - F - (Me)₃(s)Me(s)Me.

A nucleic acid comprising a first strand that is at least partially complementary to a portion of RNA transcribed from the target gene, and a second strand that is at least partially complementary to the first strand, wherein said first and second strands form a duplex region of at least 17 nucleosides in length, and wherein nucleosides of said second strand comprise a 2' sugar, and abasic modification pattern as follows (5'-3'):
ia-ia-Me(s)Me(s) (Me)₆ - (F)₃ - (Me)₁₀, wherein ia represents an inverted abasic nucleoside, and (s) represents a phosphorothioate linkage, and
wherein nucleosides of said first strand comprise a 2' sugar modification pattern as follows (5'-3'):
   Me(s)F(s)(Me)₃ - F - Me - (F)₂ - (Me)₄ - (F - Me)₂ - (Me)₂ - F - Me(s)Me(s)Me.

Preferred modifications are as follows:
Modification pattern 1:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - Me - Me - X₁ - Me - Me - Me - Me - Me - Me - Me - F - Me - F - Me - Me - Me - Me - Me - Me - Me, wherein X₁ is a thermally destabilising modification;
Or Modification pattern 2:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - F - Me - F - Me - Me - Me - Me - Me - Me - Me - F - Me - F - Me - Me - Me - Me - Me - Me - Me;
Or Modification pattern 3:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - Me - Me - F - Me - Me - Me - Me - Me - Me - Me-F - Me - F - Me - F - Me - Me - Me - Me - Me;
Or Modification pattern 4:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - Me - Me - F - Me - Me - Me - Me - Me - Me - Me - F - Me - F - Me - Me - Me - F - Me - Me - Me;
Or Modification pattern 5:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - Me - Me - X₁ - Me -F - F - Me - Me - Me - Me - F - Me - F - Me - Me - Me - Me - Me - Me - Me, wherein X₁ is a thermally destabilising modification;
Or Modification pattern 6:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me -F -Me - F - Me -F - F - Me -Me - Me - Me -F - Me - F - Me - Me - Me - Me - Me - Me - Me;
Or Modification pattern 7:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - Me - Me - F - Me - F - F - Me - Me - Me - Me - F - Me - F - Me - F - Me - Me - Me - Me - Me;
Or Modification pattern 8:
   Second strand (5'-3'): ia - ia - Me - Me - Me - Me - Me - Me - Me - Me - F - F - F - Me - Me - Me - Me - Me - Me - Me - Me - Me - Me,
   First strand (5'-3'): Me - F - Me - Me - Me - F - Me - F - F - Me - Me - Me - Me - F - Me - F - Me - Me - Me - F - Me - Me - Me
wherein ia represents an inverted abasic nucleoside.

Further preferred modifications are as follows:
Modification pattern 1:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - Me - Me - X₁ - Me - Me - Me - Me - Me - Me - Me - F - Me - F - Me - Me - Me - Me - Me(s)Me(s)Me, wherein X₁ is a thermally destabilising modification;
Or Modification pattern 2:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - F - Me - F - Me - Me - Me - Me - Me - Me - Me - F - Me - F - Me - Me - Me - Me - Me(s)Me(s)Me;
Or Modification pattern 3:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - Me - Me - F - Me - Me - Me - Me - Me - Me - Me - F - Me - F - Me - F - Me - Me - Me(s)Me(s)Me;
Or Modification pattern 4:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - Me - Me - F - Me - Me - Me - Me - Me - Me - Me - F - Me - F - Me - Me - Me - F - Me(s)Me(s)Me;
Or Modification pattern 5:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - Me - Me - X₁ - Me - F - F - Me - Me - Me - Me - F - Me - F - Me - Me - Me - Me - Me(s)Me(s)Me, wherein X₁ is a thermally destabilising modification;
Or Modification pattern 6:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - F - Me - F - Me - F - F - Me - Me - Me - Me - F - Me - F - Me - Me - Me - Me - Me(s)Me(s)Me;
Or Modification pattern 7:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - Me - Me - F - Me - F - F - Me - Me - Me - Me - F - Me - F - Me - F - Me - Me - Me(s)Me(s)Me;
Or Modification pattern 8:
   Second strand (5'-3'): ia - ia - Me(s)Me(s)Me - Me - Me - Me - Me - Me - F - F - F - Me - Me - Me - Me - Me - Me - Me - Me - Me - Me,
   First strand (5'-3'): Me(s)F(s)Me - Me - Me - F - Me - F - F - Me - Me - Me - Me - F - Me - F - Me - Me - Me - F - Me(s)Me(s)Me;
wherein (s) is a phosphorothioate internucleoside linkage and ia represents an inverted abasic nucleoside.

### Conjugation

Another modification of the nucleic acid e.g. RNA e.g. an siRNA of the invention involves linking the nucleic acid e.g. the siRNA to one or more ligand moieties e.g. to enhance the activity, cellular distribution, or cellular uptake of the nucleic acid e.g. siRNA e.g. into a cell.

**In** some embodiments, the ligand moiety described can be attached to a nucleic acid e.g. an siRNA oligonucleoside, via a linker that can be cleavable or non-cleavable. The term "linker" or "linking group" means an organic moiety that connects two parts of a compound, e.g., covalently attaches two parts of a compound.

The ligand can be attached to the 3' or 5' end of the sense strand.

The ligand is preferably conjugated to 3' end of the sense strand of the nucleic acid e.g. an siRNA agent.

The invention therefore relates in a further aspect to a conjugate for inhibiting expression of a target gene in a cell, said conjugate comprising a nucleic acid portion and one or more ligand moieties, said nucleic acid portion comprising a nucleic acid as disclosed herein.

In one aspect the second strand of the nucleic acid is conjugated directly or indirectly (e.g. via a linker) to the one or more ligand moiety(s), wherein said ligand moiety is typically present at a terminal region of the second strand, preferably at the 3' terminal region thereof.

In certain embodiments, the ligand moiety comprises a GalNAc or GalNAc derivative attached to the nucleic acid eg dsiRNA through a linker.

Therefore the invention relates to a conjugate wherein the ligand moiety comprises
i) one or more GalNAc ligands; and / or
ii) one or more GalNAc ligand derivatives; and / or
iii) one or more GalNAc ligands conjugated to said nucleic acid through a linker.

Said GalNAc ligand may be conjugated directly or indirectly to the 5' or 3' terminal region of the second strand of the nucleic acid, preferably at the 3' terminal region thereof.

GalNAc ligands are well known in the art and described in, inter alia, EP3775207A1.

In some embodiments, the GalNAc ligand is comprised in any one of the linkers shown in Figures 1 to 4 or Figure 5 (Formula XI), wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in the linker shown in Figure 3, wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in the linker shown in Figure 5 (Formula XI), wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in any one of the linkers shown in Figures 1 to 4 or Figure 5 (Formula XI), wherein the "oligonucleotide" represents a nucleic acid according to the invention, wherein the nucleic acid according to the invention comprises a modified or unmodified second strand comprising or consisting of SEQ ID NO:265 or SEQ ID NO:268, preferably wherein the linker is conjugated to the 3' terminal region of the second strand, i.e., to the 3' terminal region of SEQ ID NO:265 or SEQ ID NO:268, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified or unmodified second strand comprising or consisting of SEQ ID NO:265 or SEQ ID NO:268, preferably wherein the linker is conjugated to the 3' terminal region of the second strand, i.e., to the 3' terminal region of SEQ ID NO:265 or SEQ ID NO:268, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified or unmodified second strand comprising or consisting of SEQ ID NO:265 or SEQ ID NO:268, preferably wherein the linker is conjugated to the 3' terminal region of the second strand, i.e., to the 3' terminal region of SEQ ID NO:265 or SEQ ID NO:268, via a phosphodiester bond.

In some embodiments, the GalNAc ligand is comprised in any one of the linkers shown in Figures 1 to 4 or Figure 5 (Formula XI), wherein the "oligonucleotide" represents a nucleic acid according to the invention, wherein the nucleic acid according to the invention comprises a modified second strand comprising or consisting of SEQ ID NO:774 or SEQ ID NO:776, preferably wherein the linker is conjugated to the 3' terminal region of the second strand, i.e., to the 3' terminal region of SEQ ID NO:774 or SEQ ID NO:776, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified second strand comprising or consisting of SEQ ID NO:774 or SEQ ID NO:776, preferably wherein the linker is conjugated to the 3' terminal region of the second strand, i.e., to the 3' terminal region of SEQ ID NO:774 or SEQ ID NO:776, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified second strand comprising or consisting of SEQ ID NO:774 or SEQ ID NO:776, preferably wherein the linker is conjugated to the 3' terminal region of the second strand, i.e., to the 3' terminal region of SEQ ID NO:774 or SEQ ID NO:776, via a phosphodiester bond.

In some embodiments, the GalNAc ligand is comprised in any one of the linkers shown in Figures 1 to 4 or Figure 5 (Formula XI), wherein the "oligonucleotide" represents a nucleic acid according to the invention, wherein the nucleic acid according to the invention comprises a modified first strand comprising or consisting of SEQ ID NO:764 and a modified second strand comprising or consisting of SEQ ID NO:774, preferably wherein the linker is conjugated to the 3' terminal region of the second strand, i.e., to the 3' terminal region of SEQ ID NO:774, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified first strand comprising or consisting of SEQ ID NO:764 and a modified second strand comprising or consisting of SEQ ID NO:774, preferably wherein the linker is conjugated to the 3' terminal region of the second strand, i.e., to the 3' terminal region of SEQ ID NO:774, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified first strand comprising or consisting of SEQ ID NO:764 and a modified second strand comprising or consisting of SEQ ID NO:774, preferably wherein the linker is conjugated to the 3' terminal region of the second strand, i.e., to the 3' terminal region of SEQ ID NO:774, via a phosphodiester bond.

In some embodiments, the GalNAc ligand is comprised in any one of the linkers shown in Figures 1 to 4 or Figure 5 (Formula XI), wherein the "oligonucleotide" represents a nucleic acid according to the invention, wherein the nucleic acid according to the invention comprises a modified first strand comprising or consisting of SEQ ID NO:766 and a modified second strand comprising or consisting of SEQ ID NO:776, preferably wherein the linker is conjugated to the 3' terminal region of the second strand, i.e., to the 3' terminal region of SEQ ID NO:776, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified first strand comprising or consisting of SEQ ID NO:766 and a modified second strand comprising or consisting of SEQ ID NO:776, preferably wherein the linker is conjugated to the 3' terminal region of the second strand, i.e., to the 3' terminal region of SEQ ID NO:776, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified first strand comprising or consisting of SEQ ID NO:766 and a modified second strand comprising or consisting of SEQ ID NO:776, preferably wherein the linker is conjugated to the 3' terminal region of the second strand, i.e., to the 3' terminal region of SEQ ID NO:776, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified first strand comprising or consisting of SEQ ID NO:764 and a modified second strand comprising or consisting of SEQ ID NO:774, wherein the second strand has the following structure 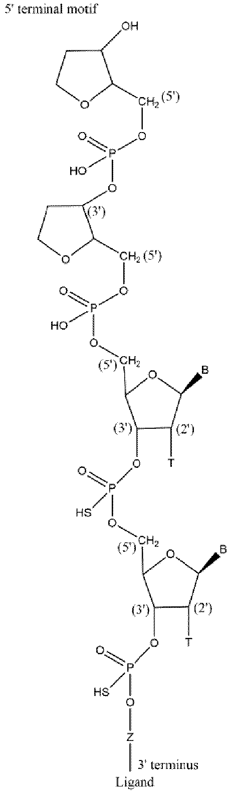 wherein:
T represents a 2'Me ribose modification,
B represents the nucleoside bases of the first two basic nucleosides in the 5' terminal region of SEQ ID NO:774, and
Z represents the remaining 19 contiguous basic nucleosides of SEQ ID NO:774.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified first strand comprising or consisting of SEQ ID NO:764 and a modified second strand comprising or consisting of SEQ ID NO:774, wherein the second strand has the following structure 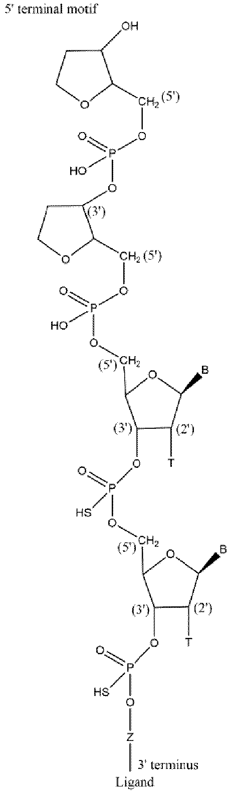 wherein:
T represents a 2'Me ribose modification,
B represents the nucleoside bases of the first two basic nucleosides in the 5' terminal region of SEQ ID NO:774, and
Z represents the remaining 19 contiguous basic nucleosides of SEQ ID NO:774.

In some embodiments, the GalNAc ligand is comprised in the linker shown in Figure 3, wherein the "oligonucleotide" represents a nucleic acid according to the invention, wherein the nucleic acid according to the invention comprises a modified first strand comprising or consisting of SEQ ID NO:766 and a modified second strand comprising or consisting of SEQ ID NO:776, wherein the second strand has the following structure 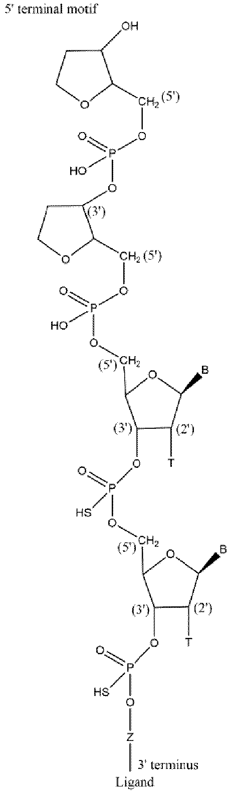 wherein:
T represents a 2'Me ribose modification,
B represents the nucleoside bases of the first two basic nucleosides in the 5' terminal region of SEQ ID NO:776, and
Z represents the remaining 19 contiguous basic nucleosides of SEQ ID NO:776.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified first strand comprising or consisting of SEQ ID NO:766 and a modified second strand comprising or consisting of SEQ ID NO:776, wherein the second strand has the following structure 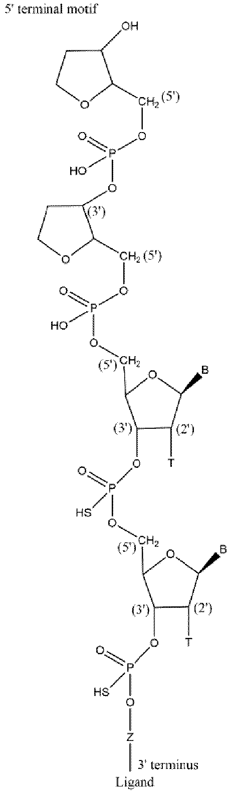 wherein:
T represents a 2'Me ribose modification,
B represents the nucleoside bases of the first two basic nucleosides in the 5' terminal region of SEQ ID NO:776, and
Z represents the remaining 19 contiguous basic nucleosides of SEQ ID NO:776.

### Vector And Cell

In one aspect, the disclosure provides a cell containing a nucleic acid, such as inhibitory RNA [RNAi] as described herein.

In one aspect, the disclosure provides a cell comprising a vector as described herein.

### Pharmaceutically Acceptable Compositions

In one aspect, the invention provides a pharmaceutical composition for inhibiting expression of a target gene, the composition comprising a nucleic acid as disclosed herein.

The pharmaceutically acceptable composition may comprise an excipient and or carrier.

Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen- free water; (17) isotonic saline; (18) Ringer's solution; (19) ethyl alcohol; (20) pH buffered solutions; (21) polyesters, polycarbonates and/or poly anhydrides; (22) bulking agents, such as polypeptides and amino acids (23) serum component, such as serum albumin, HDL and LDL; and (22) other nontoxic compatible substances employed in pharmaceutical formulations.

Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g. , magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g. , starch, sodium starch glycolate, etc.); and wetting agents (e.g., sodium lauryl sulphate, etc).

Pharmaceutically acceptable organic or inorganic excipients suitable for non-parenteral administration which do not deleteriously react with nucleic acids can also be used to formulate the compositions of the present disclosure. Suitable pharmaceutically acceptable excipients include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone, and the like.

Formulations for topical administration of nucleic acids can include sterile and non-sterile aqueous solutions, non-aqueous solutions in common solvents such as alcohols, or solutions of the nucleic acids in liquid or solid oil bases. The solutions can also contain buffers, diluents and other suitable additives. Pharmaceutically acceptable organic or inorganic excipients suitable for non- parenteral administration which do not deleteriously react with nucleic acids can be used.

In one embodiment, the nucleic acid or composition is administered in an unbuffered solution. In certain embodiments, the unbuffered solution is saline or water. In other embodiments, the nucleic acid e.g. siRNA agent is administered in a buffered solution. In such embodiments, the buffer solution can comprise acetate, citrate, prolamine, carbonate, or phosphate, or any combination thereof. For example, the buffer solution can be phosphate buffered saline (PBS).

### Dosages

The pharmaceutical compositions of the disclosure may be administered in dosages sufficient to inhibit expression of a gene. In general, a suitable dose of a nucleic acid e.g. an siRNA of the disclosure will be in the range of about 0.001 to about 200.0 milligrams per kilogram body weight of the recipient per day, generally in the range of about 1 to 50 mg per kilogram body weight per day. Typically, a suitable dose of a nucleic acid e.g. an siRNA of the disclosure will be in the range of about 0.1 mg/kg to about 5.0 mg/kg, e.g., about 0.3 mg/kg and about 3.0 mg/kg.

A repeat-dose regimen may include administration of a therapeutic amount of a nucleic acid e.g. siRNA on a regular basis, such as every other day or once a year. In certain embodiments, the nucleic acid e.g. siRNA is administered about once per month to about once per quarter (i.e., about once every three months).

In various embodiments, the nucleic acid e.g. siRNA agent is administered at a dose of about 0.01 mg/kg to about 10 mg/kg or about 0.5 mg/kg to about 50 mg/kg. In some embodiments, the nucleic acid e.g. siRNA agent is administered at a dose of about 10 mg/kg to about 30 mg/kg. In certain embodiments, the nucleic acid e.g. siRNA agent is administered at a dose selected from about 0.5 mg/kg 1 mg/kg, 1.5 mg/kg, 3 mg/kg, 5 mg/kg, 10 mg/kg, and 30 mg/kg. In certain embodiments, the nucleic acid e.g. agent is administered about once per week, once per month, once every other two months, or once a quarter (i.e., once every three months) at a dose of about 0.1 mg/kg to about 5.0 mg/kg. In certain embodiments, the nucleic acid e.g. siRNA agent is administered to the subject once a week. In certain embodiments, the nucleic acid e.g. siRNA agent is administered to the subject once a month. In certain embodiments, the nucleic acid e.g. siRNA agent is administered once per quarter (i.e. , every three months).

After an initial treatment regimen, the treatments can be administered on a less frequent basis. For example, after administration weekly or biweekly for three months, administration can be repeated once per month, for six months, or a year; or longer.

The pharmaceutical composition can be administered once daily, or administered as two, three, or more sub-doses at appropriate intervals throughout the day or even using continuous infusion or delivery through a controlled release formulation. In that case, the nucleic acid e.g. siRNA contained in each sub-dose must be correspondingly smaller in order to achieve the total daily dosage. The dosage unit can also be compounded for delivery over several days, e.g. , using a conventional sustained release formulation which provides sustained release of the nucleic acid e.g. siRNA over a several day period. Sustained release formulations are well known in the art and are particularly useful for delivery of agents at a particular site, such as could be used with the agents of the present invention. In this embodiment, the dosage unit contains a corresponding multiple of the daily dose.

In other embodiments, a single dose of the pharmaceutical compositions can be long lasting, such that subsequent doses are administered at not more than 3, 4, or 5 day intervals, or at not more than 1, 2, 3, or 4 week intervals. In some embodiments of the invention, a single dose of the pharmaceutical compositions of the invention is administered once per week. In other embodiments of the invention, a single dose of the pharmaceutical compositions of the invention is administered bimonthly. In certain embodiments, the siRNA is administered about once per month to about once per quarter (i.e. , about once every three months), or even every 6 months or 12 months.

Estimates of effective dosages and in vivo half-lives for the individual nucleic acid e.g. siRNAs encompassed by the invention can be made using conventional methodologies or on the basis of in vivo testing using an appropriate animal model, as known in the art.

The pharmaceutical compositions of the present invention can be administered in a number of ways depending upon whether local or systemic treatment is desired and upon the area to be treated. Administration can be topical {e.g. , by a transdermal patch), pulmonary, e.g. , by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal, or intramuscular injection or infusion; subdermal, e.g. , via an implanted device; or intracranial, e.g. , by intraparenchymal, intrathecal or intraventricular administration. In certain preferred embodiments, the compositions are administered by intravenous infusion or injection. In certain embodiments, the compositions are administered by subcutaneous injection.

In one embodiment, the nucleic acid e.g. agent is administered to the subject subcutaneously.

The nucleic acid e.g. siRNA can be delivered in a manner to target a particular tissue (e.g. in particular liver cells).

### Methods For Inhibiting ZPI Gene Expression

The present disclosure also provides methods of inhibiting expression of ZPI gene in a cell. Said methods are not part of the claimed invention. The methods include contacting a cell with a nucleic acid of the disclosure e.g. siRNA agent, such as double stranded siRNA agent, in an amount effective to inhibit expression of the ZPI gene in the cell, thereby inhibiting expression of the ZPI gene in the cell. It is to be noted that a nucleic acid "for inhibiting the expression of ZPI" is a nucleic acid that is capable of inhibiting ZPI expression, preferably as described herein below.

Contacting of a cell with the nucleic acid e.g. an siRNA, such as a double stranded siRNA agent, may be done in vitro or in vivo. Said contacting of a cell with the nucleic acid is not part of the claimed invention. Contacting a cell in vivo with nucleic acid e.g. includes contacting a cell or group of cells within a subject, e.g., a human subject, with the nucleic acid e.g. siRNA. Combinations of in vitro and in vivo methods of contacting a cell are also possible. Contacting a cell may be direct or indirect, as discussed above. Furthermore, contacting a cell may be accomplished via a targeting ligand moiety, including any ligand moiety described herein or known in the art. In preferred embodiments, the targeting ligand moiety is a carbohydrate moiety, e.g. a GalNAc3 ligand, or any other ligand moiety that directs the siRNA agent to a site of interest.

The term "inhibiting," as used herein, is used interchangeably with "reducing," "silencing," "downregulating", "suppressing", and other similar terms, and includes any level of inhibition.

In some embodiments of the methods of the disclosure, expression of ZPI gene is inhibited by at least 30%, 35%, 40%, 45%, 50%, 55%, 60%, 65%, 70%, 75%, 80%, 85%, 90%, or 95%, or to below the level of detection of the assay, preferably when determined by qPCR as described herein and/or when the siRNA is introduced into the target cell by transfection. In certain embodiments, the methods include a clinically relevant inhibition of expression of ZPI target gene e.g. as demonstrated by a clinically relevant outcome after treatment of a subject with an agent to reduce the expression of the gene.

In some embodiments, when transfected into the cells, the nucleic acid of the invention inhibits expression of the ZPI gene with an IC50 value lower than 2500 pM, 2400 pM, 2300 pM, 2200 pM, 2100 pM, 2000 pM, 1900 pM, 1800 pM, 1700 pM, 1600 pM, 1500 pM, 1400 pM, 1300 pM, 1200 pM, 1100 pM, 1000 pM, 900 pM, 800 pM, 700 pM, 600 pM, 500 pM, 400 pM, 300 pM, 200 pM or 100 pM, preferably when determined by qPCR, more preferably by reverse transcriptase (RT)-qPCR, as described herein.

In a preferred embodiment, when transfected into the cells, the nucleic acid of the invention inhibits expression of the ZPI gene with an IC50 value lower than 2500 pM. In a more preferred embodiment, when transfected into the cells, the nucleic acid of the invention inhibits expression of the ZPI gene with an IC50 value lower than 1000 pM. In an even more preferred embodiment, when transfected into the cells, the nucleic acid of the invention inhibits expression of the ZPI gene with an IC50 value lower than 500 pM. In a most preferred embodiment, when transfected into the cells, the nucleic acid of the invention inhibits expression of the ZPI gene with an IC50 value lower than 100 pM.

Inhibition of expression of the ZPI gene may be quantified by the following method:

Huh7 cells (human hepatocyte-derived cell line, obtained from JCRB Cell Bank) may be maintained in Dulbecco's Modified Eagle Medium (DMEM) supplemented with 10% FBS at 37°C in an atmosphere of 5% CO₂. Cells may then be transfected with siRNA duplexes targeting ZPI mRNA or a negative control siRNA (siRNA-control; sense strand 5'-UUCUCCGAACGUGUCACGUTT-3' (SEQ ID NO:794), antisense strand 5'-ACGUGACACGUUCGGAGAATT-3' (SEQ ID NO:790)) using 10x3-fold serial dilutions over a final duplex concentration range of 20 nM to 1 pM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a RNeasy 96 Kit (Qiagen). Each duplex may be tested by transfection in duplicate wells in a single experiment.

cDNA synthesis may be performed using FastQuant RT (with gDNase) Kit (Tiangen). Real-time quantitative PCR (qPCR) may be performed on an ABI Prism 7900HT or ABI QuantStudio 7 with primers specific for human ZPI (Hs01547819_m1) and human GAPDH (Hs02786624_g1) using FastStart Universal Probe Master Kit (Roche).

qPCR may be performed in duplicate on cDNA derived from each well and the mean cycle threshold (Ct) calculated. Relative ZPI expression may be calculated from mean Ct values using the comparative Ct (ΔΔCt) method, normalised to GAPDH and relative to untreated cells. Maximum percent inhibition of ZPI expression and IC50 values may be calculated using a four parameter (variable slope) model using GraphPad Prism 9.

Alternatively or in addition, inhibition of expression of the ZPI gene may be characterized by a reduction of mean relative expression of the ZPI gene.

In some embodiments, when cells are transfected with 0.1 nM of the nucleic acid of the invention, the mean relative expression of ZPI is below 1, 0.9, 0.8, 0.7, 0.6, 0.5, or 0.4, preferably when determined by qPCR, more preferably by reverse transcriptase (RT)-qPCR, as described herein.

In some embodiments, when cells are transfected with 5 nM of the nucleic acid of the invention, the mean relative expression of ZPI is below 1, 0.9, 0.8, 0.7, 0.6, 0.5, 0.4 or 0.3, preferably when determined by qPCR, more preferably by reverse transcriptase (RT)-qPCR, as described herein.

Mean relative expression of the ZPI gene may be quantified by the following method:

Huh7 cells (human hepatocyte-derived cell line, obtained from JCRB Cell Bank) may be maintained in Dulbecco's Modified Eagle Medium (DMEM) supplemented with 10% FBS at 37°C in at atmosphere of 5% CO₂. Cells may be transfected with siRNA duplexes targeting ZPI mRNA or a negative control siRNA (siRNA-control; sense strand 5'-UUCUCCGAACGUGUCACGUTT-3' (SEQ ID NO:794), antisense strand 5'-ACGUGACACGUUCGGAGAATT-3' (SEQ ID NO:790)) at a final duplex concentration of 5 nM and 0.1 nM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a RNeasy 96 Kit (Qiagen). Each duplex may be tested by transfection in duplicate wells in two independent experiments.

cDNA synthesis may be performed using FastQuant RT (with gDNase) Kit (Tiangen). Real-time quantitative PCR (qPCR) may be performed on an ABI Prism 7900HT or ABI QuantStudio 7 with primers specific for human ZPI (Hs01547819_m1) and human GAPDH (Hs02786624_g1) using FastStart Universal Probe Master Kit (Roche).

qPCR may be performed in duplicate on cDNA derived from each well and the mean Ct calculated. Relative ZPI expression may be calculated from mean Ct values using the comparative Ct (ΔΔCt) method, normalised to GAPDH and relative to untreated cells.

Inhibition of the expression of ZPI gene may be manifested by a reduction of the amount of mRNA of the target ZPI gene in comparison to a suitable control.

In other embodiments, inhibition of the expression of ZPI gene may be assessed in terms of a reduction of a parameter that is functionally linked to gene expression, e.g , protein expression or signaling pathways.

### Methods Of Treating Or Preventing Diseases Associated With ZPI Gene Expression

The present disclosure also provides methods of using nucleic acid e.g. an siRNA of the disclosure or a composition containing nucleic acid e.g. an siRNA of the disclosure to reduce or inhibit ZPI gene expression in a cell. The methods include contacting the cell with a nucleic acid e.g. dsiRNA of the disclosure and maintaining the cell for a time sufficient to obtain degradation of the mRNA transcript of ZPI, thereby inhibiting expression of the ZPI gene in the cell. Reduction in gene expression can be assessed by any methods known in the art.

In the methods of the disclosure the cell may be contacted in vitro or in vivo, i.e., the cell may be within a subject. Said methods are not part of the claimed invention.

A cell suitable for treatment using the methods of the disclosure may be any cell that expresses a gene of interest associated with disease related to a disorder of haemostasis, such as a disease related to a disorder of haemostasis, such as haemophilia.

The in vivo methods of the disclosure may include administering to a subject a composition containing a nucleic acid of the invention e.g. an siRNA, where the nucleic acid e.g. siRNA includes a nucleoside sequence that is complementary to at least a part of an RNA transcript of ZPI gene of the mammal to be treated.

The present disclosure further provides methods of treatment of a subject in need thereof. The treatment methods of the disclosure include administering a nucleic acid such as an siRNA of the disclosure to a subject, e.g., a subject that would benefit from a reduction or inhibition of the expression of ZPI gene, in a therapeutically effective amount e.g. a nucleic acid such as an siRNA targeting ZPI or a pharmaceutical composition comprising the nucleic acid targeting ZPI. The disease to be treated is related to a disorder of haemostasis, such as a disease related to a disorder of haemostasis, such as haemophilia.

Haemophilia, or hemophilia is a mostly inherited genetic disorder that impairs the body's ability to make blood clots, a process needed to stop bleeding. This results in subjects bleeding for a longer time after an injury, easy bruising, and an increased risk of bleeding inside joints or the brain. Subjects with a mild case of the disease may have symptoms only after an accident or during surgery. Bleeding into a joint, also referred to as haemarthrosis, can result in permanent damage while bleeding in the brain can result in long term headaches, seizures, or a decreased level of consciousness.

There are two main types of haemophilia: haemophilia A, which occurs due to low amounts of clotting factor VIII, and haemophilia B, which occurs due to low levels of clotting factor IX. They are typically inherited from one's parents through an X chromosome carrying a nonfunctional gene. Rarely a new mutation may occur during early development or haemophilia may develop later in life due to antibodies forming against a clotting factor. Other types include haemophilia C, which occurs due to low levels of factor XI, Von Willebrand disease, which occurs due to low levels of a substance called von Willebrand factor, and parahaemophilia, which occurs due to low levels of factor V. Haemophilia A, B, and C prevent the intrinsic pathway from functioning properly; this clotting pathway is necessary when there is damage to the endothelium of a blood vessel. Acquired haemophilia is associated with cancers, autoimmune disorders, and pregnancy. Diagnosis is by testing the blood for its ability to clot and its levels of clotting factors.

In certain embodiments, the nucleic acid of the present invention is suitable for treatment, or for treatment of haemophilia A, B and/or C. In certain embodiments, the nucleic acid of the present invention is suitable for treatment, or for treatment of haemophilia A and/or B. In certain embodiments, the nucleic acid of the present invention is suitable for treatment, or for treatment of acquired haemophilia. In certain embodiments, the nucleic acid of the present invention is suitable for treatment, or for treatment of Willebrand disease. In certain embodiments, the nucleic acid of the present invention is suitable for treatment, or for treatment of parahaemophilia.

Without wishing to being bound by theory, treatment with the nucleic acid of the invention results in a boost of clotting factor levels such that bleeding can be reduced or prevented, as demonstrated herein in Fig. 12. Thus, in a preferred embodiment, treatment with the nucleic acid of the invention reduces or prevents bleeding episodes in a subject suffering from haemophilia. In another preferred embodiment, treatment with the nucleic acid of the invention reduces or prevents bleeding into a joint of a subject suffering from haemophilia. In certain embodiments, treatment with the nucleic acid of the invention reduces or prevents bleeding into a muscle or into the brain of a subject suffering from haemophilia.

Alternatively or in addition, treatment of a subject, preferably a subject having a disorder of haemostasis, such as haemophilia, with the nucleic acid of the invention may result in one or more of more of the following:

In certain embodiments, treatment of a subject, preferably a subject having a disorder of haemostasis, such as haemophilia, with the nucleic acid of the invention results in reduced bone marrow hyperplasia. As shown in Fig. 14A, treatment of Haem A mice with a nucleic acid of the invention significantly reduced bone marrow hyperplasia in said mice.

In certain embodiments, treatment of a subject, preferably a subject having a disorder of haemostasis, such as haemophilia, with the nucleic acid of the invention results in reduced osteoarthritis. As shown in Fig. 14B, treatment of Haem A mice with a nucleic acid of the invention significantly reduced osteoarthritis in said mice.

In certain embodiments, treatment of a subject, preferably a subject having a disorder of haemostasis, such as haemophilia, with the nucleic acid of the invention results in reduced chondrocyte degeneration/necrosis. As shown in Fig. 14C, treatment of Haem A mice with a nucleic acid of the invention significantly reduced chondrocyte degeneration/necrosis in said mice.

In certain embodiments, treatment of a subject, preferably a subject having a disorder of haemostasis, such as haemophilia, with the nucleic acid of the invention results in reduced haemorrhage. As shown in Fig. 14D, treatment of Haem A mice with a nucleic acid of the invention significantly reduced haemorrhage in said mice.

**In** certain embodiments, treatment of a subject, preferably a subject having a disorder of haemostasis, such as haemophilia, with the nucleic acid of the invention results in reduced haemosiderin deposition. As shown in Fig. 14E, treatment of Haem A mice with a nucleic acid of the invention significantly reduced haemosiderin deposition in said mice.

**In** certain embodiments, treatment of a subject, preferably a subject having a disorder of haemostasis, such as haemophilia, with the nucleic acid of the invention results in reduced occurrence of haematoma. As shown in Fig. 14F, treatment of Haem A mice with a nucleic acid of the invention significantly reduced haematoma in said mice.

**In** certain embodiments, treatment of a subject, preferably a subject having a disorder of haemostasis, such as haemophilia, with the nucleic acid of the invention results in reduced osteoclastogenic bone resorption. As shown in Fig. 14G, treatment of Haem A mice with a nucleic acid of the invention significantly reduced osteoclastogenic bone resorption in said mice.

In certain embodiments, treatment of a subject, preferably a subject having a disorder of haemostasis, such as haemophilia, with the nucleic acid of the invention results in reduced osteolysis. As shown in Fig. 14H, treatment of Haem A mice with a nucleic acid of the invention significantly reduced osteolysis in said mice.

In certain embodiments, treatment of a subject, preferably a subject having a disorder of haemostasis, such as haemophilia, with the nucleic acid of the invention results in reduced periostitis. As shown in Fig.14I, treatment of Haem A mice with a nucleic acid of the invention significantly reduced periostitis in said mice.

In certain embodiments, treatment of a subject, preferably a subject having a disorder of haemostasis, such as haemophilia, with the nucleic acid of the invention results in reduced sub-chondral bone sclerosis. As shown in Fig. 14J, treatment of Haem A mice with a nucleic acid of the invention significantly reduced sub-chondral bone sclerosis in said mice.

In certain embodiments, treatment of a subject, preferably a subject having a disorder of haemostasis, such as haemophilia, with the nucleic acid of the invention results in reduced tendon degeneration. As shown in Fig. 14K, treatment of Haem A mice with a nucleic acid of the invention significantly reduced tendon degeneration in said mice.

In certain embodiments, treatment of a subject, preferably a subject having a disorder of haemostasis, such as haemophilia, with the nucleic acid of the invention results in reduced tendonitis. As shown in Fig. 14L, treatment of Haem A mice with a nucleic acid of the invention significantly reduced tendonitis in said mice.

In certain embodiments, treatment of a subject, preferably a subject having a disorder of haemostasis, such as haemophilia, with the nucleic acid of the invention results in reduced tenosynovitis. As shown in Fig. 14M, treatment of Haem A mice with a nucleic acid of the invention significantly reduced tenosynovitis in said mice.

Thus, in a particular embodiment, the invention relates to a nucleic acid suitable for use, or for use, in treatment of haemophilia, wherein the treatment of haemophilia is characterized by reduced bleeding and one or more of: reduced bone marrow hyperplasia, reduced osteoarthritis, reduced chondrocyte degeneration/necrosis, reduced haemorrhage, reduced haemosiderin deposition, reduced haematoma, reduced osteoclastogenic bone resorption, reduced osteolysis, reduced periostitis, reduced sub-chondral bone sclerosis, reduced tendon degeneration, reduced tendonitis, and/or reduced tenosynovitis. An nucleic acid e.g. siRNA of the invention may be administered as a "free" nucleic acid or "free siRNA, administered in the absence of a pharmaceutical composition. The naked nucleic acid may be in a suitable buffer solution. The buffer solution may comprise acetate, citrate, prolamine, carbonate, or phosphate, or any combination thereof. In one embodiment, the buffer solution is phosphate buffered saline (PBS). The pH and osmolarity of the buffer solution can be adjusted such that it is suitable for administering to a subject.

Alternatively, a nucleic acid e.g. siRNA of the invention may be administered as a pharmaceutical composition, such as a dsiRNA liposomal formulation.

In one embodiment, the method includes administering a composition featured herein such that expression of ZPI gene is decreased, such as for about 1, 2, 3, 4, 5, 6, 7, 8, 12, 16, 18, 24 hours, 28, 32, or about 36 hours. In one embodiment, expression of ZPI target gene is decreased for an extended duration, e.g., at least about two, three, four days or more, e.g. , about one week, two weeks, three weeks, or four weeks or longer, e.g., about 1 month, 2 months, or 3 months.

Subjects can be administered a therapeutic amount of nucleic acid e.g. siRNA, such as about 0.01 mg/kg to about 200 mg/kg, so as to treat disease related to a disorder of haemostasis, such as a disease related to a disorder of haemostasis, such as haemophilia.

The nucleic acid e.g. siRNA can be administered by intravenous infusion over a period of time, on a regular basis. In certain embodiments, after an initial treatment regimen, the treatments can be administered on a less frequent basis. Administration of the siRNA can reduce gene product levels of ZPI target gene, e.g., in a cell or tissue of the patient by at least about 20%, 25%, 30%, 35%, 40%, 45%, 50%, 55%, 60%, 65%, 70%, 75%, 80%, 85%, 90%, or 95%, or below the level of detection of the assay method used. In certain embodiments, administration results in clinical stabilization or preferably clinically relevant reduction of at least one sign or symptom of a ZPI gene- associated disorder.

Alternatively, the nucleic acid e.g. siRNA can be administered subcutaneously, i.e. , by subcutaneous injection. One or more injections may be used to deliver the desired daily dose of nucleic acid e.g. siRNA to a subject. The injections may be repeated over a period of time. The administration may be repeated on a regular basis. In certain embodiments, after an initial treatment regimen, the treatments can be administered on a less frequent basis. A repeat-dose regimen may include administration of a therapeutic amount of nucleic acid on a regular basis, such as every other day or to once a year. In certain embodiments, the nucleic acid is administered about once per month to about once per quarter (i.e. , about once every three months).

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the purview of this application and scope of the appended claims.

### Example 1: Synthesis of tether 1

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Sfär Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 x 100 mm) at 60 °C. The solvent system consisted of solvent A with H₂O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25 °C. N₂ pressure: 35.1 psi. Filter: Corona.

¹H and ¹³C NMR spectra were recorded at room temperature on a Varian spectrometer at 500 MHz (¹H NMR) and 125 MHz (¹³C NMR). Chemical shifts are given in ppm referenced to the solvent residual peak (CDCl₃ - ¹H NMR: δ at 7.26 ppm and ¹³C NMR δ at 77.2 ppm; DMSO-*d*₆ - 1H NMR: δ at 2.50 ppm and ¹³C NMR δ at 39.5 ppm). Coupling constants are given in Hertz. Signal splitting patterns are described as singlet (s), doublet (d), triplet (t) or multiplet (m).

Synthesis route for the conjugate building block TriGalNAc _Tether1:

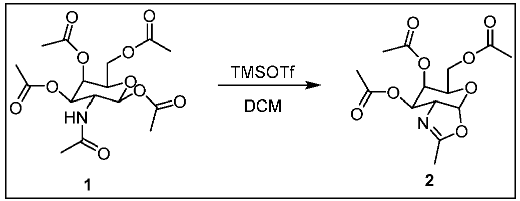

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO3 (100 mL) and water (100 mL). The organic layer was separated, dried over Na₂SO₄ and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

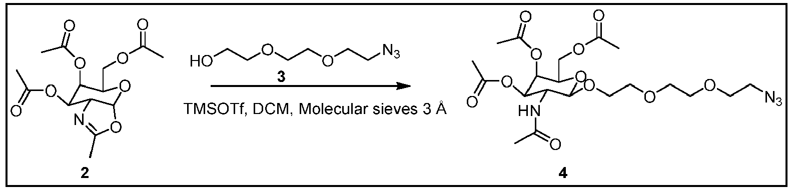

Preparation of compound 4: Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na₂SO₄ and the solvent was removed under reduced pressure. The crude material was purified by flash chromatography (gradient elution: 0-3% MeOH in DCM in 10 CV) to afford the title product as light yellow oil (3.10 g, 88%, rf = 0.25 (2% MeOH in DCM)). MS: calculated for C₂₀H₃₂N₄O₁₁, 504.21. Found 505.4. ¹H NMR (500 MHz, CDCl₃) δ 6.21-6.14 (m, 1H), 5.30 (dd, *J =* 3.4, 1.1 Hz, 1H), 5.04 (dd, *J =* 11.2, 3.4 Hz,1H), 4.76 (d, *J =* 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, *J=* 4.2 Hz, 6H). ¹³C NMR (125 MHz, CDCl₃) δ 170.6 (C), 170.5 (C), 170.4 (C), 170.3 (C), 102.1 (CH), 71.6 (CH), 70.8 (CH), 70.6 (CH), 70.5 (CH), 70.3 (CH₂), 69.7 (CH₂), 68.5 (CH₂), 66.6 (CH₂), 61.5 (CH₂), 23.1 (CH₃), 20.7 (3xCH₃).

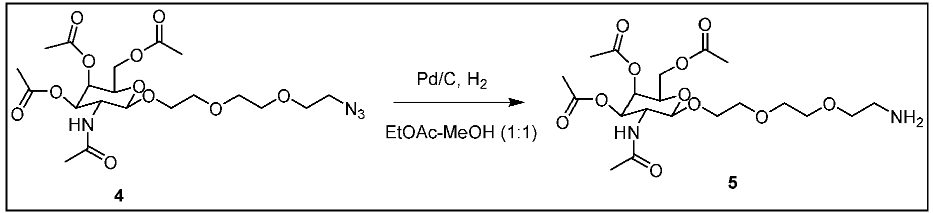

Preparation of compound 5: Compound 4 (1.00 g, 1.98 mmol, 1.0 eq.) was dissolved in a mixture of ethyl acetate (EtOAc) and MeOH (30 mL 1: 1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C_{2oH34}N₂0₁₁, 478.2. Found 479.4.

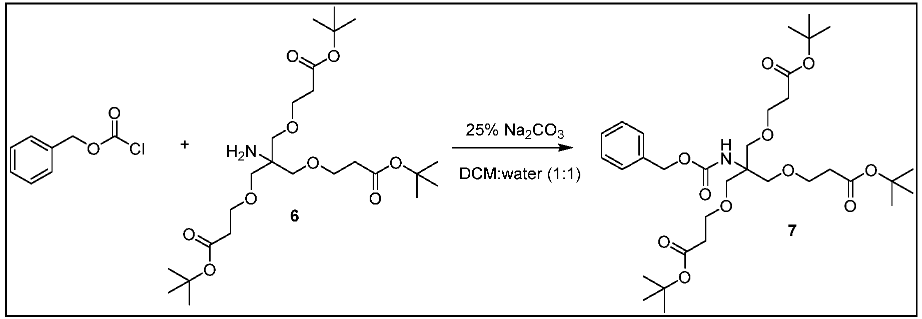

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na₂SO₄. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. ¹H NMR (500 MHz, DMSO-*d₆*) δ 7.38-7.26 (m, 5H), 4.97 (s, 2H), 3.54 (t, 6H), 3.50 (s, 6H), 2.38 (t, 6H), 1.39 (s, 27H). ¹³C NMR (125 MHz, DMSO-*d₆*) δ 170.3 (3xC), 154.5 (C), 137.1 (C), 128.2 (2xCH), 127.7 (CH), 127.6 (2xCH), 79.7 (3xC), 68.4 (3xCH₂), 66.8 (3xCH₂), 64.9 (C), 58.7 (CH₂), 35.8 (3xCH₂), 27.7 (9xCH₃).

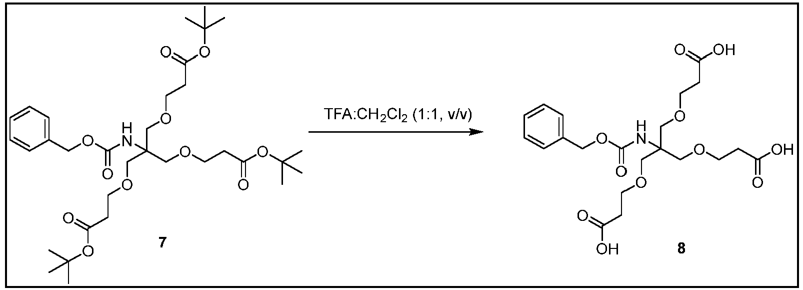

Preparation of compound 8: Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

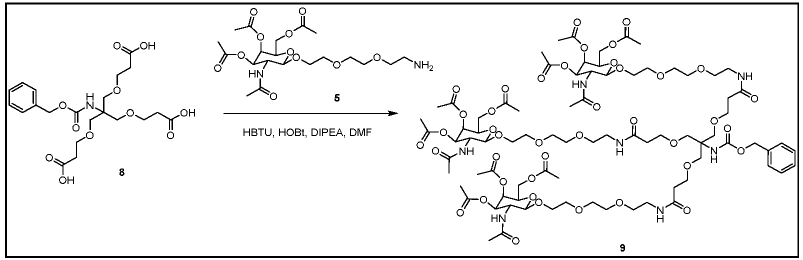

Preparation of compound 9: CbzNH-tris-COOH **8** (0.72 g, 1.49 mmol, 1.0 eq.) and GalNAc-PEG3-NH₂ 5 (3.56 g, 7.44 mmol, 5.0 eq.) were dissolved in *N,N-*dimethylformamide (DMF) (25 mL). Then *N*,*N*,*N'*,*N'*-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and *N,N-*diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO₃ (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. ¹H NMR (500 MHz, DMSO-*d₆*) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). ¹³C NMR (125 MHz, DMSO-*d₆*) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH₂), 69.0 (CH₂), 68.2 (CH₂), 67.2 (CH₂), 66.7 (CH₂), 61.4 (CH₂), 22.6 (CH₂), 22.4 (3xCH₃), 20.7 (9xCH₃).

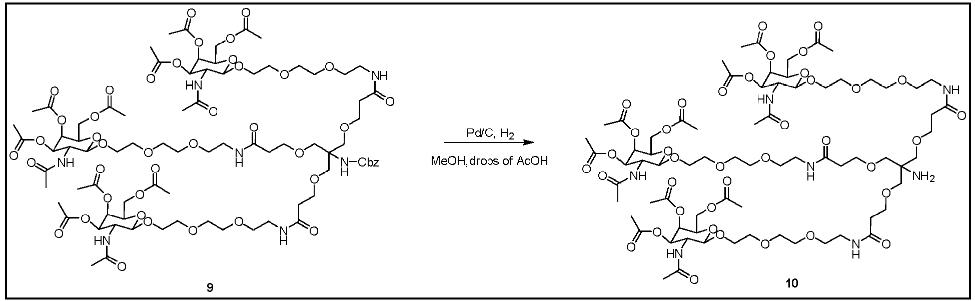

Preparation of compound 10: Triantennary GalNAc compound 9 (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

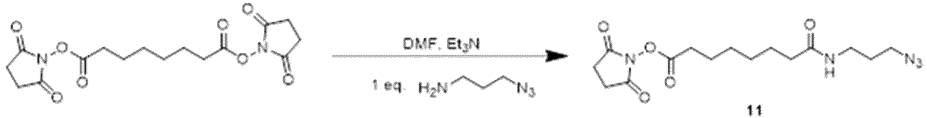

Preparation of compound 11: Commercially available suberic acid bis(N-hydroxysuccinimide ester) (3.67 g, 9.9 mmol, 1.0 eq.) was dissolved in DMF (5 mL) and triethylamine (1.2 mL) was added. To this solution was added dropwise a solution of 3-azido-1-propylamine (1.0 g, 9.9 mmol, 1.0 eq.) in DMF (5 mL). The reaction was stirred at room temperature for 3 h. The reaction mixture was diluted with EtOAc (100 mL) and washed with water (50 mL). The organic layer was separated, dried over Na₂SO₄ and the solvent was removed under reduced pressure. The crude material was purified by flash chromatography (gradient elution: 0-5% MeOH in DCM in 16 CV). The product was obtained as white solid (1.54 g, 43%, rf = 0.71 (5% MeOH in DCM)). MS: calculated for C₁₅H₂₃N₅O₅, 353.4. Found 354.3.

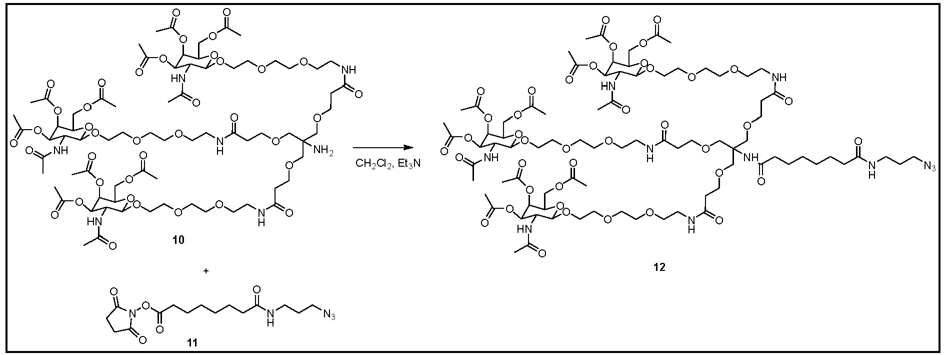

Preparation of TriGalNAc (12): Triantennary GalNAc compound 10 (0.35 g, 0.24 mmol, 1.0 eq.) and compound 11 (0.11 g, 0.31 mmol, 1.5 eq.) were dissolved in DCM (5 mL) under argon and triethylamine (0.1 mL, 0.61 mmol, 3.0 eq.) was added. The reaction was stirred at room temperature overnight. The solvent was removed under reduced pressure, the residue was dissolved in EtOAc (100 mL) and washed with water (100 mL). The organic layer was separated and dried over Na₂SO₄. The solvent was evaporated and the resulting crude material was purified by flash chromatography (elution gradient: 0-10% MeOH in DCM in 20 CV) to afford the title compound as white fluffy solid (0.27 g, 67%, rf = 0.5 (10% MeOH in DCM)). MS: calculated for C₈₄H₁₃₇N₁₁O₄₁, 1957.1. Found 1959.6.

### Conjugation of Tether 1 to a siRNA strand: Monofluoro cyclooctyne (MFCO) conjugation at 5'-or 3'-end

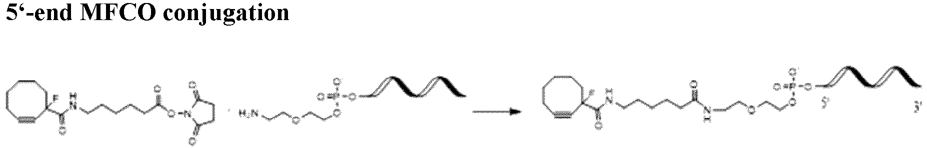

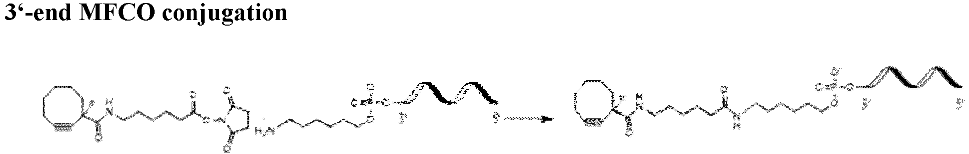

General conditions for MFCO conjugation: Amine-modified single strand was dissolved at 700 OD/mL in 50 mM carbonate/bicarbonate buffer pH 9.6/dimethyl sulfoxide (DMSO) 4:6 (v/v) and to this solution was added one molar equivalent of a 35 mM solution of MFCO-C6-NHS ester (Berry&Associates, Cat. # LK 4300) in DMF. The reaction was carried out at room temperature and after 1 h another molar equivalent of the MFCO solution was added. The reaction was allowed to proceed for an additional hour and was monitored by LC/MS. At least two molar equivalent excess of the MFCO NHS ester reagent relative to the amino modified oligonucleotide were needed to achieve quantitative consumption of the starting material. The reaction mixture was diluted 15-fold with water, filtered through a 1.2 µm filter from Sartorius and then purified by reserve phase (RP HPLC) on an Äkta Pure instrument (GE Healthcare).

Purification was performed using a XBridge C18 Prep 19 x 50 mm column from Waters. Buffer A was 100 mM TEAAc pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full length conjugated oligonucleotide were pooled, precipitated in the freezer with 3 M NaOAc, pH 5.2 and 85% ethanol and the collected pellet was dissolved in water. Samples were desalted by size exclusion chromatography and concentrated using a speed-vac concentrator to yield the conjugated oligonucleotide in an isolated yield of 40-80%.

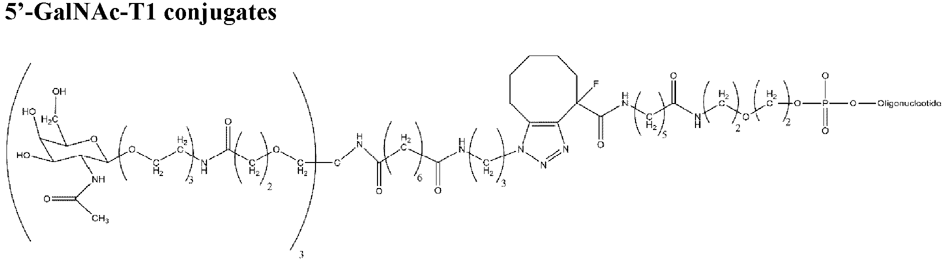

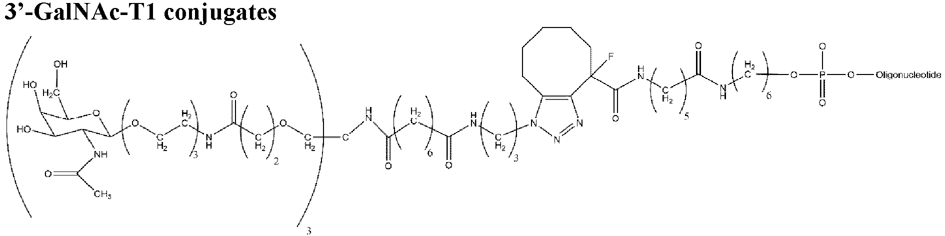

General procedure for TriGalNAc conjugation: MFCO-modified single strand was dissolved at 2000 OD/mL in water and to this solution was added one equivalent solution of compound 12 (10 mM) in DMF. The reaction was carried out at room temperature and after 3 h 0.7 molar equivalent of the compound 12 solution was added. The reaction was allowed to proceed overnight and completion was monitored by LCMS. The conjugate was diluted 15-fold in water, filtered through a 1.2 µm filter from Sartorius and then purified by RP HPLC on an Äkta Pure instrument (GE Healthcare).

RP HPLC purification was performed using a XBridge C18 Prep 19 x 50 mm column from Waters. Buffer A was 100 mM triethylammonium acetate pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleotide were pooled, precipitated in the freezer with 3 M NaOAc, pH 5.2 and 85% ethanol and the collected pellet was dissolved in water to give an oligonucleotide solution of about 1000 OD/mL. The O-acetates were removed by adding 20% aqueous ammonia. Quantitative removal of these protecting groups was verified by LC-MS.

The conjugates were desalted by size exclusion chromatography using Sephadex G25 Fine resin (GE Healthcare) on an Äkta Pure (GE Healthcare) instrument to yield the conjugated oligonucleotides in an isolated yield of 50-70%.

The following schemes further set out the routes of synthesis:

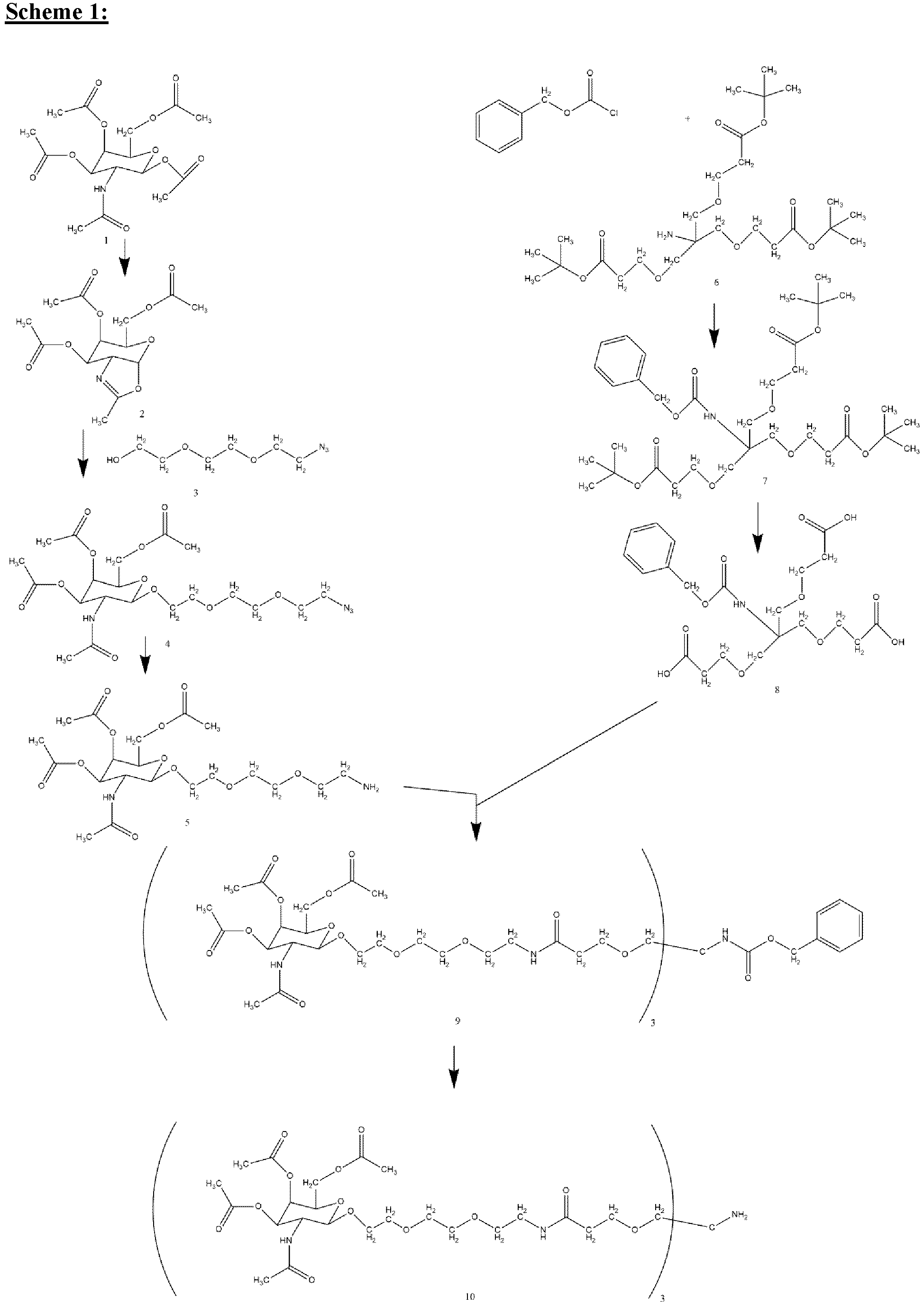

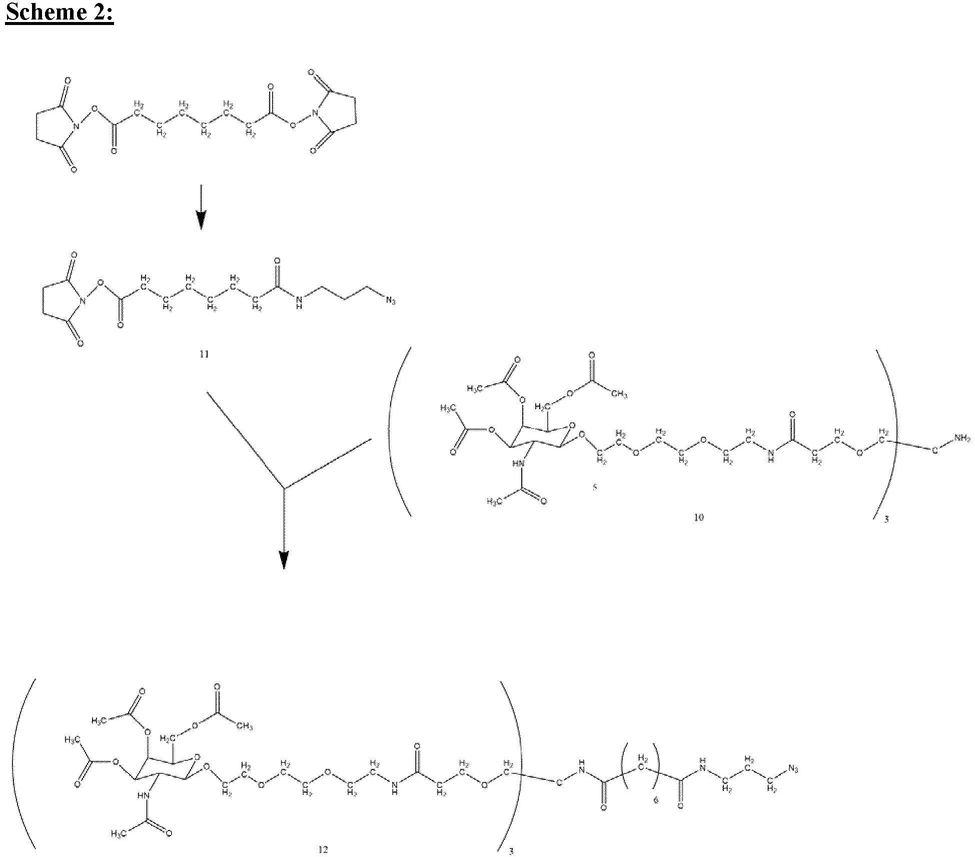

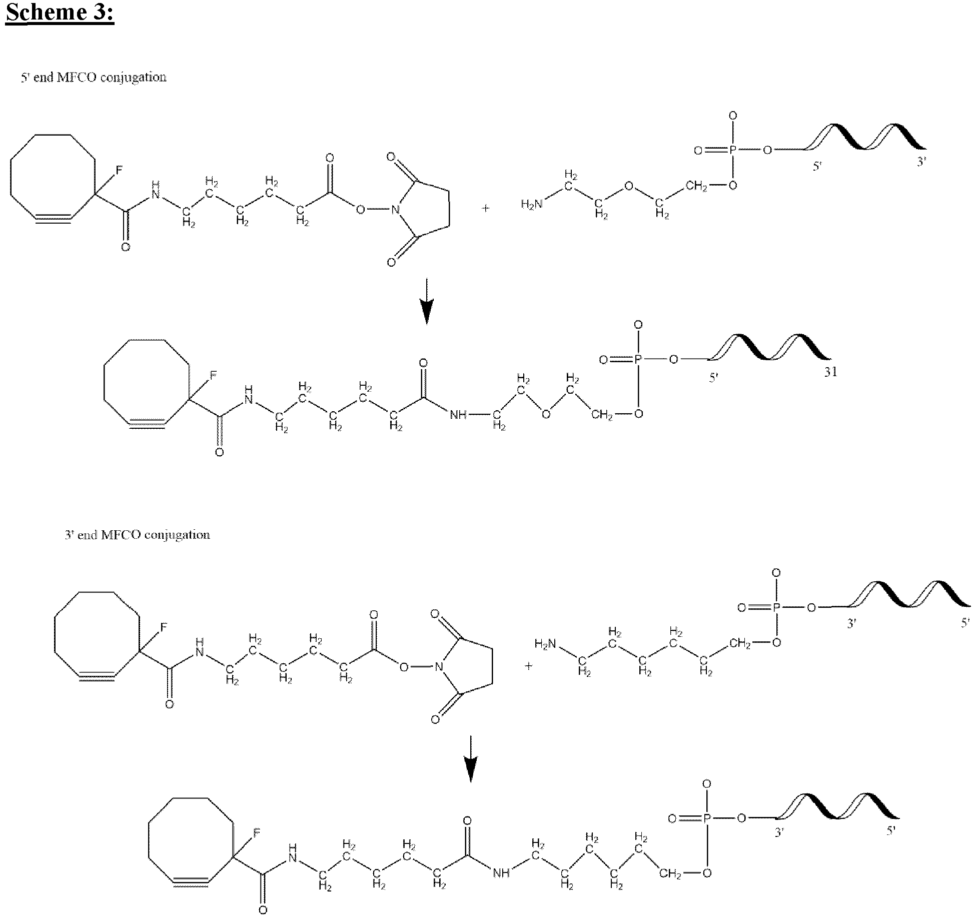

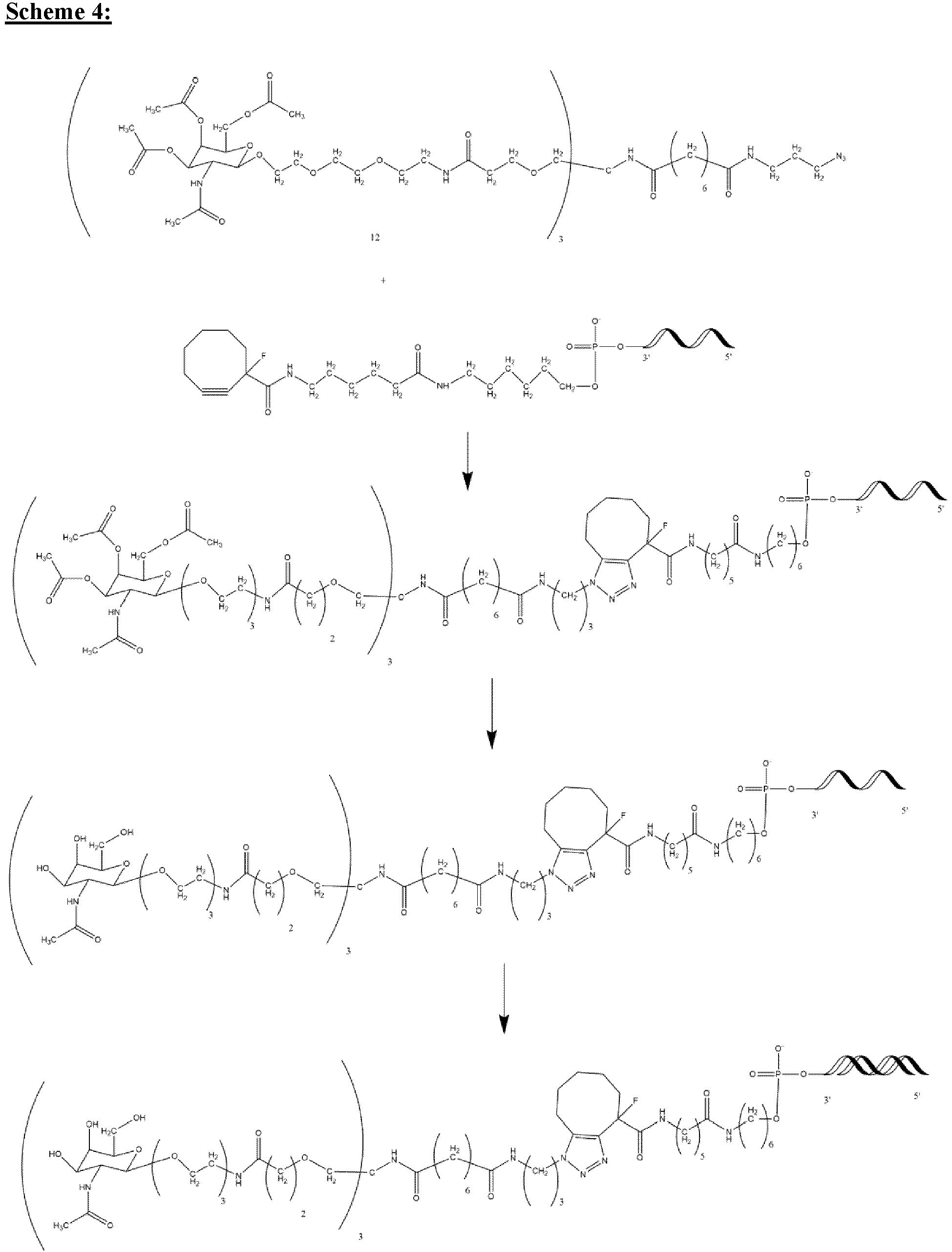

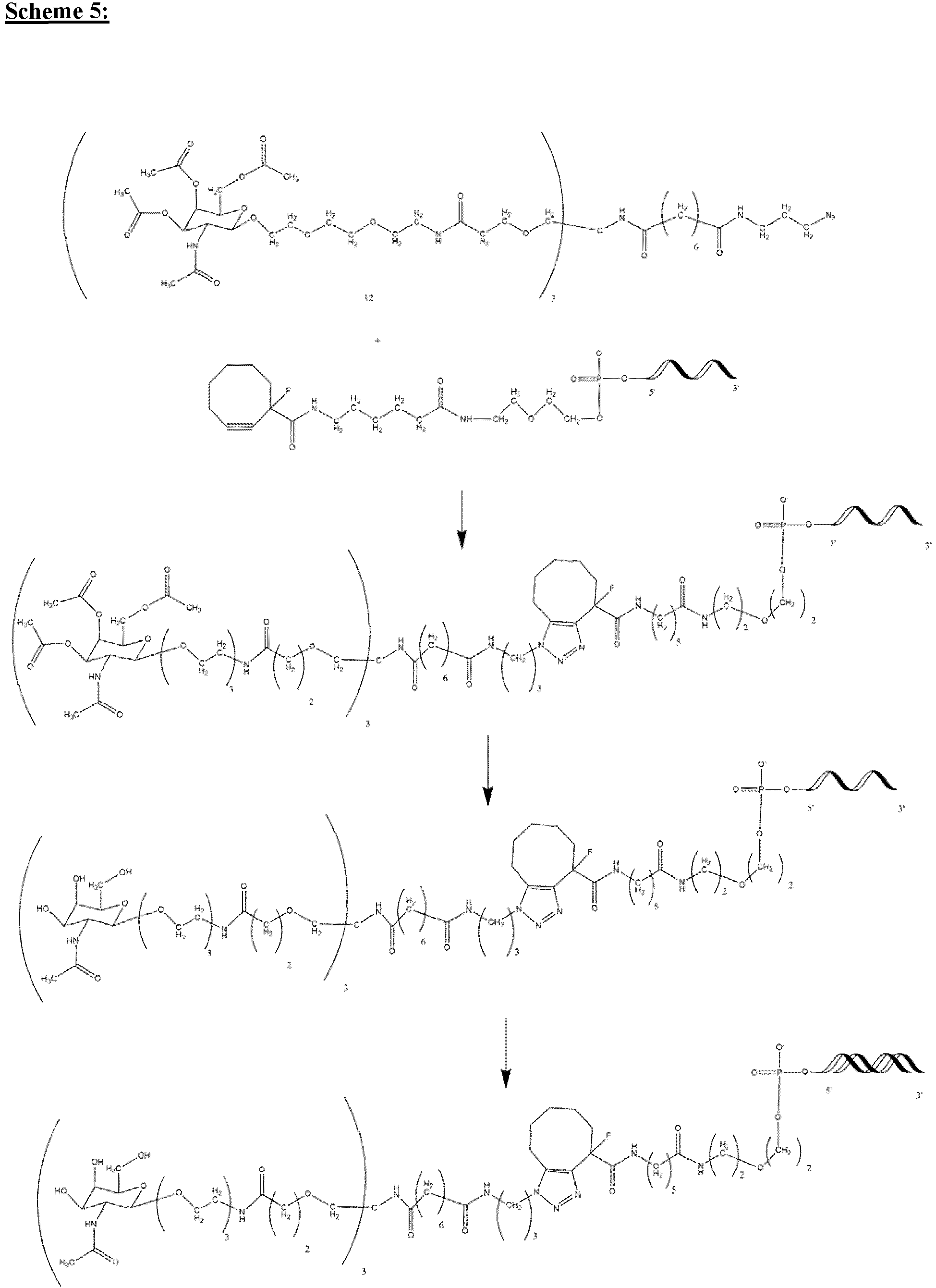

### Example 2: Duplex Annealing

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### Example 3: Synthesis of tether 2

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Sfär Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 x 100 mm) at 60 °C. The solvent system consisted of solvent A with H₂O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25°C. N₂ pressure: 35.1 psi. Filter: Corona.

¹H and ¹³C NMR spectra were recorded at room temperature on a Varian spectrometer at 500 MHz (¹H NMR) and 125 MHz (¹³C NMR). Chemical shifts are given in ppm referenced to the solvent residual peak (CDCl₃ - ¹H NMR: δ at 7.26 ppm and ¹³C NMR δ at 77.2 ppm; DMSO-*d*₆ - ¹H NMR: δ at 2.50 ppm and ¹³C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc_Tether2:

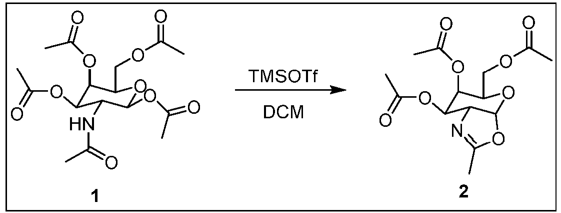

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO₃ (100 mL) and water (100 mL). The organic layer was separated, dried over Na₂SO₄, and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

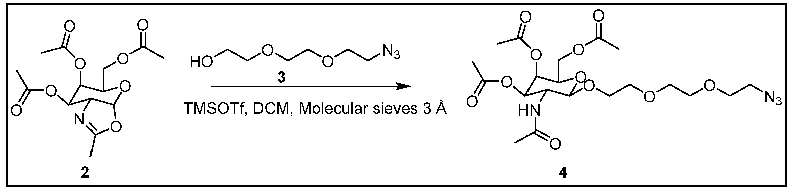

Preparation of compound 4: Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na₂SO₄ and the solvent was removed under reduced pressure. The crude material was purified by flash chromatography (gradient elution: 0-3% MeOH in DCM in 10 CV) to afford the title product as light-yellow oil (3.10 g, 88%, rf = 0.25 (2% MeOH in DCM)). MS: calculated for C₂₀H₃₂N₄O₁₁, 504.21. Found 505.4. ¹H NMR (500 MHz, CDCl₃) δ 6.21-6.14 (m, 1H), 5.30 (dd, *J =* 3.4, 1.1 Hz, 1H), 5.04 (dd, *J =* 11.2, 3.4 Hz,1H), 4.76 (d, *J =* 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, *J =* 4.2 Hz, 6H). ¹³C NMR (125 MHz, CDCl₃) δ 170.6 (C), 170.5 (C), 170.4 (C), 170.3 (C), 102.1 (CH), 71.6 (CH), 70.8 (CH), 70.6 (CH), 70.5 (CH), 70.3 (CH₂), 69.7 (CH₂), 68.5 (CH₂), 66.6 (CH₂), 61.5 (CH₂), 23.1 (CH₃), 20.7 (3xCH₃).

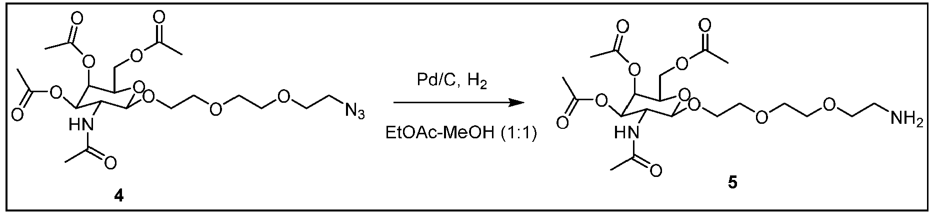

Preparation of compound 5: Compound 4 (1.00 g, 1.98 mmol, 1.0 eq.) was dissolved in a mixture of ethyl acetate (EtOAc) and MeOH (30 mL 1:1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

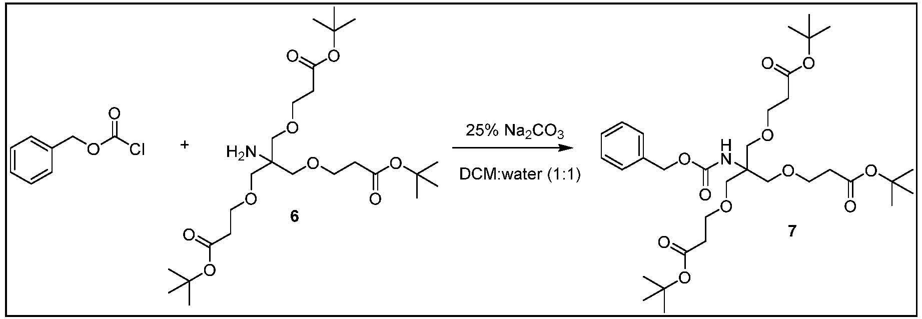

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na₂SO₄. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. ¹H NMR (500 MHz, DMSO-*d₆*) δ 7.38-7.26 (m, 5H), 4.97 (s, 2H), 3.54 (t, 6H), 3.50 (s, 6H), 2.38 (t, 6H), 1.39 (s, 27H). ¹³C NMR (125 MHz, DMSO-*d₆*) δ 170.3 (3xC), 154.5 (C), 137.1 (C), 128.2 (2xCH), 127.7 (CH), 127.6 (2xCH), 79.7 (3xC), 68.4 (3xCH₂), 66.8 (3xCH₂), 64.9 (C), 58.7 (CH₂), 35.8 (3xCH₂), 27.7 (9xCH₃).

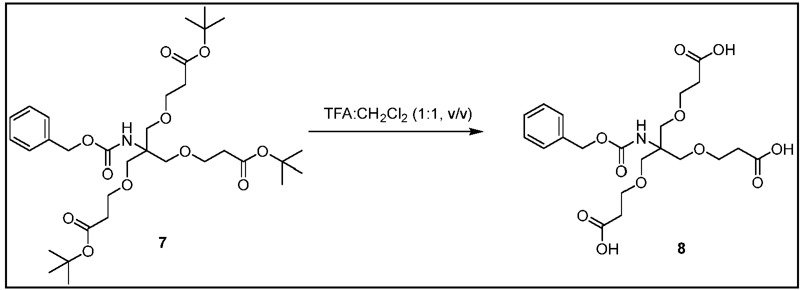

Preparation of compound 8: Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

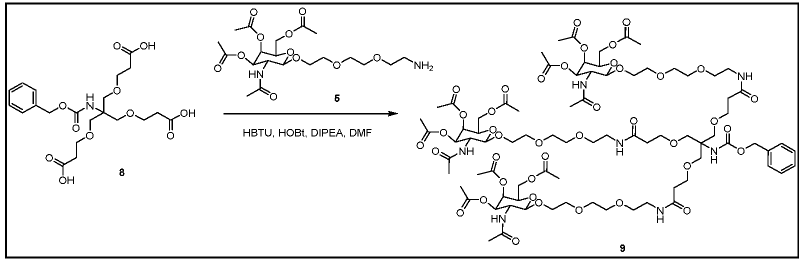

Preparation of compound 9: CbzNH-tris-COOH 8 (0.72 g, 1.49 mmol, 1.0 eq.) and GalNAc-PEG3-NH₂ 5 (3.56 g, 7.44 mmol, 5.0 eq.) were dissolved in *N,N-*dimethylformamide (DMF) (25 mL). Then *N*,*N*,*N'*,*N'*-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and *N,N-*diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO₃ (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. ¹H NMR (500 MHz, DMSO-*d₆*) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). ¹³C NMR (125 MHz, DMSO-*d₆*) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH₂), 69.0 (CH₂), 68.2 (CH₂), 67.2 (CH₂), 66.7 (CH₂), 61.4 (CH₂), 22.6 (CH₂), 22.4 (3xCH₃), 20.7 (9xCH₃).

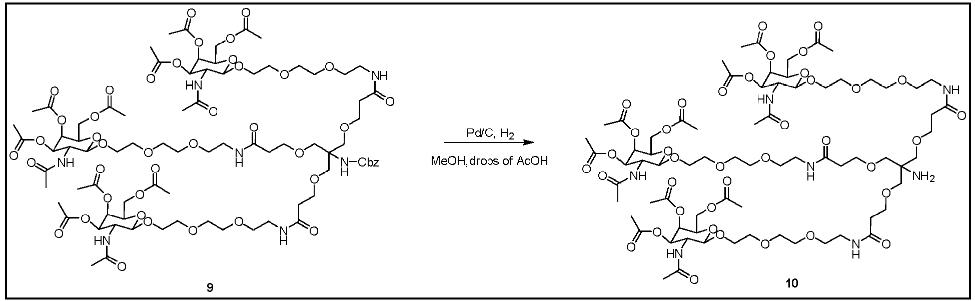

Preparation of compound 10: Triantennary GalNAc compound 9 (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated, and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

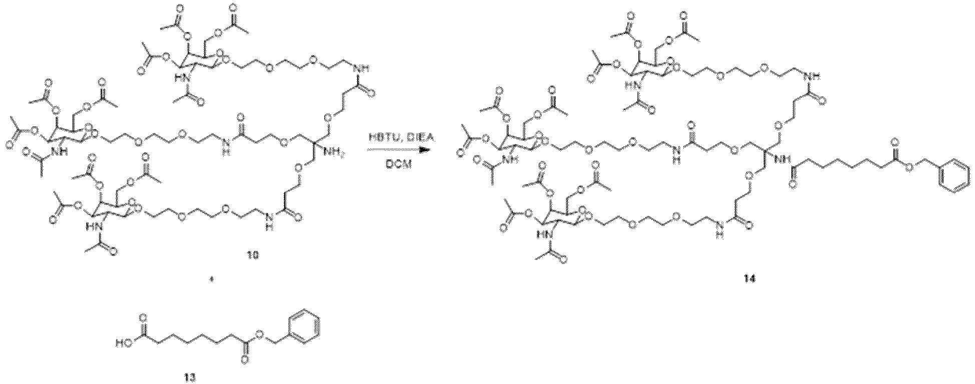

Preparation of compound 14: Triantennary GalNAc compound 10 (0.45 g, 0.26 mmol, 1.0 eq.), HBTU (0.19 g, 0.53 mmol, 2.0 eq.) and DIPEA (0.23 mL, 1.3 mmol, 5.0 eq.) were dissolved in DCM (10 mL) under argon. To this mixture, it was added dropwise a solution of compound 13 (0.14 g, 0.53 mmol, 2.0 eq.) in DCM (5 mL). The reaction was stirred at room temperature overnight. The solvent was removed, and the residue was dissolved in EtOAc (50 mL), washed with water (50 mL) and dried over Na₂SO₄. The solvent was evaporated, and the crude material was purified by flash chromatography (gradient elution: 0-5% MeOH in DCM in 20 CV). The product was obtained as white fluffy solid (0.25 g, 48%, rf = 0.4 (10% MeOH in DCM)). MS: calculated for C88H137N7O42, 1965.1. Found 1965.6.

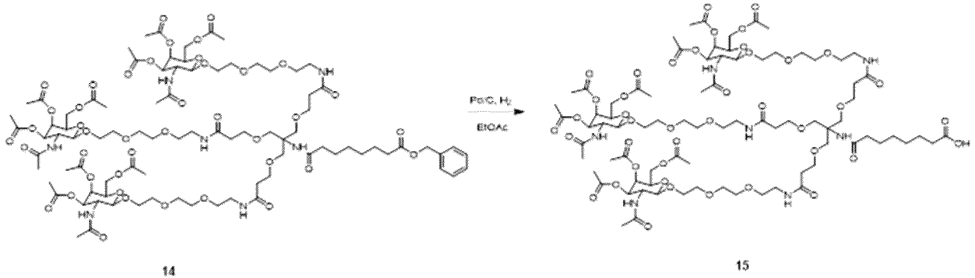

Preparation of TriGalNAc (15): Triantennary GalNAc compound 14 (0.31 g, 0.15 mmol, 1.0 eq.) was dissolved in EtOAc (15 mL) and Pd/C (40 mg) was added. The reaction mixture was degassed by using vacuum/argon cycles (3x) and hydrogenated under balloon pressure overnight. The completion of the reaction was monitored by mass spectrometry and the resulting mixture was filtered through a thin pad of celite. The solvent was removed under reduced pressure and the resulting residue was dried under high vacuum overnight. The residue was used for conjugations to oligonucleosides without further purification (0.28 g, quantitative yield). MS: calculated for C₈₁H₁₃₁N₇O₄₂, 1874.9. Found 1875.3.

### Conjugation of Tether 2 to a siRNA strand: TriGalNAc tether 2 (GalNAc-T2) conjugation at 5'-end or 3'-end

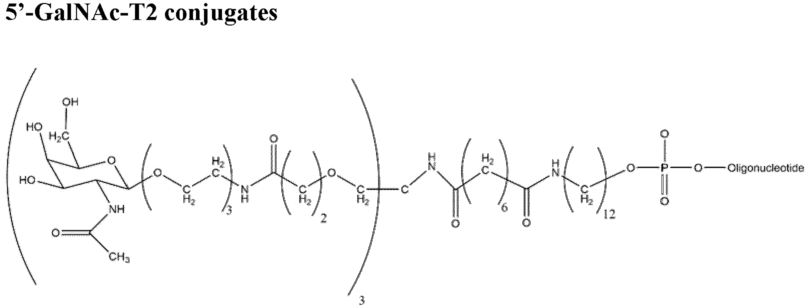

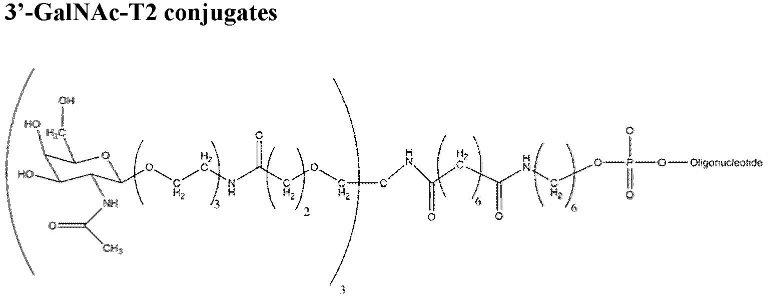

Preparation of TriGalNAc tether 2 NHS ester: To a solution of carboxylic acid tether 2 (compound 15, 227 mg, 121 µmol) in DMF (2.1 mL), N-hydroxysuccinimide (NHS) (15.3 mg, 133 µmol) and N,N'-diisopropylcarbodiimide (DIC) (19.7 µL, 127 µmol) were added. The solution was stirred at room temperature for 18 h and used without purification for the subsequent conjugation reactions.

General procedure for triGalNAc tether 2 conjugation: Amine-modified single strand was dissolved at 700 OD/mL in 50 mM carbonate/bicarbonate buffer pH 9.6/DMSO 4:6 (v/v) and to this solution was added one molar equivalent of Tether 2 NHS ester (57 mM) solution in DMF. The reaction was carried out at room temperature and after 1 h another molar equivalent of the NHS ester solution was added. The reaction was allowed to proceed for one more hour and reaction progress was monitored by LCMS. At least two molar equivalent excess of the NHS ester reagent relative to the amino modified oligonucleoside were needed to achieve quantitative consumption of the starting material. The reaction mixture was diluted 15-fold with water, filtered once through 1.2 µm filter from Sartorius and then purified by reserve phase (RP HPLC) on an Äkta Pure (GE Healthcare) instrument.

The purification was performed using a XBridge C18 Prep 19 x 50 mm column from Waters. Buffer A was 100 mM TEAA pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleosides were pooled together, precipitated in the freezer with 3 M NaOAc, pH 5.2 and 85% ethanol and then dissolved at 1000 OD/mL in water. The O-acetates were removed with 20% ammonium hydroxide in water until completion (monitored by LC-MS).

The conjugates were desalted by size exclusion chromatography using Sephadex G25 Fine resin (GE Healthcare) on an Äkta Pure (GE Healthcare) instrument to yield the conjugated oligonucleotides in an isolated yield of 60-80%.

The conjugates were characterized by HPLC-MS analysis with a 2.1 x 50 mm XBridge C18 column (Waters) on a Dionex Ultimate 3000 (Thermo Fisher Scientific) HPLC system equipped with a Compact ESI-Qq-TOF mass spectrometer (Bruker Daltonics). Buffer A was 16.3 mM triethylamine, 100 mM HFIP in 1% MeOH in H2O and buffer B contained 95% MeOH in buffer A. A flow rate of 250 µL/min and a temperature of 60°C were employed. UV traces at 260 and 280 nm were recorded. A gradient of 1-100% B within 31 min was employed.

The following schemes further set out the routes of synthesis:

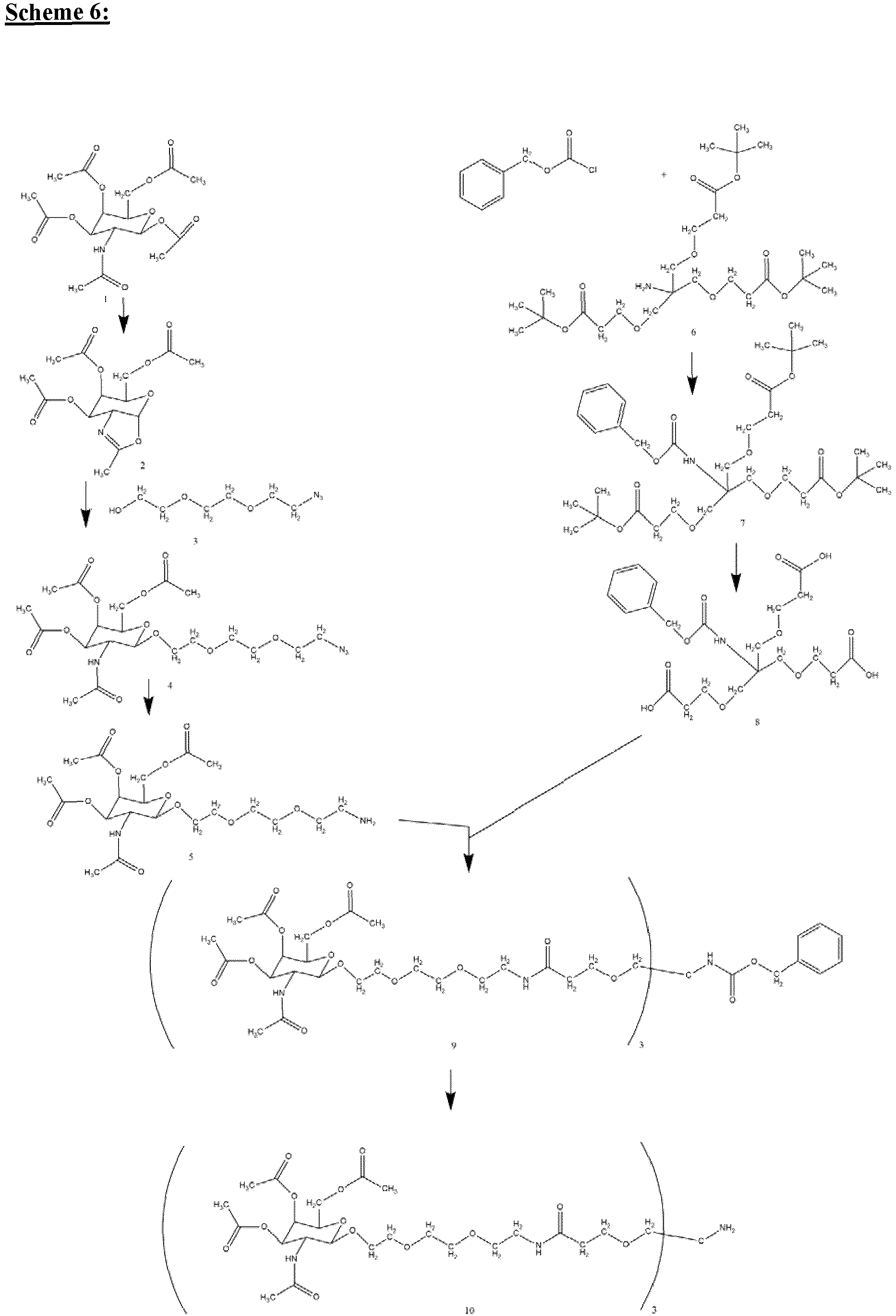

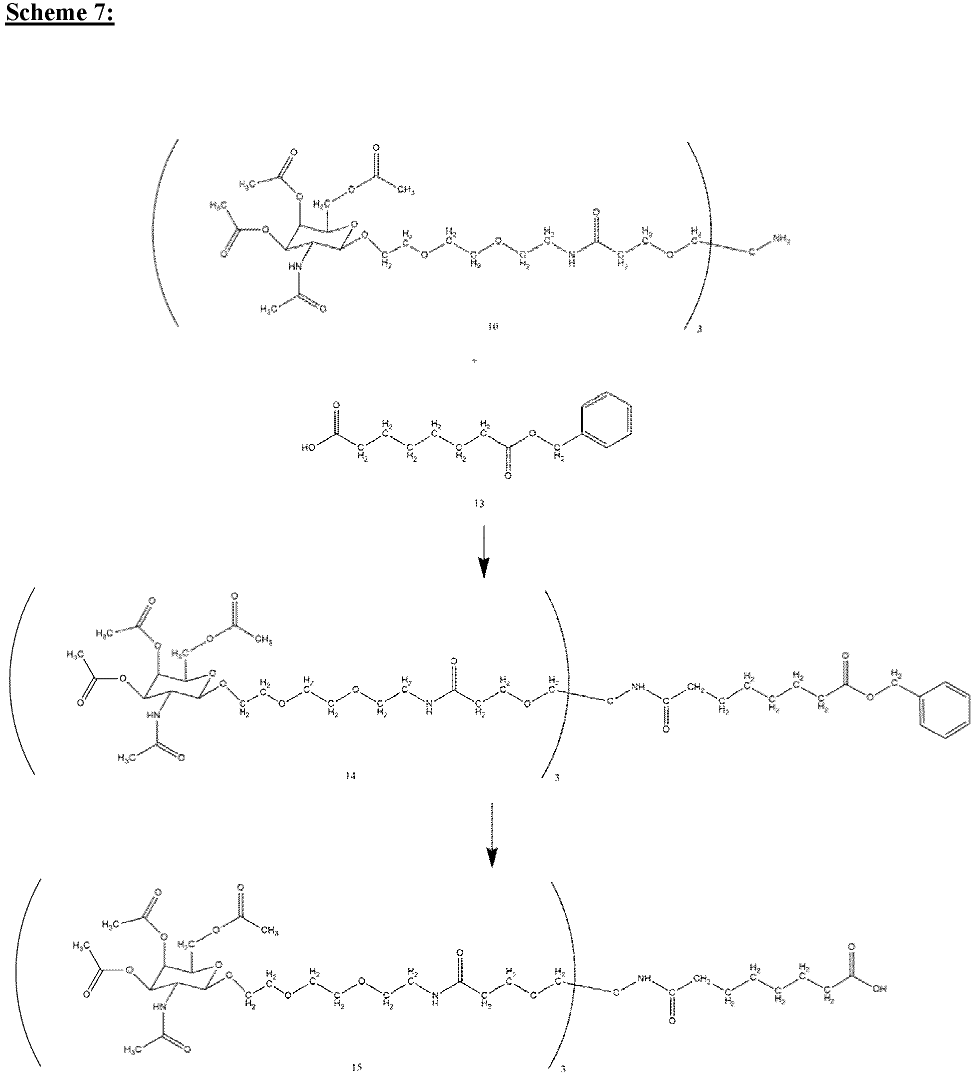

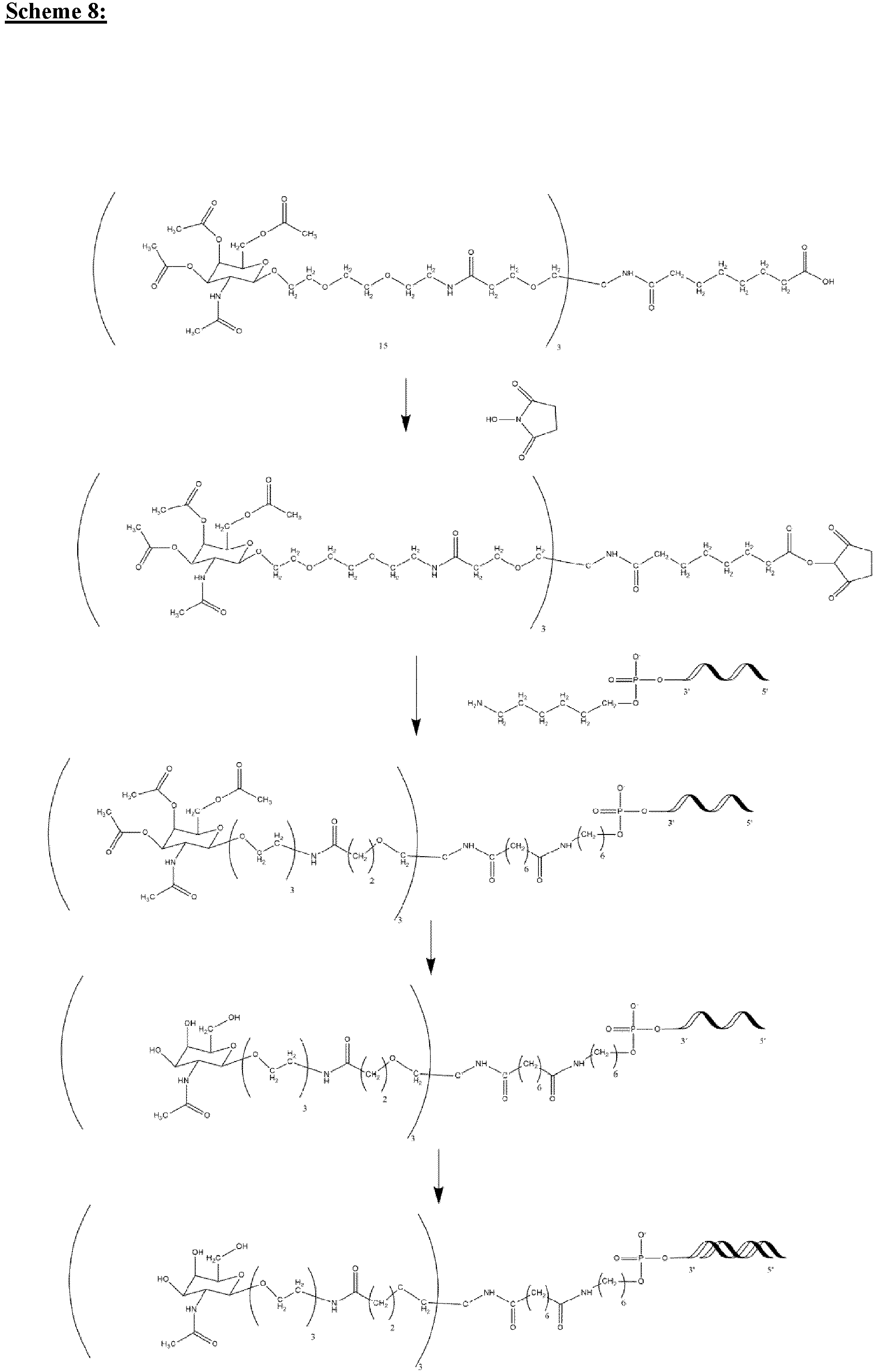

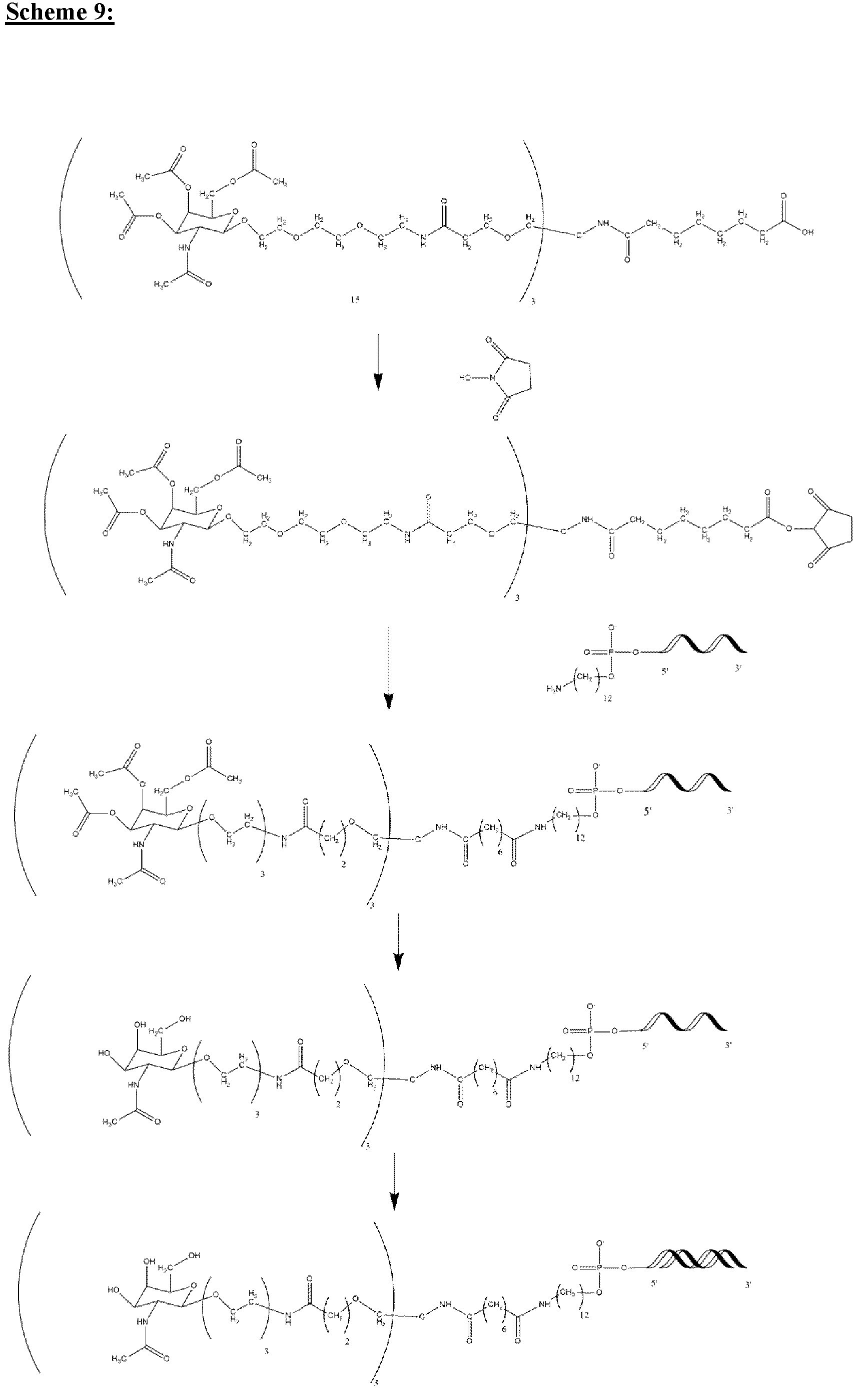

### Example 4: Duplex Annealing

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### Example 5: Alternative synthesis route for the conjugate building block TriGalNAc _Tether2:

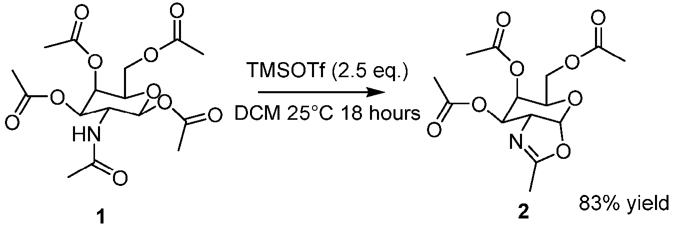

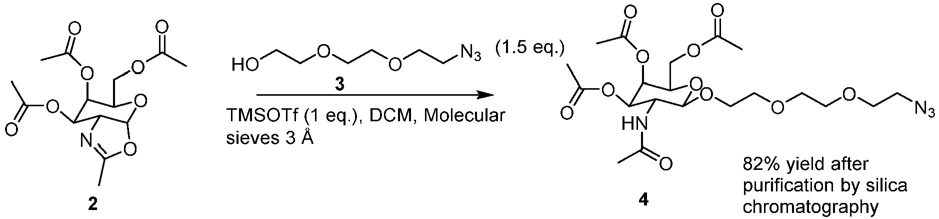

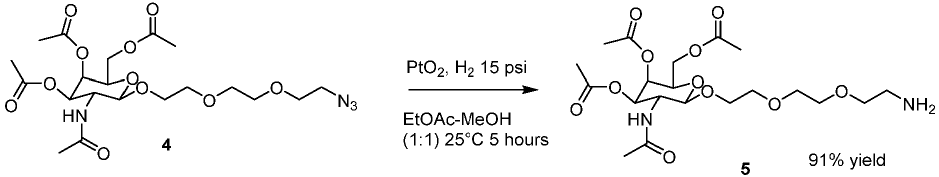

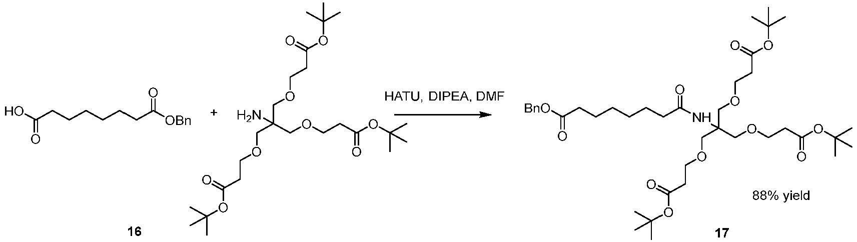

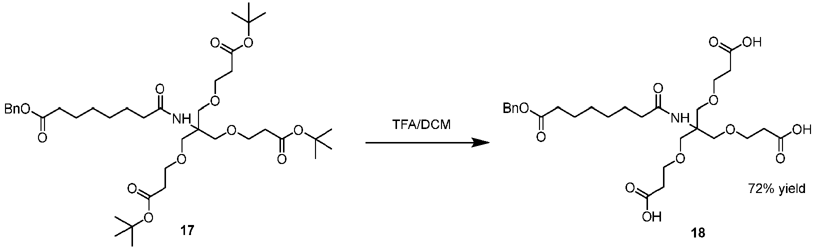

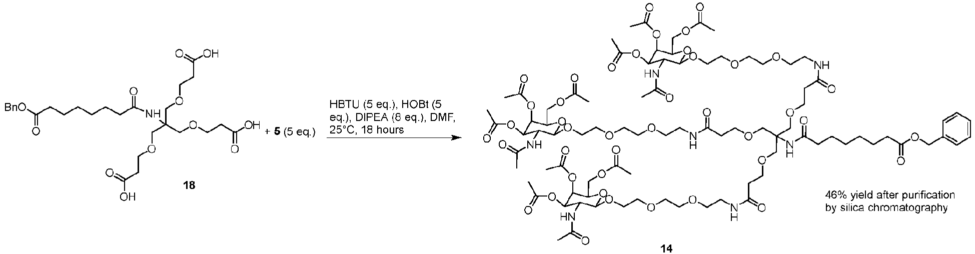

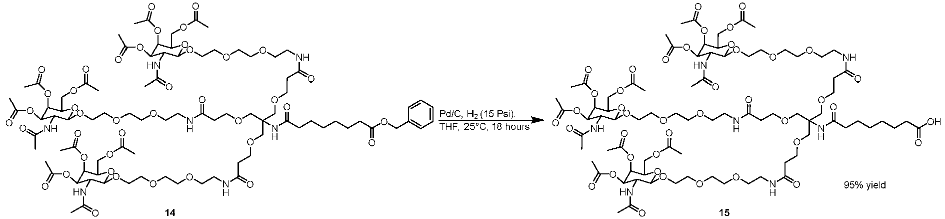

### Conjugation of Tether 2 to a siRNA strand: TriGalNAc tether 2 (GalNAc-T2) conjugation at 5'-end or 3'-end

### Conjugation conditions

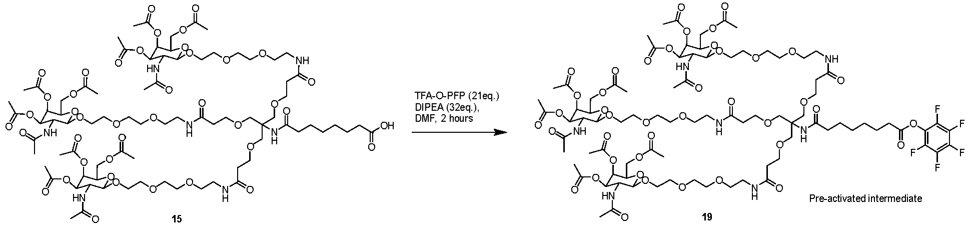

**Pre-activation:** To a solution of compound 15 (16 umol, 4 eq.) in DMF (160 µL) was added TFA-O-PFP (15 µl, 21 eq.) followed by DIPEA (23 µl, 32 eq.) at 25°C. The tube was shaken for 2 h at 25°C. The reaction was quenched with H₂O (10 µL).

**Coupling:** The resulting mixture was diluted with DMF (400 µl), followed by addition of oligo-amine solution (4.0 µmol in 10 x PBS, pH 7.4, 500 µL; final oligo concentration in organic and aqueous solution: 4 µmol/ml = 4 mM). The tube was shaken at 25°C for 16 h and the reaction was analysed by LCMS. The resulting mixture was treated with 28% NH₄OH (4.5 ml) and shaken for 2 h at 25°C. The mixture was analysed by LCMS, concentrated, and purified by IP-RP HPLC to produce the oligonucleotides conjugated to tether 2 GalNAc.

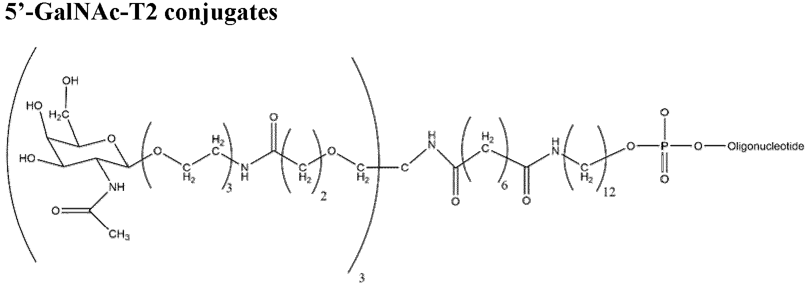

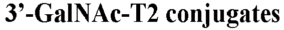

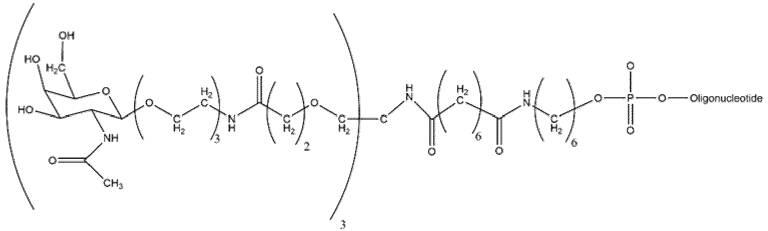

### Example 6: Solid phase synthesis method: scale ≤1µmol

Syntheses of siRNA sense and antisense strands were performed on a MerMade192X synthesiser with commercially available solid supports made of controlled pore glass with universal linker (Universal CPG, with a loading of 40 µmol/g; LGC Biosearch or Glen Research).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H20 (DNAchem) was used as oxidizing reagent. Thiolation for phosphorothioate linkages was performed with 0.2 M PADS (TCI) in acetonitrile/pyridine 1:1 v/v. 5-Ethyl thiotetrazole (ETT), 0.25M mM in acetonitrile was used as activator solution.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

At each cycle, the DMT was removed by deblock solution, 3% TCA in DCM (DNAchem).

The coupling time was 180 seconds. The oxidizer contact time was set to 80 seconds and thiolation time was 2*100 seconds.

At the end of the synthesis, the oligonucleotides were cleaved from the solid support using a NH₄OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by ultracentrifugation using Amicon Ultra-2 Centrifugal Filter Unit; PBS buffer (10x, Teknova, pH 7.4, Sterile) or by EtOH precipitation from 1M sodium acetate.

The single strands identity were assessed by MS ESI- and then, were annealed in water to form the final duplex siRNA and duplex purity were assessed by size exclusion chromatography.

### Example 7: Solid phase synthesis method: scale ≥5 µmol

Syntheses of siRNA sense and antisense strands were performed on a MerMade12 synthesiser with commercially available solid supports made of controlled pore glass with universal linker (Universal CPG, with a loading of 40 µmol/g; LGC Biosearch or Glen Research) at 5 µmol scale. Sense strand destined to 3' conjugation were sytnthesised at 12 µmol on 3'-PT-Amino-Modifier C6 CPG 500 Å solid support with a loading of 86 µmol/g (LGC).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H₂O (DNAchem) was used as oxidizing reagent. Thiolation for phosphorothioate linkages was performed with 0.2 M PADS (TCI) in acetonitrile/pyridine 1:1 v/v. 5-Ethyl thiotetrazole (ETT), 0.25M mM in acetonitrile was used as activator solution.

At each cycle, the DMT was removed by deblock solution, 3% TCA in DCM (DNAchem).

For strands synthesised on universal CPG the coupling was performed with 8 eq. of amidite for 130 seconds. The oxidation time was 47 seconds, the thiolation time was 210 seconds.

For strands synthesised on 3'-PT-Amino-Modifier C6 CPG the coupling was performed with 8 eq. of amidite for 2*150 seconds. The oxidation time was 47 seconds, the thiolation time was 250 seconds

At the end of the synthesis, the oligonucleotides were cleaved from the solid support using a NH₄OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by EtOH precipitation from 1M sodium acetate.

The single strand oligonucleotides were purified by IP-RP HPLC on Xbridge BEH C18 5 µm, 130 Å, 19x150 mm (Waters) column with an increasing gradient of B in A. Mobile phase A: 240 mM HFIP, 7 mM TEA and 5% methanol in water; mobile phase B: 240 mM HFIP, 7 mM TEA in methanol.

The single strands purity and identity were assessed by UPLC/MS ESI- on Xbridge BEH C18 2.5 µm, 3x50 mm (Waters) column with an increasing gradient of B in A. Mobile phase A: 100 mM HFIP, 5 mM TEA in water; mobile phase B: 20% mobile phase A: 80% Acetonitrile (v/v).

Sense strand were conjugated as per protocols provided in any of examples 1, 3 or 5.

Sense and Antisense strands were then annealed in water to form the final duplex siRNA and duplex purity were assessed by size exclusion chromatography.

### Example 8: Nucleic acid sequences

siRNA oligonucleosides according to the present invention target ZPI. The full DNA sequence of the ZPI target is as follows (SEQ ID NO: 1):

AATGTGGGTTGGAGCCCCCATACAGAATCTCTATGGGGGCACTGCCTAGTGGAGCTGTGAGAAGACG
GCCACCGTCCTCCAGACCCCTGAATGGTAGATCCACCGACAGCTTGCGCCATTTATCCGGAAAAGCCA
CAGACACTCAACGCCAGCCCGTGAAAGCAGCCAGGAGGGAGGCTGTACCCTGCAAAGCCACAGGGGC
AGAGCTGCCCAAGACCAAGGGAAGCTACCTTTTGCATCAACGTGACCTGGACTCAAAGGAGATCATTT
TGGAGCTTTAAAATTTGACTGACCTGCTGGATTTCAGACTTGCATGGGCCCTGTAACCACTTCGTTTAG
GCCAATTTCTCCCATTTGGAACAGCCGTATTTACCCAATACCTGTAACCCCATTGTATCTAGGCAGTAA
CTAGCTTGCTTTTGATTTTACAGGCTCATAGGCAGAAGGGACTTGCCTTATCTCAGGTGAGACTTTGGA
TTGTGGACTTTTGGGTTAATGATGAAATGAGTTAAGACTTTGGGGGACTGTTGAGAAGGCATGATTGG
TTTTGAAATGTGAGGACATGAGATTTGGCAGGGCCAGAGGCGGAATGATATGGTTTGGCTCTGTATCC
CCACCCAAATCTCATCTTGAATTGTACTCCCATAATTCCCACATGTTGTGGGAAGGGACCCAGTGGGA
GATAATTTGAATCATGGGGGTGGTTCCGCCATACTGTTCTTGTGATAGTGAATAAGTCTCACAAGATCT
GATGCCTTTATTGGGGGTTTCTGCTTTTGCGTCTTCCTCATTTTCTCTTGCCGCCACCAGGTAAGCAGTG
CCTTTTGCCTCCCACCATGATTCTGAGGCCTCCCCAGCCACGTGGAGCTGTAAGTGCATTTAAACCTCT
TTCTCTTCCCAGTCTCGGGTATGTCTTTATCAGCGGCGTGAAAATGGACTAATACACTGTGGTTATGTA
TTATAGTCATATGATATTTTCATATTTTTGGAAGCTGGGTGAAGGGTAGATGTGGAGACCATGATTTTT
GCAAATTTTTTTAAGTTTAAAGTTATTTCTAAATTAGAAGTTTAAAAAGAAGAAATCACATAAGCCAT
AACACAATAGAAAGATGTCTTTAAAGTTCAAGGCAGGAGGGATGTCTGGAAATCAGCGAGAAATTTG
CACCTGTGTGTGCATGTGCATATGTGTGTGTGTATGTTGCAAGGACTTGGAAAGCCCTTTTTTTCCTAC
CTCTGTACTACTGTGGGGGGAGGCTAAACTTGACTTCTTCCCATCTTAGTTCTTTTTTGGGATAGACTC
CTGTAACAAAAGACAGACAAGAGAAAAATCAGCTTACAACATGGGCCATGCACTTCACACAGGAGAA
ACCTGCATGAAAAGTAACTCAAAATGGTGCCTTAGAACTCCACTTACCTTTAGTAAAGAGCAATAAAT
TAGCAGGAAAATCATGGATCGGGACAAGGGAAGTGGTTTTATGCTTCCAAGGGCAGGAAATCATGGA
AGGTAAATATATGGGAGGAAACTAAAGGAATAAGGCTTGTTTGCATATTCCTCTGATGCCATCTCTGG
GTTGATAAGAGTCTAGAGTCATTTCCAGTAAAGATGAATTTTTATCTGTCTTTAGGAAGAAAGGGGGA
AAGATAGAGAAAACTATTTCTCCATTTGCTGTTTCTTAATTACCTTCAGTTCAAAAATAATTTTTATATC
AGAAAGGCATATTTAGAGGTATGTTAGTTTATTTTCACACTGCTAATAAAGACATACCCAAGACTGGG
TAATTTATAAAGAAAAAGAGGTTTAATGGACTCACCGTTCCACATGGTTGGAGAGGCCTCACAATCAA

GGCAGGTCTTACATGGCAGCAGGCAAGAGGGAGAATGAGAGCCAAGCGAAAGGAATTTCCCCTTAAA
AATCCCCTTATAAAACCATCAGATCTCGTGAGACTTACTCACTACCACAAGAACAGTATGGGGGAAAC
CACCTCTATGATTCAATGATCTCCCACTGGGTACCCCCCAACAACACGTGGGAATTATGGGAGCTACA
ATTCAAGATAAGATTTGGGTGGGGACACAGACAGACCATATCAAGGGGTAACATAGTCTGGTTTCCTT
TACTACCCACCTACCCAAACACCCCCTTCATCTGATCCACACAAAGTAAACTCTTGCAGTTCTCTCACT
GTTTCCTGGAGTCTGCTTTTGGTCTCATAGGACTGCCCTAACGCTTGTTTTTCAGACGTTTAACCCTGTA
GGTCTCTGGACAAATTTGCTTTAGAAGCCCCTCGATGTCGCCCTGAAGAGTGGCTTTCAGAAGTTGTGC
CTCCTGCCTGAGGGGAGTTCCAGGAAGGGTTCTGCATCGCCTATGAGTTTATCTGGATCACCAGAGGC
CTTCCCGTCAGAGCTTTCCCAATCGTTTTTGGCCAAGGAGTGTGAGAAGCTAAAGTTCATAACAACTG
GAAGTCAGACAGCCTGGTCTATTCTGCTTTAACTCTAGCAGGAAAGGCCTTCATGGTGGGGCCTGAAT
ATCTTCCTTTATAAAATCAAAGCCTGGGGACAGGGTTACTTACTTCTGAGGTTCAATCTGGCTCTAAAA
TTATGCAACAAATGCCATTCCTTTAGCACTTCCTTCCTACCGGGCGAGATACTCAACTCCACAGGCACC
ACCTCAGTTCATCCTCTCAGAAGTCCTAACAGCTCAGCCTGGGGCACCCCATTTTACAGATTAGTAAAC
TGAGGCTAAGAGAGGTTAGGTAGCTTGTTCAGGGTCATGCTGCTGGTAAAAGAGCTCAGGCTACAGTG
CTATGCATTGAGTTTTCTCACTTTCCCATCTAACTGGAGGGCTAAAGGTCAAAGAGTGGGCAGCTCCCT
TGTTGGGAGCTGTACAGGAATAATGTCCTCCCTGAAGGAGGGGGACTTCTGAGCCACACCCTGGGGTC
CAGGGCTCACAGCCTTAGGAGCAAAATCGTCCACCCCCTTCCTGGTTCCTCGGTGCTGCAGAGATATT
CATAGGACAGAGTCTGAGTTCTGGCCACTTAACAGAGGAAGAAAGGCTGGCTCGGTGAGGTTAACTT
ACATCCCAGCAGCTAGGAACCGGGAGCAGAGGACCTCAGATTCACACCAGGGCAGGAGGCAATGGCC
TGGCTGAAGCCTTCACAATCTTCCCAATATACTCCGCTGCCTTCCTTTATAAGGATCCATTTCTGAAAC
CCTGTGCCCTGGCCAGGCACGGTGGCTCACACCTGTAATTCCAGTACTTTGGGAGGCCAAGGCAGGAG
GACCACGAGGTCAGGAGTTTGAGACCAGCCTGGCCAATATGGTGAAACCCCGTCTCTACTAAAAATAG
AAAAATTAGCGTGGTGGCAGGCGCCTGTAATCCCAGCTACTCGGGAGGCTGAGGCAGGAGAACTGTT
TGAACCTGGGAGGTGGAGGTCTCAGTGAGCTGAGACAGACAGTGCCTGGGTGACAGACAGAGACTCC
GTCTCAAAAAAAAAAAAAAAAAGAAAGAAACCCTGTGCCCTAAGACCTGCACACTCGCTGGCTCCGC
TCAGACATTTAGCAAAGCAGACACCTTCCCAGGCCTGGAGGAAACAGCCCCTGCTTTTTGGGAATCCA
CAAGCCCGCAGCTGCAGAGCTCGACCTGGATGGGCAGGCAAAGGCTGACTCCTGTGCGTGGTGTGAG
TCCAGCCTGGCCCCTCTACACCCTCACTTTCACCTCTTAAAGAACTGCCTATTAACAGAGCAGGTACTG
CCCAAAAGGAACACTCTGGAAACTTGTTGGGACACTTCTGCCTTTCACAAACGTTTGGGGGGAGTACT
ACTAGCATTTAAGGATTGAGGGTTAGCAATGCCAGACATACCAGAACACGCAGGGCAGTCTCCCATG
ATGAAGAGGCCGCCGGGTTCCCCAGGACTCACATGTCCACCTCAAGTTCACGTGGGATTATCTGAGCC
TAGACTGTCAGTCCTGGGGCTGCTTTATTTCATATAAAAATATAATATTTATCCAAGGTTTTACTACAC
ACTGCATTTTCTGTGAAGACAATGACCGTGTAAATCAGGGAAAGATCTATATTTTATTTTGTTTGAAAC
TTTACCAAGCATTATTTACCATTTCAAAAGCTCTATCCCTGGTAGTACCATTGGTTTTCTTGTTCACCGG
CCAGCAGTGAGCAGCACACAAGCGACCTCCCGTGGGCTCCACATTGGACAGCCTCACTGCACCTGCCC
AGGCCCTTAGGCCACAGCACTGCCATATTCAGGGACACATTATTCTCTTTTATTATGCCTCCATATTAT
CATTACAGCATTATCTTTTTTTTAATTTTGTGGGTAGATTATATTAGCTATACGTTTCACTTCAATGGTA
GTAGTAAGGGGCACATAACAAAATATTTACTTATATATATTAAAAAAGAGAGTCTGAGAAGTCTGAA
AAGTTTTGCCATAAACGGTCTCCACCAGCCTCAACTCTGAGTGCCCGAGGATTCAGTCTCAAGTCCAG
CAACATTGTGAAGCAGGAAATTTACCTTGAAAGGAGCTATGTACTCTAAGTAGTGATTTACCTGTCTG
CCTCCCCCACTGGATTGACCAGTTCCTTGGGGGCTGAGAGAACAGGTCCTGAACATTTCTGCTGTGCCC
CCCAACCCACATCCTCATAGTGTCCAGTACCAGGCTGGGGACTCAGGAAGCATCCATGGGATCCCCCA
GTGCCTTCTTTCTCGAGGTGTTCAGCACCTAGAACAGCTCAAGACAAATTCCCCACACCCCACCCAGA
CAGAGCTGAATCTTACTGGGGCGAAGCCTTGAGTTGCAAGGCAGAAGCTCTCGTGATGGGATTTGGGT
CATATTCCGGGTTATAGGAGGAGCTGGGGAGTATGGGAAGCCTCCCACTTGGTCTTTGGTTTTCCAGA
AACTCCACCATCACAAGCAGGATGTTAATCAGTAACCGTCCCACAGGGGATCATACTTTGGAATAGCA
AATATTTGCTGAAGGTTCTGGGCTGCAAAGCTGAAGCTTTGGTTTCTGCTCTAAATGAAGGACTTTTCC
AGGACCCAAGGCCACACACTGGTAAGAGGCAGTGGGTTACAGGAGACCTTCAATGAGTCTAATCAGG
GAGGGACCGGGAAGGATGGTATCATCCCTGGGCGGGCTCCAACGTGAGGGCTGTGTGGCTGAGCAGT
GCAAAGACCTCCATCCTACACTCCACAGGGACTGTACATACAGATTGGGAGCTGGAGTGGGGTAAGA
GGCGAATTATAGACACAAGGGGCTCCTCTGCAGGAAGGAGGCCAAGGGAAAGAGGCTTGAAAGGCTT
GATATTTCACCCACCACCACTCACTGCCGGAGTAAGCAGGTCTCCCCTTCCCAGGGCTGAGGGGAGGC
AGGGATGTGTGCTGTCCCAGGGCTGAGAAGTGGCAGGTGAGCTGGTGATTCCTTACTGCCCAGGTTCT
GTCTAGGAAGGTGCGTCCTCACCATGCTGGATGGTGTCCTAGTCCAGGAGCACCCCCTGAGCTCCTGG
CCTAGACTCCAAAGGGTTGGGTAGATGAGCAAAGACTTTACAAAGACCTTAGGCGATATATGTCCAGG
AGCACCCAGGAATTACTGGGCTACCACTGCAGACTGCAGGACAAGCTCCAAGAACAGGAAGGTAAGA
CTCAGCATTTGGAGGTGGTGACATCTAGTTGGCGTGCTGGGCTAATTTCCTGACCATTGTACAGGGAG
AAGTAACCTTGAATTCAGGAGTATTCTGTGTGGTCTTAATGTAGAAAGTAGCACTAAATGATGCCACG
TAATCGTTTTAGCTCAGGCTCCTCTAACAAAACACCACAGGCTGGGTGGCTCCAACAGCCATTGATTTT
TCACAGTTTTGGAGGCTGAAAGTCCGAGTCAGGGTGCCAGCGTGGCCGGATTCTGGTAGGGCTGTCTT
CTTGGCTTGCAGATGGCCACCTTCGCACCGTGTCCTCCCATGGAGAGGAGGTGCGGAGGGGGACTCTG
CTCTCTTCTTATGACAGCACTAGTGCTATCACAGGGGCCTTGCCCTCACGACCTCATCTAAACCTAATC
ACCTCCCAAGCGCCCCAACTCTATTGCCATCACAATGGTGGTTCGGGCTTCAACTTATTAATTCTCAGG

GGACACATTCAGTCCATAACAATAAAAGCGTGAAACTGGGCTGCGTTTACACTGAAAGAGCTATTTAC
CCAACGTTTACAATACTTGGGTGACCTGTTGAATGCAGGCTTGCCATTTAGAGTCAAAAAGAGCTTCC
TCAACAGTGTCCTTTGGGAAACACAGTGGAAGTATTTCACTGCTTCTACAGGGGAGAGGGTAGTGCCG
TTCAGACTGCAGAGTGAGGCCCTGAATTCCGGGGTGCCATTCAGCCCGAGCAAGGGGCAACATGCTG
GGCCCTGGCGCTGGAGGCGGTTTTGTCCCAGGCATAGATAAGGACTCAGCCCCTGCATCAGGAAGAG
GCCTGGCAGCACCGCCTGTCAATACATTTTGCCGCAGGTGACCTTGGTCAAGAATAAGGGTCTCTGCT
GATGGGAACTACTGTGAGGCCGGCAGCATCCACCCTGCGCTCACTGGGCTGGGTGGCCTACCCCACCC
AGACCCTCCCAGGGCAGTGGGCCCAGAGAGAGGATGAGGGAGGGCAGGTGTCCCAGGGGTTCTGCCC
AGCCAGCCTCTGGGATCAGGCCTGCAGTGTGGCTGAACACCAGAACTGAGTTTGGACACAGCCAGGT
GGCCCAGGCCAGTCCCAAGCCATGTATTTGGATGGAAAACATGGAAGTATTCAGGAGCCAGGCTCTGT
GTCCAAGGATGTGGAGGGAGCCTAAAAGGCGACAGAGAAGGGGACAGCTAACGGTGAAGAAGTGTA
GCTCCCACACTGCAGCCTAGGACAGTGAGAACCGGCATGCAGCCCAGGTGGCTGAGGGCTCTATGAA
GCCACAGTGGAGGGAGCCCAGAAGTGGGTTGTATGAATTGCGGGGCCTCCTGCTACCCGGGAGCTGC
AGCTATAGGAAGGAAGGAAGGAAGGAAGACCTCCAAGGAACTGTGTAGCAGAGGTGCAGTGCAAAG
AGAATTTTGATAAAAAATCCAGGAAAGCTCCAATACTTTCCCCCTTCCTTGCCTAACGGGCATGCAGG
CACTCCAATCCCCAGCCAAACAGGGCACTGGGCAAGGCCGGCCACCCATCTGGATGGGCAGCCTGAC
GACCAGATGGTCAGGGCAGTGAATGAAGCAGATCAAGGAAAGGTGTGTGAGGACCCCTGATTCCACC
TGCTTGGACCCCCACCTTCTGTGCTGCCTCCTGCTCCCAGAGTGGACTCTCTTGCCCTGGCCCTCAGGG
AGGAGACGGGATGAATGAAAACGGGGTCAGGACTGAGAGCTGCCTGCCGGCCTGGCAGGGAATGGG
AACTGGAGGAGGTTTTGCTCTGTGAAATAATGTCCCCTCTTTGGGTGAGCAAATGTCACCCACACTTGC
TCTAGGTCTCCCTGGGGCAGGGCTAACCTACTTGAGCCACAGGAAGGAGGCAGGGTCCCTGAAGAAG
CTTTTACTATCCACAAAGACATTTTAGGAGGCATTAAAACCATCTCTATCCTCTCCTCTCCACAGGAAG
TCTTGCAGCTGAAGGGAGGCACTCCTTGGCCTCCGCAGCCGATCACATGAAGGTGGTGCCAAGTCTCC
TGCTCTCCGTCCTCCTGGCACAGGTGTGGCTGGTACCCGGCTTGGCCCCCAGTCCTCAGTCGCCAGAGA
CCCCAGCCCCTCAGAACCAGACCAGCAGGGTAGTGCAGGCTCCCAAGGAGGAAGAGGAAGATGAGCA
GGAGGCCAGCGAGGAGAAGGCCAGTGAGGAAGAGAAAGCCTGGCTGATGGCCAGCAGGCAGCAGCT
TGCCAAGGAGACTTCAAACTTCGGATTCAGCCTGCTGCGAAAGATCTCCATGAGGCACGATGGCAACA
TGGTCTTCTCTCCATTTGGCATGTCCTTGGCCATGACAGGCTTGATGCTGGGGGCCACAGGGCCGACTG
AAACCCAGATCAAGAGAGGGCTCCACTTGCAGGCCCTGAAGCCCACCAAGCCCGGGCTCCTGCCTTCC
CTCTTTAAGGGACTCAGAGAGACCCTCTCCCGCAACCTGGAACTGGGCCTCACACAGGGGAGTTTTGC
CTTCATCCACAAGGATTTTGATGTCAAAGAGACTTTCTTCAATTTATCCAAGAGGTATTTTGATACAGA
GTGCGTGCCTATGAATTTTCGCAATGCCTCACAGGCCAAAAGGCTCATGAATCATTACATTAACAAAG
AGACTCGGGGGAAAATTCCCAAACTGTTTGATGAGATTAATCCTGAAACCAAATTAATTCTTGTGGAT
TACATCTTGTTCAAAGGTACTTTGATAATGTTCTGCTCTCCCAAGGCCACAGGGCCCTACGATTGTCTC
TCCCTTTCCTTTCGTTAGGCCAGCATATGATTAACGCTACGTGATTTTCTATGAATGTGTTTTCACGTTT
CAAAAACAGATTGATACACATATTGAACAGTGCCAGACGCTGTCATTTGAGGCCCTTCCCTGGTATCC
TATGTGCTTGTAGTCCTTATTATTTTCAGAGCACTCTACATAGCTCCCCTCTGACACTTAGAAGCATAG
GGTCTTTCCAAAAAACAGGGGGCTGGGGGATTATCTGGGGGATTTAGGATTGCATCATTGCTCCTTCA
TTTTTACTTTTTGACCAACTCTCTGCCCTTAGATTCCTATTATAGAAAATAGGGACACTCCACCTACTAC
AGTGTTAGAGGCTAAATGAGACAATGAATGTAAAGTGCCCAGATGGGCTTGGCACATAGCAGACACT
GAGTATCTATTGTTTACTTGTTCTTCCAAACTGCCAATCAGCAGGTAGAGCAGGAGTTGTCTCCTTTCT
AAAGATGAAACCAGCTCAGAGACGTTAGCTTGATCAAGGTCACACAGTAAGTGGCAGAGGCAAAACC
CAAACAAGGGCCTCCTGACCCCCTGATCCTAGGTTCTGTCCAGCCCTGCCTCCCTAATGGGGCACTGG
ACGTGGGTTGGATGCCACTTTCGCAGAGCTGGCACCAGACTTACAAAGCCCCGGCAGGGGAAGCCAC
TTTACAACCAGCCAGGCCACACCCCCAGGGCAGACGTTTATGTAGAGAGTAATGTACCTGCCTGCTAG
TAGCCTCTGCATTGTGGGGCCTTCTCTCAGAACCACACTAAACAGTGGGTGGGTGAGAAGTGTCACTC
CTGCCACCTTGGACTCTGCATGTGCTTGTGCCTGGTGTGAATGAGACAAAGTGGCAGTCAGAGGTGCC
AGGCAAAGGCTTTTCTCTAAGCTGGAGCCAACTATGAGGGAACGACTGTGAATTCCGTTCAGGTCCAG
GACAATGAGAGGAGCCAGGGATTGTTAGGAAACATTTCCCTGCTTTCGTGTGCGATTCCCAATAGGGC
CTGCGAGTGGAGCTGCATTTTGCTAGCTGGGCTAGAGGACGGGGAAAATTTTGGGGAAATTTATTTTG
CCTGCCTGAGCTGTGGAAAAGCCAACCCAATTAGGGAACGCCTTTCCTAGTTGGAACGAGAAGACGA
GAAGTGAGAGAAGTGAGATAGAAGGCTCCCTCTCTATTATTTGAGCAAGAACAATGCTTTTCAAAGAG
GGAATTTCTGCAATGAGTTCTTCTCTTACTTGTTCAGGGAAATGGTTGACCCCATTTGACCCTGTCTTC
ACCGAAGTCGACACTTTCCACCTGGACAAGTACAAGACCATTAAGGTGCCCATGATGTACGGTGCAGG
CAAGTTTGCCTCCACCTTTGACAAGAATTTTCGTTGTCATGTCCTCAAACTGCCCTACCAAGGAAATGC
CACCATGCTGGTGGTCCTCATGGAGAAAATGGGTGACCACCTCGCCCTTGAAGACTACCTGACCACAG
ACTTGGTGGAGACATGGCTCAGAAACATGAAAACCAGGTACAACTCTTGCCCACACCCTATACAAACT
CTACCTTTCTGTACTGGCAAACGCTCAGCACAATTTCATTGAATGCACCGTGATTTAATGTCTCCTCCA
GTGAGCTATAAGTTTCCTGAAGGCAGGGCAGCATTTGTCTTTTTTTCCACTCTATCCCCAGCATCTGTC
ACAGGGTGCCTGGCTGATTCATTCATTGAGTCCATCAGTATTTTACGTTCTGCGACTGTGATAAATATA
TGATGCCAGGGATCCATCAGCAAACAAAACAGGCAAAATTAGTCTGCCCTCATGCAGCTTACATTCTA
TTGAAGGAAGACAAAGAGTAAATTAAAAATAGGTAATAATGCAGGGAAGGGGACAAGAAGCATCAT
CAGGATGCAGATGGAGGTTAGACAAGGCCTCTCCAAGAAGGTAACAGTAAGCAAACATCTGAAGATG

AAGGATAAACCATGTGGATATATTCGGGGAGAGAAGTGTTATGTTACAGGCAGAAGTGTACAAGTTCT
GGGATGGGAGTGTACCTGGTGGGTTTGAAGAACATCAAGGAGACAAGTGTGGCTTCAGCAGTTGGAG
ATAAAATCAGAGAGGAAACAGGGGCCCAGTCCCCAGAAAAGACTTGGGCTTTCCTGAGAGAGGCAGG
AAGCCACTGGATGGTTCTGAGTAGAGGAGCAACCTGATTTTGACTTCTGTTTTTAAAGGATCACATAA
GCTCCTGTGTTGAGAAAAGACACTAGGGGGTAAGGATGGAAGCAAGGGAGAGTGGTTAGAAAGTTAC
TAGCAATCCAGGTAGAGATGCTGCTACCTGGACTGCGGTGGTGGTAGTGGAAGTGGTGAGAAGTGGC
TGGATTCTGGATCTATTAGGAAGTGCAGGATCTGCTAATCGATTGGATGTGGGTGAGAGAGGTGTCAA
AGGTGATCACAAAGTTTTTGGCCTTAGCAACTGGAAAGACGGATTTGCCATTTACTGAAAGGGGGAGG
AACAGGTCTGGGGTAAGTGCAGAAGTTCAGTCTTAAACACTTGGATCAGAAATATCTATTAGACATCC
AAGTTGAGATGTCAAGACGACAGGTGGATCTGGAGTCTAGGGTGAGGTCCAGGCCGGAGATATAAAT
TCGGTCATCAACACAGAACTAGAATCTAGACACATGACAGGGTTGGGGTCTGTAAATATAGAGGAGA
GGAAAAGAAAGCACAGAGTGGGCACTGAAATGTCTGCCCAATAAATTAATCCACCTATTGGAGTACA
AGGAAAATGGCTGCAATACGAATTCCATGGCTATGGCTTCTGAATCCTGTGACTCAGATTTTGGCAGA
CAAGTGCAGCTAAAGGTCCCCAGGGTTAGTTTTATCTTCATTATTCTTCTTTCATTTTTCTTCATATCTT
TAGCACCTAACAATGAACCCCAAACATCATAAGCCCTCAAGTAATGTTTGCTGAATGAATAACTTTTT
AAATTAATCTTCAAGACACGTCATGTCCTCAATTATTTTTAAATAAATAAAAAAATTTTATTTTGAGCC
ACAGAACTCATCTTTTCAAGCAACATATTTTCAAAGGAGGACTCCAGTATACAAAATAGATGGTATCA
GAGCTTCTCTGGCTAAAGACGGGTAGGGGTTGAAAGTTTTCTTTGCTCCCCTCCCCATCCATCCCCAGA
CTCCTCGGGTCTGCAGAATCCAGGAGCTGAAAACAGCCATCATCCAGGAGGCTGCAGGACTGCTGAA
AGCAGCTGTTAACTCAGGTTTTTTTTAAAATATAGGGAAATGAACACATAAGTACTTTGCTAAAGAAA
ACGTGAGTCACTGGCTGAGGAATAAAACTCATTCACTGAAGCTGAAGTACTATTTGATAAGCTAGAAA
TATTTTCCCTGAGTAGACCACTGTAAAAGAATGGCATGAACTACATAGTCAACTGAAAGACTCATTAA
TGGAAATAATCTTAAAGAACAAAAATTGTGACCTTTTTGGTGTCCACAGACTAGGGCTTTGTCTACATT
TCACCATCATCTGTTCTTGTACCACAGAAACATGGAAGTTTTCTTTCCGAAGTTCAAGCTAGATCAGAA
GTATGAGATGCATGAGCTGCTTAGGCAGATGGGAATCAGAAGAATCTTCTCACCCTTTGCTGACCTTA
GTGAACTCTCAGCTACTGGAAGAAATCTCCAAGTATCCAGGGTAAGTCAGGATCTTTCATCAGAGCCC
AACCTCAGCATGAAATGTCACCAAAACAAATGCTTTTACAAACCATTTAACTTTGATAAAATACCTAA
TTGTAGTGGAAAATTAGATTTAAGTCCCAAATACTTGAAATAGCACCCAGGTTGGATGTTTTAAGAAT
TTCAAGCAACTTCATTAAAATAACTTTTCAACTAATTTATTTTAAGCAGACCTCTCCCCCTCTGCTTAA
AGTGCTCAGGGAGAAATTTGACCCTGAAATAGAACTGGTTTACAGAGGCATCATCATTTATGTTGAAT
ACAACTTGAATAGTTCATGAAATTACACCACCTTTACAATGAAACAAACCCCTAGACATCATCTAGCC
CAACTTCTCCCTCCTTGTGGAAATCCCCTCCATAGCCCTACGAAATAGCCCTCCAACTTCTCTTCCTCTT
CATGCTTCCAGTGACATCAAACTCACCATTTCTTTGAAGAGCTGCCCAATCCACAAATAGCTAAAATT
GTTATATGTATATATATATATGTGTGTATATATATGTATATATGTATGTGTGTATAAATGTATATGTGTG
TATATGTGTGTGTGTATATATATATACACACACATATATATATATATGGAGAGAGACATACATATATAT
ATGGAGAGAGAGAGAGAGAGAGTCCTGTAACTTCTGATTCATACTTTTTGGTCCTAGTTCTATCTCTAA
AACTTCTAAGAACAAGTTTAGTCACCATCCACATAGAATCCCTTCAGTTACTCAGTGTTTCTCAGTGGA
AGGGTTCTTGGTTTTGAGGGGAACTGCTTGTTGTCCAGAGCAGTTGTGCATGTTGCAGGGAACTGGTT
AGCATTGCTGGCCCATGTTCACTAATGCCAGTAGGAAACTCCAGTCATCACTATAAAAATGCTCCCAC
ACATTTCCAAATGGCAGCTACATCTCTCTACATTCTTCCTTAGCTGTGTGGTTTAATATTTTCTTATACA
ATTGCAATTTTCAATTCCAAGAGAGACTAAAAATGGCATCCACTTAAGTAGGACACAGTAGGGTAACT
GTGGCCTGGAATCAGGTCTTACAACCTCAAGAGAGGTAAGACAATTAAATAAAACAATCCGTCAGAC
CAGCACCTGAAAGTGTTTCTGCTATGAACACATGAAAAACTGAAATGCGCTGCTGCTTTATGAAGGGT
CATCATGAAATTTAAACTGTAAATGATTAAATATTCTCCCTCTGTTTGCTCTGGGGAATTAATTTTCCTC
TAGGAAATCAGGGAATTTCCTGGAGTGAAAATCAGTGTAATTACATGTTATGTTTTCATTATCTCTTAT
AACACAGTAATTATATAGGTACATCACTCATATCACATCTTGTTTCTGTAAAAAAGGGCCTCCCAAAC
ATAGCAAGCAGCCACAGTATAGGCAGCCAGAATTCAGGAAGGCTCCAGGGACCCCTGGGCTTGGCCC
AGAAAAATGCCTCAGAGTAGTACCAGGTGCTGGGAAGCTGCTACAGAAGACTAGCCATTCCCTGCCTC
CACCTTGCCTGCCAAAAGGAAAGTCAGAGGACTCAAGGGATCCAGGGATCAAGGGATCCAGGCAGCT
TGAAAACCTTTTAGGAGCACCAGCTCAGCTCAAGAATTAGTAGCATAAATTACATGCTCAATAAAGAT
TTGATGCATGAGTGCATCCTGAGTCCATGCCCGGAATGTGTTTCACATATTCCACAATACTTCACATTG
GGTTCCTGAGGTCTCCTGGTATTGTTTAAGACTCCTGTGGCAGTCCCTGGTGCAACCCCAGACCACTCC
TCTTAACGTAGATGGGCCTGCTCCACTAAATCCCAGGAGCATGACCCCATGGGTAGGACCACTGTGAA
GAATTTCAAGGGGCTCATTTAATTCCTCCTTTGCACTGCCACACAAATGGTTTTTCACATTATTTCCTTT
TTCCAGGTTTTACAAAGAACAGTGATTGAAGTTGATGAAAGGGGCACTGAGGCAGTGGCAGGAATCTT
GTCAGAAATTACTGCTTATTCCATGCCTCCTGTCATCAAAGTGGACCGGCCATTTCATTTCATGATCTA
TGAAGAAACCTCTGGAATGCTTCTGTTTCTGGGCAGGGTGGTGAATCCGACTCTCCTATAATTCAGGA
CACGCATAAGCACTTCGTGCTGTAGTAGATGCTGAATCTGAGGTATCAAACACACACAGGATACCAGC
AATGGATGGCAGGGGAGAGTGTTCCTTTTGTTCTTAACTAGTTTAGGGTGTTCTCAAATAAATACAGTA
GTCCCCACTTATCTGAGGGGGATACATTCAAAGACCCCCAGCAGATGCCTGAAACGGTGGACAGTGCT
GAACCTTATATATATTTTTTCCTACACATACATACCTATGATAAAGTTTAATTTATAAATTAGGCACAG
TAAGAGATTAACAATAATAACAACATTAAGTAAAATGAGTTACTTGAATGCAAGCACTGCAATACCAT
AACAGTCAAACTGATTATAGAGAAGGCTACTAAGTGACTCATGGGCGAGGAGCATAGACAGTGTGGA

GACATTGGGCAAGGGGAGAATTCACATCCTGGGTGGGACAGAGCAGGACAATGCAAGATTCCATCCC
ACTACTCAGAATGGCATGCTGCTTAAGACTTTTAGATTGTTTATTTCTGGAATTTTTCATTTAATGTTTT
TGGACCATGGTTGACCATGGTTAACTGAGACTGCAGAAAGCAAAACCATGGATAAGGGAGGACTACT
ACAAAAGCATTAAATTGATACATATTTTTTAAGATGTTTGTGCAATCTGTCTGGTATTTTAAGCTTGTTT
CTAAGAACCTTAGTTACTTGGCTAAAGACTAGCTGGGTAGAATATCTTTTCTCTGTTGCTCACATATTT
TCATTTTTAAAAAGTTGCAGATGAGAACACTATGTCAAGATAAAGCCTTTGGGAGGAACACATGTAAA
CATTCTCCTTGAGTCATGTGCTTCTCTCTCTTTCCTTCTCTCTGGTGCAAAATAAGTGTTTTATTTTAATC
TATTACGGAGTCATTTCTTGCTGACTGACATCAGAAGAAAATAGCTCTAACCAGTCCTGATCACAGCA
TCTGCTTCCATGGTGCATCAAATCGCTTGGCAGAGGCATTGGCTGAATCACAGATCATCTAGTTCAATA
CCTTCATTTTACAAAGGAAAGAAAGAGGGACCCAGAAACAGGTCCATATTCTTACTTTCATGGGCCCT
AGGCACGTTTAACCTTGTAGACTCCTCCTTCCTTCATGAAGATATATATGTTCTATGGCTGCATTGGTA
GAAAGATGAATATATTCGTCTTTCAAAGTTGCATATCTAGCTTCAAAGTTATATGTCTAGCATATGGCA
ATAAGCAAAACACCTTCATGGGCCCTTACAGTACTGTCAGCCTTGGGCACTGTGTCTTCTGCATCTAGT
GGATAAGTCATACCTTATATACCAGTGGGAACAAAATACTTGTCCAAGGTCTTCCAGTGTGGCAATGG
CAGAGTCAGAAGCCTACCTTTCCTGAGTCTAGTCTCCAAGCCCTTTTTACTCTTCCTTCCATCTAAAAC
ATCTGATGGGGACCAGGTAAACAGCATGCACTACAGCTACCCATGGGGGTTAAACAGAATATAAGCA
TGAACTTTGTCCCAGGGTGAAAAGGAAAATCGTAAATATCCCTGATCTTCCTTAGGCAGTTATTTTCTG
TCACAGAAACAGAAAAGACTATATTCAGAGAATCCTGAATAGAGCTGATTTACAGTGTGAACTATGTT
AACTAAATGCCTAATTGGATTTCTGTCTGTCTGCTATCTAATGTTTAAAAAAACCTAAAATTCATTTAT
TGATTAGTTGTTTAATATAATTCAGAGTAATGTGAATAGGTAATAATATTAATATGCAGTCTAAATACT
GACTTTTCATCATTCCATAACCTGGACTGATGAAAAGTCAGTATTTAGACTGCATATTAATAAAATAA
AATTCATTCCTGTATTCATTCCAAGAGTACTAATTGACACTTATGAAGGGACAGGCAATTCTAGGCCCT
AGAGGGCCAAAGACAGAGGACTAACTCTATCTGACATTCTTAAGTCACCTTGTTTGTGTTCAATTAGTC
AGATTTGTTTGTGGAAAAATAGTAGAAAGAGGAATAAAGTAGCATCCAGTCCAATTTCCCACTTTTAA
GAGATGAAATCTGGAAAAATAAGTCTGTGAGAGCACAATACTCACTGAAATCAATATGGCCAAACCC
AGTAATAAAAAGGTACATTATTATTGAAGGATTCATATAGCATGCAGATAAAAAACTCCTGCCTTCTT
CCCACCACATACACTGCAAAGCAACAACAGCATAATAATTGTATTTAATATACTACTCTTTAAGGTAG
AAAATGGACCTATTCTATATTTTAAATATACTTTTTAATGTTCCCTCACATTTGCTTTAAGAAGTTCCTA
AGACACTCAGTTTCAGATTTCCCAAGTACACAGGCATGACAGAAAAACGCAGACCAATAAAAAATGT
AACTTACCTTACACAAATACATACACACAAATTCAGGGTTTCCAACCGAGCGGGGGAAATCTTAACAT
TGTAGAAGTCTTCACTATATATGTGTCGAGTTTTTGTTTTTGTTTTTGTTTTTGTTTTGAGACAGAGTCTT
GCTCTGTCACCCAGGCTGGAGTGCAGTGGTGCGATCTCAGCTCACTGCAACCTCCACCTCCCGGGTTC
GTGCCATTCTCCTGCCTCAGCCTCCCGAGTAGCTGGGACTACAGGCACCTGCCACCACGACCGGCTAA
TTTTTTGTATTTTAAGTAGAGATGGGGTTTCACTGTGTTAGTCAGGATGGTCTTGATCTCCTGACCTTGT
GATCTGCCCGCCTCGGCCTCCCAAAGTGCTGGGATTACAGGCATGAGCCACCACGCCCGGCCAAGTGT
CGAGTCTTAAAAATTGTTCCTACACAGACACACTCAACCACACGTTCTCACATATATATGCTGTAACA
ACTGAGAACAGGTTACTGACTTAATCTAATTCATTCTATCTTCATTGTAAAACTTCCACTCCAGCTGAA
GAGCCTGTTTCATTTCAATTCAAAGATTTCTCATATATCCACTAATTGTATGGCAAAACTGACTCATCT
CCAGACTAAGATATTCAAGCTCAGGAAGTCAAATAATAGAAATGATTTTTTAAATGTGTAAGAGGTTA
TAAAGAAAAACTTTATGTGCTCCTTATTTAACCTCTATTAAGTAAAATCCTTTATAGACCTATCTCCAT
TTCTGCAGTAAAAGTGAGCTCTACAGTTAGCTTGTAAGGCTAACTAGTGAAATTCCTGGACTTGTTCTT
AAAAATGCAAGTTTTAGTAATTAACAAAATGATGATGAAGATGTCCCTTTTCCCTACAACTACAGATG
GAGGGAGATTTTTCTTTGCCATACAACTAGCTTAAAGGATTAATTTGATAAGTTGTTAAACTGAGAACT
TTCACAAAAGTATCCATCTTGTTTTTGATATAAATGGAGATACATGTAGTTATTCATAACTGTCAGTAA
TTTGCTGTTTATCCTGTTTCTATATATCTGTCCTTGAGAGTATAATTTTAATAAATATTTCAAAGATTTT
AGGAAATGTCATGTTCTGTTAAAAAACTTCCAAAAGTAATTTTGATGAACAGTTTTGATAACTTAGTAC
TAACTAGGACTAAGACTGCAATTGACTGCTCTACATTCCTGAACTTTATAAGCAGTAGTTGTTTCTCTC
TGTCAAATCAGTGTCCCCTTTTCCCATTTGCATCATGGGAAAGTGAAACCTTATAATTCTGCTAAATTT
ATTATAACAAATACATTGAAATTCTCCATTTTATTAAATTAATAGAATGTTATGAATCAAAGCACCAA
AAAAACTGATGCAATTTTGATGTCTCGTTCTGTACCACATTCTCCAGATCTTAATATATTCAGTTCCAC
ATTATTGGTGCTAGTAGGAGACATAATGAAAACAGTTAAATGAAATCCACAGCGAGTATACTGATTAA
CCAGTACTGTCAAATTTCTCATACCTATTGAATTTTAACTACTGACAAAATGAGCAGTAACAATTCCAT
TTACCTGATTGTCCTTTGGCAAAGGATATTATTAAGAATCACTAAAAATAGCCATAAAGAAGCCATAT
GGAAGGAAGAAGGAAAACAAATGGCATGAAAAGGTCTCTCACTGAGTAACTATGCTCTTATAGTTGA
CGCTGGTATATTTCTTTTATTCACTACCTAAAAATGAACTATCTTACTCTTTAATTATAGAATAAAAAC
TGCAGGAAAGTATTTAAGACTTTTTTTCACAAACACAGGTATCTCATTAACCTATGTTTTATTTTGAGT
AAATTCATTATTCATTATTTCACATTATAAAAAGTAACCACACATACATATGCATTCACAAATTAGATC
ATCTTTATCATACATCAATATATTTTAAAAAACAAATATCTTCTAATATCAATATAGTTATATGCTGAT
TGCATTTTGAAATAGAGAAGCTGACAATAGCTTCACACGGTATATCTCAAGAACTGACAGTTTAAAAT
TAAGAACTGTATATATTCCACAGGCAAATTTTGATGGAAATATTAGCATTAGTACAAATAAATGCTGT
TGACATAGCTTAAGCATGATAGCTTGGAATAACAGCTGATTCAGACTAGATTCATCATTTTAAATAAA
GACAAGTACAATCTAAAATGTAAACAAAGTATTTATAAAATAAATTCTCTAGGAAATAAAGAAAATC
ATCAATCTATTATTTTTAAGGTATTTATAGCTCAAAGTTACCAGAAATCTTTGTGGAATTTTCACTGCC

AAATTTAAATTTGGGAATGTCCGGGTACAACATATTGTCACCACAATCCGGAGGGCCGCCAAAATCGC
AGACGGCTATTTGCATCCTTTCAGTGTGACTTTTCAAGTGGGCTTGGAGACTCATGAGAAAATGCAGT
ATCTTTCTCACCTTCCAAGTCCCCCTCCAAGTGCTTATCAAGCTAGGACAATTCAGCTGATGTAGACTT
TCATACGATTTTTAAATGCTAAAACTCTAGAACAATTAAATGGCTGGTTTCCTGCACAAATAAATGCA
GACTTGTCTCTTTTGCAGCAGTGGTTAAAGCACATTCCTAGAGATGTTTTTCATTACACTTCACTATAA
CATTGGAATTCCGTAACCACATTATTACTCAAGAAATATATATTATACCTCCTAGGGAATCTAATTTGA
AATATGAAAAGTTTAACATCAGCTGTCATTATGTCTCTCTTTCTGCTCATTAACAACAACAAAAAAAA
AAACCCAAAATTTAAAAACAAAGCCCCAGCCACTGCTTTAGCTTTTGTGTACCAATCACATTATCTCCT
GCTGCCTTTGTTTTGCCTCCTTCATCAAGCAGTTGATTTAAGGATTGGATTTTCTGGATTTTCTTTGGGA
AGAAAGAAATGAAGGAAGAGAGGGAGGGTGGGGAAGGAGGGAGTGAGAAAGGGAGAAAAAGAAAA
AAATATGAAAAATGTTATTCATATAATGTGTACAAAGTAAATTAAAAATATATAGATACTCTACTTTG
AATAATTCTAATATATGAGAAGT

Following Table 1 provides oligonucleoside mRNA target sequences of ZPI, together with the corresponding positions in transcript NM_016186.3. It is to be understood that SEQ ID NO: 2 to 121 refer to human (Homo sapiens) mRNA sequences.

**Table 1**

| SEQ ID NO | Oligonucleoside mRNA target sequence 5' → 3' | Starting position on NM_016186.3 |
|---|---|---|
| SEQ ID NO: 2 | UUUGCCUUCAUCCACAAGGAUUU | 991 |
| SEQ ID NO: 3 | GCUGCGAAAGAUCUCCAUGAGGC | 756 |
| SEQ ID NO: 4 | AUGCUGGUGGUCCUCAUGGAGAA | 1390 |
| SEQ ID NO: 5 | CUGCGAAAGAUCUCCAUGAGGCA | 757 |
| SEQ ID NO: 6 | AAGUAUGAGAUGCAUGAGCUGCU | 1531 |
| SEQ ID NO: 7 | CUGUUUGAUGAGAUUAAUCCUGA | 1156 |
| SEQ ID NO: 8 | GAUGAGAUUAAUCCUGAAACCAA | 1162 |
| SEQ ID NO: 9 | UUUGAUGAGAUUAAUCCUGAAAC | 1159 |
| SEQ ID NO: 10 | UGAUGAGAUUAAUCCUGAAACCA | 1161 |
| SEQ ID NO: 11 | UUGAUGAGAUUAAUCCUGAAACC | 1160 |
| SEQ ID NO: 12 | AACUGUUUGAUGAGAUUAAUCCU | 1154 |
| SEQ ID NO: 13 | AGUUUUGCCUUCAUCCACAAGGA | 988 |
| SEQ ID NO: 14 | UGCGAAAGAUCUCCAUGAGGCAC | 758 |
| SEQ ID NO: 15 | CAUGCUGGUGGUCCUCAUGGAGA | 1389 |
| SEQ ID NO: 16 | UGCCUUCAUCCACAAGGAUUUUG | 993 |
| SEQ ID NO: 17 | GCGAAAGAUCUCCAUGAGGCACG | 759 |
| SEQ ID NO: 18 | CCUUCAUCCACAAGGAUUUUGAU | 995 |
| SEQ ID NO: 19 | UGUUUGAUGAGAUUAAUCCUGAA | 1157 |
| SEQ ID NO: 20 | UUUUGCCUUCAUCCACAAGGAUU | 990 |
| SEQ ID NO: 21 | AAGAUCUCCAUGAGGCACGAUGG | 763 |
| SEQ ID NO: 22 | ACCAUGCUGGUGGUCCUCAUGGA | 1387 |
| SEQ ID NO: 23 | GUUUUGCCUUCAUCCACAAGGAU | 989 |
| SEQ ID NO: 24 | UUGCCUUCAUCCACAAGGAUUUU | 992 |
| SEQ ID NO: 25 | CCUACCAAGGAAAUGCCACCAUG | 1370 |
| SEQ ID NO: 26 | GUUUGAUGAGAUUAAUCCUGAAA | 1158 |
| SEQ ID NO: 27 | GCCUUCAUCCACAAGGAUUUUGA | 994 |
| SEQ ID NO: 28 | CGAAAGAUCUCCAUGAGGCACGA | 760 |
| SEQ ID NO: 29 | ACUGUUUGAUGAGAUUAAUCCUG | 1155 |
| SEQ ID NO: 30 | CCAUGCUGGUGGUCCUCAUGGAG | 1388 |
| SEQ ID NO: 31 | GAGUUUUGCCUUCAUCCACAAGG | 987 |
| SEQ ID NO: 32 | UGCCACCAUGCUGGUGGUCCUCA | 1383 |
| SEQ ID NO: 33 | GCCACCAUGCUGGUGGUCCUCAU | 1384 |
| SEQ ID NO: 34 | GAAAGAUCUCCAUGAGGCACGAU | 761 |
| SEQ ID NO: 35 | GGAGUUUUGCCUUCAUCCACAAG | 986 |
| SEQ ID NO: 36 | CCACCAUGCUGGUGGUCCUCAUG | 1385 |
| SEQ ID NO: 37 | AAAGAUCUCCAUGAGGCACGAUG | 762 |
| SEQ ID NO: 38 | CACCAUGCUGGUGGUCCUCAUGG | 1386 |
| SEQ ID NO: 39 | UUUGCCUCCACCUUUGACAAGAA | 1321 |
| SEQ ID NO: 40 | UGCCUCCACCUUUGACAAGAAUU | 1323 |
| SEQ ID NO: 41 | ACCAUUAAGGUGCCCAUGAUGUA | 1285 |
| SEQ ID NO: 42 | AACUGCCCUACCAAGGAAAUGCC | 1364 |
| SEQ ID NO: 43 | CUCAAACUGCCCUACCAAGGAAA | 1360 |
| SEQ ID NO: 44 | CCUCAAACUGCCCUACCAAGGAA | 1359 |
| SEQ ID NO: 45 | GCCUCCACCUUUGACAAGAAUUU | 1324 |
| SEQ ID NO: 46 | GUCGACACUUUCCACCUGGACAA | 1255 |
| SEQ ID NO: 47 | ACACUUUCCACCUGGACAAGUAC | 1259 |
| SEQ ID NO: 48 | UCAAACUGCCCUACCAAGGAAAU | 1361 |
| SEQ ID NO: 49 | AAACUGCCCUACCAAGGAAAUGC | 1363 |
| SEQ ID NO: 50 | GACACUUUCCACCUGGACAAGUA | 1258 |
| SEQ ID NO: 51 | GACCAUUAAGGUGCCCAUGAUGU | 1284 |
| SEQ ID NO: 52 | GUUUGCCUCCACCUUUGACAAGA | 1320 |
| SEQ ID NO: 53 | ACUUUCCACCUGGACAAGUACAA | 1261 |
| SEQ ID NO: 54 | UGUCCUCAAACUGCCCUACCAAG | 1356 |
| SEQ ID NO: 55 | CAAACUGCCCUACCAAGGAAAUG | 1362 |
| SEQ ID NO: 56 | GUCCUCAAACUGCCCUACCAAGG | 1357 |
| SEQ ID NO: 57 | AUGUCCUCAAACUGCCCUACCAA | 1355 |
| SEQ ID NO: 58 | UCGACACUUUCCACCUGGACAAG | 1256 |
| SEQ ID NO: 59 | AGUUUGCCUCCACCUUUGACAAG | 1319 |
| SEQ ID NO: 60 | CGACACUUUCCACCUGGACAAGU | 1257 |
| SEQ ID NO: 61 | CUUUCCACCUGGACAAGUACAAG | 1262 |
| SEQ ID NO: 62 | CACUUUCCACCUGGACAAGUACA | 1260 |
| SEQ ID NO: 63 | UCCUCAAACUGCCCUACCAAGGA | 1358 |
| SEQ ID NO: 64 | GAUUACAUCUUGUUCAAAGGGAA | 1198 |
| SEQ ID NO: 65 | AAUGCCACCAUGCUGGUGGUCCU | 1381 |
| SEQ ID NO: 66 | UUUAUCCAAGAGGUAUUUUGAUA | 1038 |
| SEQ ID NO: 67 | GGAAAUGCCACCAUGCUGGUGGU | 1378 |
| SEQ ID NO: 68 | GGAUUACAUCUUGUUCAAAGGGA | 1197 |
| SEQ ID NO: 69 | AUCUCCAUGAGGCACGAUGGCAA | 766 |
| SEQ ID NO: 70 | AUUCCAUGCCUCCUGUCAUCAAA | 1721 |
| SEQ ID NO: 71 | UUAUUCCAUGCCUCCUGUCAUCA | 1719 |
| SEQ ID NO: 72 | ACCAAGGAAAUGCCACCAUGCUG | 1373 |
| SEQ ID NO: 73 | GCUGGUGGUCCUCAUGGAGAAAA | 1392 |
| SEQ ID NO: 74 | ACAUCUUGUUCAAAGGGAAAUGG | 1202 |
| SEQ ID NO: 75 | CCAAGGAAAUGCCACCAUGCUGG | 1374 |
| SEQ ID NO: 76 | UUGCCUCCACCUUUGACAAGAAU | 1322 |
| SEQ ID NO: 77 | GGGAGUUUUGCCUUCAUCCACAA | 985 |
| SEQ ID NO: 78 | CUGCUGCGAAAGAUCUCCAUGAG | 754 |
| SEQ ID NO: 79 | CAAGUUUGCCUCCACCUUUGACA | 1317 |
| SEQ ID NO: 80 | AAGUUUGCCUCCACCUUUGACAA | 1318 |
| SEQ ID NO: 81 | UACAUCUUGUUCAAAGGGAAAUG | 1201 |
| SEQ ID NO: 82 | GGGGAGUUUUGCCUUCAUCCACA | 984 |
| SEQ ID NO: 83 | UGCUGCGAAAGAUCUCCAUGAGG | 755 |
| SEQ ID NO: 84 | UUCCAUGCCUCCUGUCAUCAAAG | 1722 |
| SEQ ID NO: 85 | UCUGUUUCUGGGCAGGGUGGUGA | 1794 |
| SEQ ID NO: 86 | CAGCCUGCUGCGAAAGAUCUCCA | 750 |
| SEQ ID NO: 87 | CAAACUGUUUGAUGAGAUUAAUC | 1152 |
| SEQ ID NO: 88 | CCAUGCCUCCUGUCAUCAAAGUG | 1724 |
| SEQ ID NO: 89 | GAAGUAUGAGAUGCAUGAGCUGC | 1530 |
| SEQ ID NO: 90 | AGAAGUAUGAGAUGCAUGAGCUG | 1529 |
| SEQ ID NO: 91 | CAUGUCCUCAAACUGCCCUACCA | 1354 |
| SEQ ID NO: 92 | AAACUGUUUGAUGAGAUUAAUCC | 1153 |
| SEQ ID NO: 93 | AAGACCAUUAAGGUGCCCAUGAU | 1282 |
| SEQ ID NO: 94 | AGACCAUUAAGGUGCCCAUGAUG | 1283 |
| SEQ ID NO: 95 | CUGUUUCUGGGCAGGGUGGUGAA | 1795 |
| SEQ ID NO: 96 | GCUUCUGUUUCUGGGCAGGGUGG | 1791 |
| SEQ ID NO: 97 | AGUUUUCUUUCCGAAGUUCAAGC | 1500 |
| SEQ ID NO: 98 | GUUUUCUUUCCGAAGUUCAAGCU | 1501 |
| SEQ ID NO: 99 | CUUCUGUUUCUGGGCAGGGUGGU | 1792 |
| SEQ ID NO: 100 | UCAUGUCCUCAAACUGCCCUACC | 1353 |
| SEQ ID NO: 101 | AGUCGACACUUUCCACCUGGACA | 1254 |
| SEQ ID NO: 102 | CCUUCAUCCACAAGGAUUU | 995 |
| SEQ ID NO: 103 | CGAAAGAUCUCCAUGAGGC | 760 |
| SEQ ID NO: 104 | UGGUGGUCCUCAUGGAGAA | 1394 |
| SEQ ID NO: 105 | GAAAGAUCUCCAUGAGGCA | 761 |
| SEQ ID NO: 106 | AUGAGAUGCAUGAGCUGCU | 1535 |
| SEQ ID NO: 107 | UUGAUGAGAUUAAUCCUGA | 1160 |
| SEQ ID NO: 108 | AGAUUAAUCCUGAAACCAA | 1166 |
| SEQ ID NO: 109 | AUGAGAUUAAUCCUGAAAC | 1163 |
| SEQ ID NO: 110 | GAGAUUAAUCCUGAAACCA | 1165 |
| SEQ ID NO: 111 | UGAGAUUAAUCCUGAAACC | 1164 |
| SEQ ID NO: 112 | GUUUGAUGAGAUUAAUCCU | 1158 |
| SEQ ID NO: 113 | UUGCCUUCAUCCACAAGGA | 992 |
| SEQ ID NO: 114 | AAAGAUCUCCAUGAGGCAC | 762 |
| SEQ ID NO: 115 | CUGGUGGUCCUCAUGGAGA | 1393 |
| SEQ ID NO: 116 | UUCAUCCACAAGGAUUUUG | 997 |
| SEQ ID NO: 117 | AAGAUCUCCAUGAGGCACG | 763 |
| SEQ ID NO: 118 | CAUCCACAAGGAUUUUGAU | 999 |
| SEQ ID NO: 119 | UGAUGAGAUUAAUCCUGAA | 1161 |
| SEQ ID NO: 120 | GCCUUCAUCCACAAGGAUU | 994 |
| SEQ ID NO: 121 | UCUCCAUGAGGCACGAUGG | 767 |

Table 2 provides the unmodified first (antisense) and corresponding unmodified second (sense) strand sequences for siRNA oligonucleosides according to the present invention, together with the corresponding positions in the overall gene sequence of SEQ ID NO: 1 as follows.

**Table 2**

| SEQ ID NO (AS) | First (Antisense) Strand Base Sequence 5' → 3' | SEQ ID NO (SS) | Second (Sense) Strand Base Sequence 5' → 3' | Corresponding positions on NM_016186.3 |
|---|---|---|---|---|
| | (Shown as an Unmodified Nucleoside Sequence) | | (Shown as an Unmodified Nucleoside Sequence) | |
| SEQ ID NO: 145 | CAUGGUGGCAUUUCCUUGG UAGG | SEQ ID NO: 265 | UACCAAGGAAAUGCCAC CAUG | 1370-1391 |
| SEQ ID NO: 148 | UCGUGCCUCAUGGAGAUCU UUCG | SEQ ID NO: 268 | AAAGAUCUCCAUGAGGC ACGA | 760-781 |
| SEQ ID NO: 122 | AAAUCCUUGUGGAUGAAGG CAAA | SEQ ID NO: 242 | UGCCUUCAUCCACAAGG AUUU | 991-1012 |
| SEQ ID NO: 123 | GCCUCAUGGAGAUCUUUCG CAGC | SEQ ID NO: 243 | UGCGAAAGAUCUCCAUG AGGC | 756-777 |
| SEQ ID NO: 124 | UUCUCCAUGAGGACCACCA GCAU | SEQ ID NO: 244 | GCUGGUGGUCCUCAUGG AGAA | 1390-1411 |
| SEQ ID NO: 125 | UGCCUCAUGGAGAUCUUUC GCAG | SEQ ID NO: 245 | GCGAAAGAUCUCCAUGA GGCA | 757-778 |
| SEQ ID NO: 126 | AGCAGCUCAUGCAUCUCAU ACUU | SEQ ID NO: 246 | GUAUGAGAUGCAUGAGC UGCU | 1531-1552 |
| SEQ ID NO: 127 | UCAGGAUUAAUCUCAUCAA ACAG | SEQ ID NO: 247 | GUUUGAUGAGAUUAAUC CUGA | 1156-1177 |
| SEQ ID NO: 128 | UUGGUUUCAGGAUUAAUCU CAUC | SEQ ID NO: 248 | UGAGAUUAAUCCUGAAA CCAA | 1162-1183 |
| SEQ ID NO: 129 | GUUUCAGGAUUAAUCUCAU CAAA | SEQ ID NO: 249 | UGAUGAGAUUAAUCCUG AAAC | 1159-1180 |
| SEQ ID NO: 130 | UGGUUUCAGGAUUAAUCUC AUCA | SEQ ID NO: 250 | AUGAGAUUAAUCCUGAA ACCA | 1161-1182 |
| SEQ ID NO: 131 | GGUUUCAGGAUUAAUCUCA UCAA | SEQ ID NO: 251 | GAUGAGAUUAAUCCUGA AACC | 1160-1181 |
| SEQ ID NO: 132 | AGGAUUAAUCUCAUCAAAC AGUU | SEQ ID NO: 252 | CUGUUUGAUGAGAUUAA UCCU | 1154-1175 |
| SEQ ID NO: 133 | UCCUUGUGGAUGAAGGCAA AACU | SEQ ID NO: 253 | UUUUGCCUUCAUCCACA AGGA | 988-1009 |
| SEQ ID NO: 134 | GUGCCUCAUGGAGAUCUUU CGCA | SEQ ID NO: 254 | CGAAAGAUCUCCAUGAG GCAC | 758-779 |
| SEQ ID NO: 135 | UCUCCAUGAGGACCACCAG CAUG | SEQ ID NO: 255 | UGCUGGUGGUCCUCAUG GAGA | 1389-1410 |
| SEQ ID NO: 136 | CAAAAUCCUUGUGGAUGAA GGCA | SEQ ID NO: 256 | CCUUCAUCCACAAGGAU UUUG | 993-1014 |
| SEQ ID NO: 137 | CGUGCCUCAUGGAGAUCUU UCGC | SEQ ID NO: 257 | GAAAGAUCUCCAUGAGG CACG | 759-780 |
| SEQ ID NO: 138 | AUCAAAUCCUUGUGGAUG AAGG | SEQ ID NO: 258 | UUCAUCCACAAGGAUUU UGAU | 995-1016 |
| SEQ ID NO: 139 | UUCAGGAUUAAUCUCAUCA AACA | SEQ ID NO: 259 | UUUGAUGAGAUUAAUCC UGAA | 1157-1178 |
| SEQ ID NO: 140 | AAUCCUUGUGGAUGAAGGC AAAA | SEQ ID NO: 260 | UUGCCUUCAUCCACAAG GAUU | 990-1011 |
| SEQ ID NO: 141 | CCAUCGUGCCUCAUGGAGA UCUU | SEQ ID NO: 261 | GAUCUCCAUGAGGCACG AUGG | 763-784 |
| SEQ ID NO: 142 | UCCAUGAGGACCACCAGCA UGGU | SEQ ID NO: 262 | CAUGCUGGUGGUCCUCA UGGA | 1387-1408 |
| SEQ ID NO: 143 | AUCCUUGUGGAUGAAGGCA AAAC | SEQ ID NO: 263 | UUUGCCUUCAUCCACAA GGAU | 989-1010 |
| SEQ ID NO: 144 | AAAAUCCUUGUGGAUGAAG GCAA | SEQ ID NO: 264 | GCCUUCAUCCACAAGGA UUUU | 992-1013 |
| SEQ ID NO: 146 | UUUCAGGAUUAAUCUCAUC AAAC | SEQ ID NO: 266 | UUGAUGAGAUUAAUCCU GAAA | 1158-1179 |
| SEQ ID NO: 147 | UCAAAAUCCUUGUGGAUGA AGGC | SEQ ID NO: 267 | CUUCAUCCACAAGGAUU UUGA | 994-1015 |
| SEQ ID NO: 149 | CAGGAUUAAUCUCAUCAAA CAGU | SEQ ID NO: 269 | UGUUUGAUGAGAUUAAU CCUG | 1155-1176 |
| SEQ ID NO: 150 | CUCCAUGAGGACCACCAGC AUGG | SEQ ID NO: 270 | AUGCUGGUGGUCCUCAU GGAG | 1388-1409 |
| SEQ ID NO: 151 | CCUUGUGGAUGAAGGCAAA ACUC | SEQ ID NO: 271 | GUUUUGCCUUCAUCCAC AAGG | 987-1008 |
| SEQ ID NO: 152 | UGAGGACCACCAGCAUGGU GGCA | SEQ ID NO: 272 | CCACCAUGCUGGUGGUC CUCA | 1383-1404 |
| SEQ ID NO: 153 | AUGAGGACCACCAGCAUGG UGGC | SEQ ID NO: 273 | CACCAUGCUGGUGGUCC UCAU | 1384-1405 |
| SEQ ID NO: 154 | AUCGUGCCUCAUGGAGAUC UUUC | SEQ ID NO: 274 | AAGAUCUCCAUGAGGCA CGAU | 761-782 |
| SEQ ID NO: 155 | CUUGUGGAUGAAGGCAAAA CUCC | SEQ ID NO: 275 | AGUUUUGCCUUCAUCCA CAAG | 986-1007 |
| SEQ ID NO: 156 | CAUGAGGACCACCAGCAUG GUGG | SEQ ID NO: 276 | ACCAUGCUGGUGGUCCU CAUG | 1385-1406 |
| SEQ ID NO: 157 | CAUCGUGCCUCAUGGAGAU CUUU | SEQ ID NO: 277 | AGAUCUCCAUGAGGCAC GAUG | 762-783 |
| SEQ ID NO: 158 | CCAUGAGGACCACCAGCAU GGUG | SEQ ID NO: 278 | CCAUGCUGGUGGUCCUC AUGG | 1386-1407 |
| SEQ ID NO: 159 | UUCUUGUCAAAGGUGGAGG CAAA | SEQ ID NO: 279 | UGCCUCCACCUUUGACA AGAA | 1321-1342 |
| SEQ ID NO: 160 | AAUUCUUGUCAAAGGUGGA GGCA | SEQ ID NO: 280 | CCUCCACCUUUGACAAG AAUU | 1323-1344 |
| SEQ ID NO: 161 | UACAUCAUGGGCACCUUAA UGGU | SEQ ID NO: 281 | CAUUAAGGUGCCCAUGA UGUA | 1285-1306 |
| SEQ ID NO: 162 | GGCAUUUCCUUGGUAGGGC AGUU | SEQ ID NO: 282 | CUGCCCUACCAAGGAAA UGCC | 1364-1385 |
| SEQ ID NO: 163 | UUUCCUUGGUAGGGCAGUU UGAG | SEQ ID NO: 283 | CAAACUGCCUACCAAG GAAA | 1360-1381 |
| SEQ ID NO: 164 | UUCCUUGGUAGGCAGUUU GAGG | SEQ ID NO: 284 | UCAAACUGCCCUACCAA GGAA | 1359-1380 |
| SEQ ID NO: 165 | AAAUUCUUGUCAAAGGUGG AGGC | SEQ ID NO: 285 | CUCCACCUUUGACAAGA AUUU | 1324-1345 |
| SEQ ID NO: 166 | UUGUCCAGGUGGAAAGUGU CGAC | SEQ ID NO: 286 | CGACACUUUCCACCUGG ACAA | 1255-1276 |
| SEQ ID NO: 167 | GUACUUGUCCAGGUGGAAA GUGU | SEQ ID NO: 287 | ACUUUCCACCUGGACAA GUAC | 1259-1280 |
| SEQ ID NO: 168 | AUUUCCUUGGUAGGCAGU UUGA | SEQ ID NO: 288 | AAACUGCCCUACCAAGG AAAU | 1361-1382 |
| SEQ ID NO: 169 | GCAUUUCCUUGGUAGGGCA GUUU | SEQ ID NO: 289 | ACUGCCCUACCAAGGAA AUGC | 1363-1384 |
| SEQ ID NO: 170 | UACUUGUCCAGGUGGAAAG UGUC | SEQ ID NO: 290 | CACUUUCCACCUGGACA AGUA | 1258-1279 |
| SEQ ID NO: 171 | ACAUCAUGGGCACCUUAAU GGUC | SEQ ID NO: 291 | CCAUUAAGGUGCCCAUG AUGU | 1284-1305 |
| SEQ ID NO: 172 | UCUUGUCAAAGGUGGAGGC AAAC | SEQ ID NO: 292 | UUGCCUCCACCUUUGAC AAGA | 1320-1341 |
| SEQ ID NO: 173 | UUGUACUUGUCCAGGUGGA AAGU | SEQ ID NO: 293 | UUUCCACCUGGACAAGU ACAA | 1261-1282 |
| SEQ ID NO: 174 | CUUGGUAGGCAGUUUGAG GACA | SEQ ID NO: 294 | UCCUCAAACUGCCCUAC CAAG | 1356-1377 |
| SEQ ID NO: 175 | CAUUUCCUUGGUAGGCAG UUUG | SEQ ID NO: 295 | AACUGCCCUACCAAGGA AAUG | 1362-1383 |
| SEQ ID NO: 176 | CCUUGGUAGGCAGUUUGA GGAC | SEQ ID NO: 296 | CCUCAAACUGCCCUACC AAGG | 1357-1378 |
| SEQ ID NO: 177 | UUGGUAGGGCAGUUUGAGG ACAU | SEQ ID NO: 297 | GUCCUCAAACUGCCCUA CCAA | 1355-1376 |
| SEQ ID NO: 178 | CUUGUCCAGGUGGAAAGUG UCGA | SEQ ID NO: 298 | GACACUUUCCACCUGGA CAAG | 1256-1277 |
| SEQ ID NO: 179 | CUUGUCAAAGGUGGAGGCA AACU | SEQ ID NO: 299 | UUUGCCUCCACCUUUGA CAAG | 1319-1340 |
| SEQ ID NO: 180 | ACUUGUCCAGGUGGAAAGU GUCG | SEQ ID NO: 300 | ACACUUUCCACCUGGAC AAGU | 1257-1278 |
| SEQ ID NO: 181 | CUUGUACUUGUCCAGGUGG AAAG | SEQ ID NO: 301 | UUCCACCUGGACAAGUA CAAG | 1262-1283 |
| SEQ ID NO: 182 | UGUACUUGUCCAGGUGGAA AGUG | SEQ ID NO: 302 | CUUUCCACCUGGACAAG UACA | 1260-1281 |
| SEQ ID NO: 183 | UCCUUGGUAGGGCAGUUUG AGGA | SEQ ID NO: 303 | CUCAAACUGCCCUACCA AGGA | 1358-1379 |
| SEQ ID NO: 184 | UUCCCUUUGAACAAGAUGU AAUC | SEQ ID NO: 304 | UUACAUCUUGUUCAAAG GGAA | 1198-1219 |
| SEQ ID NO: 185 | AGGACCACCAGCAUGGUGG CAUU | SEQ ID NO: 305 | UGCCACCAUGCUGGUGG UCCU | 1381-1402 |
| SEQ ID NO: 186 | UAUCAAAUACCUCUUGGA UAAA | SEQ ID NO: 306 | UAUCCAAGAGGUAUUUU GAUA | 1038-1059 |
| SEQ ID NO: 187 | ACCACCAGCAUGGUGGCAU UUCC | SEQ ID NO: 307 | AAAUGCCACCAUGCUGG UGGU | 1378-1399 |
| SEQ ID NO: 188 | UCCCUUUGAACAAGAUGUA AUCC | SEQ ID NO: 308 | AUUACAUCUUGUUCAAA GGGA | 1197-1218 |
| SEQ ID NO: 189 | UUGCCAUCGUGCCUCAUGG AGAU | SEQ ID NO: 309 | CUCCAUGAGGCACGAUG GCAA | 766-787 |
| SEQ ID NO: 190 | UUUGAUGACAGGAGGCAUG GAAU | SEQ ID NO: 310 | UCCAUGCCUCCUGUCAU CAAA | 1721-1742 |
| SEQ ID NO: 191 | UGAUGACAGGAGGCAUGGA AUAA | SEQ ID NO: 311 | AUUCCAUGCCUCCUGUC AUCA | 1719-1740 |
| SEQ ID NO: 192 | CAGCAUGGUGGCAUUUCCU UGGU | SEQ ID NO: 312 | CAAGGAAAUGCCACCAU GCUG | 1373-1394 |
| SEQ ID NO: 193 | UUUUCUCCAUGAGGACCAC CAGC | SEQ ID NO: 313 | UGGUGGUCCUCAUGGAG AAAA | 1392-1413 |
| SEQ ID NO: 194 | CCAUUUCCCUUUGAACAAG AUGU | SEQ ID NO: 314 | AUCUUGUUCAAAGGGAA AUGG | 1202-1223 |
| SEQ ID NO: 195 | CCAGCAUGGUGGCAUUUCC UUGG | SEQ ID NO: 315 | AAGGAAAUGCCACCAUG CUGG | 1374-1395 |
| SEQ ID NO: 196 | AUUCUUGUCAAAGGUGGAG GCAA | SEQ ID NO: 316 | GCCUCCACCUUUGACAA GAAU | 1322-1343 |
| SEQ ID NO: 197 | UUGUGGAUGAAGGCAAAAC UCCC | SEQ ID NO: 317 | GAGUUUUGCCUUCAUCC ACAA | 985-1006 |
| SEQ ID NO: 198 | CUCAUGGAGAUCUUUCGCA GCAG | SEQ ID NO: 318 | GCUGCGAAAGAUCUCCA UGAG | 754-775 |
| SEQ ID NO: 199 | UGUCAAAGGUGGAGGCAAA CUUG | SEQ ID NO: 319 | AGUUUGCCUCCACCUUU GACA | 1317-1338 |
| SEQ ID NO: 200 | UUGUCAAAGGUGGAGGCAA ACUU | SEQ ID NO: 320 | GUUUGCCUCCACCUUUG ACAA | 1318-1339 |
| SEQ ID NO: 201 | CAUUUCCCUUUGAACAAGA UGUA | SEQ ID NO: 321 | CAUCUUGUUCAAAGGA AAUG | 1201-1222 |
| SEQ ID NO: 202 | UGUGGAUGAAGGCAAAACU CCCC | SEQ ID NO: 322 | GGAGUUUUGCCUUCAUC CACA | 984-1005 |
| SEQ ID NO: 203 | CCUCAUGGAGAUCUUUCGC AGCA | SEQ ID NO: 323 | CUGCGAAAGAUCUCCAU GAGG | 755-776 |
| SEQ ID NO: 204 | CUUUGAUGACAGGAGGCAU GGAA | SEQ ID NO: 324 | CCAUGCCUCCUGUCAUC AAAG | 1722-1743 |
| SEQ ID NO: 205 | UCACCACCCUGCCCAGAAA CAGA | SEQ ID NO: 325 | UGUUUCUGGGCAGGGUG GUGA | 1794-1815 |
| SEQ ID NO: 206 | UGGAGAUCUUUCGCAGCAG GCUG | SEQ ID NO: 326 | GCCUGCUGCGAAAGAUC UCCA | 750-771 |
| SEQ ID NO: 207 | GAUUAAUCUCAUCAAACAG UUUG | SEQ ID NO: 327 | AACUGUUUGAUGAGAUU AAUC | 1152-1173 |
| SEQ ID NO: 208 | CACUUUGAUGACAGGAGGC AUGG | SEQ ID NO: 328 | AUGCCUCCUGUCAUCAA AGUG | 1724-1745 |
| SEQ ID NO: 209 | GCAGCUCAUGCAUCUCAUA CUUC | SEQ ID NO: 329 | AGUAUGAGAUGCAUGAG CUGC | 1530-1551 |
| SEQ ID NO: 210 | CAGCUCAUGCAUCUCAUAC UUCU | SEQ ID NO: 330 | AAGUAUGAGAUGCAUGA GCUG | 1529-1550 |
| SEQ ID NO: 211 | UGGUAGGGCAGUUUGAGGA CAUG | SEQ ID NO: 331 | UGUCCUCAAACUGCCCU ACCA | 1354-1375 |
| SEQ ID NO: 212 | GGAUUAAUCUCAUCAAACA GUUU | SEQ ID NO: 332 | ACUGUUUGAUGAGAUUA AUCC | 1153-1174 |
| SEQ ID NO: 213 | AUCAUGGGCACCUUAAUGG UCUU | SEQ ID NO: 333 | GACCAUUAAGGUGCCCA UGAU | 1282-1303 |
| SEQ ID NO: 214 | CAUCAUGGGCACCUUAAUG GUCU | SEQ ID NO: 334 | ACCAUUAAGGUGCCCAU GAUG | 1283-1304 |
| SEQ ID NO: 215 | UUCACCACCCUGCCCAGAA ACAG | SEQ ID NO: 335 | GUUUCUGGCAGGGUGG UGAA | 1795-1816 |
| SEQ ID NO: 216 | CCACCCUGCCCAGAAACAG AAGC | SEQ ID NO: 336 | UUCUGUUUCUGGGCAGG GUGG | 1791-1812 |
| SEQ ID NO: 217 | GCUUGAACUUCGGAAAGAA AACU | SEQ ID NO: 337 | UUUUCUUUCCGAAGUUC AAGC | 1500-1521 |
| SEQ ID NO: 218 | AGCUUGAACUUCGGAAAGA AAAC | SEQ ID NO: 338 | UUUCUUUCCGAAGUUCA AGCU | 1501-1522 |
| SEQ ID NO: 219 | ACCACCCUGCCCAGAAACA GAAG | SEQ ID NO: 339 | UCUGUUUCUGGGCAGGG UGGU | 1792-1813 |
| SEQ ID NO: 220 | GGUAGGGCAGUUUGAGGAC AUGA | SEQ ID NO: 340 | AUGUCCUCAAACUGCCC UACC | 1353-1374 |
| SEQ ID NO: 221 | UGUCCAGGUGGAAAGUGUC GACU | SEQ ID NO: 341 | UCGACACUUUCCACCUG GACA | 1254-1275 |
| SEQ ID NO: 222 | AAAUCCUUGUGGAUGAAGG | SEQ ID NO: 342 | CCUUCAUCCACAAGGAU UU | 995-1014 |
| SEQ ID NO: 223 | GCCUCAUGGAGAUCUUUCG | SEQ ID NO: 343 | CGAAAGAUCUCCAUGAG GC | 760-779 |
| SEQ ID NO: 224 | UUCUCCAUGAGGACCACCA | SEQ ID NO: 344 | UGGUGGUCCUCAUGGAG AA | 1394-1413 |
| SEQ ID NO: 225 | UGCCUCAUGGAGAUCUUUC | SEQ ID NO: 345 | GAAAGAUCUCCAUGAGG CA | 761-780 |
| SEQ ID NO: 226 | AGCAGCUCAUGCAUCUCAU | SEQ ID NO: 346 | AUGAGAUGCAUGAGCUG CU | 1535-1554 |
| SEQ ID NO: 227 | UCAGGAUUAAUCUCAUCAA | SEQ ID NO: 347 | UUGAUGAGAUUAAUCCU GA | 1160-1179 |
| SEQ ID NO: 228 | UUGGUUUCAGGAUUAAUCU | SEQ ID NO: 348 | AGAUUAAUCCUGAAACC AA | 1166-1185 |
| SEQ ID NO: 229 | GUUUCAGGAUUAAUCUCAU | SEQ ID NO: 349 | AUGAGAUUAAUCCUGAA AC | 1163-1182 |
| SEQ ID NO: 230 | UGGUUUCAGGAUUAAUCUC | SEQ ID NO: 350 | GAGAUUAAUCCUGAAAC CA | 1165-1184 |
| SEQ ID NO: 231 | GGUUUCAGGAUUAAUCUCA | SEQ ID NO: 351 | UGAGAUUAAUCCUGAAA CC | 1164-1183 |
| SEQ ID NO: 232 | AGGAUUAAUCUCAUCAAAC | SEQ ID NO: 352 | GUUUGAUGAGAUUAAUC CU | 1158-1177 |
| SEQ ID NO: 233 | UCCUUGUGGAUGAAGGCAA | SEQ ID NO: 353 | UUGCCUUCAUCCACAAG GA | 992-1011 |
| SEQ ID NO: 234 | GUGCCUCAUGGAGAUCUUU | SEQ ID NO: 354 | AAAGAUCUCCAUGAGGC AC | 762-781 |
| SEQ ID NO: 235 | UCUCCAUGAGGACCACCAG | SEQ ID NO: 355 | CUGGUGGUCCUCAUGGA GA | 1393-1412 |
| SEQ ID NO: 236 | CAAAAUCCUUGUGGAUGAA | SEQ ID NO: 356 | UUCAUCCACAAGGAUUU UG | 997-1016 |
| SEQ ID NO: 237 | CGUGCCUCAUGGAGAUCUU | SEQ ID NO: 357 | AAGAUCUCCAUGAGGCA CG | 763-782 |
| SEQ ID NO: 238 | AUCAAAAUCCUUGUGGAUG | SEQ ID NO: 358 | CAUCCACAAGGAUUUUG AU | 999-1018 |
| SEQ ID NO: 239 | UUCAGGAUUAAUCUCAUCA | SEQ ID NO: 359 | UGAUGAGAUUAAUCCUG AA | 1161-1180 |
| SEQ ID NO: 240 | AAUCCUUGUGGAUGAAGGC | SEQ ID NO: 360 | GCCUUCAUCCACAAGGA UU | 994-1013 |
| SEQ ID NO: 241 | CCAUCGUGCCUCAUGGAGA | SEQ ID NO: 361 | UCUCCAUGAGGCACGAU GG | 767-786 |
| SEQ ID NO: 787 | AAAGUCCUUGUGGAUGAAG GCAA | SEQ ID NO: 791 | GCCUUCAUCCACAAGGA CUUU | NA |
| SEQ ID NO: 788 | AACUUCUUGUCAAAGGUGG AGGC | SEQ ID NO: 792 | CUCCACCUUUGACAAGA AGUU | NA |
| SEQ ID NO: 789 | UGUGGAUGAAGGCAAAGCU ACCC | SEQ ID NO: 793 | GUAGCUUUGCCUUCAUC CACA | NA |

Table 3 provides the modified first (antisense) sequences, together with the corresponding unmodified first (antisense) sequences for siRNA oligonucleosides according to the present invention as follows.

**Table 3**

| Antisense strand ID | Modified First (Antisense) Strand 5' → 3' | SEQ ID NO (AS - mod) | Underlying Base Sequence 5' → 3' | SEQ ID NO (AS - unmod) |
|---|---|---|---|---|
| | | | (Shown as an Unmodified Nucleoside Sequence) | |
| ETXS1036 | CmsAfsUmGfGmUfGmGmCmAmUmUm UmCfCmUfUmGmGmUmAmsGmsGm | SEQ ID NO: 764 | CAUGGUGGCAUUUCC UUGGUAGG | SEQ ID NO: 145 |
| ETXS1040 | UmsCfsGmUfGmCfCmUmCmAmUmGm GmAfGmAfUmCmUmUmUmsCmsGm | SEQ ID NO: 766 | UCGUGCCUCAUGGAG AUCUUUCG | SEQ ID NO: 148 |
| ETXS632 | AmsAfsAmUmCmCfUmUfGfUmGmGm AmUfGmAfAmGmGmCmAmsAmsAm | SEQ ID NO: 362 | AAAUCCUUGUGGAUG AAGGCAAA | SEQ ID NO: 122 |
| ETXS634 | GmsCfsCmUmCmAfUmGfGfAmGmAm UmCfUmUfUmCmGmCmAmsGmsCm | SEQ ID NO: 363 | GCCUCAUGGAGAUCU UUCGCAGC | SEQ ID NO: 123 |
| ETXS636 | UmsUfsCmUmCmCfAmUfGfAmGmGm AmCfCmAfCmCmAmGmCmsAmsUm | SEQ ID NO: 364 | UUCUCCAUGAGGACC ACCAGCAU | SEQ ID NO: 124 |
| ETXS638 | UmsGfsCmCmUmCfAmUfGfGmAmGm AmUfCmUfUmUmCmGmCmsAmsGm | SEQ ID NO: 365 | UGCCUCAUGGAGAUC UUUCGCAG | SEQ ID NO: 125 |
| ETXS640 | AmsGfsCmAmGmCfUmCfAfUmGmCm AmUfCmUfCmAmUmAmCmsUmsUm | SEQ ID NO: 366 | AGCAGCUCAUGCAUC UCAUACUU | SEQ ID NO: 126 |
| ETXS642 | UmsCfsAmGmGmAfUmUfAfAmUmCm UmCfAmUfCmAmAmAmCmsAmsGm | SEQ ID NO: 367 | UCAGGAUUAAUCUCA UCAAACAG | SEQ ID NO: 127 |
| ETXS644 | UmsUfsGmGmUmUfUmCfAfGmGmAm UmUfAmAfUmCmUmCmAmsUmsCm | SEQ ID NO: 368 | UUGGUUUCAGGAUUA AUCUCAUC | SEQ ID NO: 128 |
| ETXS646 | GmsUfsUmUmCmAfGmGfAfUmUmAm AmUfCmUfCmAmUmCmAmsAmsAm | SEQ ID NO: 369 | GUUUCAGGAUUAAUC UCAUCAAA | SEQ ID NO: 129 |
| ETXS648 | UmsGfsGmUmUmUfCmAfGfGmAmUm UmAfAmUfCmUmCmAmUmsCmsAm | SEQ ID NO: 370 | UGGUUUCAGGAUUAA UCUCAUCA | SEQ ID NO: 130 |
| ETXS650 | GmsGfsUmUmUmCfAmGfGfAmUmUm AmAfUmCfUmCmAmUmCmsAmsAm | SEQ ID NO: 371 | GGUUUCAGGAUUAAU CUCAUCAA | SEQ ID NO: 131 |
| ETXS652 | AmsGfsGmAmUmUfAmAfUfCmUmCm AmUfCmAfAmAmCmAmGmsUmsUm | SEQ ID NO: 372 | AGGAUUAAUCUCAUC AAACAGUU | SEQ ID NO: 132 |
| ETXS654 | UmsCfsCmUmUmGfUmGfGfAmUmGm AmAfGmGfCmAmAmAmAmsCmsUm | SEQ ID NO: 373 | UCCUUGUGGAUGAAG GCAAAACU | SEQ ID NO: 133 |
| ETXS656 | GmsUfsGmCmCmUfCmAfUfGmGmAm GmAfUmCfUmUmUmCmGmsCmsAm | SEQ ID NO: 374 | GUGCCUCAUGGAGAU CUUUCGCA | SEQ ID NO: 134 |
| ETXS658 | UmsCfsUmCmCmAfUmGfAfGmGmAm CmCfAmCfCmAmGmCmAmsUmsGm | SEQ ID NO: 375 | UCUCCAUGAGGACCA CCAGCAUG | SEQ ID NO: 135 |
| ETXS660 | CmsAfsAmAmAmUfCmCfUfUmGmUm GmGfAmUfGmAmAmGmGmsCmsAm | SEQ ID NO: 376 | CAAAAUCCUUGUGGA UGAAGGCA | SEQ ID NO: 136 |
| ETXS662 | CmsGfsUmGmCmCfUmCfAfUmGmGm AmGfAmUfCmUmUmUmCmsGmsCm | SEQ ID NO: 377 | CGUGCCUCAUGGAGA UCUUUCGC | SEQ ID NO: 137 |
| ETXS664 | AmsUfsCmAmAmAfAmUfCfCmUmUm GmUfGmGfAmUmGmAmAmsGmsGm | SEQ ID NO: 378 | AUCAAAAUCCUUGUG GAUGAAGG | SEQ ID NO: 138 |
| ETXS666 | UmsUfsCmAmGmGfAmUfUfAmAmUm CmUfCmAfUmCmAmAmAmsCmsAm | SEQ ID NO: 379 | UUCAGGAUUAAUCUC AUCAAACA | SEQ ID NO: 139 |
| ETXS668 | AmsAfsUmCmCmUfUmGfUfGmGmAm UmGfAmAfGmGmCmAmAmsAmsAm | SEQ ID NO: 380 | AAUCCUUGUGGAUGA AGGCAAAA | SEQ ID NO: 140 |
| ETXS670 | CmsCfsAmUmCmGfUmGfCfCmUmCm AmUfGmGfAmGmAmUmCmsUmsUm | SEQ ID NO: 381 | CCAUCGUGCCUCAUG GAGAUCUU | SEQ ID NO: 141 |
| ETXS672 | UmsCfsCmAmUmGfAmGfGfAmCmCm AmCfCmAfGmCmAmUmGmsGmsUm | SEQ ID NO: 382 | UCCAUGAGGACCACC AGCAUGGU | SEQ ID NO: 142 |
| ETXS674 | AmsUfsCmCmUmUfGmUfGfGmAmUm GmAfAmGfGmCmAmAmAmsAmsCm | SEQ ID NO: 383 | AUCCUUGUGGAUGAA GGCAAAAC | SEQ ID NO: 143 |
| ETXS676 | AmsAfsAmAmUmCfCmUfUfGmUmGm GmAfUmGfAmAmGmGmCmsAmsAm | SEQ ID NO: 384 | AAAAUCCUUGUGGAU GAAGGCAA | SEQ ID NO: 144 |
| ETXS678 | CmsAfsUmGmGmUfGmGfCfAmUmUm UmCfCmUfUmGmGmUmAmsGmsGm | SEQ ID NO: 385 | CAUGGUGGCAUUUCC UUGGUAGG | SEQ ID NO: 145 |
| ETXS680 | UmsUfsUmCmAmGfGmAfUfUmAmAm UmCfUmCfAmUmCmAmAmsAmsCm | SEQ ID NO: 386 | UUUCAGGAUUAAUCU CAUCAAAC | SEQ ID NO: 146 |
| ETXS682 | UmsCfsAmAmAmAfUmCfCfUmUmGm UmGfGmAfUmGmAmAmGmsGmsCm | SEQ ID NO: 387 | UCAAAAUCCUUGUGG AUGAAGGC | SEQ ID NO: 147 |
| ETXS684 | UmsCfsGmUmGmCfCmUfCfAmUmGm GmAfGmAfUmCmUmUmUmsCmsGm | SEQ ID NO: 388 | UCGUGCCUCAUGGAG AUCUUUCG | SEQ ID NO: 148 |
| ETXS686 | CmsAfsGmGmAmUfUmAfAfUmCmUm CmAfUmCfAmAmAmCmAmsGmsUm | SEQ ID NO: 389 | CAGGAUUAAUCUCAU CAAACAGU | SEQ ID NO: 149 |
| ETXS688 | CmsUfsCmCmAmUfGmAfGfGmAmCm CmAfCmCfAmGmCmAmUmsGmsGm | SEQ ID NO: 390 | CUCCAUGAGGACCAC CAGCAUGG | SEQ ID NO: 150 |
| ETXS690 | CmsCfsUmUmGmUfGmGfAfUmGmAm AmGfGmCfAmAmAmAmCmsUmsCm | SEQ ID NO: 391 | CCUUGUGGAUGAAGG CAAAACUC | SEQ ID NO: 151 |
| ETXS692 | UmsGfsAmGmGmAfCmCfAfCmCmAm GmCfAmUfGmGmUmGmGmsCmsAm | SEQ ID NO: 392 | UGAGGACCACCAGCA UGGUGGCA | SEQ ID NO: 152 |
| ETXS694 | AmsUfsGmAmGmGfAmCfCfAmCmCm AmGfCmAfUmGmGmUmGmsGmsCm | SEQ ID NO: 393 | AUGAGGACCACCAGC AUGGUGGC | SEQ ID NO: 153 |
| ETXS696 | AmsUfsCmGmUmGfCmCfUfCmAmUm GmGfAmGfAmUmCmUmUmsUmsCm | SEQ ID NO: 394 | AUCGUGCCUCAUGGA GAUCUUUC | SEQ ID NO: 154 |
| ETXS698 | CmsUfsUmGmUmGfGmAfUfGmAmAm GmGfCmAfAmAmAmCmUmsCmsCm | SEQ ID NO: 395 | CUUGUGGAUGAAGGC AAAACUCC | SEQ ID NO: 155 |
| ETXS700 | CmsAfsUmGmAmGfGmAfCfCmAmCm CmAfGmCfAmUmGmGmUmsGmsGm | SEQ ID NO: 396 | CAUGAGGACCACCAG CAUGGUGG | SEQ ID NO: 156 |
| ETXS702 | CmsAfsUmCmGmUfGmCfCfUmCmAm UmGfGmAfGmAmUmCmUmsUmsUm | SEQ ID NO: 397 | CAUCGUGCCUCAUGG AGAUCUUU | SEQ ID NO: 157 |
| ETXS704 | CmsCfsAmUmGmAfGmGfAfCmCmAm CmCfAmGfCmAmUmGmGmsUmsGm | SEQ ID NO: 398 | CCAUGAGGACCACCA GCAUGGUG | SEQ ID NO: 158 |
| ETXS706 | UmsUfsCmUmUmGfUmCfAfAmAmGm GmUfGmGfAmGmGmCmAmsAmsAm | SEQ ID NO: 399 | UUCUUGUCAAAGGUG GAGGCAAA | SEQ ID NO: 159 |
| ETXS708 | AmsAfsUmUmCmUfUmGfUfCmAmAm AmGfGmUfGmGmAmGmGmsCmsAm | SEQ ID NO: 400 | AAUUCUUGUCAAAGG UGGAGGCA | SEQ ID NO: 160 |
| ETXS710 | UmsAfsCmAmUmCfAmUfGfGmGmCm AmCfCmUfUmAmAmUmGmsGmsUm | SEQ ID NO: 401 | UACAUCAUGGGCACC UUAAUGGU | SEQ ID NO: 161 |
| ETXS712 | GmsGfsCmAmUmUfUmCfCfUmUmGm GmUfAmGfGmGmCmAmGmsUmsUm | SEQ ID NO: 402 | GGCAUUUCCUUGGUA GGGCAGUU | SEQ ID NO: 162 |
| ETXS714 | UmsUfsUmCmCmUfUmGfGfUmAmGm GmGfCmAfGmUmUmUmGmsAmsGm | SEQ ID NO: 403 | UUUCCUUGGUAGGGC AGUUUGAG | SEQ ID NO: 163 |
| ETXS716 | UmsUfsCmCmUmUfGmGfUfAmGmGm GmCfAmGfUmUmUmGmAmsGmsGm | SEQ ID NO: 404 | UUCCUUGGUAGGGCA GUUUGAGG | SEQ ID NO: 164 |
| ETXS718 | AmsAfsAmUmUmCfUmUfGfUmCmAm AmAfGmGfUmGmGmAmGmsGmsCm | SEQ ID NO: 405 | AAAUUCUUGUCAAAG GUGGAGGC | SEQ ID NO: 165 |
| ETXS720 | UmsUfsGmUmCmCfAmGfGfUmGmGm AmAfAmGfUmGmUmCmGmsAmsCm | SEQ ID NO: 406 | UUGUCCAGGUGGAAA GUGUCGAC | SEQ ID NO: 166 |
| ETXS722 | GmsUfsAmCmUmUfGmUfCfCmAmGm GmUfGmGfAmAmAmGmUmsGmsUm | SEQ ID NO: 407 | GUACUUGUCCAGGUG GAAAGUGU | SEQ ID NO: 167 |
| ETXS724 | AmsUfsUmUmCmCfUmUfGfGmUmAm GmGfGmCfAmGmUmUmUmsGmsAm | SEQ ID NO: 408 | AUUUCCUUGGUAGGG CAGUUUGA | SEQ ID NO: 168 |
| ETXS726 | GmsCfsAmUmUmUfCmCfUfUmGmGm UmAfGmGfGmCmAmGmUmsUmsUm | SEQ ID NO: 409 | GCAUUUCCUUGGUAG GGCAGUUU | SEQ ID NO: 169 |
| ETXS728 | UmsAfsCmUmUmGfUmCfCfAmGmGm UmGfGmAfAmAmGmUmGmsUmsCm | SEQ ID NO: 410 | UACUUGUCCAGGUGG AAAGUGUC | SEQ ID NO: 170 |
| ETXS730 | AmsCfsAmUmCmAfUmGfGfGmCmAm CmCfUmUfAmAmUmGmGmsUmsCm | SEQ ID NO: 411 | ACAUCAUGGGCACCU UAAUGGUC | SEQ ID NO: 171 |
| ETXS732 | UmsCfsUmUmGmUfCmAfAfAmGmGm UmGfGmAfGmGmCmAmAmsAmsCm | SEQ ID NO: 412 | UCUUGUCAAAGGUGG AGGCAAAC | SEQ ID NO: 172 |
| ETXS734 | UmsUfsGmUmAmCfUmUfGfUmCmCm AmGfGmUfGmGmAmAmAmsGmsUm | SEQ ID NO: 413 | UUGUACUUGUCCAGG UGGAAAGU | SEQ ID NO: 173 |
| ETXS736 | CmsUfsUmGmGmUfAmGfGfGmCmAm GmUfUmUfGmAmGmGmAmsCmsAm | SEQ ID NO: 414 | CUUGGUAGGGCAGUU UGAGGACA | SEQ ID NO: 174 |
| ETXS738 | CmsAfsUmUmUmCfCmUfUfGmGmUm AmGfGmGfCmAmGmUmUmsUmsGm | SEQ ID NO: 415 | CAUUUCCUUGGUAGG GCAGUUUG | SEQ ID NO: 175 |
| ETXS740 | CmsCfsUmUmGmGfUmAfGfGmGmCm AmGfUmUfUmGmAmGmGmsAmsCm | SEQ ID NO: 416 | CCUUGGUAGGGCAGU UUGAGGAC | SEQ ID NO: 176 |
| ETXS742 | UmsUfsGmGmUmAfGmGfGfCmAmGm UmUfUmGfAmGmGmAmCmsAmsUm | SEQ ID NO: 417 | UUGGUAGGCAGUUU GAGGACAU | SEQ ID NO: 177 |
| ETXS744 | CmsUfsUmGmUmCfCmAfGfGmUmGm GmAfAmAfGmUmGmUmCmsGmsAm | SEQ ID NO: 418 | CUUGUCCAGGUGGAA AGUGUCGA | SEQ ID NO: 178 |
| ETXS746 | CmsUfsUmGmUmCfAmAfAfGmGmUm GmGfAmGfGmCmAmAmAmsCmsUm | SEQ ID NO: 419 | CUUGUCAAAGGUGGA GGCAAACU | SEQ ID NO: 179 |
| ETXS748 | AmsCfsUmUmGmUfCmCfAfGmGmUm GmGfAmAfAmGmUmGmUmsCmsGm | SEQ ID NO: 420 | ACUUGUCCAGGUGGA AAGUGUCG | SEQ ID NO: 180 |
| ETXS750 | CmsUfsUmGmUmAfCmUfUfGmUmCm CmAfGmGfUmGmGmAmAmsAmsGm | SEQ ID NO: 421 | CUUGUACUUGUCCAG GUGGAAAG | SEQ ID NO: 181 |
| ETXS752 | UmsGfsUmAmCmUfUmGfUfCmCmAm GmGfUmGfGmAmAmAmGmsUmsGm | SEQ ID NO: 422 | UGUACUUGUCCAGGU GGAAAGUG | SEQ ID NO: 182 |
| ETXS754 | UmsCfsCmUmUmGfGmUfAfGmGmGm CmAfGmUfUmUmGmAmGmsGmsAm | SEQ ID NO: 423 | UCCUUGGUAGGGCAG UUUGAGGA | SEQ ID NO: 183 |
| ETXS756 | UmsUfsCmCmCmUfUmUfGfAmAmCm AmAfGmAfUmGmUmAmAmsUmsCm | SEQ ID NO: 424 | UUCCCUUUGAACAAG AUGUAAUC | SEQ ID NO: 184 |
| ETXS758 | AmsGfsGmAmCmCfAmCfCfAmGmCm AmUfGmGfUmGmGmCmAmsUmsUm | SEQ ID NO: 425 | AGGACCACCAGCAUG GUGGCAUU | SEQ ID NO: 185 |
| ETXS760 | UmsAfsUmCmAmAfAmAfUfAmCmCm UmCfUmUfGmGmAmUmAmsAmsAm | SEQ ID NO: 426 | UAUCAAAAUACCUCU UGGAUAAA | SEQ ID NO: 186 |
| ETXS762 | AmsCfsCmAmCmCfAmGfCfAmUmGm GmUfGmGfCmAmUmUmUmsCmsCm | SEQ ID NO: 427 | ACCACCAGCAUGGUG GCAUUUCC | SEQ ID NO: 187 |
| ETXS764 | UmsCfsCmCmUmUfUmGfAfAmCmAm AmGfAmUfGmUmAmAmUmsCmsCm | SEQ ID NO: 428 | UCCCUUUGAACAAGA UGUAAUCC | SEQ ID NO: 188 |
| ETXS766 | UmsUfsGmCmCmAfUmCfGfUmGmCm CmUfCmAfUmGmGmAmGmsAmsUm | SEQ ID NO: 429 | UUGCCAUCGUGCCUC AUGGAGAU | SEQ ID NO: 189 |
| ETXS768 | UmsUfsUmGmAmUfGmAfCfAmGmGm AmGfGmCfAmUmGmGmAmsAmsUm | SEQ ID NO: 430 | UUUGAUGACAGGAGG CAUGGAAU | SEQ ID NO: 190 |
| ETXS770 | UmsGfsAmUmGmAfCmAfGfGmAmGm GmCfAmUfGmGmAmAmUmsAmsAm | SEQ ID NO: 431 | UGAUGACAGGAGGCA UGGAAUAA | SEQ ID NO: 191 |
| ETXS772 | CmsAfsGmCmAmUfGmGfUfGmGmCm AmUfUmUfCmCmUmUmGmsGmsUm | SEQ ID NO: 432 | CAGCAUGGUGGCAUU UCCUUGGU | SEQ ID NO: 192 |
| ETXS774 | UmsUfsUmUmCmUfCmCfAfUmGmAm GmGfAmCfCmAmCmCmAmsGmsCm | SEQ ID NO: 433 | UUUUCUCCAUGAGGA CCACCAGC | SEQ ID NO: 193 |
| ETXS776 | CmsCfsAmUmUmUfCmCfCfUmUmUm GmAfAmCfAmAmGmAmUmsGmsUm | SEQ ID NO: 434 | CCAUUUCCCUUUGAA CAAGAUGU | SEQ ID NO: 194 |
| ETXS778 | CmsCfsAmGmCmAfUmGfGfUmGmGm CmAfUmUfUmCmCmUmUmsGmsGm | SEQ ID NO: 435 | CCAGCAUGGUGGCAU UUCCUUGG | SEQ ID NO: 195 |
| ETXS780 | AmsUfsUmCmUmUfGmUfCfAmAmAm GmGfUmGfGmAmGmGmCmsAmsAm | SEQ ID NO: 436 | AUUCUUGUCAAAGGU GGAGGCAA | SEQ ID NO: 196 |
| ETXS782 | UmsUfsGmUmGmGfAmUfGfAmAmGm GmCfAmAfAmAmCmUmCmsCmsCm | SEQ ID NO: 437 | UUGUGGAUGAAGGCA AAACUCCC | SEQ ID NO: 197 |
| ETXS784 | CmsUfsCmAmUmGfGmAfGfAmUmCm UmUfUmCfGmCmAmGmCmsAmsGm | SEQ ID NO: 438 | CUCAUGGAGAUCUUU CGCAGCAG | SEQ ID NO: 198 |
| ETXS786 | UmsGfsUmCmAmAfAmGfGfUmGmGm AmGfGmCfAmAmAmCmUmsUmsGm | SEQ ID NO: 439 | UGUCAAAGGUGGAGG CAAACUUG | SEQ ID NO: 199 |
| ETXS788 | UmsUfsGmUmCmAfAmAfGfGmUmGm GmAfGmGfCmAmAmAmCmsUmsUm | SEQ ID NO: 440 | UUGUCAAAGGUGGAG GCAAACUU | SEQ ID NO: 200 |
| ETXS790 | CmsAfsUmUmUmCfCmCfUfUmUmGm AmAfCmAfAmGmAmUmGmsUmsAm | SEQ ID NO: 441 | CAUUUCCCUUUGAAC AAGAUGUA | SEQ ID NO: 201 |
| ETXS792 | UmsGfsUmGmGmAfUmGfAfAmGmGm CmAfAmAfAmCmUmCmCmsCmsCm | SEQ ID NO: 442 | UGUGGAUGAAGGCAA AACUCCCC | SEQ ID NO: 202 |
| ETXS794 | CmsCfsUmCmAmUfGmGfAfGmAmUm CmUfUmUfCmGmCmAmGmsCmsAm | SEQ ID NO: 443 | CCUCAUGGAGAUCUU UCGCAGCA | SEQ ID NO: 203 |
| ETXS796 | CmsUfsUmUmGmAfUmGfAfCmAmGm GmAfGmGfCmAmUmGmGmsAmsAm | SEQ ID NO: 444 | CUUUGAUGACAGGAG GCAUGGAA | SEQ ID NO: 204 |
| ETXS798 | UmsCfsAmCmCmAfCmCfCfUmGmCmC mCfAmGfAmAmAmCmAmsGmsAm | SEQ ID NO: 445 | UCACCACCCUGCCCA GAAACAGA | SEQ ID NO: 205 |
| ETXS800 | UmsGfsGmAmGmAfUmCfUfUmUmCm GmCfAmGfCmAmGmGmCmsUmsGm | SEQ ID NO: 446 | UGGAGAUCUUUCGCA GCAGGCUG | SEQ ID NO: 206 |
| ETXS802 | GmsAfsUmUmAmAfUmCfUfCmAmUm CmAfAmAfCmAmGmUmUmsUmsGm | SEQ ID NO: 447 | GAUUAAUCUCAUCAA ACAGUUUG | SEQ ID NO: 207 |
| ETXS804 | CmsAfsCmUmUmUfGmAfUfGmAmCm AmGfGmAfGmGmCmAmUmsGmsGm | SEQ ID NO: 448 | CACUUUGAUGACAGG AGGCAUGG | SEQ ID NO: 208 |
| ETXS806 | GmsCfsAmGmCmUfCmAfUfGmCmAm UmCfUmCfAmUmAmCmUmsUmsCm | SEQ ID NO: 449 | GCAGCUCAUGCAUCU CAUACUUC | SEQ ID NO: 209 |
| ETXS808 | CmsAfsGmCmUmCfAmUfGfCmAmUm CmUfCmAfUmAmCmUmUmsCmsUm | SEQ ID NO: 450 | CAGCUCAUGCAUCUC AUACUUCU | SEQ ID NO: 210 |
| ETXS810 | UmsGfsGmUmAmGfGmGfCfAmGmUm UmUfGmAfGmGmAmCmAmsUmsGm | SEQ ID NO: 451 | UGGUAGGGCAGUUUG AGGACAUG | SEQ ID NO: 211 |
| ETXS812 | GmsGfsAmUmUmAfAmUfCfUmCmAm UmCfAmAfAmCmAmGmUmsUmsUm | SEQ ID NO: 452 | GGAUUAAUCUCAUCA AACAGUUU | SEQ ID NO: 212 |
| ETXS814 | AmsUfsCmAmUmGfGmGfCfAmCmCm UmUfAmAfUmGmGmUmCmsUmsUm | SEQ ID NO: 453 | AUCAUGGGCACCUUA AUGGUCUU | SEQ ID NO: 213 |
| ETXS816 | CmsAfsUmCmAmUfGmGfGfCmAmCm CmUfUmAfAmUmGmGmUmsCmsUm | SEQ ID NO: 454 | CAUCAUGGGCACCUU AAUGGUCU | SEQ ID NO: 214 |
| ETXS818 | UmsUfsCmAmCmCfAmCfCfCmUmGmC mCfCmAfGmAmAmAmCmsAmsGm | SEQ ID NO: 455 | UUCACCACCCUGCCC AGAAACAG | SEQ ID NO: 215 |
| ETXS820 | CmsCfsAmCmCmCfUmGfCfCmCmAmG mAfAmAfCmAmGmAmAmsGmsCm | SEQ ID NO: 456 | CCACCCUGCCCAGAA ACAGAAGC | SEQ ID NO: 216 |
| ETXS822 | GmsCfsUmUmGmAfAmCfUfUmCmGm GmAfAmAfGmAmAmAmAmsCmsUm | SEQ ID NO: 457 | GCUUGAACUUCGGAA AGAAAACU | SEQ ID NO: 217 |
| ETXS824 | AmsGfsCmUmUmGfAmAfCfUmUmCm GmGfAmAfAmGmAmAmAmsAmsCm | SEQ ID NO: 458 | AGCUUGAACUUCGGA AAGAAAAC | SEQ ID NO: 218 |
| ETXS826 | AmsCfsCmAmCmCfCmUfGfCmCmCmA mGfAmAfAmCmAmGmAmsAmsGm | SEQ ID NO: 459 | ACCACCCUGCCCAGA AACAGAAG | SEQ ID NO: 219 |
| ETXS828 | GmsGfsUmAmGmGfGmCfAfGmUmUm UmGfAmGfGmAmCmAmUmsGmsAm | SEQ ID NO: 460 | GGUAGGGCAGUUUGA GGACAUGA | SEQ ID NO: 220 |
| ETXS830 | UmsGfsUmCmCmAfGmGfUfGmGmAm AmAfGmUfGmUmCmGmAmsCmsUm | SEQ ID NO: 461 | UGUCCAGGUGGAAAG UGUCGACU | SEQ ID NO: 221 |
| ETXS832 | AmsAfsAmUfCmCfUmUfGmUfGmGfA mUfGmAfAmsGfsGm | SEQ ID NO: 462 | AAAUCCUUGUGGAUG AAGG | SEQ ID NO: 222 |
| ETXS834 | GmsCfsCmUfCmAfUmGfGmAfGmAfU mCfUmUfUmsCfsGm | SEQ ID NO: 463 | GCCUCAUGGAGAUCU UUCG | SEQ ID NO: 223 |
| ETXS836 | UmsUfsCmUfCmCfAmUfGmAfGmGfA mCfCmAfCmsCfsAm | SEQ ID NO: 464 | UUCUCCAUGAGGACC ACCA | SEQ ID NO: 224 |
| ETXS838 | UmsGfsCmCfUmCfAmUfGmGfAmGfA mUfCmUfUmsUfsCm | SEQ ID NO: 465 | UGCCUCAUGGAGAUC UUUC | SEQ ID NO: 225 |
| ETXS840 | AmsGfsCmAfGmCfUmCfAmUfGmCfA mUfCmUfCmsAfsUm | SEQ ID NO: 466 | AGCAGCUCAUGCAUC UCAU | SEQ ID NO: 226 |
| ETXS842 | UmsCfsAmGfGmAfUmUfAmAfUmCfU mCfAmUfCmsAfsAm | SEQ ID NO: 467 | UCAGGAUUAAUCUCA UCAA | SEQ ID NO: 227 |
| ETXS844 | UmsUfsGmGfUmUfUmCfAmGfGmAfU mUfAmAfUmsCfsUm | SEQ ID NO: 468 | UUGGUUUCAGGAUUA AUCU | SEQ ID NO: 228 |
| ETXS846 | GmsUfsUmUfCmAfGmGfAmUfUmAfA mUfCmUfCmsAfsUm | SEQ ID NO: 469 | GUUUCAGGAUUAAUC UCAU | SEQ ID NO: 229 |
| ETXS848 | UmsGfsGmUfUmUfCmAfGmGfAmUfU mAfAmUfCmsUfsCm | SEQ ID NO: 470 | UGGUUUCAGGAUUAA UCUC | SEQ ID NO: 230 |
| ETXS850 | GmsGfsUmUfUmCfAmGfGmAfUmUfA mAfUmCfUmsCfsAm | SEQ ID NO: 471 | GGUUUCAGGAUUAAU CUCA | SEQ ID NO: 231 |
| ETXS852 | AmsGfsGmAfUmUfAmAfUmCfUmCfA mUfCmAfAmsAfsCm | SEQ ID NO: 472 | AGGAUUAAUCUCAUC AAAC | SEQ ID NO: 232 |
| ETXS854 | UmsCfsCmUfUmGfUmGfGmAfUmGfA mAfGmGfCmsAfsAm | SEQ ID NO: 473 | UCCUUGUGGAUGAAG GCAA | SEQ ID NO: 233 |
| ETXS856 | GmsUfsGmCfCmUfCmAfUmGfGmAfG mAfUmCfUmsUfsUm | SEQ ID NO: 474 | GUGCCUCAUGGAGAU CUUU | SEQ ID NO: 234 |
| ETXS858 | UmsCfsUmCfCmAfUmGfAmGfGmAfC mCfAmCfCmsAfsGm | SEQ ID NO: 475 | UCUCCAUGAGGACCA CCAG | SEQ ID NO: 235 |
| ETXS860 | CmsAfsAmAfAmUfCmCfUmUfGmUfG mGfAmUfGmsAfsAm | SEQ ID NO: 476 | CAAAAUCCUUGUGGA UGAA | SEQ ID NO: 236 |
| ETXS862 | CmsGfsUmGfCmCfUmCfAmUfGmGfA mGfAmUfCmsUfsUm | SEQ ID NO: 477 | CGUGCCUCAUGGAGA UCUU | SEQ ID NO: 237 |
| ETXS864 | AmsUfsCmAfAmAfAmUfCmCfUmUfG mUfGmGfAmsUfsGm | SEQ ID NO: 478 | AUCAAAAUCCUUGUG GAUG | SEQ ID NO: 238 |
| ETXS866 | UmsUfsCmAfGmGfAmUfUmAfAmUfC mUfCmAfUmsCfsAm | SEQ ID NO: 479 | UUCAGGAUUAAUCUC AUCA | SEQ ID NO: 239 |
| ETXS868 | AmsAfsUmCfCmUfUmGfUmGfGmAfU mGfAmAfGmsGfsCm | SEQ ID NO: 480 | AAUCCUUGUGGAUGA AGGC | SEQ ID NO: 240 |
| ETXS870 | CmsCfsAmUfCmGfUmGfCmCfUmCfAm UfGmGfAmsGfsAm | SEQ ID NO: 481 | CCAUCGUGCCUCAUG GAGA | SEQ ID NO: 241 |
| ETXS872 | AmsAfsAmUfCmCfUmUfGfUmGmGmA mUfGmAfAmGmGmCmAmsAmsAm | SEQ ID NO: 482 | AAAUCCUUGUGGAUG AAGGCAAA | SEQ ID NO: 122 |
| ETXS874 | GmsCfsCmUfCmAfUmGfGfAmGmAmU mCfUmUfUmCmGmCmAmsGmsCm | SEQ ID NO: 483 | GCCUCAUGGAGAUCU UUCGCAGC | SEQ ID NO: 123 |
| ETXS876 | UmsUfsCmUfCmCfAmUfGfAmGmGmA mCfCmAfCmCmAmGmCmsAmsUm | SEQ ID NO: 484 | UUCUCCAUGAGGACC ACCAGCAU | SEQ ID NO: 124 |
| ETXS878 | UmsGfsCmCfUmCfAmUfGfGmAmGmA mUfCmUfUmUmCmGmCmsAmsGm | SEQ ID NO: 485 | UGCCUCAUGGAGAUC UUUCGCAG | SEQ ID NO: 125 |
| ETXS880 | AmsGfsCmAfGmCfUmCfAfUmGmCmA mUfCmUfCmAmUmAmCmsUmsUm | SEQ ID NO: 486 | AGCAGCUCAUGCAUC UCAUACUU | SEQ ID NO: 126 |
| ETXS882 | UmsCfsAmGfGmAfUmUfAfAmUmCmU mCfAmUfCmAmAmAmCmsAmsGm | SEQ ID NO: 487 | UCAGGAUUAAUCUCA UCAAACAG | SEQ ID NO: 127 |
| ETXS884 | UmsUfsGmGfUmUfUmCfAfGmGmAmU mUfAmAfUmCmUmCmAmsUmsCm | SEQ ID NO: 488 | UUGGUUUCAGGAUUA AUCUCAUC | SEQ ID NO: 128 |
| ETXS886 | GmsUfsUmUfCmAfGmGfAfUmUmAmA mUfCmUfCmAmUmCmAmsAmsAm | SEQ ID NO: 489 | GUUUCAGGAUUAAUC UCAUCAAA | SEQ ID NO: 129 |
| ETXS888 | UmsGfsGmUfUmUfCmAfGfGmAmUmU mAfAmUfCmUmCmAmUmsCmsAm | SEQ ID NO: 490 | UGGUUUCAGGAUUAA UCUCAUCA | SEQ ID NO: 130 |
| ETXS890 | GmsGfsUmUfUmCfAmGfGfAmUmUmA mAfUmCfUmCmAmUmCmsAmsAm | SEQ ID NO: 491 | GGUUUCAGGAUUAAU CUCAUCAA | SEQ ID NO: 131 |
| ETXS892 | AmsGfsGmAfUmUfAmAfUfCmUmCmA mUfCmAfAmAmCmAmGmsUmsUm | SEQ ID NO: 492 | AGGAUUAAUCUCAUC AAACAGUU | SEQ ID NO: 132 |
| ETXS894 | UmsCfsCmUfUmGfUmGfGfAmUmGmA mAfGmGfCmAmAmAmAmsCmsUm | SEQ ID NO: 493 | UCCUUGUGGAUGAAG GCAAAACU | SEQ ID NO: 133 |
| ETXS896 | GmsUfsGmCfCmUfCmAfUfGmGmAmG mAfUmCfUmUmUmCmGmsCmsAm | SEQ ID NO: 494 | GUGCCUCAUGGAGAU CUUUCGCA | SEQ ID NO: 134 |
| ETXS898 | UmsCfsUmCfCmAfUmGfAfGmGmAmC mCfAmCfCmAmGmCmAmsUmsGm | SEQ ID NO: 495 | UCUCCAUGAGGACCA CCAGCAUG | SEQ ID NO: 135 |
| ETXS900 | CmsAfsAmAfAmUfCmCfUfUmGmUmG mGfAmUfGmAmAmGmGmsCmsAm | SEQ ID NO: 496 | CAAAAUCCUUGUGGA UGAAGGCA | SEQ ID NO: 136 |
| ETXS902 | CmsGfsUmGfCmCfUmCfAfUmGmGmA mGfAmUfCmUmUmUmCmsGmsCm | SEQ ID NO: 497 | CGUGCCUCAUGGAGA UCUUUCGC | SEQ ID NO: 137 |
| ETXS904 | AmsUfsCmAfAmAfAmUfCfCmUmUmG mUfGmGfAmUmGmAmAmsGmsGm | SEQ ID NO: 498 | AUCAAAAUCCUUGUG GAUGAAGG | SEQ ID NO: 138 |
| ETXS906 | UmsUfsCmAfGmGfAmUfUfAmAmUmC mUfCmAfUmCmAmAmAmsCmsAm | SEQ ID NO: 499 | UUCAGGAUUAAUCUC AUCAAACA | SEQ ID NO: 139 |
| ETXS908 | AmsAfsUmCfCmUfUmGfUfGmGmAmU mGfAmAfGmGmCmAmAmsAmsAm | SEQ ID NO: 500 | AAUCCUUGUGGAUGA AGGCAAAA | SEQ ID NO: 140 |
| ETXS910 | CmsCfsAmUfCmGfUmGfCfCmUmCmA mUfGmGfAmGmAmUmCmsUmsUm | SEQ ID NO: 501 | CCAUCGUGCCUCAUG GAGAUCUU | SEQ ID NO: 141 |
| ETXS912 | AmsAfsAmUfCmCfUmUfGfUmGmGmA mUfGmAfAmGmGmCmAmsAmsAm | SEQ ID NO: 502 | AAAUCCUUGUGGAUG AAGGCAAA | SEQ ID NO: 122 |
| ETXS914 | GmsCfsCmUfCmAfUmGfGfAmGmAmU mCfUmUfUmCmGmCmAmsGmsCm | SEQ ID NO: 503 | GCCUCAUGGAGAUCU UUCGCAGC | SEQ ID NO: 123 |
| ETXS916 | UmsUfsCmUfCmCfAmUfGfAmGmGmA mCfCmAfCmCmAmGmCmsAmsUm | SEQ ID NO: 504 | UUCUCCAUGAGGACC ACCAGCAU | SEQ ID NO: 124 |
| ETXS918 | UmsGfsCmCfUmCfAmUfGfGmAmGmA mUfCmUfUmUmCmGmCmsAmsGm | SEQ ID NO: 505 | UGCCUCAUGGAGAUC UUUCGCAG | SEQ ID NO: 125 |
| ETXS920 | AmsGfsCmAfGmCfUmCfAfUmGmCmA mUfCmUfCmAmUmAmCmsUmsUm | SEQ ID NO: 506 | AGCAGCUCAUGCAUC UCAUACUU | SEQ ID NO: 126 |
| ETXS922 | UmsCfsAmGfGmAfUmUfAfAmUmCmU mCfAmUfCmAmAmAmCmsAmsGm | SEQ ID NO: 507 | UCAGGAUUAAUCUCA UCAAACAG | SEQ ID NO: 127 |
| ETXS924 | UmsUfsGmGfUmUfUmCfAfGmGmAmU mUfAmAfUmCmUmCmAmsUmsCm | SEQ ID NO: 508 | UUGGUUUCAGGAUUA AUCUCAUC | SEQ ID NO: 128 |
| ETXS926 | GmsUfsUmUfCmAfGmGfAfUmUmAmA mUfCmUfCmAmUmCmAmsAmsAm | SEQ ID NO: 509 | GUUUCAGGAUUAAUC UCAUCAAA | SEQ ID NO: 129 |
| ETXS928 | UmsGfsGmUfUmUfCmAfGfGmAmUmU mAfAmUfCmUmCmAmUmsCmsAm | SEQ ID NO: 510 | UGGUUUCAGGAUUAA UCUCAUCA | SEQ ID NO: 130 |
| ETXS930 | GmsGfsUmUfUmCfAmGfGfAmUmUmA mAfUmCfUmCmAmUmCmsAmsAm | SEQ ID NO: 511 | GGUUUCAGGAUUAAU CUCAUCAA | SEQ ID NO: 131 |
| ETXS932 | AmsGfsGmAfUmUfAmAfUfCmUmCmA mUfCmAfAmAmCmAmGmsUmsUm | SEQ ID NO: 512 | AGGAUUAAUCUCAUC AAACAGUU | SEQ ID NO: 132 |
| ETXS934 | UmsCfsCmUfUmGfUmGfGfAmUmGmA mAfGmGfCmAmAmAmAmsCmsUm | SEQ ID NO: 513 | UCCUUGUGGAUGAAG GCAAAACU | SEQ ID NO: 133 |
| ETXS936 | GmsUfsGmCfCmUfCmAfUfGmGmAmG mAfUmCfUmUmUmCmGmsCmsAm | SEQ ID NO: 514 | GUGCCUCAUGGAGAU CUUUCGCA | SEQ ID NO: 134 |
| ETXS938 | UmsCfsUmCfCmAfUmGfAfGmGmAmC mCfAmCfCmAmGmCmAmsUmsGm | SEQ ID NO: 515 | UCUCCAUGAGGACCA CCAGCAUG | SEQ ID NO: 135 |
| ETXS940 | CmsAfsAmAfAmUfCmCfUfUmGmUmG mGfAmUfGmAmAmGmGmsCmsAm | SEQ ID NO: 516 | CAAAAUCCUUGUGGA UGAAGGCA | SEQ ID NO: 136 |
| ETXS942 | CmsGfsUmGfCmCfUmCfAfUmGmGmA mGfAmUfCmUmUmUmCmsGmsCm | SEQ ID NO: 517 | CGUGCCUCAUGGAGA UCUUUCGC | SEQ ID NO: 137 |
| ETXS944 | AmsUfsCmAfAmAfAmUfCfCmUmUmG mUfGmGfAmUmGmAmAmsGmsGm | SEQ ID NO: 518 | AUCAAAAUCCUUGUG GAUGAAGG | SEQ ID NO: 138 |
| ETXS946 | UmsUfsCmAfGmGfAmUfUfAmAmUmC mUfCmAfUmCmAmAmAmsCmsAm | SEQ ID NO: 519 | UUCAGGAUUAAUCUC AUCAAACA | SEQ ID NO: 139 |
| ETXS948 | AmsAfsUmCfCmUfUmGfUfGmGmAmU mGfAmAfGmGmCmAmAmsAmsAm | SEQ ID NO: 520 | AAUCCUUGUGGAUGA AGGCAAAA | SEQ ID NO: 140 |
| ETXS950 | CmsCfsAmUfCmGfUmGfCfCmUmCmA mUfGmGfAmGmAmUmCmsUmsUm | SEQ ID NO: 521 | CCAUCGUGCCUCAUG GAGAUCUU | SEQ ID NO: 141 |
| ETXS952 | AmsAfsAmUfCmCfUmUfGfUmGmGmA mUfGmAfAmGmGmCmAmsAmsAm | SEQ ID NO: 522 | AAAUCCUUGUGGAUG AAGGCAAA | SEQ ID NO: 122 |
| ETXS954 | GmsCfsCmUfCmAfUmGfGfAmGmAmU mCfUmUfUmCmGmCmAmsGmsCm | SEQ ID NO: 523 | GCCUCAUGGAGAUCU UUCGCAGC | SEQ ID NO: 123 |
| ETXS956 | UmsUfsCmUfCmCfAmUfGfAmGmGmA mCfCmAfCmCmAmGmCmsAmsUm | SEQ ID NO: 524 | UUCUCCAUGAGGACC ACCAGCAU | SEQ ID NO: 124 |
| ETXS958 | UmsGfsCmCfUmCfAmUfGfGmAmGmA mUfCmUfUmUmCmGmCmsAmsGm | SEQ ID NO: 525 | UGCCUCAUGGAGAUC UUUCGCAG | SEQ ID NO: 125 |
| ETXS960 | AmsGfsCmAfGmCfUmCfAfUmGmCmA mUfCmUfCmAmUmAmCmsUmsUm | SEQ ID NO: 526 | AGCAGCUCAUGCAUC UCAUACUU | SEQ ID NO: 126 |
| ETXS962 | UmsCfsAmGfGmAfUmUfAfAmUmCmU mCfAmUfCmAmAmAmCmsAmsGm | SEQ ID NO: 527 | UCAGGAUUAAUCUCA UCAAACAG | SEQ ID NO: 127 |
| ETXS964 | UmsUfsGmGfUmUfUmCfAfGmGmAmU mUfAmAfUmCmUmCmAmsUmsCm | SEQ ID NO: 528 | UUGGUUUCAGGAUUA AUCUCAUC | SEQ ID NO: 128 |
| ETXS966 | GmsUfsUmUfCmAfGmGfAfUmUmAmA mUfCmUfCmAmUmCmAmsAmsAm | SEQ ID NO: 529 | GUUUCAGGAUUAAUC UCAUCAAA | SEQ ID NO: 129 |
| ETXS968 | UmsGfsGmUfUmUfCmAfGfGmAmUmU mAfAmUfCmUmCmAmUmsCmsAm | SEQ ID NO: 530 | UGGUUUCAGGAUUAA UCUCAUCA | SEQ ID NO: 130 |
| ETXS970 | GmsGfsUmUfUmCfAmGfGfAmUmUmA mAfUmCfUmCmAmUmCmsAmsAm | SEQ ID NO: 531 | GGUUUCAGGAUUAAU CUCAUCAA | SEQ ID NO: 131 |
| ETXS972 | AmsGfsGmAfUmUfAmAfUfCmUmCmA mUfCmAfAmAmCmAmGmsUmsUm | SEQ ID NO: 532 | AGGAUUAAUCUCAUC AAACAGUU | SEQ ID NO: 132 |
| ETXS974 | UmsCfsCmUfUmGfUmGfGfAmUmGmA mAfGmGfCmAmAmAmAmsCmsUm | SEQ ID NO: 533 | UCCUUGUGGAUGAAG GCAAAACU | SEQ ID NO: 133 |
| ETXS976 | GmsUfsGmCfCmUfCmAfUfGmGmAmG mAfUmCfUmUmUmCmGmsCmsAm | SEQ ID NO: 534 | GUGCCUCAUGGAGAU CUUUCGCA | SEQ ID NO: 134 |
| ETXS978 | UmsCfsUmCfCmAfUmGfAfGmGmAmC mCfAmCfCmAmGmCmAmsUmsGm | SEQ ID NO: 535 | UCUCCAUGAGGACCA CCAGCAUG | SEQ ID NO: 135 |
| ETXS980 | CmsAfsAmAfAmUfCmCfUfUmGmUmG mGfAmUfGmAmAmGmGmsCmsAm | SEQ ID NO: 536 | CAAAAUCCUUGUGGA UGAAGGCA | SEQ ID NO: 136 |
| ETXS982 | CmsGfsUmGfCmCfUmCfAfUmGmGmA mGfAmUfCmUmUmUmCmsGmsCm | SEQ ID NO: 537 | CGUGCCUCAUGGAGA UCUUUCGC | SEQ ID NO: 137 |
| ETXS984 | AmsUfsCmAfAmAfAmUfCfCmUmUmG mUfGmGfAmUmGmAmAmsGmsGm | SEQ ID NO: 538 | AUCAAAAUCCUUGUG GAUGAAGG | SEQ ID NO: 138 |
| ETXS986 | UmsUfsCmAfGmGfAmUfUfAmAmUmC mUfCmAfUmCmAmAmAmsCmsAm | SEQ ID NO: 539 | UUCAGGAUUAAUCUC AUCAAACA | SEQ ID NO: 139 |
| ETXS988 | AmsAfsUmCfCmUfUmGfUfGmGmAmU mGfAmAfGmGmCmAmAmsAmsAm | SEQ ID NO: 540 | AAUCCUUGUGGAUGA AGGCAAAA | SEQ ID NO: 140 |
| ETXS990 | CmsCfsAmUfCmGfUmGfCfCmUmCmA mUfGmGfAmGmAmUmCmsUmsUm | SEQ ID NO: 541 | CCAUCGUGCCUCAUG GAGAUCUU | SEQ ID NO: 141 |
| ETXS992 | AmsAfsAmUfCmCfUmUmGmUmGmGm AmUfGmAfAmGmGmCmAmsAmsAm | SEQ ID NO: 542 | AAAUCCUUGUGGAUG AAGGCAAA | SEQ ID NO: 122 |
| ETXS994 | GmsCfsCmUfCmAfUmGmGmAmGmAm UmCfUmUfUmCmGmCmAmsGmsCm | SEQ ID NO: 543 | GCCUCAUGGAGAUCU UUCGCAGC | SEQ ID NO: 123 |
| ETXS996 | UmsUfsCmUfCmCfAmUmGmAmGmGm AmCfCmAfCmCmAmGmCmsAmsUm | SEQ ID NO: 544 | UUCUCCAUGAGGACC ACCAGCAU | SEQ ID NO: 124 |
| ETXS998 | UmsGfsCmCfUmCfAmUmGmGmAmGm AmUfCmUfUmUmCmGmCmsAmsGm | SEQ ID NO: 545 | UGCCUCAUGGAGAUC UUUCGCAG | SEQ ID NO: 125 |
| ETXS1000 | AmsGfsCmAfGmCfUmCmAmUmGmCm AmUfCmUfCmAmUmAmCmsUmsUm | SEQ ID NO: 546 | AGCAGCUCAUGCAUC UCAUACUU | SEQ ID NO: 126 |
| ETXS1002 | UmsCfsAmGfGmAfUmUmAmAmUmCm UmCfAmUfCmAmAmAmCmsAmsGm | SEQ ID NO: 547 | UCAGGAUUAAUCUCA UCAAACAG | SEQ ID NO: 127 |
| ETXS1004 | UmsUfsGmGfUmUfUmCmAmGmGmA mUmUfAmAfUmCmUmCmAmsUmsCm | SEQ ID NO: 548 | UUGGUUUCAGGAUUA AUCUCAUC | SEQ ID NO: 128 |
| ETXS1006 | GmsUfsUmUfCmAfGmGmAmUmUmA mAmUfCmUfCmAmUmCmAmsAmsAm | SEQ ID NO: 549 | GUUUCAGGAUUAAUC UCAUCAAA | SEQ ID NO: 129 |
| ETXS1008 | UmsGfsGmUfUmUfCmAmGmGmAmU mUmAfAmUfCmUmCmAmUmsCmsAm | SEQ ID NO: 550 | UGGUUUCAGGAUUAA UCUCAUCA | SEQ ID NO: 130 |
| ETXS1010 | GmsGfsUmUfUmCfAmGmGmAmUmU mAmAfUmCfUmCmAmUmCmsAmsAm | SEQ ID NO: 551 | GGUUUCAGGAUUAAU CUCAUCAA | SEQ ID NO: 131 |
| ETXS1012 | AmsGfsGmAfUmUfAmAmUmCmUmCm AmUfCmAfAmAmCmAmGmsUmsUm | SEQ ID NO: 552 | AGGAUUAAUCUCAUC AAACAGUU | SEQ ID NO: 132 |
| ETXS1014 | UmsCfsCmUfUmGfUmGmGmAmUmGm AmAfGmGfCmAmAmAmAmsCmsUm | SEQ ID NO: 553 | UCCUUGUGGAUGAAG GCAAAACU | SEQ ID NO: 133 |
| ETXS1016 | GmsUfsGmCfCmUfCmAmUmGmGmAm GmAfUmCfUmUmUmCmGmsCmsAm | SEQ ID NO: 554 | GUGCCUCAUGGAGAU CUUUCGCA | SEQ ID NO: 134 |
| ETXS1018 | UmsCfsUmCfCmAfUmGmAmGmGmAm CmCfAmCfCmAmGmCmAmsUmsGm | SEQ ID NO: 555 | UCUCCAUGAGGACCA CCAGCAUG | SEQ ID NO: 135 |
| ETXS1020 | CmsAfsAmAfAmUfCmCmUmUmGmUm GmGfAmUfGmAmAmGmGmsCmsAm | SEQ ID NO: 556 | CAAAAUCCUUGUGGA UGAAGGCA | SEQ ID NO: 136 |
| ETXS1022 | CmsGfsUmGfCmCfUmCmAmUmGmGm AmGfAmUfCmUmUmUmCmsGmsCm | SEQ ID NO: 557 | CGUGCCUCAUGGAGA UCUUUCGC | SEQ ID NO: 137 |
| ETXS1024 | AmsUfsCmAfAmAfAmUmCmCmUmUm GmUfGmGfAmUmGmAmAmsGmsGm | SEQ ID NO: 558 | AUCAAAAUCCUUGUG GAUGAAGG | SEQ ID NO: 138 |
| ETXS1026 | UmsUfsCmAfGmGfAmUmUmAmAmU mCmUfCmAfUmCmAmAmAmsCmsAm | SEQ ID NO: 559 | UUCAGGAUUAAUCUC AUCAAACA | SEQ ID NO: 139 |
| ETXS1028 | AmsAfsUmCfCmUfUmGmUmGmGmAm UmGfAmAfGmGmCmAmAmsAmsAm | SEQ ID NO: 560 | AAUCCUUGUGGAUGA AGGCAAAA | SEQ ID NO: 140 |
| ETXS1030 | CmsCfsAmUfCmGfUmGmCmCmUmCm AmUfGmGfAmGmAmUmCmsUmsUm | SEQ ID NO: 561 | CCAUCGUGCCUCAUG GAGAUCUU | SEQ ID NO: 141 |
| ETXS1032 | AmsAfsAmAfUmCfCmUmUmGmUmGm GmAfUmGfAmAmGmGmCmsAmsAm | SEQ ID NO: 762 | AAAAUCCUUGUGGAU GAAGGCAA | SEQ ID NO: 144 |
| ETXS1034 | AmsAfsAmAmUmCfCmUmUfGmUmGm GmAfUmGfAmAmGmGmCmsAmsAm | SEQ ID NO: 763 | AAAAUCCUUGUGGAU GAAGGCAA | SEQ ID NO: 144 |
| ETXS1038 | CmsAfsUmGmGmUfGmGmCfAmUmUm UmCfCmUfUmGmGmUmAmsGmsGm | SEQ ID NO: 765 | CAUGGUGGCAUUUCC UUGGUAGG | SEQ ID NO: 145 |
| ETXS1042 | UmsCfsGmUmGmCfCmUmCfAmUmGm GmAfGmAfUmCmUmUmUmsCmsGm | SEQ ID NO: 767 | UCGUGCCUCAUGGAG AUCUUUCG | SEQ ID NO: 148 |
| ETXS1044 | AmsAfsAmUfUmCfUmUmGmUmCmAm AmAfGmGfUmGmAmGmsGmsCm | SEQ ID NO: 768 | AAAUUCUUGUCAAAG GUGGAGGC | SEQ ID NO: 165 |
| ETXS1046 | AmsAfsAmUmUmCfUmUmGfUmCmAm AmAfGmGfUmGmGmAmGmsGmsCm | SEQ ID NO: 769 | AAAUUCUUGUCAAAG GUGGAGGC | SEQ ID NO: 165 |
| ETXS1048 | UmsGfsUmGfGmAfUmGmAmAmGmG mCmAfAmAfAmCmUmCmCmsCmsCm | SEQ ID NO: 770 | UGUGGAUGAAGGCAA AACUCCCC | SEQ ID NO: 202 |
| ETXS1050 | UmsGfsUmGmGmAfUmGmAfAmGmG mCmAfAmAfAmCmUmCmCmsCmsCm | SEQ ID NO: 771 | UGUGGAUGAAGGCAA AACUCCCC | SEQ ID NO: 202 |
| ETXS1051 | AmsAfsAmAmUmCfCmUmUmGmUmG mGmAfUmGfAmAfGmGmCmsAmsAm | SEQ ID NO: 782 | AAAAUCCUUGUGGAU GAAGGCAA | SEQ ID NO: 144 |
| ETXS1052 | CmsAfsUmGmGmUfGmGmCmAmUmU mUmCfCmUfUmGfGmUmAmsGmsGm | SEQ ID NO: 783 | CAUGGUGGCAUUUCC UUGGUAGG | SEQ ID NO: 145 |
| ETXS1053 | UmsCfsGmUmGmCfCmUmCmAmUmG mGmAfGmAfUmCfUmUmUmsCmsGm | SEQ ID NO: 784 | UCGUGCCUCAUGGAG AUCUUUCG | SEQ ID NO: 148 |
| ETXS1054 | AmsAfsAmUmUmCfUmUmGmUmCmA mAmAfGmGfUmGfGmAmGmsGmsCm | SEQ ID NO: 785 | AAAUUCUUGUCAAAG GUGGAGGC | SEQ ID NO: 165 |
| ETXS1055 | UmsGfsUmGmGmAfUmGmAmAmGmG mCmAfAmAfAmCfUmCmCmsCmsCm | SEQ ID NO: 786 | UGUGGAUGAAGGCAA AACUCCCC | SEQ ID NO: 202 |
| ETXS2128 | AmsAfsAmGmUmCfCmUfUfGmUmGm GmAfUmGfAmAmGmGmCmsAmsAm | SEQ ID NO: 795 | AAAGUCCUUGUGGAU GAAGGCAA | SEQ ID NO: 787 |
| ETXS2144 | AmsAfsCmUmUmCfUmUfGfUmCmAm AmAfGmGfUmGmGmAmGmsGmsCm | SEQ ID NO: 796 | AACUUCUUGUCAAAG GUGGAGGC | SEQ ID NO: 788 |
| ETXS2152 | UmsGfsUmGmGmAfUmGfAfAmGmGm CmAfAmAfGmCmUmAmCmsCmsCm | SEQ ID NO: 797 | UGUGGAUGAAGGCAA AGCUACCC | SEQ ID NO: 789 |
| ETXS2398 | CmsAfsUmGmGmUfGmGmCfAmUmUm UmCfCmUfUmGmGmUmAmsGmsGm | SEQ ID NO: 801 | CAUGGUGGCAUUUCC UUGGUAGG | SEQ ID NO: 145 |
| ETSX2400 | CmsAfsUmGmGmUfGmGmCmAmUmU mUmCfCmUfUmGfGmUmAmsGmsGm | SEQ ID NO: 802 | CAUGGUGGCAUUUCC UUGGUAGG | SEQ ID NO: 145 |
| ETXS2402 | CmsAfsUmGmGmUoGmGmCmAmUmU mUmCfCmUfUmGmGmUmAmsGmsGm | SEQ ID NO: 803 | CAUGGUGGCAUUUCC UUGGUAGG | SEQ ID NO: 145 |
| ETXS2404 | CmsAfsUmGmGmUoGmGfCfAmUmUm UmCfCmUfUmGmGmUmAmsGmsGm | SEQ ID NO: 804 | CAUGGUGGCAUUUCC UUGGUAGG | SEQ ID NO: 145 |
| ETXS2406 | CmsAfsUmGmGmUfGmGmCmAmUmU mUmCfCmUfUmGmGmUfAmsGmsGm | SEQ ID NO: 805 | CAUGGUGGCAUUUCC UUGGUAGG | SEQ ID NO: 145 |
| ETXS2408 | CmsAfsUmGfGmUfGmGmCmAmUmUm UmCfCmUfUmGmGmUmAmsGmsGm | SEQ ID NO: 806 | CAUGGUGGCAUUUCC UUGGUAGG | SEQ ID NO: 145 |
| ETXS2410 | CmsAfsUmGfGmUfGmGfCfAmUmUmU mCfCmUfUmGmGmUmAmsGmsGm | SEQ ID NO: 807 | CAUGGUGGCAUUUCC UUGGUAGG | SEQ ID NO: 145 |
| ETXS2412 | CmsAfsUmGmGmUfGmGfCfAmUmUm UmCfCmUfUmGfGmUmAmsGmsGm | SEQ ID NO: 808 | CAUGGUGGCAUUUCC UUGGUAGG | SEQ ID NO: 145 |
| ETXS2414 | CmsAfsUmGmGmUfGmGfCfAmUmUm UmCfCmUfUmGmGmUfAmsGmsGm | SEQ ID NO: 809 | CAUGGUGGCAUUUCC UUGGUAGG | SEQ ID NO: 145 |
| ETXS2416 | UmsCfsGmUmGmCfCmUmCfAmUmGm GmAfGmAfUmCmUmUmUmsCmsGm | SEQ ID NO: 810 | UCGUGCCUCAUGGAG AUCUUUCG | SEQ ID NO: 148 |
| ETXS2418 | UmsCfsGmUmGmCfCmUmCmAmUmG mGmAfGmAfUmCfUmUmUmsCmsGm | SEQ ID NO: 811 | UCGUGCCUCAUGGAG AUCUUUCG | SEQ ID NO: 148 |
| ETXS2420 | UmsCfsGmUmGmCoCmUmCmAmUmG mGmAfGmAfUmCmUmUmUmsCmsGm | SEQ ID NO: 812 | UCGUGCCUCAUGGAG AUCUUUCG | SEQ ID NO: 148 |
| ETXS2422 | UmsCfsGmUmGmCoCmUfCfAmUmGm GmAfGmAfUmCmUmUmUmsCmsGm | SEQ ID NO: 813 | UCGUGCCUCAUGGAG AUCUUUCG | SEQ ID NO: 148 |
| ETXS2424 | UmsCfsGmUmGmCfCmUmCmAmUmG mGmAfGmAfUmCmUmUfUmsCmsGm | SEQ ID NO: 814 | UCGUGCCUCAUGGAG AUCUUUCG | SEQ ID NO: 148 |
| ETXS2426 | UmsCfsGmUfGmCfCmUmCmAmUmGm GmAfGmAfUmCmUmUmUmsCmsGm | SEQ ID NO: 815 | UCGUGCCUCAUGGAG AUCUUUCG | SEQ ID NO: 148 |
| ETXS2428 | UmsCfsGmUfGmCfCmUfCfAmUmGmG mAfGmAfUmCmUmUmUmsCmsGm | SEQ ID NO: 816 | UCGUGCCUCAUGGAG AUCUUUCG | SEQ ID NO: 148 |
| ETXS2430 | UmsCfsGmUmGmCfCmUfCfAmUmGm GmAfGmAfUmCfUmUmUmsCmsGm | SEQ ID NO: 817 | UCGUGCCUCAUGGAG AUCUUUCG | SEQ ID NO: 148 |
| ETXS2432 | UmsCfsGmUmGmCfCmUfCfAmUmGm GmAfGmAfUmCmUmUfUmsCmsGm | SEQ ID NO: 818 | UCGUGCCUCAUGGAG AUCUUUCG | SEQ ID NO: 148 |

Table 4 provides the modified second (sense) sequences, together with the corresponding unmodified second (sense) sequences for siRNA oligonucleosides according to the present invention as follows.

**Table 4**

| Sense strand ID | Modified Second (Sense) Strand 5' → 3' | SEQ ID NO (SS - mod) | Underlying Base Sequence 5' → 3' | SEQ ID NO (SS - unmod) |
|---|---|---|---|---|
| | | | (Shown as an Unmodified Nucleoside Sequence) | |
| ETXS1035 | iaiaUmsAmsCmCmAmAmGfGmAfAfA fUfGmCmCmAmCmCmAmUmGm | SEQ ID NO: 774 | UACCAAGGAAAUGCCAC CAUG | SEQ ID NO: 265 |
| ETXS1039 | iaiaAmsAmsAmGmAmUmCfUmCfCfA fUfGmAmGmGmCmAmCmGmAm | SEQ ID NO: 776 | AAAGAUCUCCAUGAGGC ACGA | SEQ ID NO: 268 |
| ETXS631 | UmsGmsCmCmUmUmCfAmUfCfCfA mCmAmAmGmGmAmUmUmUm | SEQ ID NO: 562 | UGCCUUCAUCCACAAGG AUUU | SEQ ID NO: 242 |
| ETXS633 | UmsGmsCmGmAmAmAfGmAfUfCfU mCmCmAmUmGmAmGmGmCm | SEQ ID NO: 563 | UGCGAAAGAUCUCCAUG AGGC | SEQ ID NO: 243 |
| ETXS635 | GmsCmsUmGmGmUmGfGmUfCfCfU mCmAmUmGmGmAmGmAmAm | SEQ ID NO: 564 | GCUGGUGGUCCUCAUGG AGAA | SEQ ID NO: 244 |
| ETXS637 | GmsCmsGmAmAmAmGfAmUfCfUfC mCmAmUmGmAmGmGmCmAm | SEQ ID NO: 565 | GCGAAAGAUCUCCAUGA GGCA | SEQ ID NO: 245 |
| ETXS639 | GmsUmsAmUmGmAmGfAmUfGfCfA mUmGmAmGmCmUmGmCmUm | SEQ ID NO: 566 | GUAUGAGAUGCAUGAGC UGCU | SEQ ID NO: 246 |
| ETXS641 | GmsUmsUmUmGmAmUfGmAfGfAfU mUmAmAmUmCmCmUmGmAm | SEQ ID NO: 567 | GUUUGAUGAGAUUAAUC CUGA | SEQ ID NO: 247 |
| ETXS643 | UmsGmsAmGmAmUmUfAmAfUfCfC mUmGmAmAmAmCmCmAmAm | SEQ ID NO: 568 | UGAGAUUAAUCCUGAAA CCAA | SEQ ID NO: 248 |
| ETXS645 | UmsGmsAmUmGmAmGfAmUfUfAfA mUmCmCmUmGmAmAmAmCm | SEQ ID NO: 569 | UGAUGAGAUUAAUCCUG AAAC | SEQ ID NO: 249 |
| ETXS647 | AmsUmsGmAmGmAmUfUmAfAfUfC mCmUmGmAmAmAmCmCmAm | SEQ ID NO: 570 | AUGAGAUUAAUCCUGAA ACCA | SEQ ID NO: 250 |
| ETXS649 | GmsAmsUmGmAmGmAfUmUfAfAfU mCmCmUmGmAmAmAmCmCm | SEQ ID NO: 571 | GAUGAGAUUAAUCCUGA AACC | SEQ ID NO: 251 |
| ETXS651 | CmsUmsGmUmUmUmGfAmUfGfAfG mAmUmUmAmAmUmCmCmUm | SEQ ID NO: 572 | CUGUUUGAUGAGAUUAA UCCU | SEQ ID NO: 252 |
| ETXS653 | UmsUmsUmUmGmCmCfUmUfCfAfU mCmCmAmCmAmAmGmGmAm | SEQ ID NO: 573 | UUUUGCCUUCAUCCACA AGGA | SEQ ID NO: 253 |
| ETXS655 | CmsGmsAmAmAmGmAfUmCfUfCfC mAmUmGmAmGmGmCmAmCm | SEQ ID NO: 574 | CGAAAGAUCUCCAUGAG GCAC | SEQ ID NO: 254 |
| ETXS657 | UmsGmsCmUmGmGmUfGmGfUfCfC mUmCmAmUmGmGmAmGmAm | SEQ ID NO: 575 | UGCUGGUGGUCCUCAUG GAGA | SEQ ID NO: 255 |
| ETXS659 | CmsCmsUmUmCmAmUfCmCfAfCfA mAmGmGmAmUmUmUmGm | SEQ ID NO: 576 | CCUUCAUCCACAAGGAU UUUG | SEQ ID NO: 256 |
| ETXS661 | GmsAmsAmAmGmAmUfCmUfCfCfA mUmGmAmGmGmCmAmCmGm | SEQ ID NO: 577 | GAAAGAUCUCCAUGAGG CACG | SEQ ID NO: 257 |
| ETXS663 | UmsUmsCmAmUmCmCfAmCfAfAfG mGmAmUmUmUmUmGmAmUm | SEQ ID NO: 578 | UUCAUCCACAAGGAUUU UGAU | SEQ ID NO: 258 |
| ETXS665 | UmsUmsUmGmAmUmGfAmGfAfUfU mAmAmUmCmCmUmGmAmAm | SEQ ID NO: 579 | UUUGAUGAGAUUAAUCC UGAA | SEQ ID NO: 259 |
| ETXS667 | UmsUmsGmCmCmUmUfCmAfUfCfC mAmCmAmAmGmGmAmUmUm | SEQ ID NO: 580 | UUGCCUUCAUCCACAAG GAUU | SEQ ID NO: 260 |
| ETXS669 | GmsAmsUmCmUmCmCfAmUfGfAfG mGmCmAmCmGmAmUmGmGm | SEQ ID NO: 581 | GAUCUCCAUGAGGCACG AUGG | SEQ ID NO: 261 |
| ETXS671 | CmsAmsUmGmCmUmGfGmUfGfGfU mCmCmUmCmAmUmGmGmAm | SEQ ID NO: 582 | CAUGCUGGUGGUCCUCA UGGA | SEQ ID NO: 262 |
| ETXS673 | UmsUmsUmGmCmCmUfUmCfAfUfC mCmAmCmAmAmGmGmAmUm | SEQ ID NO: 583 | UUUGCCUUCAUCCACAA GGAU | SEQ ID NO: 263 |
| ETXS675 | GmsCmsCmUmUmCmAfUmCfCfAfC mAmAmGmGmAmUmUmUmUm | SEQ ID NO: 584 | GCCUUCAUCCACAAGGA UUUU | SEQ ID NO: 264 |
| ETXS677 | UmsAmsCmCmAmAmGfGmAfAfAfU mGmCmCmAmCmCmAmUmGm | SEQ ID NO: 585 | UACCAAGGAAAUGCCAC CAUG | SEQ ID NO: 265 |
| ETXS679 | UmsUmsGmAmUmGmAfGmAfUfUfA mAmUmCmCmUmGmAmAmAm | SEQ ID NO: 586 | UUGAUGAGAUUAAUCCU GAAA | SEQ ID NO: 266 |
| ETXS681 | CmsUmsUmCmAmUmCfCmAfCfAfA mGmGmAmUmUmUmUmGmAm | SEQ ID NO: 587 | CUUCAUCCACAAGGAUU UUGA | SEQ ID NO: 267 |
| ETXS683 | AmsAmsAmGmAmUmCfUmCfCfAfU mGmAmGmGmCmAmCmGmAm | SEQ ID NO: 588 | AAAGAUCUCCAUGAGGC ACGA | SEQ ID NO: 268 |
| ETXS685 | UmsGmsUmUmUmGmAfUmGfAfGfA mUmUmAmAmUmCmCmUmGm | SEQ ID NO: 589 | UGUUUGAUGAGAUUAAU CCUG | SEQ ID NO: 269 |
| ETXS687 | AmsUmsGmCmUmGmGfUmGfGfUfC mCmUmCmAmUmGmGmAmGm | SEQ ID NO: 590 | AUGCUGGUGGUCCUCAU GGAG | SEQ ID NO: 270 |
| ETXS689 | GmsUmsUmUmUmGmCfCmUfUfCfA mUmCmCmAmCmAmAmGmGm | SEQ ID NO: 591 | GUUUUGCCUUCAUCCAC AAGG | SEQ ID NO: 271 |
| ETXS691 | CmsCmsAmCmCmAmUfGmCfUfGfG mUmGmGmUmCmCmUmCmAm | SEQ ID NO: 592 | CCACCAUGCUGGUGGUC CUCA | SEQ ID NO: 272 |
| ETXS693 | CmsAmsCmCmAmUmGfCmUfGfGfU mGmGmUmCmCmUmCmAmUm | SEQ ID NO: 593 | CACCAUGCUGGUGGUCC UCAU | SEQ ID NO: 273 |
| ETXS695 | AmsAmsGmAmUmCmUfCmCfAfUfG mAmGmGmCmAmCmGmAmUm | SEQ ID NO: 594 | AAGAUCUCCAUGAGGCA CGAU | SEQ ID NO: 274 |
| ETXS697 | AmsGmsUmUmUmUmGfCmCfUfUfC mAmUmCmCmAmCmAmAmGm | SEQ ID NO: 595 | AGUUUUGCCUUCAUCCA CAAG | SEQ ID NO: 275 |
| ETXS699 | AmsCmsCmAmUmGmCfUmGfGfUfG mGmUmCmCmUmCmAmUmGm | SEQ ID NO: 596 | ACCAUGCUGGUGGUCCU CAUG | SEQ ID NO: 276 |
| ETXS701 | AmsGmsAmUmCmUmCfCmAfUfGfA mGmGmCmAmCmGmAmUmGm | SEQ ID NO: 597 | AGAUCUCCAUGAGGCAC GAUG | SEQ ID NO: 277 |
| ETXS703 | CmsCmsAmUmGmCmUfGmGfUfGfG mUmCmCmUmCmAmUmGmGm | SEQ ID NO: 598 | CCAUGCUGGUGGUCCUC AUGG | SEQ ID NO: 278 |
| ETXS705 | UmsGmsCmCmUmCmCfAmCfCfUfU mUmGmAmCmAmAmGmAmAm | SEQ ID NO: 599 | UGCCUCCACCUUUGACA AGAA | SEQ ID NO: 279 |
| ETXS707 | CmsCmsUmCmCmAmCfCmUfUfUfG mAmCmAmAmGmAmAmUmUm | SEQ ID NO: 600 | CCUCCACCUUUGACAAG AAUU | SEQ ID NO: 280 |
| ETXS709 | CmsAmsUmUmAmAmGfGmUfGfCfC mCmAmUmGmAmUmGmUmAm | SEQ ID NO: 601 | CAUUAAGGUGCCCAUGA UGUA | SEQ ID NO: 281 |
| ETXS711 | CmsUmsGmCmCmCmUfAmCfCfAfA mGmGmAmAmAmUmGmCmCm | SEQ ID NO: 602 | CUGCCCUACCAAGGAAA UGCC | SEQ ID NO: 282 |
| ETXS713 | CmsAmsAmAmCmUmGfCmCfCfUfA mCmCmAmAmGmGmAmAmAm | SEQ ID NO: 603 | CAAACUGCCCUACCAAG GAAA | SEQ ID NO: 283 |
| ETXS715 | UmsCmsAmAmAmCmUfGmCfCfCfU mAmCmCmAmAmGmGmAmAm | SEQ ID NO: 604 | UCAAACUGCCCUACCAA GGAA | SEQ ID NO: 284 |
| ETXS717 | CmsUmsCmCmAmCmCfUmUfUfGfA mCmAmAmGmAmAmUmUmUm | SEQ ID NO: 605 | CUCCACCUUUGACAAGA AUUU | SEQ ID NO: 285 |
| ETXS719 | CmsGmsAmCmAmCmUfUmUfCfCfA mCmCmUmGmGmAmCmAmAm | SEQ ID NO: 606 | CGACACUUUCCACCUGG ACAA | SEQ ID NO: 286 |
| ETXS721 | AmsCmsUmUmUmCmCfAmCfCfUfG mGmAmCmAmAmGmUmAmCm | SEQ ID NO: 607 | ACUUUCCACCUGGACAA GUAC | SEQ ID NO: 287 |
| ETXS723 | AmsAmsAmCmUmGmCfCmCfUfAfC mCmAmAmGmGmAmAmAmUm | SEQ ID NO: 608 | AAACUGCCCUACCAAGG AAAU | SEQ ID NO: 288 |
| ETXS725 | AmsCmsUmGmCmCmCfUmAfCfCfA mAmGmGmAmAmAmUmGmCm | SEQ ID NO: 609 | ACUGCCCUACCAAGGAA AUGC | SEQ ID NO: 289 |
| ETXS727 | CmsAmsCmUmUmUmCfCmAfCfCfU mGmGmAmCmAmAmGmUmAm | SEQ ID NO: 610 | CACUUUCCACCUGGACA AGUA | SEQ ID NO: 290 |
| ETXS729 | CmsCmsAmUmUmAmAfGmGfUfGfC mCmCmAmUmGmAmUmGmUm | SEQ ID NO: 611 | CCAUUAAGGUGCCCAUG AUGU | SEQ ID NO: 291 |
| ETXS731 | UmsUmsGmCmCmUmCfCmAfCfCfU mUmUmGmAmCmAmAmGmAm | SEQ ID NO: 612 | UUGCCUCCACCUUUGAC AAGA | SEQ ID NO: 292 |
| ETXS733 | UmsUmsUmCmCmAmCfCmUfGfGfA mCmAmAmGmUmAmCmAmAm | SEQ ID NO: 613 | UUUCCACCUGGACAAGU ACAA | SEQ ID NO: 293 |
| ETXS735 | UmsCmsCmUmCmAmAfAmCfUfGfC mCmCmUmAmCmCmAmAmGm | SEQ ID NO: 614 | UCCUCAAACUGCCCUAC CAAG | SEQ ID NO: 294 |
| ETXS737 | AmsAmsCmUmGmCmCfCmUfAfCfC mAmAmGmGmAmAmAmUmGm | SEQ ID NO: 615 | AACUGCCCUACCAAGGA AAUG | SEQ ID NO: 295 |
| ETXS739 | CmsCmsUmCmAmAmAfCmUfGfCfC mCmUmAmCmCmAmAmGmGm | SEQ ID NO: 616 | CCUCAAACUGCCCUACC AAGG | SEQ ID NO: 296 |
| ETXS741 | GmsUmsCmCmUmCmAfAmAfCfUfG mCmCmCmUmAmCmCmAmAm | SEQ ID NO: 617 | GUCCUCAAACUGCCCUA CCAA | SEQ ID NO: 297 |
| ETXS743 | GmsAmsCmAmCmUmUfUmCfCfAfC mCmUmGmGmAmCmAmAmGm | SEQ ID NO: 618 | GACACUUUCCACCUGGA CAAG | SEQ ID NO: 298 |
| ETXS745 | UmsUmsUmGmCmCmUfCmCfAfCfC mUmUmUmGmAmCmAmAmGm | SEQ ID NO: 619 | UUUGCCUCCACCUUUGA CAAG | SEQ ID NO: 299 |
| ETXS747 | AmsCmsAmCmUmUmUfCmCfAfCfC mUmGmGmAmCmAmAmGmUm | SEQ ID NO: 620 | ACACUUUCCACCUGGAC AAGU | SEQ ID NO: 300 |
| ETXS749 | UmsUmsCmCmAmCmCfUmGfGfAfC mAmAmGmUmAmCmAmAmGm | SEQ ID NO: 621 | UUCCACCUGGACAAGUA CAAG | SEQ ID NO: 301 |
| ETXS751 | CmsUmsUmUmCmCmAfCmCfUfGfG mAmCmAmAmGmUmAmCmAm | SEQ ID NO: 622 | CUUUCCACCUGGACAAG UACA | SEQ ID NO: 302 |
| ETXS753 | CmsUmsCmAmAmAmCfUmGfCfCfC mUmAmCmCmAmAmGmGmAm | SEQ ID NO: 623 | CUCAAACUGCCCUACCA AGGA | SEQ ID NO: 303 |
| ETXS755 | UmsUmsAmCmAmUmCfUmUfGfUfU mCmAmAmAmGmGmGmAmAm | SEQ ID NO: 624 | UUACAUCUUGUUCAAAG GGAA | SEQ ID NO: 304 |
| ETXS757 | UmsGmsCmCmAmCmCfAmUfGfCfU mGmGmUmGmGmUmCmCmUm | SEQ ID NO: 625 | UGCCACCAUGCUGGUGG UCCU | SEQ ID NO: 305 |
| ETXS759 | UmsAmsUmCmCmAmAfGmAfGfGfU mAmUmUmUmUmGmAmUmAm | SEQ ID NO: 626 | UAUCCAAGAGGUAUUUU GAUA | SEQ ID NO: 306 |
| ETXS761 | AmsAmsAmUmGmCmCfAmCfCfAfU mGmCmUmGmGmUmGmGmUm | SEQ ID NO: 627 | AAAUGCCACCAUGCUGG UGGU | SEQ ID NO: 307 |
| ETXS763 | AmsUmsUmAmCmAmUfCmUfUfGfU mUmCmAmAmAmGmGmGmAm | SEQ ID NO: 628 | AUUACAUCUUGUUCAAA GGGA | SEQ ID NO: 308 |
| ETXS765 | CmsUmsCmCmAmUmGfAmGfGfCfA mCmGmAmUmGmGmCmAmAm | SEQ ID NO: 629 | CUCCAUGAGGCACGAUG GCAA | SEQ ID NO: 309 |
| ETXS767 | UmsCmsCmAmUmGmCfCmUfCfCfU mGmUmCmAmUmCmAmAmAm | SEQ ID NO: 630 | UCCAUGCCUCCUGUCAU CAAA | SEQ ID NO: 310 |
| ETXS769 | AmsUmsUmCmCmAmUfGmCfCfUfC mCmUmGmUmCmAmUmCmAm | SEQ ID NO: 631 | AUUCCAUGCCUCCUGUC AUCA | SEQ ID NO: 311 |
| ETXS771 | CmsAmsAmGmGmAmAfAmUfGfCfC mAmCmCmAmUmGmCmUmGm | SEQ ID NO: 632 | CAAGGAAAUGCCACCAU GCUG | SEQ ID NO: 312 |
| ETXS773 | UmsGmsGmUmGmGmUfCmCfUfCfA mUmGmGmAmGmAmAmAmAm | SEQ ID NO: 633 | UGGUGGUCCUCAUGGAG AAAA | SEQ ID NO: 313 |
| ETXS775 | AmsUmsCmUmUmGmUfUmCfAfAfA mGmGmGmAmAmAmUmGmGm | SEQ ID NO: 634 | AUCUUGUUCAAAGGAA AUGG | SEQ ID NO: 314 |
| ETXS777 | AmsAmsGmGmAmAmAfUmGfCfCfA mCmCmAmUmGmCmUmGmGm | SEQ ID NO: 635 | AAGGAAAUGCCACCAUG CUGG | SEQ ID NO: 315 |
| ETXS779 | GmsCmsCmUmCmCmAfCmCfUfUfU mGmAmCmAmAmGmAmAmUm | SEQ ID NO: 636 | GCCUCCACCUUUGACAA GAAU | SEQ ID NO: 316 |
| ETXS781 | GmsAmsGmUmUmUmUfGmCfCfUfU mCmAmUmCmCmAmCmAmAm | SEQ ID NO: 637 | GAGUUUUGCCUUCAUCC ACAA | SEQ ID NO: 317 |
| ETXS783 | GmsCmsUmGmCmGmAfAmAfGfAfU mCmUmCmCmAmUmGmAmGm | SEQ ID NO: 638 | GCUGCGAAAGAUCUCCA UGAG | SEQ ID NO: 318 |
| ETXS785 | AmsGmsUmUmUmGmCfCmUfCfCfA mCmCmUmUmUmGmAmCmAm | SEQ ID NO: 639 | AGUUUGCCUCCACCUUU GACA | SEQ ID NO: 319 |
| ETXS787 | GmsUmsUmUmGmCmCfUmCfCfAfC mCmUmUmUmGmAmCmAmAm | SEQ ID NO: 640 | GUUUGCCUCCACCUUUG ACAA | SEQ ID NO: 320 |
| ETXS789 | CmsAmsUmCmUmUmGfUmUfCfAfA mAmGmGmGmAmAmAmUmGm | SEQ ID NO: 641 | CAUCUUGUUCAAAGGA AAUG | SEQ ID NO: 321 |
| ETXS791 | GmsGmsAmGmUmUmUfUmGfCfCfU mUmCmAmUmCmCmAmCmAm | SEQ ID NO: 642 | GGAGUUUUGCCUUCAUC CACA | SEQ ID NO: 322 |
| ETXS793 | CmsUmsGmCmGmAmAfAmGfAfUfC mUmCmCmAmUmGmAmGmGm | SEQ ID NO: 643 | CUGCGAAAGAUCUCCAU GAGG | SEQ ID NO: 323 |
| ETXS795 | CmsCmsAmUmGmCmCfUmCfCfUfG mUmCmAmUmCmAmAmAmGm | SEQ ID NO: 644 | CCAUGCCUCCUGUCAUC AAAG | SEQ ID NO: 324 |
| ETXS797 | UmsGmsUmUmUmCmUfGmGfGfCfA mGmGmGmUmGmGmUmGmAm | SEQ ID NO: 645 | UGUUUCUGGGCAGGGUG GUGA | SEQ ID NO: 325 |
| ETXS799 | GmsCmsCmUmGmCmUfGmCfGfAfA mAmGmAmUmCmUmCmCmAm | SEQ ID NO: 646 | GCCUGCUGCGAAAGAUC UCCA | SEQ ID NO: 326 |
| ETXS801 | AmsAmsCmUmGmUmUfUmGfAfUfG mAmGmAmUmUmAmAmUmCm | SEQ ID NO: 647 | AACUGUUUGAUGAGAUU AAUC | SEQ ID NO: 327 |
| ETXS803 | AmsUmsGmCmCmUmCfCmUfGfUfC mAmUmCmAmAmAmGmUmGm | SEQ ID NO: 648 | AUGCCUCCUGUCAUCAA AGUG | SEQ ID NO: 328 |
| ETXS805 | AmsGmsUmAmUmGmAfGmAfUfGfC mAmUmGmAmGmCmUmGmCm | SEQ ID NO: 649 | AGUAUGAGAUGCAUGAG CUGC | SEQ ID NO: 329 |
| ETXS807 | AmsAmsGmUmAmUmGfAmGfAfUfG mCmAmUmGmAmGmCmUmGm | SEQ ID NO: 650 | AAGUAUGAGAUGCAUGA GCUG | SEQ ID NO: 330 |
| ETXS809 | UmsGmsUmCmCmUmCfAmAfAfCfU mGmCmCmCmUmAmCmCmAm | SEQ ID NO: 651 | UGUCCUCAAACUGCCCU ACCA | SEQ ID NO: 331 |
| ETXS811 | AmsCmsUmGmUmUmUfGmAfUfGfA mGmAmUmUmAmAmUmCmCm | SEQ ID NO: 652 | ACUGUUUGAUGAGAUUA AUCC | SEQ ID NO: 332 |
| ETXS813 | GmsAmsCmCmAmUmUfAmAfGfGfU mGmCmCmAmUmGmAmUm | SEQ ID NO: 653 | GACCAUUAAGGUGCCCA UGAU | SEQ ID NO: 333 |
| ETXS815 | AmsCmsCmAmUmUmAfAmGfGfUfG mCmCmCmAmUmGmAmUmGm | SEQ ID NO: 654 | ACCAUUAAGGUGCCCAU GAUG | SEQ ID NO: 334 |
| ETXS817 | GmsUmsUmUmCmUmGfGmGfCfAfG mGmGmUmGmGmUmGmAmAm | SEQ ID NO: 655 | GUUUCUGGGCAGGGUGG UGAA | SEQ ID NO: 335 |
| ETXS819 | UmsUmsCmUmGmUmUfUmCfUfGfG mGmCmAmGmGmGmUmGmGm | SEQ ID NO: 656 | UUCUGUUUCUGGGCAGG GUGG | SEQ ID NO: 336 |
| ETXS821 | UmsUmsUmUmCmUmUfUmCfCfGfA mAmGmUmUmCmAmAmGmCm | SEQ ID NO: 657 | UUUUCUUUCCGAAGUUC AAGC | SEQ ID NO: 337 |
| ETXS823 | UmsUmsUmCmUmUmUfCmCfGfAfA mGmUmUmCmAmAmGmCmUm | SEQ ID NO: 658 | UUUCUUUCCGAAGUUCA AGCU | SEQ ID NO: 338 |
| ETXS825 | UmsCmsUmGmUmUmUfCmUfGfGfG mCmAmGmGmGmUmGmGmUm | SEQ ID NO: 659 | UCUGUUUCUGGGCAGGG UGGU | SEQ ID NO: 339 |
| ETXS827 | AmsUmsGmUmCmCmUfCmAfAfAfC mUmGmCmCmCmUmAmCmCm | SEQ ID NO: 660 | AUGUCCUCAAACUGCCC UACC | SEQ ID NO: 340 |
| ETXS829 | UmsCmsGmAmCmAmCfUmUfUfCfC mAmCmCmUmGmGmAmCmAm | SEQ ID NO: 661 | UCGACACUUUCCACCUG GACA | SEQ ID NO: 341 |
| ETXS831 | CfsCmsUfUmCfAmUfCmCfAmCfAmA fGmGfAmUfUmUf | SEQ ID NO: 662 | CCUUCAUCCACAAGGAU UU | SEQ ID NO: 342 |
| ETXS833 | CfsGmsAfAmAfGmAfUmCfUmCfCmA fUmGfAmGfGmCf | SEQ ID NO: 663 | CGAAAGAUCUCCAUGAG GC | SEQ ID NO: 343 |
| ETXS835 | UfsGmsGfUmGfGmUfCmCfUmCfAm UfGmGfAmGfAmAf | SEQ ID NO: 664 | UGGUGGUCCUCAUGGAG AA | SEQ ID NO: 344 |
| ETXS837 | GfsAmsAfAmGfAmUfCmUfCmCfAm UfGmAfGmGfCmAf | SEQ ID NO: 665 | GAAAGAUCUCCAUGAGG CA | SEQ ID NO: 345 |
| ETXS839 | AfsUmsGfAmGfAmUfGmCfAmUfGm AfGmCfUmGfCmUf | SEQ ID NO: 666 | AUGAGAUGCAUGAGCUG CU | SEQ ID NO: 346 |
| ETXS841 | UfsUmsGfAmUfGmAfGmAfUmUfAm AfUmCfCmUfGmAf | SEQ ID NO: 667 | UUGAUGAGAUUAAUCCU GA | SEQ ID NO: 347 |
| ETXS843 | AfsGmsAfUmUfAmAfUmCfCmUfGm AfAmAfCmCfAmAf | SEQ ID NO: 668 | AGAUUAAUCCUGAAACC AA | SEQ ID NO: 348 |
| ETXS845 | AfsUmsGfAmGfAmUfUmAfAmUfCm CfUmGfAmAfAmCf | SEQ ID NO: 669 | AUGAGAUUAAUCCUGAA AC | SEQ ID NO: 349 |
| ETXS847 | GfsAmsGfAmUfUmAfAmUfCmCfUm GfAmAfAmCfCmAf | SEQ ID NO: 670 | GAGAUUAAUCCUGAAAC CA | SEQ ID NO: 350 |
| ETXS849 | UfsGmsAfGmAfUmUfAmAfUmCfCm UfGmAfAmAfCmCf | SEQ ID NO: 671 | UGAGAUUAAUCCUGAAA CC | SEQ ID NO: 351 |
| ETXS851 | GfsUmsUfUmGfAmUfGmAfGmAfUm UfAmAfUmCfCmUf | SEQ ID NO: 672 | GUUUGAUGAGAUUAAUC CU | SEQ ID NO: 352 |
| ETXS853 | UfsUmsGfCmCfUmUfCmAfUmCfCmA fCmAfAmGfGmAf | SEQ ID NO: 673 | UUGCCUUCAUCCACAAG GA | SEQ ID NO: 353 |
| ETXS855 | AfsAmsAfGmAfUmCfUmCfCmAfUm GfAmGfGmCfAmCf | SEQ ID NO: 674 | AAAGAUCUCCAUGAGGC AC | SEQ ID NO: 354 |
| ETXS857 | CfsUmsGfGmUfGmGfUmCfCmUfCmA fUmGfGmAfGmAf | SEQ ID NO: 675 | CUGGUGGUCCUCAUGGA GA | SEQ ID NO: 355 |
| ETXS859 | UfsUmsCfAmUfCmCfAmCfAmAfGmG fAmUfUmUfUmGf | SEQ ID NO: 676 | UUCAUCCACAAGGAUUU UG | SEQ ID NO: 356 |
| ETXS861 | AfsAmsGfAmUfCmUfCmCfAmUfGm AfGmGfCmAfCmGf | SEQ ID NO: 677 | AAGAUCUCCAUGAGGCA CG | SEQ ID NO: 357 |
| ETXS863 | CfsAmsUfCmCfAmCfAmAfGmGfAmU fUmUfUmGfAmUf | SEQ ID NO: 678 | CAUCCACAAGGAUUUUG AU | SEQ ID NO: 358 |
| ETXS865 | UfsGmsAfUmGfAmGfAmUfUmAfAm UfCmCfUmGfAmAf | SEQ ID NO: 679 | UGAUGAGAUUAAUCCUG AA | SEQ ID NO: 359 |
| ETXS867 | GfsCmsCfUmUfCmAfUmCfCmAfCmA fAmGfGmAfUmUf | SEQ ID NO: 680 | GCCUUCAUCCACAAGGA UU | SEQ ID NO: 360 |
| ETXS869 | UfsCmsUfCmCfAmUfGmAfGmGfCmA fCmGfAmUfGmGf | SEQ ID NO: 681 | UCUCCAUGAGGCACGAU GG | SEQ ID NO: 361 |
| ETXS871 | UmsGmsCmCmUmUmCfAfUfCfCfAm CmAmAmGmGmAmUfUmUm | SEQ ID NO: 682 | UGCCUUCAUCCACAAGG AUUU | SEQ ID NO: 242 |
| ETXS873 | UmsGmsCmGmAmAmAfGfAfUfCfUm CmCmAmUmGmAmGfGmCm | SEQ ID NO: 683 | UGCGAAAGAUCUCCAUG AGGC | SEQ ID NO: 243 |
| ETXS875 | GmsCmsUmGmGmUmGfGfUfCfCfUm CmAmUmGmGmAmGfAmAm | SEQ ID NO: 684 | GCUGGUGGUCCUCAUGG AGAA | SEQ ID NO: 244 |
| ETXS877 | GmsCmsGmAmAmAmGfAfUfCfUfCm CmAmUmGmAmGmGfCmAm | SEQ ID NO: 685 | GCGAAAGAUCUCCAUGA GGCA | SEQ ID NO: 245 |
| ETXS879 | GmsUmsAmUmGmAmGfAfUfGfCfAm UmGmAmGmCmUmGfCmUm | SEQ ID NO: 686 | GUAUGAGAUGCAUGAGC UGCU | SEQ ID NO: 246 |
| ETXS881 | GmsUmsUmUmGmAmUfGfAfGfAfUm UmAmAmUmCmCmUfGmAm | SEQ ID NO: 687 | GUUUGAUGAGAUUAAUC CUGA | SEQ ID NO: 247 |
| ETXS883 | UmsGmsAmGmAmUmUfAfAfUfCfCm UmGmAmAmAmCmCfAmAm | SEQ ID NO: 688 | UGAGAUUAAUCCUGAAA CCAA | SEQ ID NO: 248 |
| ETXS885 | UmsGmsAmUmGmAmGfAfUfUfAfAm UmCmCmUmGmAmAfAmCm | SEQ ID NO: 689 | UGAUGAGAUUAAUCCUG AAAC | SEQ ID NO: 249 |
| ETXS887 | AmsUmsGmAmGmAmUfUfAfAfUfCm CmUmGmAmAmAmCfCmAm | SEQ ID NO: 690 | AUGAGAUUAAUCCUGAA ACCA | SEQ ID NO: 250 |
| ETXS889 | GmsAmsUmGmAmGmAfUfUfAfAfUm CmCmUmGmAmAmAfCmCm | SEQ ID NO: 691 | GAUGAGAUUAAUCCUGA AACC | SEQ ID NO: 251 |
| ETXS891 | CmsUmsGmUmUmUmGfAfUfGfAfGm AmUmUmAmAmUmCfCmUm | SEQ ID NO: 692 | CUGUUUGAUGAGAUUAA UCCU | SEQ ID NO: 252 |
| ETXS893 | UmsUmsUmUmGmCmCfUfUfCfAfUm CmCmAmCmAmAmGfGmAm | SEQ ID NO: 693 | UUUUGCCUUCAUCCACA AGGA | SEQ ID NO: 253 |
| ETXS895 | CmsGmsAmAmAmGmAfUfCfUfCfCm AmUmGmAmGmGmCfAmCm | SEQ ID NO: 694 | CGAAAGAUCUCCAUGAG GCAC | SEQ ID NO: 254 |
| ETXS897 | UmsGmsCmUmGmGmUfGfGfUfCfCm UmCmAmUmGmGmAfGmAm | SEQ ID NO: 695 | UGCUGGUGGUCCUCAUG GAGA | SEQ ID NO: 255 |
| ETXS899 | CmsCmsUmUmCmAmUfCfCfAfCfAm AmGmGmAmUmUmUfUmGm | SEQ ID NO: 696 | CCUUCAUCCACAAGGAU UUUG | SEQ ID NO: 256 |
| ETXS901 | GmsAmsAmAmGmAmUfCfUfCfCfAm UmGmAmGmGmCmAfCmGm | SEQ ID NO: 697 | GAAAGAUCUCCAUGAGG CACG | SEQ ID NO: 257 |
| ETXS903 | UmsUmsCmAmUmCmCfAfCfAfAfGm GmAmUmUmUmUmGfAmUm | SEQ ID NO: 698 | UUCAUCCACAAGGAUUU UGAU | SEQ ID NO: 258 |
| ETXS905 | UmsUmsUmGmAmUmGfAfGfAfUfUm AmAmUmCmCmUmGfAmAm | SEQ ID NO: 699 | UUUGAUGAGAUUAAUCC UGAA | SEQ ID NO: 259 |
| ETXS907 | UmsUmsGmCmCmUmUfCfAfUfCfCm AmCmAmAmGmGmAfUmUm | SEQ ID NO: 700 | UUGCCUUCAUCCACAAG GAUU | SEQ ID NO: 260 |
| ETXS909 | GmsAmsUmCmUmCmCfAfUfGfAfGm GmCmAmCmGmAmUfGmGm | SEQ ID NO: 701 | GAUCUCCAUGAGGCACG AUGG | SEQ ID NO: 261 |
| ETXS911 | UmsGmsCmCmUmUfCfAmUfCfCfAfC mAmAmGmGmAmUmUmUm | SEQ ID NO: 702 | UGCCUUCAUCCACAAGG AUUU | SEQ ID NO: 242 |
| ETXS913 | UmsGmsCmGmAmAfAfGmAfUfCfUf CmCmAmUmGmAmGmGmCm | SEQ ID NO: 703 | UGCGAAAGAUCUCCAUG AGGC | SEQ ID NO: 243 |
| ETXS915 | GmsCmsUmGmGmUfGfGmUfCfCfUfC mAmUmGmGmAmGmAmAm | SEQ ID NO: 704 | GCUGGUGGUCCUCAUGG AGAA | SEQ ID NO: 244 |
| ETXS917 | GmsCmsGmAmAmAfGfAmUfCfUfCfC mAmUmGmAmGmGmCmAm | SEQ ID NO: 705 | GCGAAAGAUCUCCAUGA GGCA | SEQ ID NO: 245 |
| ETXS919 | GmsUmsAmUmGmAfGfAmUfGfCfAf UmGmAmGmCmUmGmCmUm | SEQ ID NO: 706 | GUAUGAGAUGCAUGAGC UGCU | SEQ ID NO: 246 |
| ETXS921 | GmsUmsUmUmGmAfUfGmAfGfAfUf UmAmAmUmCmCmUmGmAm | SEQ ID NO: 707 | GUUUGAUGAGAUUAAUC CUGA | SEQ ID NO: 247 |
| ETXS923 | UmsGmsAmGmAmUfUfAmAfUfCfCf UmGmAmAmAmCmCmAmAm | SEQ ID NO: 708 | UGAGAUUAAUCCUGAAA CCAA | SEQ ID NO: 248 |
| ETXS925 | UmsGmsAmUmGmAfGfAmUfUfAfAf UmCmCmUmGmAmAmAmCm | SEQ ID NO: 709 | UGAUGAGAUUAAUCCUG AAAC | SEQ ID NO: 249 |
| ETXS927 | AmsUmsGmAmGmAfUfUmAfAfUfCf CmUmGmAmAmAmCmCmAm | SEQ ID NO: 710 | AUGAGAUUAAUCCUGAA ACCA | SEQ ID NO: 250 |
| ETXS929 | GmsAmsUmGmAmGfAfUmUfAfAfUf CmCmUmGmAmAmAmCmCm | SEQ ID NO: 711 | GAUGAGAUUAAUCCUGA AACC | SEQ ID NO: 251 |
| ETXS931 | CmsUmsGmUmUmUfGfAmUfGfAfGf AmUmUmAmAmUmCmCmUm | SEQ ID NO: 712 | CUGUUUGAUGAGAUUAA UCCU | SEQ ID NO: 252 |
| ETXS933 | UmsUmsUmUmGmCfCfUmUfCfAfUfC mCmAmCmAmAmGmGmAm | SEQ ID NO: 713 | UUUUGCCUUCAUCCACA AGGA | SEQ ID NO: 253 |
| ETXS935 | CmsGmsAmAmAmGfAfUmCfUfCfCfA mUmGmAmGmGmCmAmCm | SEQ ID NO: 714 | CGAAAGAUCUCCAUGAG GCAC | SEQ ID NO: 254 |
| ETXS937 | UmsGmsCmUmGmGfUfGmGfUfCfCf UmCmAmUmGmGmAmGmAm | SEQ ID NO: 715 | UGCUGGUGGUCCUCAUG GAGA | SEQ ID NO: 255 |
| ETXS939 | CmsCmsUmUmCmAfUfCmCfAfCfAfA mGmGmAmUmUmUmUmGm | SEQ ID NO: 716 | CCUUCAUCCACAAGGAU UUUG | SEQ ID NO: 256 |
| ETXS941 | GmsAmsAmAmGmAfUfCmUfCfCfAf UmGmAmGmGmCmAmCmGm | SEQ ID NO: 717 | GAAAGAUCUCCAUGAGG CACG | SEQ ID NO: 257 |
| ETXS943 | UmsUmsCmAmUmCfCfAmCfAfAfGfG mAmUmUmUmUmGmAmUm | SEQ ID NO: 718 | UUCAUCCACAAGGAUUU UGAU | SEQ ID NO: 258 |
| ETXS945 | UmsUmsUmGmAmUfGfAmGfAfUfUf AmAmUmCmCmUmGmAmAm | SEQ ID NO: 719 | UUUGAUGAGAUUAAUCC UGAA | SEQ ID NO: 259 |
| ETXS947 | UmsUmsGmCmCmUfUfCmAfUfCfCfA mCmAmAmGmGmAmUmUm | SEQ ID NO: 720 | UUGCCUUCAUCCACAAG GAUU | SEQ ID NO: 260 |
| ETXS949 | GmsAmsUmCmUmCfCfAmUfGfAfGf GmCmAmCmGmAmUmGmGm | SEQ ID NO: 721 | GAUCUCCAUGAGGCACG AUGG | SEQ ID NO: 261 |
| ETXS951 | UmsGmsCmCmUmUmCfAmUfCfCfAf CmAmAmGmGmAmUmUmUm | SEQ ID NO: 722 | UGCCUUCAUCCACAAGG AUUU | SEQ ID NO: 242 |
| ETXS953 | UmsGmsCmGmAmAmAfGmAfUfCfUf CmCmAmUmGmAmGmGmCm | SEQ ID NO: 723 | UGCGAAAGAUCUCCAUG AGGC | SEQ ID NO: 243 |
| ETXS955 | GmsCmsUmGmGmUmGfGmUfCfCfUf CmAmUmGmGmAmGmAmAm | SEQ ID NO: 724 | GCUGGUGGUCCUCAUGG AGAA | SEQ ID NO: 244 |
| ETXS957 | GmsCmsGmAmAmAmGfAmUfCfUfCf CmAmUmGmAmGmGmCmAm | SEQ ID NO: 725 | GCGAAAGAUCUCCAUGA GGCA | SEQ ID NO: 245 |
| ETXS959 | GmsUmsAmUmGmAmGfAmUfGfCfAf UmGmAmGmCmUmGmCmUm | SEQ ID NO: 726 | GUAUGAGAUGCAUGAGC UGCU | SEQ ID NO: 246 |
| ETXS961 | GmsUmsUmUmGmAmUfGmAfGfAfUf UmAmAmUmCmCmUmGmAm | SEQ ID NO: 727 | GUUUGAUGAGAUUAAUC CUGA | SEQ ID NO: 247 |
| ETXS963 | UmsGmsAmGmAmUmUfAmAfUfCfCf UmGmAmAmAmCmCmAmAm | SEQ ID NO: 728 | UGAGAUUAAUCCUGAAA CCAA | SEQ ID NO: 248 |
| ETXS965 | UmsGmsAmUmGmAmGfAmUfUfAfAf UmCmCmUmGmAmAmAmCm | SEQ ID NO: 729 | UGAUGAGAUUAAUCCUG AAAC | SEQ ID NO: 249 |
| ETXS967 | AmsUmsGmAmGmAmUfUmAfAfUfCf CmUmGmAmAmAmCmCmAm | SEQ ID NO: 730 | AUGAGAUUAAUCCUGAA ACCA | SEQ ID NO: 250 |
| ETXS969 | GmsAmsUmGmAmGmAfUmUfAfAfUf CmCmUmGmAmAmAmCmCm | SEQ ID NO: 731 | GAUGAGAUUAAUCCUGA AACC | SEQ ID NO: 251 |
| ETXS971 | CmsUmsGmUmUmUmGfAmUfGfAfGf AmUmUmAmAmUmCmCmUm | SEQ ID NO: 732 | CUGUUUGAUGAGAUUAA UCCU | SEQ ID NO: 252 |
| ETXS973 | UmsUmsUmUmGmCmCfUmUfCfAfUf CmCmAmCmAmAmGmGmAm | SEQ ID NO: 733 | UUUUGCCUUCAUCCACA AGGA | SEQ ID NO: 253 |
| ETXS975 | CmsGmsAmAmAmGmAfUmCfUfCfCf AmUmGmAmGmGmCmAmCm | SEQ ID NO: 734 | CGAAAGAUCUCCAUGAG GCAC | SEQ ID NO: 254 |
| ETXS977 | UmsGmsCmUmGmGmUfGmGfUfCfCf UmCmAmUmGmGmAmGmAm | SEQ ID NO: 735 | UGCUGGUGGUCCUCAUG GAGA | SEQ ID NO: 255 |
| ETXS979 | CmsCmsUmUmCmAmUfCmCfAfCfAf AmGmGmAmUmUmUmUmGm | SEQ ID NO: 736 | CCUUCAUCCACAAGGAU UUUG | SEQ ID NO: 256 |
| ETXS981 | GmsAmsAmAmGmAmUfCmUfCfCfAf UmGmAmGmGmCmAmCmGm | SEQ ID NO: 737 | GAAAGAUCUCCAUGAGG CACG | SEQ ID NO: 257 |
| ETXS983 | UmsUmsCmAmUmCmCfAmCfAfAfGf GmAmUmUmUmUmGmAmUm | SEQ ID NO: 738 | UUCAUCCACAAGGAUUU UGAU | SEQ ID NO: 258 |
| ETXS985 | UmsUmsUmGmAmUmGfAmGfAfUfUf AmAmUmCmCmUmGmAmAm | SEQ ID NO: 739 | UUUGAUGAGAUUAAUCC UGAA | SEQ ID NO: 259 |
| ETXS987 | UmsUmsGmCmCmUmUfCmAfUfCfCf AmCmAmAmGmGmAmUmUm | SEQ ID NO: 740 | UUGCCUUCAUCCACAAG GAUU | SEQ ID NO: 260 |
| ETXS989 | GmsAmsUmCmUmCmCfAmUfGfAfGf GmCmAmCmGmAmUmGmGm | SEQ ID NO: 741 | GAUCUCCAUGAGGCACG AUGG | SEQ ID NO: 261 |
| ETXS991 | UmsGmsCmCmUmUmCfAmUfCfCfAf CmAmAmGmGmAmUmUmUm | SEQ ID NO: 742 | UGCCUUCAUCCACAAGG AUUU | SEQ ID NO: 242 |
| ETXS993 | UmsGmsCmGmAmAmAfGmAfUfCfUf CmCmAmUmGmAmGmGmCm | SEQ ID NO: 743 | UGCGAAAGAUCUCCAUG AGGC | SEQ ID NO: 243 |
| ETXS995 | GmsCmsUmGmGmUmGfGmUfCfCfUf CmAmUmGmGmAmGmAmAm | SEQ ID NO: 744 | GCUGGUGGUCCUCAUGG AGAA | SEQ ID NO: 244 |
| ETXS997 | GmsCmsGmAmAmAmGfAmUfCfUfCf CmAmUmGmAmGmGmCmAm | SEQ ID NO: 745 | GCGAAAGAUCUCCAUGA GGCA | SEQ ID NO: 245 |
| ETXS999 | GmsUmsAmUmGmAmGfAmUfGfCfAf UmGmAmGmCmUmGmCmUm | SEQ ID NO: 746 | GUAUGAGAUGCAUGAGC UGCU | SEQ ID NO: 246 |
| ETXS1001 | GmsUmsUmUmGmAmUfGmAfGfAfUf UmAmAmUmCmCmUmGmAm | SEQ ID NO: 747 | GUUUGAUGAGAUUAAUC CUGA | SEQ ID NO: 247 |
| ETXS1003 | UmsGmsAmGmAmUmUfAmAfUfCfCf UmGmAmAmAmCmCmAmAm | SEQ ID NO: 748 | UGAGAUUAAUCCUGAAA CCAA | SEQ ID NO: 248 |
| ETXS1005 | UmsGmsAmUmGmAmGfAmUfUfAfAf UmCmCmUmGmAmAmAmCm | SEQ ID NO: 749 | UGAUGAGAUUAAUCCUG AAAC | SEQ ID NO: 249 |
| ETXS1007 | AmsUmsGmAmGmAmUfUmAfAfUfCf CmUmGmAmAmAmCmCmAm | SEQ ID NO: 750 | AUGAGAUUAAUCCUGAA ACCA | SEQ ID NO: 250 |
| ETXS1009 | GmsAmsUmGmAmGmAfUmUfAfAfUf CmCmUmGmAmAmAmCmCm | SEQ ID NO: 751 | GAUGAGAUUAAUCCUGA AACC | SEQ ID NO: 251 |
| ETXS1011 | CmsUmsGmUmUmUmGfAmUfGfAfGf AmUmUmAmAmUmCmCmUm | SEQ ID NO: 752 | CUGUUUGAUGAGAUUAA UCCU | SEQ ID NO: 252 |
| ETXS1013 | UmsUmsUmUmGmCmCfUmUfCfAfUf CmCmAmCmAmAmGmGmAm | SEQ ID NO: 753 | UUUUGCCUUCAUCCACA AGGA | SEQ ID NO: 253 |
| ETXS1015 | CmsGmsAmAmAmGmAfUmCfUfCfCf AmUmGmAmGmGmCmAmCm | SEQ ID NO: 754 | CGAAAGAUCUCCAUGAG GCAC | SEQ ID NO: 254 |
| ETXS1017 | UmsGmsCmUmGmGmUfGmGfUfCfCf UmCmAmUmGmGmAmGmAm | SEQ ID NO: 755 | UGCUGGUGGUCCUCAUG GAGA | SEQ ID NO: 255 |
| ETXS1019 | CmsCmsUmUmCmAmUfCmCfAfCfAf AmGmGmAmUmUmUmUmGm | SEQ ID NO: 756 | CCUUCAUCCACAAGGAU UUUG | SEQ ID NO: 256 |
| ETXS1021 | GmsAmsAmAmGmAmUfCmUfCfCfAf UmGmAmGmGmCmAmCmGm | SEQ ID NO: 757 | GAAAGAUCUCCAUGAGG CACG | SEQ ID NO: 257 |
| ETXS1023 | UmsUmsCmAmUmCmCfAmCfAfAfGf GmAmUmUmUmUmGmAmUm | SEQ ID NO: 758 | UUCAUCCACAAGGAUUU UGAU | SEQ ID NO: 258 |
| ETXS1025 | UmsUmsUmGmAmUmGfAmGfAfUfUf AmAmUmCmCmUmGmAmAm | SEQ ID NO: 759 | UUUGAUGAGAUUAAUCC UGAA | SEQ ID NO: 259 |
| ETXS1027 | UmsUmsGmCmCmUmUfCmAfUfCfCf AmCmAmAmGmGmAmUmUm | SEQ ID NO: 760 | UUGCCUUCAUCCACAAG GAUU | SEQ ID NO: 260 |
| ETXS1029 | GmsAmsUmCmUmCmCfAmUfGfAfGf GmCmAmCmGmAmUmGmGm | SEQ ID NO: 761 | GAUCUCCAUGAGGCACG AUGG | SEQ ID NO: 261 |
| ETXS1031 | iaiaGmsCmsCmUmUmCmAfUmCfCfA fCfAmAmGmGmAmUmUmUmUm | SEQ ID NO: 772 | GCCUUCAUCCACAAGGA UUUU | SEQ ID NO: 264 |
| ETXS1033 | iaiaGmsCmsCmUmUmCmAmUmCfCf AfCmAmAmGmGmAmUmUmUmUm | SEQ ID NO: 773 | GCCUUCAUCCACAAGGA UUUU | SEQ ID NO: 264 |
| ETXS1037 | iaiaUmsAmsCmCmAmAmGmGmAfAf AfUmGmCmCmAmCmCmAmUmGm | SEQ ID NO: 775 | UACCAAGGAAAUGCCAC CAUG | SEQ ID NO: 265 |
| ETXS1041 | iaiaAmsAmsAmGmAmUmCmUmCfCf AfUmGmAmGmGmCmAmCmGmAm | SEQ ID NO: 777 | AAAGAUCUCCAUGAGGC ACGA | SEQ ID NO: 268 |
| ETXS1043 | iaiaCmsUmsCmCmAmCmCfUmUfUfG fAfCmAmAmGmAmAmUmUmUm | SEQ ID NO: 778 | CUCCACCUUUGACAAGA AUUU | SEQ ID NO: 285 |
| ETXS1045 | iaiaCmsUmsCmCmAmCmCmUmUfUf GfAmCmAmAmGmAmAmUmUmUm | SEQ ID NO: 779 | CUCCACCUUUGACAAGA AUUU | SEQ ID NO: 285 |
| ETXS1047 | iaiaGmsGmsAmGmUmUmUfUmGfCfC fUfUmCmAmUmCmCmAmCmAm | SEQ ID NO: 780 | GGAGUUUUGCCUUCAUC CACA | SEQ ID NO: 322 |
| ETXS1049 | iaiaGmsGmsAmGmUmUmUmUmGfCf CfUmUmCmAmUmCmCmAmCmAm | SEQ ID NO: 781 | GGAGUUUUGCCUUCAUC CACA | SEQ ID NO: 322 |
| ETXS2127 | GmsCmsCmUmUmCmAfUmCfCfAfC mAmAmGmGmAmCmUmUmUm | SEQ ID NO: 798 | GCCUUCAUCCACAAGGA CUUU | SEQ ID NO: 791 |
| ETXS2143 | CmsUmsCmCmAmCmCfUmUfUfGfA mCmAmAmGmAmAmGmUmUm | SEQ ID NO: 799 | CUCCACCUUUGACAAGA AGUU | SEQ ID NO: 792 |
| ETXS2151 | GmsUmsAmGmCmUmUfUmGfCfCfU mUmCmAmUmCmCmAmCmAm | SEQ ID NO: 800 | GUAGCUUUGCCUUCAUC CACA | SEQ ID NO: 793 |
| ETXS2397 | iaiaUmsAmsCmCmAmAmGmGmAfAf AfUmGmCmCmAmCmCmAmUmGm | SEQ ID NO: 819 | UACCAAGGAAAUGCCAC CAUG | SEQ ID NO: 265 |
| ETXS2399 | iaiaAmsAmsAmGmAmUmCmUmCfCf AfUmGmAmGmGmCmAmCmGmAm | SEQ ID NO: 820 | AAAGAUCUCCAUGAGGC ACGA | SEQ ID NO: 268 |

Some of the modified second strand sequences as illustrated above in Table 4 include the preferred 5' iaia motif. However, it should also be understood that the scope of these modified second strand sequences additionally includes the Me / F modified second strand in the absence of the 5'iaia motif.

Table 5 identifies duplexes with Duplex IDs referencing the modified antisense and sense IDs from previous Tables 3 and 4.

**Table 5**

| Duplex ID | First (Antisense) strand ID | Second (Sense) strand ID |
|---|---|---|
| ETXM1184 | ETXS1036 | ETXS1035 |
| ETXM1157 | ETXS1040 | ETXS1039 |
| ETXM316 | ETXS632 | ETXS631 |
| ETXM317 | ETXS634 | ETXS633 |
| ETXM318 | ETXS636 | ETXS635 |
| ETXM319 | ETXS638 | ETXS637 |
| ETXM320 | ETXS640 | ETXS639 |
| ETXM321 | ETXS642 | ETXS641 |
| ETXM322 | ETXS644 | ETXS643 |
| ETXM323 | ETXS646 | ETXS645 |
| ETXM324 | ETXS648 | ETXS647 |
| ETXM325 | ETXS650 | ETXS649 |
| ETXM326 | ETXS652 | ETXS651 |
| ETXM327 | ETXS654 | ETXS653 |
| ETXM328 | ETXS656 | ETXS655 |
| ETXM329 | ETXS658 | ETXS657 |
| ETXM330 | ETXS660 | ETXS659 |
| ETXM331 | ETXS662 | ETXS661 |
| ETXM332 | ETXS664 | ETXS663 |
| ETXM333 | ETXS666 | ETXS665 |
| ETXM334 | ETXS668 | ETXS667 |
| ETXM335 | ETXS670 | ETXS669 |
| ETXM336 | ETXS672 | ETXS671 |
| ETXM337 | ETXS674 | ETXS673 |
| ETXM338 | ETXS676 | ETXS675 |
| ETXM339 | ETXS678 | ETXS677 |
| ETXM340 | ETXS680 | ETXS679 |
| ETXM341 | ETXS682 | ETXS681 |
| ETXM342 | ETXS684 | ETXS683 |
| ETXM343 | ETXS686 | ETXS685 |
| ETXM344 | ETXS688 | ETXS687 |
| ETXM345 | ETXS690 | ETXS689 |
| ETXM346 | ETXS692 | ETXS691 |
| ETXM347 | ETXS694 | ETXS693 |
| ETXM348 | ETXS696 | ETXS695 |
| ETXM349 | ETXS698 | ETXS697 |
| ETXM350 | ETXS700 | ETXS699 |
| ETXM351 | ETXS702 | ETXS701 |
| ETXM352 | ETXS704 | ETXS703 |
| ETXM353 | ETXS706 | ETXS705 |
| ETXM354 | ETXS708 | ETXS707 |
| ETXM355 | ETXS710 | ETXS709 |
| ETXM356 | ETXS712 | ETXS711 |
| ETXM357 | ETXS714 | ETXS713 |
| ETXM358 | ETXS716 | ETXS715 |
| ETXM359 | ETXS718 | ETXS717 |
| ETXM360 | ETXS720 | ETXS719 |
| ETXM361 | ETXS722 | ETXS721 |
| ETXM362 | ETXS724 | ETXS723 |
| ETXM363 | ETXS726 | ETXS725 |
| ETXM364 | ETXS728 | ETXS727 |
| ETXM365 | ETXS730 | ETXS729 |
| ETXM366 | ETXS732 | ETXS731 |
| ETXM367 | ETXS734 | ETXS733 |
| ETXM368 | ETXS736 | ETXS735 |
| ETXM369 | ETXS738 | ETXS737 |
| ETXM370 | ETXS740 | ETXS739 |
| ETXM371 | ETXS742 | ETXS741 |
| ETXM372 | ETXS744 | ETXS743 |
| ETXM373 | ETXS746 | ETXS745 |
| ETXM374 | ETXS748 | ETXS747 |
| ETXM375 | ETXS750 | ETXS749 |
| ETXM376 | ETXS752 | ETXS751 |
| ETXM377 | ETXS754 | ETXS753 |
| ETXM378 | ETXS756 | ETXS755 |
| ETXM379 | ETXS758 | ETXS757 |
| ETXM380 | ETXS760 | ETXS759 |
| ETXM381 | ETXS762 | ETXS761 |
| ETXM382 | ETXS764 | ETXS763 |
| ETXM383 | ETXS766 | ETXS765 |
| ETXM384 | ETXS768 | ETXS767 |
| ETXM385 | ETXS770 | ETXS769 |
| ETXM386 | ETXS772 | ETXS771 |
| ETXM387 | ETXS774 | ETXS773 |
| ETXM388 | ETXS776 | ETXS775 |
| ETXM389 | ETXS778 | ETXS777 |
| ETXM390 | ETXS780 | ETXS779 |
| ETXM391 | ETXS782 | ETXS781 |
| ETXM392 | ETXS784 | ETXS783 |
| ETXM393 | ETXS786 | ETXS785 |
| ETXM394 | ETXS788 | ETXS787 |
| ETXM395 | ETXS790 | ETXS789 |
| ETXM396 | ETXS792 | ETXS791 |
| ETXM397 | ETXS794 | ETXS793 |
| ETXM398 | ETXS796 | ETXS795 |
| ETXM399 | ETXS798 | ETXS797 |
| ETXM400 | ETXS800 | ETXS799 |
| ETXM401 | ETXS802 | ETXS801 |
| ETXM402 | ETXS804 | ETXS803 |
| ETXM403 | ETXS806 | ETXS805 |
| ETXM404 | ETXS808 | ETXS807 |
| ETXM405 | ETXS810 | ETXS809 |
| ETXM406 | ETXS812 | ETXS811 |
| ETXM407 | ETXS814 | ETXS813 |
| ETXM408 | ETXS816 | ETXS815 |
| ETXM409 | ETXS818 | ETXS817 |
| ETXM410 | ETXS820 | ETXS819 |
| ETXM411 | ETXS822 | ETXS821 |
| ETXM412 | ETXS824 | ETXS823 |
| ETXM413 | ETXS826 | ETXS825 |
| ETXM414 | ETXS828 | ETXS827 |
| ETXM415 | ETXS830 | ETXS829 |
| ETXM416 | ETXS832 | ETXS831 |
| ETXM417 | ETXS834 | ETXS833 |
| ETXM418 | ETXS836 | ETXS835 |
| ETXM419 | ETXS838 | ETXS837 |
| ETXM420 | ETXS840 | ETXS839 |
| ETXM421 | ETXS842 | ETXS841 |
| ETXM422 | ETXS844 | ETXS843 |
| ETXM423 | ETXS846 | ETXS845 |
| ETXM424 | ETXS848 | ETXS847 |
| ETXM425 | ETXS850 | ETXS849 |
| ETXM426 | ETXS852 | ETXS851 |
| ETXM427 | ETXS854 | ETXS853 |
| ETXM428 | ETXS856 | ETXS855 |
| ETXM429 | ETXS858 | ETXS857 |
| ETXM430 | ETXS860 | ETXS859 |
| ETXM431 | ETXS862 | ETXS861 |
| ETXM432 | ETXS864 | ETXS863 |
| ETXM433 | ETXS866 | ETXS865 |
| ETXM434 | ETXS868 | ETXS867 |
| ETXM435 | ETXS870 | ETXS869 |
| ETXM436 | ETXS872 | ETXS871 |
| ETXM437 | ETXS874 | ETXS873 |
| ETXM438 | ETXS876 | ETXS875 |
| ETXM439 | ETXS878 | ETXS877 |
| ETXM440 | ETXS880 | ETXS879 |
| ETXM441 | ETXS882 | ETXS881 |
| ETXM442 | ETXS884 | ETXS883 |
| ETXM443 | ETXS886 | ETXS885 |
| ETXM444 | ETXS888 | ETXS887 |
| ETXM445 | ETXS890 | ETXS889 |
| ETXM446 | ETXS892 | ETXS891 |
| ETXM447 | ETXS894 | ETXS893 |
| ETXM448 | ETXS896 | ETXS895 |
| ETXM449 | ETXS898 | ETXS897 |
| ETXM450 | ETXS900 | ETXS899 |
| ETXM451 | ETXS902 | ETXS901 |
| ETXM452 | ETXS904 | ETXS903 |
| ETXM453 | ETXS906 | ETXS905 |
| ETXM454 | ETXS908 | ETXS907 |
| ETXM455 | ETXS910 | ETXS909 |
| ETXM456 | ETXS912 | ETXS911 |
| ETXM457 | ETXS914 | ETXS913 |
| ETXM458 | ETXS916 | ETXS915 |
| ETXM459 | ETXS918 | ETXS917 |
| ETXM460 | ETXS920 | ETXS919 |
| ETXM461 | ETXS922 | ETXS921 |
| ETXM462 | ETXS924 | ETXS923 |
| ETXM463 | ETXS926 | ETXS925 |
| ETXM464 | ETXS928 | ETXS927 |
| ETXM465 | ETXS930 | ETXS929 |
| ETXM466 | ETXS932 | ETXS931 |
| ETXM467 | ETXS934 | ETXS933 |
| ETXM468 | ETXS936 | ETXS935 |
| ETXM469 | ETXS938 | ETXS937 |
| ETXM470 | ETXS940 | ETXS939 |
| ETXM471 | ETXS942 | ETXS941 |
| ETXM472 | ETXS944 | ETXS943 |
| ETXM473 | ETXS946 | ETXS945 |
| ETXM474 | ETXS948 | ETXS947 |
| ETXM475 | ETXS950 | ETXS949 |
| ETXM476 | ETXS952 | ETXS951 |
| ETXM477 | ETXS954 | ETXS953 |
| ETXM478 | ETXS956 | ETXS955 |
| ETXM479 | ETXS958 | ETXS957 |
| ETXM480 | ETXS960 | ETXS959 |
| ETXM481 | ETXS962 | ETXS961 |
| ETXM482 | ETXS964 | ETXS963 |
| ETXM483 | ETXS966 | ETXS965 |
| ETXM484 | ETXS968 | ETXS967 |
| ETXM485 | ETXS970 | ETXS969 |
| ETXM486 | ETXS972 | ETXS971 |
| ETXM487 | ETXS974 | ETXS973 |
| ETXM488 | ETXS976 | ETXS975 |
| ETXM489 | ETXS978 | ETXS977 |
| ETXM490 | ETXS980 | ETXS979 |
| ETXM491 | ETXS982 | ETXS981 |
| ETXM492 | ETXS984 | ETXS983 |
| ETXM493 | ETXS986 | ETXS985 |
| ETXM494 | ETXS988 | ETXS987 |
| ETXM495 | ETXS990 | ETXS989 |
| ETXM496 | ETXS992 | ETXS991 |
| ETXM497 | ETXS994 | ETXS993 |
| ETXM498 | ETXS996 | ETXS995 |
| ETXM499 | ETXS998 | ETXS997 |
| ETXM500 | ETXS 1000 | ETXS999 |
| ETXM501 | ETXS 1002 | ETXS1001 |
| ETXM502 | ETXS 1004 | ETXS1003 |
| ETXM503 | ETXS 1006 | ETXS 1005 |
| ETXM504 | ETXS1008 | ETXS 1007 |
| ETXM505 | ETXS1010 | ETXS 1009 |
| ETXM506 | ETXS1012 | ETXS1011 |
| ETXM507 | ETXS1014 | ETXS1013 |
| ETXM508 | ETXS1016 | ETXS1015 |
| ETXM509 | ETXS1018 | ETXS1017 |
| ETXM510 | ETXS 1020 | ETXS1019 |
| ETXM511 | ETXS 1022 | ETXS 1021 |
| ETXM512 | ETXS 1024 | ETXS1023 |
| ETXM513 | ETXS 1026 | ETXS1025 |
| ETXM514 | ETXS1028 | ETXS 1027 |
| ETXM515 | ETXS1030 | ETXS 1029 |
| ETXM1064 | ETXS2128 | ETXS2127 |
| ETXM1072 | ETXS2144 | ETXS2143 |
| ETXM1076 | ETXS2152 | ETXS2151 |
| ETXM1180 | ETXS1032 | ETXS1031 |
| ETXM1181 | ETXS1034 | ETXS1033 |
| ETXM1185 | ETXS1038 | ETXS1037 |
| ETXM1162 | ETXS 1042 | ETXS1041 |
| ETXM1188 | ETXS 1044 | ETXS 1043 |
| ETXM1189 | ETXS 1046 | ETXS 1045 |
| ETXM1192 | ETXS1048 | ETXS1047 |
| ETXM1193 | ETXS 1050 | ETXS1049 |
| ETXM1194 | ETXS1051 | ETXS1033 |
| ETXM1195 | ETXS1052 | ETXS1037 |
| ETXM1196 | ETXS1053 | ETXS1041 |
| ETXM1197 | ETXS1054 | ETXS1045 |
| ETXM1198 | ETXS1055 | ETXS1049 |
| ETXM1199 | ETXS2398 | ETXS2397 |
| ETXM1200 | ETSX2400 | ETXS2397 |
| ETXM1201 | ETXS2402 | ETXS2397 |
| ETXM1202 | ETXS2404 | ETXS2397 |
| ETXM1203 | ETXS2406 | ETXS2397 |
| ETXM1204 | ETXS2408 | ETXS2397 |
| ETXM1205 | ETXS2410 | ETXS2397 |
| ETXM1206 | ETXS2412 | ETXS2397 |
| ETXM1207 | ETXS2414 | ETXS2397 |
| ETXM1208 | ETXS2416 | ETXS2399 |
| ETXM1209 | ETXS2418 | ETXS2399 |
| ETXM1210 | ETXS2420 | ETXS2399 |
| ETXM1211 | ETXS2422 | ETXS2399 |
| ETXM1212 | ETXS2424 | ETXS2399 |
| ETXM1213 | ETXS2426 | ETXS2399 |
| ETXM1214 | ETXS2428 | ETXS2399 |
| ETXM1215 | ETXS2430 | ETXS2399 |
| ETXM1216 | ETXS2432 | ETXS2399 |

For duplexes of Table 5:
ETXM316 - ETXM415, ETXM436 - ETXM515 and ETXM1180 - ETXM1216 have a duplex structure according to Figure 8a with a 2 nucleoside overhang at the 3' end of the antisense;
ETXM416 - ETXM435:have a duplex structure according to Figure 8b, namely a 19mer blunt ended construct.

Definitions as provided in the above Tables:
A - adenosine
C - cytidine
G - guanosine
T - thymidine
m - 2'-O-methyl
f - 2'fluro
s - phosphorothioate bond
o - thermally destabilised nucleoside
ia - inverted abasic nucleoside

### Example 9: Inhibition Screen for ZPI Expression in Human Huh7 Cells

Huh7 cells (human hepatocyte-derived cell line, obtained from JCRB Cell Bank) were maintained in Dulbecco's Modified Eagle Medium (DMEM) supplemented with 10% FBS at 37°C in at atmosphere of 5% CO₂. Cells were transfected with siRNA duplexes targeting ZPI mRNA or a negative control siRNA (siRNA-control; sense strand 5'-UUCUCCGAACGUGUCACGUTT-3' (SEQ ID NO:794), antisense strand 5'-ACGUGACACGUUCGGAGAATT-3' (SEQ ID NO:790)) at a final duplex concentration of 5 nM and 0.1 nM. Transfection was carried out by adding 9.7 µL Opti-MEM (ThermoFisher) plus 0.3 µL Lipofectamine RNAiMAX (ThermoFisher) to 10 µL of each siRNA duplex. The mixture was incubated at room temperature for 15 minutes before being added to 100 µL of complete growth medium containing 20,000 Huh7 cells. Cells were incubated for 24 hours at 37°C/5% CO₂ prior to total RNA purification using a RNeasy 96 Kit (Qiagen). Each duplex was tested by transfection in duplicate wells in two independent experiments.

cDNA synthesis was performed using FastQuant RT (with gDNase) Kit (Tiangen). Real-time quantitative PCR (qPCR) was performed on an ABI Prism 7900HT or ABI QuantStudio 7 with primers specific for human ZPI (Hs01547819_m1) and human GAPDH (Hs02786624_gl) using FastStart Universal Probe Master Kit (Roche).

qPCR was performed in duplicate on cDNA derived from each well and the mean Ct calculated. Relative ZPI expression was calculated from mean Ct values using the comparative Ct (ΔΔCt) method, normalised to GAPDH and relative to untreated cells. Based on the results of primary screen, siRNA duplexes displaying good activity were selected for dose-response follow-up. Results are shown in Figure 9. Sequences of RNAi molecules are depicted in Table 5.

### Example 10: Dose-response for Inhibition of ZPI Expression in Human Huh7 Cells

Huh7 cells (human hepatocyte-derived cell line, obtained from JCRB Cell Bank) were maintained in Dulbecco's Modified Eagle Medium (DMEM) supplemented with 10% FBS at 37°C in at atmosphere of 5% CO₂. Cells were transfected with siRNA duplexes targeting ZPI mRNA or a negative control siRNA (siRNA-control; sense strand 5'-UUCUCCGAACGUGUCACGUTT-3' (SEQ ID NO:794), antisense strand 5'-ACGUGACACGUUCGGAGAATT-3' (SEQ ID NO:790)) using 10x3-fold serial dilutions over a final duplex concentration range of 20 nM to 1 pM. Transfection was carried out by adding 9.7 µL Opti-MEM (ThermoFisher) plus 0.3 µL Lipofectamine RNAiMAX (ThermoFisher) to 10 µL of each siRNA duplex. The mixture was incubated at room temperature for 15 minutes before being added to 100 µL of complete growth medium containing 20,000 Huh7 cells. Cells were incubated for 24 hours at 37°C/5% CO₂ prior to total RNA purification using a RNeasy 96 Kit (Qiagen). Each duplex was tested by transfection in duplicate wells in a single experiment.

cDNA synthesis was performed using FastQuant RT (with gDNase) Kit (Tiangen). Real-time quantitative PCR (qPCR) was performed on an ABI Prism 7900HT or ABI QuantStudio 7 with primers specific for human ZPI (Hs01547819_m1) and human GAPDH (Hs02786624_gl) using FastStart Universal Probe Master Kit (Roche).

qPCR was performed in duplicate on cDNA derived from each well and the mean Ct calculated. Relative ZPI expression was calculated from mean Ct values using the comparative Ct (ΔΔCt) method, normalised to GAPDH and relative to untreated cells. Maximum percent inhibition of ZPI expression and IC50 values were calculated using a four parameter (variable slope) model using GraphPad Prism 9. Results are shown in Figure 9. Sequences of RNAi molecules are depicted in the relevant Tables herein.

**Table 6 - Relative mRNA Expression**

| Duplex ID | Antisense strand ID | SEQ ID NO (AS - mod) | Sense strand ID | SEQ ID NO (SS - mod) | Mean Relative Expression /0.1 nM | Mean Relative Expressio n/5 nM |
|---|---|---|---|---|---|---|
| ETXM316 | ETXS632 | SEQ ID NO: 362 | ETXS631 | SEQ ID NO: 562 | 0.72 | 0.38 |
| ETXM317 | ETXS634 | SEQ ID NO: 363 | ETXS633 | SEQ ID NO: 563 | 1.01 | 0.54 |
| ETXM318 | ETXS636 | SEQ ID NO: 364 | ETXS635 | SEQ ID NO: 564 | 0.97 | 0.56 |
| ETXM319 | ETXS638 | SEQ ID NO: 365 | ETXS637 | SEQ ID NO: 565 | 0.91 | 0.43 |
| ETXM320 | ETXS640 | SEQ ID NO: 366 | ETXS639 | SEQ ID NO: 566 | 0.84 | 0.34 |
| ETXM321 | ETXS642 | SEQ ID NO: 367 | ETXS641 | SEQ ID NO: 567 | 0.58 | 0.27 |
| ETXM322 | ETXS644 | SEQ ID NO: 368 | ETXS643 | SEQ ID NO: 568 | 0.5 | 0.27 |
| ETXM323 | ETXS646 | SEQ ID NO: 369 | ETXS645 | SEQ ID NO: 569 | 0.69 | 0.3 |
| ETXM324 | ETXS648 | SEQ ID NO: 370 | ETXS647 | SEQ ID NO: 570 | 0.87 | 0.46 |
| ETXM325 | ETXS650 | SEQ ID NO: 371 | ETXS649 | SEQ ID NO: 571 | 0.74 | 0.31 |
| ETXM326 | ETXS652 | SEQ ID NO: 372 | ETXS651 | SEQ ID NO: 572 | 0.99 | 0.33 |
| ETXM327 | ETXS654 | SEQ ID NO: 373 | ETXS653 | SEQ ID NO: 573 | 0.79 | 0.37 |
| ETXM328 | ETXS656 | SEQ ID NO: 374 | ETXS655 | SEQ ID NO: 574 | 0.9 | 0.49 |
| ETXM329 | ETXS658 | SEQ ID NO: 375 | ETXS657 | SEQ ID NO: 575 | 1.11 | 0.81 |
| ETXM330 | ETXS660 | SEQ ID NO: 376 | ETXS659 | SEQ ID NO: 576 | 1 | 0.83 |
| ETXM331 | ETXS662 | SEQ ID NO: 377 | ETXS661 | SEQ ID NO: 577 | 1.04 | 0.84 |
| ETXM332 | ETXS664 | SEQ ID NO: 378 | ETXS663 | SEQ ID NO: 578 | 0.42 | 0.22 |
| ETXM333 | ETXS666 | SEQ ID NO: 379 | ETXS665 | SEQ ID NO: 579 | 0.58 | 0.28 |
| ETXM334 | ETXS668 | SEQ ID NO: 380 | ETXS667 | SEQ ID NO: 580 | 0.91 | 0.57 |
| ETXM335 | ETXS670 | SEQ ID NO: 381 | ETXS669 | SEQ ID NO: 581 | 1.04 | 0.74 |
| ETXM336 | ETXS672 | SEQ ID NO: 382 | ETXS671 | SEQ ID NO: 582 | 1.07 | 0.85 |
| ETXM337 | ETXS674 | SEQ ID NO: 383 | ETXS673 | SEQ ID NO: 583 | 0.84 | 0.51 |
| ETXM338 | ETXS676 | SEQ ID NO: 384 | ETXS675 | SEQ ID NO: 584 | 0.38 | 0.23 |
| ETXM339 | ETXS678 | SEQ ID NO: 385 | ETXS677 | SEQ ID NO: 585 | 0.85 | 0.4 |
| ETXM340 | ETXS680 | SEQ ID NO: 386 | ETXS679 | SEQ ID NO: 586 | 0.75 | 0.36 |
| ETXM341 | ETXS682 | SEQ ID NO: 387 | ETXS681 | SEQ ID NO: 587 | 0.55 | 0.22 |
| ETXM342 | ETXS684 | SEQ ID NO: 388 | ETXS683 | SEQ ID NO: 588 | 0.55 | 0.42 |
| ETXM343 | ETXS686 | SEQ ID NO: 389 | ETXS685 | SEQ ID NO: 589 | 0.45 | 0.29 |
| ETXM344 | ETXS688 | SEQ ID NO: 390 | ETXS687 | SEQ ID NO: 590 | 0.98 | 1.01 |
| ETXM345 | ETXS690 | SEQ ID NO: 391 | ETXS689 | SEQ ID NO: 591 | 0.78 | 0.57 |
| ETXM346 | ETXS692 | SEQ ID NO: 392 | ETXS691 | SEQ ID NO: 592 | 1.09 | 1.12 |
| ETXM347 | ETXS694 | SEQ ID NO: 393 | ETXS693 | SEQ ID NO: 593 | 0.93 | 0.45 |
| ETXM348 | ETXS696 | SEQ ID NO: 394 | ETXS695 | SEQ ID NO: 594 | 0.91 | 0.65 |
| ETXM349 | ETXS698 | SEQ ID NO: 395 | ETXS697 | SEQ ID NO: 595 | 0.88 | 0.49 |
| ETXM350 | ETXS700 | SEQ ID NO: 396 | ETXS699 | SEQ ID NO: 596 | 0.87 | 0.75 |
| ETXM351 | ETXS702 | SEQ ID NO: 397 | ETXS701 | SEQ ID NO: 597 | 0.96 | 0.96 |
| ETXM352 | ETXS704 | SEQ ID NO: 398 | ETXS703 | SEQ ID NO: 598 | 0.95 | 1.04 |
| ETXM353 | ETXS706 | SEQ ID NO: 399 | ETXS705 | SEQ ID NO: 599 | 0.71 | 0.5 |
| ETXM354 | ETXS708 | SEQ ID NO: 400 | ETXS707 | SEQ ID NO: 600 | 0.7 | 0.43 |
| ETXM355 | ETXS710 | SEQ ID NO: 401 | ETXS709 | SEQ ID NO: 601 | 0.69 | 0.34 |
| ETXM356 | ETXS712 | SEQ ID NO: 402 | ETXS711 | SEQ ID NO: 602 | 0.92 | 0.71 |
| ETXM357 | ETXS714 | SEQ ID NO: 403 | ETXS713 | SEQ ID NO: 603 | 0.88 | 0.49 |
| ETXM358 | ETXS716 | SEQ ID NO: 404 | ETXS715 | SEQ ID NO: 604 | 0.98 | 0.5 |
| ETXM359 | ETXS718 | SEQ ID NO: 405 | ETXS717 | SEQ ID NO: 605 | 0.66 | 0.33 |
| ETXM360 | ETXS720 | SEQ ID NO: 406 | ETXS719 | SEQ ID NO: 606 | 0.75 | 0.54 |
| ETXM361 | ETXS722 | SEQ ID NO: 407 | ETXS721 | SEQ ID NO: 607 | 0.68 | 0.48 |
| ETXM362 | ETXS724 | SEQ ID NO: 408 | ETXS723 | SEQ ID NO: 608 | 0.95 | 0.9 |
| ETXM363 | ETXS726 | SEQ ID NO: 409 | ETXS725 | SEQ ID NO: 609 | 0.96 | 0.75 |
| ETXM364 | ETXS728 | SEQ ID NO: 410 | ETXS727 | SEQ ID NO: 610 | 0.88 | 0.44 |
| ETXM365 | ETXS730 | SEQ ID NO: 411 | ETXS729 | SEQ ID NO: 611 | 0.82 | 0.49 |
| ETXM366 | ETXS732 | SEQ ID NO: 412 | ETXS731 | SEQ ID NO: 612 | 0.95 | 0.6 |
| ETXM367 | ETXS734 | SEQ ID NO: 413 | ETXS733 | SEQ ID NO: 613 | 0.92 | 0.51 |
| ETXM368 | ETXS736 | SEQ ID NO: 414 | ETXS735 | SEQ ID NO: 614 | 1.02 | 0.84 |
| ETXM369 | ETXS738 | SEQ ID NO: 415 | ETXS737 | SEQ ID NO: 615 | 1.02 | 1 |
| ETXM370 | ETXS740 | SEQ ID NO: 416 | ETXS739 | SEQ ID NO: 616 | 1.37 | 0.96 |
| ETXM371 | ETXS742 | SEQ ID NO: 417 | ETXS741 | SEQ ID NO: 617 | 0.94 | 0.65 |
| ETXM372 | ETXS744 | SEQ ID NO: 418 | ETXS743 | SEQ ID NO: 618 | 0.95 | 0.67 |
| ETXM373 | ETXS746 | SEQ ID NO: 419 | ETXS745 | SEQ ID NO: 619 | 1.05 | 0.96 |
| ETXM374 | ETXS748 | SEQ ID NO: 420 | ETXS747 | SEQ ID NO: 620 | 0.97 | 0.91 |
| ETXM375 | ETXS750 | SEQ ID NO: 421 | ETXS749 | SEQ ID NO: 621 | 0.81 | 0.39 |
| ETXM376 | ETXS752 | SEQ ID NO: 422 | ETXS751 | SEQ ID NO: 622 | 0.97 | 0.76 |
| ETXM377 | ETXS754 | SEQ ID NO: 423 | ETXS753 | SEQ ID NO: 623 | 0.93 | 0.59 |
| ETXM378 | ETXS756 | SEQ ID NO: 424 | ETXS755 | SEQ ID NO: 624 | 0.93 | 0.52 |
| ETXM379 | ETXS758 | SEQ ID NO: 425 | ETXS757 | SEQ ID NO: 625 | 0.96 | 0.81 |
| ETXM380 | ETXS760 | SEQ ID NO: 426 | ETXS759 | SEQ ID NO: 626 | 0.61 | 0.31 |
| ETXM381 | ETXS762 | SEQ ID NO: 427 | ETXS761 | SEQ ID NO: 627 | 0.84 | 0.82 |
| ETXM382 | ETXS764 | SEQ ID NO: 428 | ETXS763 | SEQ ID NO: 628 | 0.8 | 0.47 |
| ETXM383 | ETXS766 | SEQ ID NO: 429 | ETXS765 | SEQ ID NO: 629 | 0.82 | 0.37 |
| ETXM384 | ETXS768 | SEQ ID NO: 430 | ETXS767 | SEQ ID NO: 630 | 0.67 | 0.38 |
| ETXM385 | ETXS770 | SEQ ID NO: 431 | ETXS769 | SEQ ID NO: 631 | 0.9 | 0.87 |
| ETXM386 | ETXS772 | SEQ ID NO: 432 | ETXS771 | SEQ ID NO: 632 | 0.91 | 0.73 |
| ETXM387 | ETXS774 | SEQ ID NO: 433 | ETXS773 | SEQ ID NO: 633 | 0.86 | 0.97 |
| ETXM388 | ETXS776 | SEQ ID NO: 434 | ETXS775 | SEQ ID NO: 634 | 0.96 | 0.7 |
| ETXM389 | ETXS778 | SEQ ID NO: 435 | ETXS777 | SEQ ID NO: 635 | 0.95 | 0.68 |
| ETXM390 | ETXS780 | SEQ ID NO: 436 | ETXS779 | SEQ ID NO: 636 | 0.87 | 0.51 |
| ETXM391 | ETXS782 | SEQ ID NO: 437 | ETXS781 | SEQ ID NO: 637 | 0.76 | 0.35 |
| ETXM392 | ETXS784 | SEQ ID NO: 438 | ETXS783 | SEQ ID NO: 638 | 0.99 | 0.76 |
| ETXM393 | ETXS786 | SEQ ID NO: 439 | ETXS785 | SEQ ID NO: 639 | 0.94 | 1.06 |
| ETXM394 | ETXS788 | SEQ ID NO: 440 | ETXS787 | SEQ ID NO: 640 | 0.85 | 0.8 |
| ETXM395 | ETXS790 | SEQ ID NO: 441 | ETXS789 | SEQ ID NO: 641 | 0.95 | 0.53 |
| ETXM396 | ETXS792 | SEQ ID NO: 442 | ETXS791 | SEQ ID NO: 642 | 0.62 | 0.27 |
| ETXM397 | ETXS794 | SEQ ID NO: 443 | ETXS793 | SEQ ID NO: 643 | 0.96 | 0.64 |
| ETXM398 | ETXS796 | SEQ ID NO: 444 | ETXS795 | SEQ ID NO: 644 | 0.93 | 0.55 |
| ETXM399 | ETXS798 | SEQ ID NO: 445 | ETXS797 | SEQ ID NO: 645 | 0.94 | 0.66 |
| ETXM400 | ETXS800 | SEQ ID NO: 446 | ETXS799 | SEQ ID NO: 646 | 0.77 | 0.57 |
| ETXM401 | ETXS802 | SEQ ID NO: 447 | ETXS801 | SEQ ID NO: 647 | 0.69 | 0.25 |
| ETXM402 | ETXS804 | SEQ ID NO: 448 | ETXS803 | SEQ ID NO: 648 | 1.05 | 0.95 |
| ETXM403 | ETXS806 | SEQ ID NO: 449 | ETXS805 | SEQ ID NO: 649 | 0.86 | 0.5 |
| ETXM404 | ETXS808 | SEQ ID NO: 450 | ETXS807 | SEQ ID NO: 650 | 0.83 | 0.35 |
| ETXM405 | ETXS810 | SEQ ID NO: 451 | ETXS809 | SEQ ID NO: 651 | 0.97 | 0.73 |
| ETXM406 | ETXS812 | SEQ ID NO: 452 | ETXS811 | SEQ ID NO: 652 | 0.84 | 0.33 |
| ETXM407 | ETXS814 | SEQ ID NO: 453 | ETXS813 | SEQ ID NO: 653 | 0.77 | 0.51 |
| ETXM408 | ETXS816 | SEQ ID NO: 454 | ETXS815 | SEQ ID NO: 654 | 0.89 | 0.51 |
| ETXM409 | ETXS818 | SEQ ID NO: 455 | ETXS817 | SEQ ID NO: 655 | 1 | 0.59 |
| ETXM410 | ETXS820 | SEQ ID NO: 456 | ETXS819 | SEQ ID NO: 656 | 0.98 | 0.71 |
| ETXM411 | ETXS822 | SEQ ID NO: 457 | ETXS821 | SEQ ID NO: 657 | 0.77 | 0.36 |
| ETXM412 | ETXS824 | SEQ ID NO: 458 | ETXS823 | SEQ ID NO: 658 | 0.97 | 0.42 |
| ETXM413 | ETXS826 | SEQ ID NO: 459 | ETXS825 | SEQ ID NO: 659 | 1 | 0.77 |
| ETXM414 | ETXS828 | SEQ ID NO: 460 | ETXS827 | SEQ ID NO: 660 | 0.98 | 0.79 |
| ETXM415 | ETXS830 | SEQ ID NO: 461 | ETXS829 | SEQ ID NO: 661 | 0.96 | 0.62 |
| ETXM416 | ETXS832 | SEQ ID NO: 462 | ETXS831 | SEQ ID NO: 662 | 0.96 | 0.52 |
| ETXM417 | ETXS834 | SEQ ID NO: 463 | ETXS833 | SEQ ID NO: 663 | 1.03 | 0.75 |
| ETXM418 | ETXS836 | SEQ ID NO: 464 | ETXS835 | SEQ ID NO: 664 | 1.11 | 0.74 |
| ETXM419 | ETXS838 | SEQ ID NO: 465 | ETXS837 | SEQ ID NO: 665 | 0.99 | 0.6 |
| ETXM420 | ETXS840 | SEQ ID NO: 466 | ETXS839 | SEQ ID NO: 666 | 0.91 | 0.54 |
| ETXM421 | ETXS842 | SEQ ID NO: 467 | ETXS841 | SEQ ID NO: 667 | 0.8 | 0.27 |
| ETXM422 | ETXS844 | SEQ ID NO: 468 | ETXS843 | SEQ ID NO: 668 | 0.82 | 0.33 |
| ETXM423 | ETXS846 | SEQ ID NO: 469 | ETXS845 | SEQ ID NO: 669 | 0.91 | 0.36 |
| ETXM424 | ETXS848 | SEQ ID NO: 470 | ETXS847 | SEQ ID NO: 670 | 0.98 | 0.62 |
| ETXM425 | ETXS850 | SEQ ID NO: 471 | ETXS849 | SEQ ID NO: 671 | 0.81 | 0.32 |
| ETXM426 | ETXS852 | SEQ ID NO: 472 | ETXS851 | SEQ ID NO: 672 | 0.91 | 0.49 |
| ETXM427 | ETXS854 | SEQ ID NO: 473 | ETXS853 | SEQ ID NO: 673 | 1.02 | 0.46 |
| ETXM428 | ETXS856 | SEQ ID NO: 474 | ETXS855 | SEQ ID NO: 674 | 1.09 | 0.94 |
| ETXM429 | ETXS858 | SEQ ID NO: 475 | ETXS857 | SEQ ID NO: 675 | 1.14 | 0.7 |
| ETXM430 | ETXS860 | SEQ ID NO: 476 | ETXS859 | SEQ ID NO: 676 | 0.92 | 0.71 |
| ETXM431 | ETXS862 | SEQ ID NO: 477 | ETXS861 | SEQ ID NO: 677 | 1.18 | 0.9 |
| ETXM432 | ETXS864 | SEQ ID NO: 478 | ETXS863 | SEQ ID NO: 678 | 0.7 | 0.26 |
| ETXM433 | ETXS866 | SEQ ID NO: 479 | ETXS865 | SEQ ID NO: 679 | 1.07 | 0.31 |
| ETXM434 | ETXS868 | SEQ ID NO: 480 | ETXS867 | SEQ ID NO: 680 | 1.11 | 0.63 |
| ETXM435 | ETXS870 | SEQ ID NO: 481 | ETXS869 | SEQ ID NO: 681 | 1 | 0.8 |
| ETXM436 | ETXS872 | SEQ ID NO: 482 | ETXS871 | SEQ ID NO: 682 | 0.72 | 0.44 |
| ETXM437 | ETXS874 | SEQ ID NO: 483 | ETXS873 | SEQ ID NO: 683 | 0.98 | 0.47 |
| ETXM438 | ETXS876 | SEQ ID NO: 484 | ETXS875 | SEQ ID NO: 684 | 1.05 | 0.75 |
| ETXM439 | ETXS878 | SEQ ID NO: 485 | ETXS877 | SEQ ID NO: 685 | 0.91 | 0.49 |
| ETXM440 | ETXS880 | SEQ ID NO: 486 | ETXS879 | SEQ ID NO: 686 | 0.91 | 0.46 |
| ETXM441 | ETXS882 | SEQ ID NO: 487 | ETXS881 | SEQ ID NO: 687 | 0.74 | 0.36 |
| ETXM442 | ETXS884 | SEQ ID NO: 488 | ETXS883 | SEQ ID NO: 688 | 0.62 | 0.36 |
| ETXM443 | ETXS886 | SEQ ID NO: 489 | ETXS885 | SEQ ID NO: 689 | 0.73 | 0.3 |
| ETXM444 | ETXS888 | SEQ ID NO: 490 | ETXS887 | SEQ ID NO: 690 | 1 | 0.59 |
| ETXM445 | ETXS890 | SEQ ID NO: 491 | ETXS889 | SEQ ID NO: 691 | 0.71 | 0.37 |
| ETXM446 | ETXS892 | SEQ ID NO: 492 | ETXS891 | SEQ ID NO: 692 | 0.73 | 0.27 |
| ETXM447 | ETXS894 | SEQ ID NO: 493 | ETXS893 | SEQ ID NO: 693 | 0.81 | 0.39 |
| ETXM448 | ETXS896 | SEQ ID NO: 494 | ETXS895 | SEQ ID NO: 694 | 0.81 | 0.61 |
| ETXM449 | ETXS898 | SEQ ID NO: 495 | ETXS897 | SEQ ID NO: 695 | 0.91 | 0.8 |
| ETXM450 | ETXS900 | SEQ ID NO: 496 | ETXS899 | SEQ ID NO: 696 | 0.97 | 0.52 |
| ETXM451 | ETXS902 | SEQ ID NO: 497 | ETXS901 | SEQ ID NO: 697 | 0.96 | 0.61 |
| ETXM452 | ETXS904 | SEQ ID NO: 498 | ETXS903 | SEQ ID NO: 698 | 0.4 | 0.24 |
| ETXM453 | ETXS906 | SEQ ID NO: 499 | ETXS905 | SEQ ID NO: 699 | 0.62 | 0.3 |
| ETXM454 | ETXS908 | SEQ ID NO: 500 | ETXS907 | SEQ ID NO: 700 | 0.81 | 0.46 |
| ETXM455 | ETXS910 | SEQ ID NO: 501 | ETXS909 | SEQ ID NO: 701 | 0.94 | 0.68 |
| ETXM456 | ETXS912 | SEQ ID NO: 502 | ETXS911 | SEQ ID NO: 702 | 0.75 | 0.36 |
| ETXM457 | ETXS914 | SEQ ID NO: 503 | ETXS913 | SEQ ID NO: 703 | 0.98 | 0.52 |
| ETXM458 | ETXS916 | SEQ ID NO: 504 | ETXS915 | SEQ ID NO: 704 | 1 | 0.61 |
| ETXM459 | ETXS918 | SEQ ID NO: 505 | ETXS917 | SEQ ID NO: 705 | 0.92 | 0.44 |
| ETXM460 | ETXS920 | SEQ ID NO: 506 | ETXS919 | SEQ ID NO: 706 | 0.86 | 0.4 |
| ETXM461 | ETXS922 | SEQ ID NO: 507 | ETXS921 | SEQ ID NO: 707 | 0.84 | 0.27 |
| ETXM462 | ETXS924 | SEQ ID NO: 508 | ETXS923 | SEQ ID NO: 708 | 0.72 | 0.33 |
| ETXM463 | ETXS926 | SEQ ID NO: 509 | ETXS925 | SEQ ID NO: 709 | 0.76 | 0.35 |
| ETXM464 | ETXS928 | SEQ ID NO: 510 | ETXS927 | SEQ ID NO: 710 | 0.95 | 0.55 |
| ETXM465 | ETXS930 | SEQ ID NO: 511 | ETXS929 | SEQ ID NO: 711 | 0.77 | 0.36 |
| ETXM466 | ETXS932 | SEQ ID NO: 512 | ETXS931 | SEQ ID NO: 712 | 0.84 | 0.33 |
| ETXM467 | ETXS934 | SEQ ID NO: 513 | ETXS933 | SEQ ID NO: 713 | 0.91 | 0.39 |
| ETXM468 | ETXS936 | SEQ ID NO: 514 | ETXS935 | SEQ ID NO: 714 | 1.14 | 0.8 |
| ETXM469 | ETXS938 | SEQ ID NO: 515 | ETXS937 | SEQ ID NO: 715 | 1.17 | 0.67 |
| ETXM470 | ETXS940 | SEQ ID NO: 516 | ETXS939 | SEQ ID NO: 716 | 1.12 | 0.79 |
| ETXM471 | ETXS942 | SEQ ID NO: 517 | ETXS941 | SEQ ID NO: 717 | 1.16 | 0.86 |
| ETXM472 | ETXS944 | SEQ ID NO: 518 | ETXS943 | SEQ ID NO: 718 | 0.49 | 0.25 |
| ETXM473 | ETXS946 | SEQ ID NO: 519 | ETXS945 | SEQ ID NO: 719 | 0.91 | 0.34 |
| ETXM474 | ETXS948 | SEQ ID NO: 520 | ETXS947 | SEQ ID NO: 720 | 1.12 | 0.68 |
| ETXM475 | ETXS950 | SEQ ID NO: 521 | ETXS949 | SEQ ID NO: 721 | 1.25 | 0.84 |
| ETXM476 | ETXS952 | SEQ ID NO: 522 | ETXS951 | SEQ ID NO: 722 | 0.87 | 0.42 |
| ETXM477 | ETXS954 | SEQ ID NO: 523 | ETXS953 | SEQ ID NO: 723 | 1.12 | 0.52 |
| ETXM478 | ETXS956 | SEQ ID NO: 524 | ETXS955 | SEQ ID NO: 724 | 1.03 | 0.62 |
| ETXM479 | ETXS958 | SEQ ID NO: 525 | ETXS957 | SEQ ID NO: 725 | 1.13 | 0.51 |
| ETXM480 | ETXS960 | SEQ ID NO: 526 | ETXS959 | SEQ ID NO: 726 | 0.93 | 0.56 |
| ETXM481 | ETXS962 | SEQ ID NO: 527 | ETXS961 | SEQ ID NO: 727 | 0.89 | 0.36 |
| ETXM482 | ETXS964 | SEQ ID NO: 528 | ETXS963 | SEQ ID NO: 728 | 0.68 | 0.46 |
| ETXM483 | ETXS966 | SEQ ID NO: 529 | ETXS965 | SEQ ID NO: 729 | 0.82 | 0.5 |
| ETXM484 | ETXS968 | SEQ ID NO: 530 | ETXS967 | SEQ ID NO: 730 | 1.06 | 0.74 |
| ETXM485 | ETXS970 | SEQ ID NO: 531 | ETXS969 | SEQ ID NO: 731 | 0.91 | 0.41 |
| ETXM486 | ETXS972 | SEQ ID NO: 532 | ETXS971 | SEQ ID NO: 732 | 0.68 | 0.23 |
| ETXM487 | ETXS974 | SEQ ID NO: 533 | ETXS973 | SEQ ID NO: 733 | 0.8 | 0.31 |
| ETXM488 | ETXS976 | SEQ ID NO: 534 | ETXS975 | SEQ ID NO: 734 | 0.89 | 0.64 |
| ETXM489 | ETXS978 | SEQ ID NO: 535 | ETXS977 | SEQ ID NO: 735 | 0.89 | 0.67 |
| ETXM490 | ETXS980 | SEQ ID NO: 536 | ETXS979 | SEQ ID NO: 736 | 0.91 | 0.56 |
| ETXM491 | ETXS982 | SEQ ID NO: 537 | ETXS981 | SEQ ID NO: 737 | 1.06 | 0.75 |
| ETXM492 | ETXS984 | SEQ ID NO: 538 | ETXS983 | SEQ ID NO: 738 | 0.43 | 0.22 |
| ETXM493 | ETXS986 | SEQ ID NO: 539 | ETXS985 | SEQ ID NO: 739 | 0.59 | 0.33 |
| ETXM494 | ETXS988 | SEQ ID NO: 540 | ETXS987 | SEQ ID NO: 740 | 0.93 | 0.59 |
| ETXM495 | ETXS990 | SEQ ID NO: 541 | ETXS989 | SEQ ID NO: 741 | 1.08 | 0.74 |
| ETXM496 | ETXS992 | SEQ ID NO: 542 | ETXS991 | SEQ ID NO: 742 | 0.73 | 0.42 |
| ETXM497 | ETXS994 | SEQ ID NO: 543 | ETXS993 | SEQ ID NO: 743 | 1.01 | 0.59 |
| ETXM498 | ETXS996 | SEQ ID NO: 544 | ETXS995 | SEQ ID NO: 744 | 0.95 | 0.59 |
| ETXM499 | ETXS998 | SEQ ID NO: 545 | ETXS997 | SEQ ID NO: 745 | 1.08 | 0.53 |
| ETXM500 | ETXS 1000 | SEQ ID NO: 546 | ETXS999 | SEQ ID NO: 746 | 0.87 | 0.46 |
| ETXM501 | ETXS1002 | SEQ ID NO: 547 | ETXS1001 | SEQ ID NO: 747 | 0.6 | 0.27 |
| ETXM502 | ETXS 1004 | SEQ ID NO: 548 | ETXS1003 | SEQ ID NO: 748 | 0.6 | 0.28 |
| ETXM503 | ETXS 1006 | SEQ ID NO: 549 | ETXS1005 | SEQ ID NO: 749 | 0.73 | 0.36 |
| ETXM504 | ETXS1008 | SEQ ID NO: 550 | ETXS1007 | SEQ ID NO: 750 | 0.9 | 0.68 |
| ETXM505 | ETXS1010 | SEQ ID NO: 551 | ETXS1009 | SEQ ID NO: 751 | 0.72 | 0.36 |
| ETXM506 | ETXS1012 | SEQ ID NO: 552 | ETXS1011 | SEQ ID NO: 752 | 0.62 | 0.25 |
| ETXM507 | ETXS1014 | SEQ ID NO: 553 | ETXS1013 | SEQ ID NO: 753 | 1.21 | 0.33 |
| ETXM508 | ETXS1016 | SEQ ID NO: 554 | ETXS1015 | SEQ ID NO: 754 | 1.08 | 0.83 |
| ETXM509 | ETXS1018 | SEQ ID NO: 555 | ETXS1017 | SEQ ID NO: 755 | 1.09 | 0.85 |
| ETXM510 | ETXS 1020 | SEQ ID NO: 556 | ETXS1019 | SEQ ID NO: 756 | 0.98 | 0.62 |
| ETXM511 | ETXS 1022 | SEQ ID NO: 557 | ETXS1021 | SEQ ID NO: 757 | 0.81 | 0.87 |
| ETXM512 | ETXS 1024 | SEQ ID NO: 558 | ETXS1023 | SEQ ID NO: 758 | 0.34 | 0.17 |
| ETXM513 | ETXS 1026 | SEQ ID NO: 559 | ETXS1025 | SEQ ID NO: 759 | 0.49 | 0.22 |
| ETXM514 | ETXS1028 | SEQ ID NO: 560 | ETXS1027 | SEQ ID NO: 760 | 0.84 | 0.56 |
| ETXM515 | ETXS1030 | SEQ ID NO: 561 | ETXS1029 | SEQ ID NO: 761 | 0.93 | 0.74 |

**Table 7 - Dose-Response Data Table**

| Duplex ID | Antisense strand ID | SEQ ID NO (AS - mod) | Sense strand ID | SEQ ID NO (SS - mod) | IC₅₀ [pM] | % Max Inhibition |
|---|---|---|---|---|---|---|
| ETXM320 | ETXS640 | SEQ ID NO: 366 | ETXS639 | SEQ ID NO: 566 | 556 | 77 |
| ETXM321 | ETXS642 | SEQ ID NO: 367 | ETXS641 | SEQ ID NO: 567 | 172 | 82 |
| ETXM322 | ETXS644 | SEQ ID NO: 368 | ETXS643 | SEQ ID NO: 568 | 101 | 79 |
| ETXM323 | ETXS646 | SEQ ID NO: 369 | ETXS645 | SEQ ID NO: 569 | 268 | 79 |
| ETXM325 | ETXS650 | SEQ ID NO: 371 | ETXS649 | SEQ ID NO: 571 | 274 | 79 |
| ETXM326 | ETXS652 | SEQ ID NO: 372 | ETXS651 | SEQ ID NO: 572 | 607 | 86 |
| ETXM332 | ETXS664 | SEQ ID NO: 378 | ETXS663 | SEQ ID NO: 578 | 81 | 85 |
| ETXM333 | ETXS666 | SEQ ID NO: 379 | ETXS665 | SEQ ID NO: 579 | 130 | 83 |
| ETXM338 | ETXS676 | SEQ ID NO: 384 | ETXS675 | SEQ ID NO: 584 | 33 | 77 |
| ETXM339 | ETXS678 | SEQ ID NO: 385 | ETXS677 | SEQ ID NO: 585 | 1180 | 78 |
| ETXM341 | ETXS682 | SEQ ID NO: 387 | ETXS681 | SEQ ID NO: 587 | 186 | 83 |
| ETXM342 | ETXS684 | SEQ ID NO: 388 | ETXS683 | SEQ ID NO: 588 | 71 | 63 |
| ETXM343 | ETXS686 | SEQ ID NO: 389 | ETXS685 | SEQ ID NO: 589 | 60 | 79 |
| ETXM420 | ETXS840 | SEQ ID NO: 466 | ETXS839 | SEQ ID NO: 666 | 2450 | 77 |
| ETXM421 | ETXS842 | SEQ ID NO: 467 | ETXS841 | SEQ ID NO: 667 | 305 | 81 |
| ETXM422 | ETXS844 | SEQ ID NO: 468 | ETXS843 | SEQ ID NO: 668 | 502 | 80 |
| ETXM423 | ETXS846 | SEQ ID NO: 469 | ETXS845 | SEQ ID NO: 669 | 1660 | 80 |
| ETXM425 | ETXS850 | SEQ ID NO: 471 | ETXS849 | SEQ ID NO: 671 | 1050 | 89 |
| ETXM426 | ETXS852 | SEQ ID NO: 472 | ETXS851 | SEQ ID NO: 672 | 1570 | 74 |
| ETXM432 | ETXS864 | SEQ ID NO: 478 | ETXS863 | SEQ ID NO: 678 | 239 | 85 |
| ETXM433 | ETXS866 | SEQ ID NO: 479 | ETXS865 | SEQ ID NO: 679 | 703 | 86 |
| ETXM452 | ETXS904 | SEQ ID NO: 498 | ETXS903 | SEQ ID NO: 698 | 82 | 78 |
| ETXM472 | ETXS944 | SEQ ID NO: 518 | ETXS943 | SEQ ID NO: 718 | 130 | 77 |
| ETXM492 | ETXS984 | SEQ ID NO: 538 | ETXS983 | SEQ ID NO: 738 | 47 | 84 |
| ETXM500 | ETXS1000 | SEQ ID NO: 546 | ETXS999 | SEQ ID NO: 746 | 607 | 70 |
| ETXM501 | ETXS1002 | SEQ ID NO: 547 | ETXS1001 | SEQ ID NO: 747 | 190 | 74 |
| ETXM502 | ETXS1004 | SEQ ID NO: 548 | ETXS1003 | SEQ ID NO: 748 | 132 | 76 |
| ETXM503 | ETXS1006 | SEQ ID NO: 549 | ETXS1005 | SEQ ID NO: 749 | 322 | 75 |
| ETXM505 | ETXS1010 | SEQ ID NO: 551 | ETXS1009 | SEQ ID NO: 751 | 199 | 72 |
| ETXM506 | ETXS1012 | SEQ ID NO: 552 | ETXS1011 | SEQ ID NO: 752 | 162 | 82 |
| ETXM512 | ETXS1024 | SEQ ID NO: 558 | ETXS1023 | SEQ ID NO: 758 | 76 | 84 |
| ETXM513 | ETXS1026 | SEQ ID NO: 559 | ETXS1025 | SEQ ID NO: 759 | 146 | 77 |

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope of the disclosure and are intended to fall within the scope of the present disclosure.

In case of ambiguity between the sequences in this specification and the sequences in the attached sequence listing, the sequences provided herein are considered to be the correct sequences.

### Example 11: Murinisation of ZPI siRNA Sequences

The 5 siRNA sequences identified in a Huh7 cell line transfection screen as being inhibitors of ZPI expression are shown in Table 8.

**Table 8**

| **Sequence ID** | **Sense (5' to 3')** | **Antisense (5' to 3')** |
|---|---|---|
| ETXM342 | AAAGAUCUCCAUGAGGCACGA (SEQ ID NO:268) | UCGUGCCUCAUGGAGAUCUUUCG (SEQ ID NO: 148) |
| ETXM339 | UACCAAGGAAAUGCCACCAUG (SEQ ID NO:265) | CAUGGUGGCAUUUCCUUGGUAGG (SEQ ID NO: 145) |
| ETXM338 | GCCUUCAUCCACAAGGAUUUU (SEQ ID NO:264) | AAAAUCCUUGUGGAUGAAGGCAA (SEQ ID NO: 144) |
| ETXM359 | CUCCACCUUUGACAAGAAUUU (SEQ ID NO:285) | AAAUUCUUGUCAAAGGUGGAGGC (SEQ ID NO:165) |
| ETXM396 | GGAGUUUUGCCUUCAUCCACA (SEQ ID NO:322) | UGUGGAUGAAGGCAAAACUCCCC (SEQ ID NO:202) |

ETXM338, ETXM359 and ETXM396 do not cross react with mouse ZPI sequences and will not be used in mouse PoC studies. These sequences were murinised so that they are homologous with mouse ZPI sequence for use in mouse studies. The siRNA sequences were aligned with mouse ZPI transcripts NM_144834.4 and NM_001301404.1 and nucleotides that mismatched the mouse sequence changed to match the mouse sequence.

ETXM338 was changed to a C at position 18 of the sense strand and a G at position 4 of the antisense strand to give ETXM1064.

ETXM359 was changed to a G at position 19 of the sense strand, a C at position 3 of the antisense strand and a C at position 23 of the antisense strand to give ETXM1072.

ETXM396 was changed to a U at position 2 of the sense strand, a C at position 5 of the sense strand, a G at position 17 of the antisense strand and an A at position 20 of the antisense strand to give ETXM1076.

The murinised sequences were checked by BLAST search to ensure that they did not cross-react with other mouse transcripts.

The murinised siRNA sequences are shown in Table 9. Positions changed to mouse sequence are underlined.

**Table 9**

| **Sequence ID** | **Sense (5' to 3')** | **Antisense (5' to 3')** |
|---|---|---|
| ETXM1064 | GCCUUCAUCCACAAGGA**C**UUU (SEQ ID NO:791) | AAA**G**UCCUUGUGGAUGAAGGCAA (SEQ ID NO:787) |
| ETXM1072 | CUCCACCUUUGACAAGAA**G**UU (SEQ ID NO:792) | AA**C**UUCUUGUCAAAGGUGGAGG**C** (SEQ ID NO:788) |
| ETXM1076 | G**U**AG**C**UUUGCCUUCAUCCACA | UGUGGAUGAAGGCAAA**G**CU**A**CCC |
| | (SEQ ID NO:793) | (SEQ ID NO:789) |

### Example 12: In vivo Efficacy data in a haemophilia mouse model

Haemarthrosis is defined as a bleeding into joint spaces that is a common feature of haemophilia. A long-term consequence of repeated haemarthrosis is the development of permanent joint disease known as haemophilic arthropathy. Around 50% of patients with haemophilia develop severe arthropathy resulting in chronic joint pain, reduced range of motion and function, and reduced quality of life. Haemophilic arthropathy is characterised by synovial hyperplasia, chronic inflammation, fibrosis, and haemosiderosis.

The model of haemarthrosis used was the induction of a knee bleed in haem A mice and the appropriate background wild-type (WT) strain, with progression of the bleed into the joint monitored up to 10 days post-injury. Identical studies were conducted twice to increase number of animals for analysis.

The objective of these repeat studies was to demonstrate that prophylactic administration of ETXM1184 could reduce haemarthrosis in Haemophilia A mice after a joint bleed injury. Fitusiran (siRNA targeting antithrombin (AT)) was used as a reference. Advate (recombinant FVIII) was used as positive control.

For that, a total of 20 Haem A mice (Bi, L., Lawler, A., Antonarakis, S. et al. Targeted disruption of the mouse factor VIII gene produces a model of haemophilia A. Nat Genet 10, 119-121 (1995). https://doi.org/10.1038/ng0595-119) and 10 WT mice were used in this study:

| **Group** | **Mouse strain** | **Treatment** | **Dosing time (pre-injury; days)** | **Termination time (post injury; days)** | **N** |
|---|---|---|---|---|---|
| 1 | WT | Vehicle | -8 | 10 | 10 |
| 2 | Haem A | Vehicle | -8 | 10 | 10 |
| 3 | Haem A | Fitusiran - 3 mg/kg s.c | -8 | 10 | 10 |
| 4 | Haem A | Fitusiran - 10 mg/kg s.c | -8 | 10 | 10 |
| 5 | Haem A | ETXM1184 - 3 mg/kg s.c. | -8 | 10 | 10 |
| 6 | Haem A | ETXM1184 - 10 mg/kg s.c. | -8 | 10 | 10 |
| 7 | Haem A | FVIII (Advate) - 300 IU/kg i.v | 15 mins | 10 | 10 |

8 days prior to induction of knee bleed, mice were injected subcutaneously (s.c.) with the GalNAc-siRNA construct ETXM1184, fitusiran or a vehicle (0.9% saline) at a dose volume of 5 ml/kg. Advate was injected intravenously 15 minutes prior to joint bleed induction.

To induce knee bleed, mice were weighed and anaesthetised using isoflurane inhaled anaesthetic. Both legs were shaved to expose the knee joint. Mice were injected s.c. with buprenorphine at 10 ml/kg for analgesia and the diameter of both knees was measured with electronic calipers. Subsequently, both knees were wiped with 70% ethanol.

A 30 G sterile hypodermic needle was inserted into the infrapatellar ligament of one knee. The injected knee was randomised between left and right, and the injected side was recorded. Mice were removed from anaesthetic and allowed to recover in a warmed cage before being returned to the home cage.

Mice were monitored regularly for the first 6 hours and were injected subcutaneously with buprenorphine at 10 ml/kg for analgesia at 6 hours post injury. The visual bleeding score (VBS) of the injured knee was assessed at 72 hours and 10 days post-injury.

All mice were carefully inspected daily for clinical signs of excessive blood loss. Mice showing clinical signs of excessive blood loss, piloerection, withdrawing from cage mates or grimacing were killed for welfare reasons.

Mice were taken off study at 10 days post-injury.

A citrated blood sample was taken by cardiac puncture, under isoflurane anaesthesia, plasma prepared and aliquots frozen on dry ice before storing at -80°C. For that, blood was collected into 3.8% Sodium Citrate at a ratio of 1 to 9 followed by centrifugation at 7000 x g for 10 minutes at 4°C. In detail, the following steps were performed:
1. Collect blood by cardiac puncture.
2. Flush the syringe and needle with sodium citrate solution (3.8%), leaving solution in the hub of the syringe (~30 µl).
3. Following blood collection, expel sample into a 1.5 ml microcentrifuge tube and ensure sufficient sodium citrate solution (3.8%) is added to achieve a 1:9 ratio of sodium citrate:blood. Add the sodium citrate solution to the side of the tube, not directly to the sample. Mix by inverting 4-6 times. If not centrifuging sample immediately, keep in a fridge if available or alternatively on a wrapped ice block and continue to invert the collection tube regularly.
4. Centrifuge the samples as soon as possible at a spin speed of 7000 x g for 10 minutes at 4°C.
5. Remove all plasma from the sample and place into a fresh microcentrifuge tube.
6. Aliquot the plasma into pre-labelled tubes (Thermo Scientific; 10775974) as follows:
   ∘ 30 µl for potential TGA assay
   ∘ 100 µl for potential APTT assay
   ∘ All remaining for potential target protein abundance analysis.
7. Place all aliquots on wet/dry ice immediately.
8. Transport samples on wet/dry ice.
9. Transfer samples to -20°C/-80°C freezer to be stored until use.

The liver was removed and up to 3 portions of each lobe were placed in RNA later and kept at 4°C for 24 to 72 hours. Tissue was then blotted dry, weighed and stored at -80°C. In detail, the following steps were performed:
1. Immediately after the cardiac puncture, kill the mouse by cervical dislocation.
2. Make an incision into the abdominal wall and remove the liver as quickly as possible.
3. Place the liver on a petri dish on wet ice, to minimise sample degradation.
4. Cut 3 x ~50 mg pieces of liver from each of the following lobes: left lateral lobe, medial lobe, right lateral lobe and caudate lobe. Place these liver pieces immediately into pre-labelled tubes (1.5 ml microcentrifuge tubes) containing 500 µl RNAlater, and place the collection tube on wet ice.
   a. Transport on wet ice and transfer to storage at 4°C.
   b. After a period of 24-72 hours, blot the liver samples and weigh. Record the weights on the terminal sheet.
   c. Transfer to -80°C for long-term storage.
5. Collect any spare liver and place into separate pre-labelled collection tubes (2 ml microcentrifuge tubes).
   a. Freeze on dry ice for potential future analyses.
   b. Transport samples on dry ice.
   c. Transfer to -80°C for long-term storage.
6. Clean all dissection tools between animals to prevent any cross contamination.

The skin was removed from the legs and the knee joint measured. Legs were subsequently placed in 10% formalin before decalcification and slide preparation. In detail, the following steps were performed:
1. Following the removal of the liver, measure and record the diameter of both the injured and uninjured knee.
2. Remove the skin from both knees. Carry out a visual bleeding score and measure knee joints.
3. Dissect the legs from the top of the femur to the ankle joint and remove some excess muscle, being careful not to cause any damage to the knee and associated structures. Place the knees in pre-labelled tubes (7 ml bijou tubes) containing 10% neutral buffered formalin to be processed for histological analysis.

Both at day 3 and day 10 after induction of knee bleed, Haem A mice that received the GalNAc-siRNA construct ETXM1184 showed a significantly reduced visual bleeding score in comparison to Haem A mice that received the vehicle (0.9% saline) (see Fig. 12A&B). Furthermore, the knee diameter of mice that received the GalNAc-siRNA construct ETXM1184 recovered faster following the induction of knee bleed compared to mice that received the vehicle (Fig 13A). This observation was confirmed by comparing the differences between the diameter of the injured and non-injured skinned knee diameter (Fig. 13B).

Analysis of the Haem A mice 10 days post injury further revealed less severe bone marrow hyperplasia (Fig. 14A), less severe osteoarthritis (Fig. 14B), less severe chondrocyte degeneration/necrosis (Fig. 14C), less severe haemorrhage (Fig. 14D), less severe haemosiderin deposition (Fig. 14E), less severe haematoma (Fig. 14F), less severe osteoclastogenic bone resorption (Fig. 14G), less severe osteolysis (Fig. 14H), less severe periostitis (Fig. 14I), less severe sub-chondral bone sclerosis (Fig. 14J), less severe tendon degeneration (Fig. 14K), less severe tendonitis (Fig. 14L) and less severe tenosynovitis (Fig. 14M) in mice that received the GalNAc-siRNA construct ETXM1184 in comparison to Haem A mice that received the vehicle (0.9% saline).

Comparative data between ETXM1184 and fitusiran is provided in Figs. 15-20.

### Example 13: Dose-response for Inhibition of ZPI Expression in Human Huh7 Cells

Huh7 cells (human hepatocyte-derived cell line, obtained from JCRB Cell Bank) were maintained in Dulbecco's Modified Eagle Medium (DMEM) supplemented with 10% FBS at 37°C in at atmosphere of 5% CO₂. Cells were transfected with siRNA duplexes designed against the target or a negative control siRNA at 0.1nM and InM. Transfection was carried out by adding 9.7 µL Opti-MEM (ThermoFisher) plus 0.3 µL Lipofectamine RNAiMAX (ThermoFisher) to 10 µL of each siRNA duplex. The mixture was incubated at room temperature for 15 minutes before being added to 100 µL of complete growth medium containing 20,000 Huh7 cells. Cells were incubated for 24 hours at 37°C/5% CO₂ prior to total RNA purification using a RNeasy 96 Kit (Qiagen). Each duplex was tested by transfection in duplicate wells and the experiment was repeated three time.

cDNA synthesis was performed using FastKing RT kit (with gDNase) Kit (Tiangen). Real-time quantitative PCR (qPCR) was performed on an ABI Prism 7900HT or ABI QuantStudio 7 with primers specific for human ZPI (Hs01547819_m1) and human GAPDH (Hs02786624_gl) using a TaqMan Gene Expression Assay Kit (ThermoFisher Scientific).

qPCR was performed in duplicate on cDNA derived from each well and the mean Ct calculated. Relative target expression was calculated from mean Ct values using the comparative Ct (ΔΔCt) method, normalised to GAPDH and relative to untreated cells.

For the inhibition of ZPI, the siRNA duplexes ETXM1184, ETXM1199, ETXM1200, ETXM1201, ETXM1202, ETXM1203, ETXM1204, ETXM1205, ETXM1206 and ETXM1207 were tested (Fig.21).

## Claims

1. A nucleic acid for use in prevention or treatment of a disease related to a disorder of haemostasis, such as haemophilia,
wherein the nucleic acid is for inhibiting expression of ZPI, and comprises a duplex region that comprises a first strand and a second strand that is at least partially complementary to the first strand, wherein said first strand is:
(i) at least partially complementary to a portion of RNA transcribed from the ZPI gene, and
(ii) comprises at least 17 contiguous nucleosides differing by 0 or 1 nucleosides from any one of the first strand sequences as listed in Table 2, and
wherein the nucleic acid comprises first and second strands that comprises, consists of, or consists essentially of a nucleoside sequence differing by 0 or 1 nucleosides from any one of the following first and second sequences:
| Unmodified first strand | Unmodified second strand |
|---|---|
| SEQ ID NO: 145 | SEQ ID NO: 265 |
| SEQ ID NO: 148 | SEQ ID NO: 268 |
| SEQ ID NO: 126 | SEQ ID NO: 246 |
| SEQ ID NO: 127 | SEQ ID NO: 247 |
| SEQ ID NO: 128 | SEQ ID NO: 248 |
| SEQ ID NO: 129 | SEQ ID NO: 249 |
| SEQ ID NO: 131 | SEQ ID NO: 251 |
| SEQ ID NO: 132 | SEQ ID NO: 252 |
| SEQ ID NO: 138 | SEQ ID NO: 258 |
| SEQ ID NO: 139 | SEQ ID NO: 259 |
| SEQ ID NO: 144 | SEQ ID NO: 264 |
| SEQ ID NO: 147 | SEQ ID NO: 267 |
| SEQ ID NO: 149 | SEQ ID NO: 269 |
| SEQ ID NO: 226 | SEQ ID NO: 346 |
| SEQ ID NO: 227 | SEQ ID NO: 347 |
| SEQ ID NO: 228 | SEQ ID NO: 348 |
| SEQ ID NO: 229 | SEQ ID NO: 349 |
| SEQ ID NO: 231 | SEQ ID NO: 351 |
| SEQ ID NO: 232 | SEQ ID NO: 352 |
| SEQ ID NO: 238 | SEQ ID NO: 358 |
| SEQ ID NO: 239 | SEQ ID NO: 359 |

2. A nucleic acid for use according to claim 1, comprising first and second strands that comprise, consist of, or consist essentially of a nucleoside sequence differing by 0 or 1 nucleosides from any one of the following first and second sequences:
| Unmodified first strand | Unmodified second strand |
|---|---|
| SEQ ID NO: 128 | SEQ ID NO: 248 |
| SEQ ID NO: 144 | SEQ ID NO: 264 |
| SEQ ID NO: 148 | SEQ ID NO: 268 |
| SEQ ID NO: 149 | SEQ ID NO: 269 |
| SEQ ID NO: 138 | SEQ ID NO: 258 |

3. A nucleic acid for use according to claim 1, comprising first and second strands that comprise, consist of, or consist essentially of a nucleoside sequence differing by 0 or 1 nucleosides from any one of the following first and second sequences:
| Unmodified first strand | Unmodified second strand |
|---|---|
| SEQ ID NO: 148 | SEQ ID NO: 268 |
| SEQ ID NO: 145 | SEQ ID NO: 265 |
| SEQ ID NO: 144 | SEQ ID NO: 264 |
| SEQ ID NO: 165 | SEQ ID NO: 285 |
| SEQ ID NO: 202 | SEQ ID NO: 322 |

4. A nucleic acid for use according to claim 1, comprising first and second strands that comprise, consist of, or consist essentially of a nucleoside sequence differing by 0 or 1 nucleosides from any one of the following first and second sequences:
| Unmodified first strand | Unmodified second strand |
|---|---|
| SEQ ID NO: 145 | SEQ ID NO: 265 |
| SEQ ID NO: 148 | SEQ ID NO: 268 |
, optionally
| Unmodified first strand | Unmodified second strand |
|---|---|
| SEQ ID NO: 145 | SEQ ID NO: 265 |

5. A nucleic acid for use according to any preceding claim, wherein the first strand has a length in the range of 17 to 30 nucleosides, preferably 19 to 25 nucleosides, more preferably 19 or 23 nucleosides.

6. A nucleic acid for use according to any preceding claim, wherein the second strand has a length in the range of 17 to 30 nucleosides, preferably 19 to 25 nucleosides, more preferably 19 or 21 nucleosides.

7. A nucleic acid for use according to any preceding claim, wherein the duplex region of the nucleic acid is between 17 and 30 nucleosides in length, more preferably is 19 or 21 nucleosides in length.

8. A nucleic acid for use according to any preceding claim, wherein the region of complementarity between the first strand and the portion of RNA transcribed from the ZPI gene is between 17 and 30 nucleosides in length.

9. A nucleic acid for use according to any preceding claim, wherein the nucleic acid comprises one or more single-stranded nucleoside overhangs, optionally wherein the overhang is present on the first or second strand, preferably at the 3' terminus of the first or second strand, and/or wherein the overhang comprises 1 to 4 nucleosides, more preferably 2 nucleosides.

10. A nucleic acid for use according to any preceding claim, wherein the nucleic acid is an siRNA oligonucleoside.

11. A nucleic acid for use according to any preceding claim, wherein one or more nucleosides on the first and/or second strand are modified, to form modified nucleosides.

12. A nucleic acid for use according to any preceding claim, which further comprises one or more abasic nucleosides, optionally wherein the one or more abasic nucleosides are in a terminal region of the second strand, and/or wherein at least one abasic nucleoside is linked to an adjacent basic nucleoside through a reversed internucleoside linkage.

13. A nucleic acid for use according to any preceding claim, wherein the nucleic acid comprises one or more phosphorothioate internucleoside linkages.

14. A nucleic acid for use according to any preceding claim, wherein the nucleic acid is conjugated directly or indirectly to one or more ligand moieties, optionally wherein said ligand moiety is present at a terminal region of the second strand, preferably at the 3' terminal region thereof.

15. A pharmaceutical composition for use in prevention or treatment of a disease related to a disorder of haemostasis, such as haemophilia comprising a nucleic acid as disclosed in any preceding claim, in combination with a pharmaceutically acceptable excipient or carrier.

## Patentansprüche

1. Nukleinsäure zur Verwendung bei der Vorbeugung oder Behandlung einer Erkrankung in Zusammenhang mit einer Störung der Hämostase wie z. B. Hämophilie,
wobei die Nukleinsäure zum Hemmen der Expression von ZPI vorgesehen ist und eine Duplexregion umfasst, die einen ersten Strang und einen zweiten Strang umfasst, der zumindest teilweise zu dem ersten Strang komplementär ist, wobei der erste Strang:
(i) zumindest teilweise zu einem Abschnitt von RNA komplementär ist, der aus dem ZPI-Gen transkribiert ist, und
(ii) zumindest 17 zusammenhängende Nukleoside umfasst, die sich um 0 oder 1 Nukleosid von einer der Sequenzen des ersten Strangs unterscheiden, wie in Tabelle 2 aufgelistet, und
wobei die Nukleinsäure erste und zweite Stränge umfasst, die eine Nukleosidsequenz umfassen, daraus bestehen oder im Wesentlichen daraus bestehen, die sich um 0 oder 1 Nukleosid von einer der nachstehenden ersten und zweiten Sequenzen unterscheidet:
| Unmodifizierter erster Strang | Unmodifizierter zweiter Strang |
|---|---|
| SEQ ID NO: 145 | SEQ ID NO: 265 |
| SEQ ID NO: 148 | SEQ ID NO: 268 |
| SEQ ID NO: 126 | SEQ ID NO: 246 |
| SEQ ID NO: 127 | SEQ ID NO: 247 |
| SEQ ID NO: 128 | SEQ ID NO: 248 |
| SEQ ID NO: 129 | SEQ ID NO: 249 |
| SEQ ID NO: 131 | SEQ ID NO: 251 |
| SEQ ID NO: 132 | SEQ ID NO: 252 |
| SEQ ID NO: 138 | SEQ ID NO: 258 |
| SEQ ID NO: 139 | SEQ ID NO: 259 |
| SEQ ID NO: 144 | SEQ ID NO: 264 |
| SEQ ID NO: 147 | SEQ ID NO: 267 |
| SEQ ID NO: 149 | SEQ ID NO: 269 |
| SEQ ID NO: 226 | SEQ ID NO: 346 |
| SEQ ID NO: 227 | SEQ ID NO: 347 |
| SEQ ID NO: 228 | SEQ ID NO: 348 |
| SEQ ID NO: 229 | SEQ ID NO: 349 |
| SEQ ID NO: 231 | SEQ ID NO: 351 |
| SEQ ID NO: 232 | SEQ ID NO: 352 |
| SEQ ID NO: 238 | SEQ ID NO: 358 |
| SEQ ID NO: 239 | SEQ ID NO: 359 |

2. Nukleinsäure zur Verwendung nach Anspruch 1, die erste und zweite Stränge umfasst, die eine Nukleosidsequenz umfassen, daraus bestehen oder im Wesentlichen daraus bestehen, die sich um 0 oder 1 Nukleosid von einer der nachstehenden ersten und zweiten Sequenzen unterscheiden:
| Unmodifizierter erster Strang | Unmodifizierter zweiter Strang |
|---|---|
| SEQ ID NO: 128 | SEQ ID NO: 248 |
| SEQ ID NO: 144 | SEQ ID NO: 264 |
| SEQ ID NO: 148 | SEQ ID NO: 268 |
| SEQ ID NO: 149 | SEQ ID NO: 269 |
| SEQ ID NO: 138 | SEQ ID NO: 258 |

3. Nukleinsäure zur Verwendung nach Anspruch 1, die erste und zweite Stränge umfasst, die eine Nukleosidsequenz umfassen, daraus bestehen oder im Wesentlichen daraus bestehen, die sich um 0 oder 1 Nukleosid von einer der nachstehenden ersten und zweiten Sequenzen unterscheiden.
| Unmodifizierter erster Strang | Unmodifizierter zweiter Strang |
|---|---|
| SEQ ID NO: 148 | SEQ ID NO: 268 |
| SEQ ID NO: 145 | SEQ ID NO: 265 |
| SEQ ID NO: 144 | SEQ ID NO: 264 |
| SEQ ID NO: 165 | SEQ ID NO: 285 |
| SEQ ID NO: 202 | SEQ ID NO: 322 |

4. Nukleinsäure zur Verwendung nach Anspruch 1, die erste und zweite Stränge umfasst, die eine Nukleosidsequenz umfassen, daraus bestehen oder im Wesentlichen daraus bestehen, die sich um 0 oder 1 Nukleosid von einer der nachstehenden ersten und zweiten Sequenzen unterscheiden.
| Unmodifizierter erster Strang | Unmodifizierter zweiter Strang |
|---|---|
| SEQ ID NO: 145 | SEQ ID NO: 265 |
| SEQ ID NO: 148 | SEQ ID NO: 268 |
optional
| Unmodifizierter erster Strang | Unmodifizierter zweiter Strang |
|---|---|
| SEQ ID NO: 145 | SEQ ID NO: 265 |

5. Nukleinsäure zur Verwendung nach einem vorstehenden Anspruch, wobei der erste Strang eine Länge in dem Bereich von 17 bis 30 Nukleosiden, vorzugsweise 19 bis 25 Nukleosiden, mehr bevorzugt 19 oder 23 Nukleoside, aufweist.

6. Nukleinsäure zur Verwendung nach einem vorstehenden Anspruch, wobei der zweite Strang eine Länge im Bereich von 17 bis 30 Nukleosiden, vorzugsweise 19 bis 25 Nukleosiden, mehr bevorzugt 19 oder 21 Nukleoside, aufweist.

7. Nukleinsäure zur Verwendung nach einem vorstehenden Anspruch, wobei die Duplexregion der Nukleinsäure eine Länge zwischen 17 und 30 Nukleosiden aufweist und mehr bevorzugt eine Länge von 19 oder 21 Nukleosiden aufweist.

8. Nukleinsäure zur Verwendung nach einem vorstehenden Anspruch, wobei die Region der Komplementarität zwischen dem ersten Strang und dem Abschnitt von RNA, der aus dem ZPI-Gen transkribiert ist, eine Länge zwischen 17 und 30 Nukleosiden aufweist.

9. Nukleinsäure zur Verwendung nach einem vorstehenden Anspruch, wobei die Nukleinsäure einen oder mehrere einzelsträngige Nukleosidüberhänge umfasst, optional wobei der Überhang auf dem ersten oder dem zweiten Strang vorhanden ist, vorzugsweise an dem 3'-Terminus des ersten oder des zweiten Strangs, und/oder wobei der Überhang 1 bis 4 Nukleoside, mehr bevorzugt 2 Nukleoside, umfasst.

10. Nukleinsäure zur Verwendung nach einem vorstehenden Anspruch, wobei die Nukleinsäure in einem siRNA-Oligonukleosid vorliegt.

11. Nukleinsäure zur Verwendung nach einem vorstehenden Anspruch, wobei ein oder mehr Nukleoside auf dem ersten und/oder dem zweiten Strang modifiziert sind, um modifizierte Nukleoside zu bilden.

12. Nukleinsäure zur Verwendung nach einem vorstehenden Anspruch, die ferner ein oder mehrere abasische Nukleoside umfasst, optional wobei das eine oder die mehreren abasischen Nukleoside in einer terminalen Region des zweiten Strangs vorliegen und/oder wobei zumindest ein abasisches Nukleosid durch eine umgekehrte Internukleosidverknüpfung mit einem benachbarten basischen Nukleosid verknüpft ist.

13. Nukleinsäure zur Verwendung nach einem vorstehenden Anspruch, wobei die Nukleinsäure ein oder mehrere Phosphorothioat-Internukleosidverknüpfungen umfasst.

14. Nukleinsäure zur Verwendung nach einem vorstehenden Anspruch, wobei die Nukleinsäure direkt oder indirekt an eine oder mehrere Ligandeinheiten konjugiert ist, optional wobei die Ligandeinheit in einer terminalen Region des zweiten Strangs vorhanden ist, vorzugsweise an der 3'-terminalen Region davon.

15. Pharmazeutische Zusammensetzung zur Verwendung bei der Vorbeugung oder Behandlung einer Erkrankung in Zusammenhang mit einer Störung der Hämostase, wie z. B. Hämophilie, die eine Nukleinsäure nach einem vorstehenden Anspruch in Kombination mit einem pharmazeutisch unbedenklichen Exzipienten oder Träger umfasst.

## Revendications

1. Acide nucléique destiné à être utilisé dans la prévention ou le traitement d'une maladie liée à un trouble de l'hémostase, tel que l'hémophilie,
dans lequel l'acide nucléique est destiné à inhiber l'expression de ZPI, et comprend une région duplex qui comprend un premier brin et un second brin qui est au moins partiellement complémentaire du premier brin, dans lequel ledit premier brin est :
(i) au moins partiellement complémentaire d'une partie de l'ARN transcrit à partir du gène ZPI, et
(ii) comprend au moins 17 nucléosides contigus différant de 0 ou 1 nucléoside de l'une quelconque des séquences du premier brin telles qu'énumérées dans le tableau 2, et
dans lequel l'acide nucléique comprend des premier et second brins qui comprennent, sont composés de ou sont constitués essentiellement d'une séquence nucléosidique différant de 0 ou 1 nucléoside de l'une quelconque des premières et secondes séquences suivantes :
| Premier brin non modifié | Second brin non modifié |
|---|---|
| SEQ ID NO: 145 | SEQ ID NO: 265 |
| SEQ ID NO: 148 | SEQ ID NO: 268 |
| SEQ ID NO: 126 | SEQ ID NO: 246 |
| SEQ ID NO: 127 | SEQ ID NO: 247 |
| SEQ ID NO: 128 | SEQ ID NO: 248 |
| SEQ ID NO: 129 | SEQ ID NO: 249 |
| SEQ ID NO: 131 | SEQ ID NO: 251 |
| SEQ ID NO: 132 | SEQ ID NO: 252 |
| SEQ ID NO: 138 | SEQ ID NO: 258 |
| SEQ ID NO: 139 | SEQ ID NO: 259 |
| SEQ ID NO: 144 | SEQ ID NO: 264 |
| SEQ ID NO: 147 | SEQ ID NO: 267 |
| SEQ ID NO: 149 | SEQ ID NO: 269 |
| SEQ ID NO: 226 | SEQ ID NO: 346 |
| SEQ ID NO: 227 | SEQ ID NO: 347 |
| SEQ ID NO: 228 | SEQ ID NO: 348 |
| SEQ ID NO: 229 | SEQ ID NO: 349 |
| SEQ ID NO: 231 | SEQ ID NO: 351 |
| SEQ ID NO: 232 | SEQ ID NO: 352 |
| SEQ ID NO: 238 | SEQ ID NO: 358 |
| SEQ ID NO: 239 | SEQ ID NO: 359 |

2. Acide nucléique destiné à être utilisé selon la revendication 1, comprenant des premier et second brins qui comprennent, sont composés de ou sont constitués essentiellement d'une séquence nucléosidique différant de 0 ou 1 nucléoside de l'une quelconque des premières et secondes séquences suivantes :
| Premier brin non modifié | Second brin non modifié |
|---|---|
| SEQ ID NO: 128 | SEQ ID NO: 248 |
| SEQ ID NO: 144 | SEQ ID NO: 264 |
| SEQ ID NO: 148 | SEQ ID NO: 268 |
| SEQ ID NO: 149 | SEQ ID NO: 269 |
| SEQ ID NO: 138 | SEQ ID NO: 258 |

3. Acide nucléique destiné à être utilisé selon la revendication 1, comprenant des premier et second brins qui comprennent, sont composés de ou sont constitués essentiellement d'une séquence nucléosidique différant de 0 ou 1 nucléoside de l'une quelconque des premières et secondes séquences suivantes :
| Premier brin non modifié | Second brin non modifié |
|---|---|
| SEQ ID NO: 148 | SEQ ID NO: 268 |
| SEQ ID NO: 145 | SEQ ID NO: 265 |
| SEQ ID NO: 144 | SEQ ID NO: 264 |
| SEQ ID NO: 165 | SEQ ID NO: 285 |
| SEQ ID NO: 202 | SEQ ID NO: 322 |

4. Acide nucléique destiné à être utilisé selon la revendication 1, comprenant des premier et second brins qui comprennent, sont composés de ou sont constitués essentiellement d'une séquence nucléosidique différant de 0 ou 1 nucléoside de l'une quelconque des premières et secondes séquences suivantes :
| Premier brin non modifié | Second brin non modifié |
|---|---|
| SEQ ID NO: 145 | SEQ ID NO: 265 |
| SEQ ID NO: 148 | SEQ ID NO: 268 |
, éventuellement
| Premier brin non modifié | Second brin non modifié |
|---|---|
| SEQ ID NO: 145 | SEQ ID NO: 265 |

5. Acide nucléique destiné à être utilisé selon une quelconque revendication précédente, dans lequel le premier brin a une longueur dans la plage de 17 à 30 nucléosides, de préférence de 19 à 25 nucléosides, plus préférablement de 19 ou 23 nucléosides.

6. Acide nucléique destiné à être utilisé selon une quelconque revendication précédente, dans lequel le second brin a une longueur dans la plage de 17 à 30 nucléosides, de préférence de 19 à 25 nucléosides, plus préférablement de 19 ou 21 nucléosides.

7. Acide nucléique destiné à être utilisé selon une quelconque revendication précédente, dans lequel la région duplex de l'acide nucléique a une longueur comprise entre 17 et 30 nucléosides, et plus préférablement une longueur de 19 ou 21 nucléosides.

8. Acide nucléique destiné à être utilisé selon une quelconque revendication précédente, dans lequel la région de complémentarité entre le premier brin et la partie d'ARN transcrite à partir du gène ZPI a une longueur comprise entre 17 et 30 nucléosides.

9. Acide nucléique destiné à être utilisé selon une quelconque revendication précédente, dans lequel l'acide nucléique comprend un ou plusieurs surplombs de nucléosides monocaténaires, éventuellement dans lequel le surplomb est présent sur le premier ou le second brin, de préférence à l'extrémité 3' du premier ou du second brin, et/ou dans lequel le surplomb comprend 1 à 4 nucléosides, plus préférablement 2 nucléosides.

10. Acide nucléique destiné à être utilisé selon une quelconque revendication précédente, dans lequel l'acide nucléique est un oligonucléoside d'ARNsi.

11. Acide nucléique destiné à être utilisé selon une quelconque revendication précédente, dans lequel un ou plusieurs nucléosides sur le premier et/ou le second brin sont modifiés, pour former des nucléosides modifiés.

12. Acide nucléique destiné à être utilisé selon une quelconque revendication précédente, qui comprend en outre un ou plusieurs nucléosides abasiques, éventuellement dans lequel les un ou plusieurs nucléosides abasiques sont situés dans une région terminale du second brin, et/ou dans lequel au moins un nucléoside abasique est lié à un nucléoside basique adjacent à travers une liaison internucléosidique inversée.

13. Acide nucléique destiné à être utilisé selon une quelconque revendication précédente, dans lequel l'acide nucléique comprend une ou plusieurs liaisons internucléosidiques phosphorothioate.

14. Acide nucléique destiné à être utilisé selon une quelconque revendication précédente, dans lequel l'acide nucléique est conjugué directement ou indirectement à une ou plusieurs fractions de ligand, éventuellement dans lequel ladite fraction de ligand est présente dans une région terminale du second brin, de préférence dans la région terminale 3' de celui-ci.

15. Composition pharmaceutique destinée à être utilisée dans la prévention ou le traitement d'une maladie liée à un trouble de l'hémostase, tel que l'hémophilie, comprenant un acide nucléique tel que décrit dans une quelconque revendication précédente, en combinaison avec un excipient ou un support pharmaceutiquement acceptable.
